# EUROPEAN PATENT APPLICATION

(11) **EP 4 541 795 A1**
(43) Date of publication of application: **23.04.2025**
(21) Application number: 23823166.6
(22) Date of filing: 14.06.2023
(51) Int. Cl.: C07D 471/10, C07D 417/14, C07D 487/08, C07D 491/056, C07D 401/14, C07D 403/14, C07D 403/12, A61K 31/4725, A61K 31/496, A61K 31/352, A61P 35/00

(54) **AROMATIC COMPOUND, METHOD FOR PREPARING SAME, AND USE THEREOF IN PREPARATION OF ESTROGEN RECEPTOR DEGRADER**

(30) Priority: 14.06.2022 CN 202210668327; 13.09.2022 CN 202211109532; 11.01.2023 CN 202310040674
(71) Applicant: Hinova Pharmaceuticals Inc., Chengdu, Sichuan 610041 (CN)
(72) Inventor: DU, Wu, Chengdu, Sichuan 610041 (CN); LI, Xinghai, Chengdu, Sichuan 610041 (CN); QIN, Dekun, Chengdu, Sichuan 610041 (CN); LI, Haibo, Chengdu, Sichuan 610041 (CN); DUAN, Jingyi, Chengdu, Sichuan 610041 (CN); LIU, Shijuan, Chengdu, Sichuan 610041 (CN); ZHANG, Meng, Chengdu, Sichuan 610041 (CN); NIE, Zhenyan, Chengdu, Sichuan 610041 (CN); YUAN, Zhihui, Chengdu, Sichuan 610041 (CN); TU, Zhilin, Chengdu, Sichuan 610041 (CN)
(74) Representative: Altmann Stößel Dick Patentanwälte PartG mbB
(86) International application number: PCT/CN2023/100055
(87) International publication number: WO 2023/241598

(57) **Abstract**

The present invention provides an aromatic compound, a method for preparing same, and use thereof in the preparation of an estrogen receptor degrader, and belongs to the technical field of chemical medicines. The aromatic compound is a compound represented by formula (I), or an optical isomer thereof, or a salt thereof, or a hydrate thereof, or a solvate thereof. The compound of the present invention can be used for preparing an estrogen receptor degrader, and a drug for treating an estrogen receptor-associated disease, such as a drug for treating and/or preventing cancer, wherein the cancer can be breast cancer.

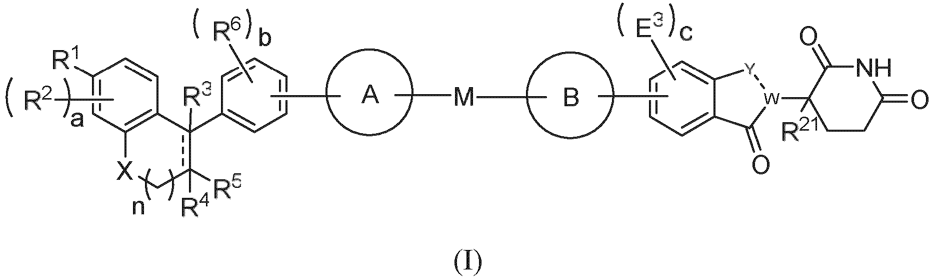

## Description

### Field of the invention

The present invention belongs to the field of chemical medicines, and specifically relates to an aromatic compound, a preparation method therefor, and uses thereof in the manufacture of estrogen receptor degraders.

### Background of the invention

Estrogen receptor (ER) is a ligand-activated transcription regulatory protein that mediates the induction of various biological effects by interaction with endogenous estrogens. Endogenous estrogens include 17β-estradiol and estrone. It has been found that ER has two different conformations, namely ER-α and ER-β. Estrogens and estrogen receptors are associated with many diseases or conditions, such as bone cancer, breast cancer, colorectal cancer, endometrial cancer, prostate cancer, ovarian cancer, uterine cancer, cervical cancer, lung cancer, and other diseases or conditions. For example, about 70% of patients with breast cancer express ER and/or progesterone receptors. The treatment of these diseases can be carried out by inhibiting ER signaling through various means, including antagonizing the binding of ligands to ER, antagonizing or downregulating ERα, blocking estrogen synthesis, etc.

Estrogen receptor degraders can compete with estrogen receptors (ER) present in estrogen-responsive tissues for binding. They are expected to be used for treating ER-related diseases. Conventional non-steroidal ER degraders, such as tamoxifen, can effectively compete for ER binding, but they are often limited by their partial agonistic effects, resulting in lower efficacy and incomplete blockade of estrogen-mediated activity. For the treatment of estrogen-dependent diseases, specific or "pure" antiestrogens with high affinity for ER and no agonist effects may offer advantages over conventional non-steroidal antiestrogens. Therefore, it is of great significance to investigate an ER degrader that reduces ER expression at the transcript or protein level.

### Content of the invention

The objective of the present invention is to provide an aromatic compound, a preparation method therefor, and uses thereof in the manufacture of ER degraders.

The present invention provides a compound represented by formula (I), or an optical isomer thereof, or a salt thereof, or a hydrate thereof, or a solvate thereof: 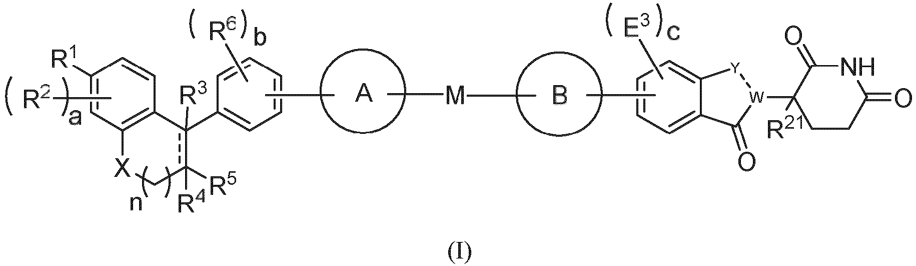 wherein,
R¹ is selected from the group consisting of H, C₁-C₆ alkyl, hydroxyl, amino, carboxyl, C₁-C₆ alkoxy, dihydroxyboranyl, and -OR';
R' is selected from the group consisting of phenyl and benzyl;
R² represents a substituent at any position of the benzene ring, and a represents the number of the substituent R²;
Each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
provided that the dashed line in the benzene ring represents a bond, R³ and R⁴ are independently selected from absence;
provided that the dashed line in the benzene ring is absent, R³ and R⁴ are each independently selected from the group consisting of H and C₁-C₆ alkyl; alternatively, R³ and R⁴ are linked to form 3-6-membered cycloalkyl;
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
n is 0, 1, or 2;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
provided that the dashed line between Y and W is absent, W represents NH, and Y represents halogen;
provided that the dashed line between Y and W is a bond, W represents N; and Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
R²¹ is selected from the group consisting of provided that the dashed line between Y and W is absent, W represents NH, and Y represents a halogen;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.

Further, the compound is as represented by formula (II): 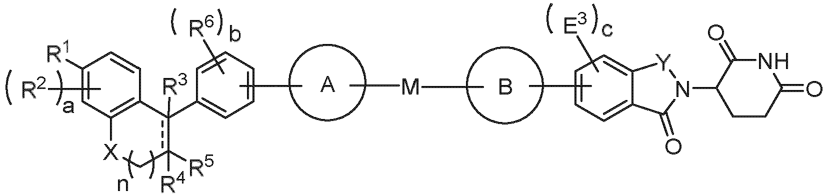 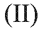 wherein,
R¹ is selected from the group consisting of H, C₁-C₆ alkyl, hydroxyl, amino, carboxyl, C₁-C₆ alkoxy, dihydroxyboranyl, and -OR';
R' is selected from the group consisting of phenyl and benzyl;
R² represents a substituent at any position of the benzene ring, and a represents the number of the substituent R²;
each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
provided that the dashed line in the benzene ring represents a bond, R³ and R⁴ are independently selected from absence;
provided that the dashed line in the benzene ring is absent, R³ and R⁴ are each independently selected from the group consisting of H and C₁-C₆ alkyl; alternatively, R³ and R⁴ are linked to form 3-6-membered cycloalkyl;
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
n is 0, 1, or 2;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.

Further, the compound is as represented by formula (III): 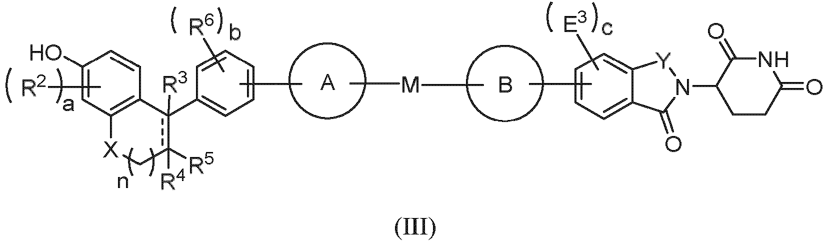 wherein,
R² represents a substituent at any position of the benzene ring, and a represents the number of the substituent R²;
each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
provided that the dashed line in the benzene ring represents a bond, R³ and R⁴ are independently selected from absence;
provided that the dashed line in the benzene ring is absent, R³ and R⁴ are each independently selected from the group consisting of H and C₁-C₆ alkyl; alternatively, R³ and R⁴ are linked to form 3-6-membered cycloalkyl;
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
n is 0, 1, or 2;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H an C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.
preferably,
R⁵ is selected from the group consisting of C₁-C₆ alkyl, 3-6-membered cycloalkyl, trifluoromethyl, trifluoroethyl, substituted or unsubstituted phenyl, substituted or unsubstituted thienyl, substituted or unsubstituted furanyl, substituted or unsubstituted pyrrolyl, substituted or unsubstituted pyrimidinyl, substituted or unsubstituted pyridazinyl, substituted or unsubstituted pyrazolyl, substituted or unsubstituted pyrazinyl; the substituent of said phenyl, thienyl, furanyl, pyrrolyl, pyrimidinyl, pyridazinyl, pyrazolyl, or pyrazinyl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

Further, the compound is as represented by formula (IV): 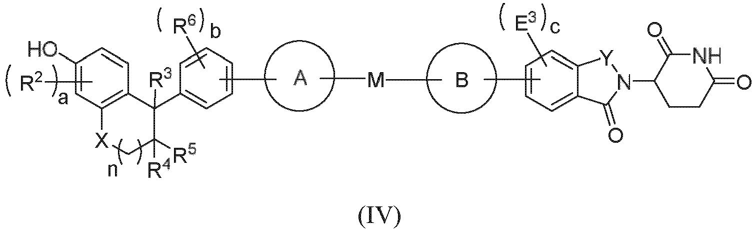 wherein,
R² represents a substituent at any position of the benzene ring, and a represents the number of the substituent R²;
each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
R³ and R⁴ are each independently selected from the group consisting of H and C₁-C₆ alkyl; alternatively, R³ and R⁴ are linked to form 3-6-membered cycloalkyl;
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
n is 0, 1, or 2;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.

Preferably,
R⁵ is selected from the group consisting of C₁-C₆ alkyl, 3-6-membered cycloalkyl, trifluoromethyl, trifluoroethyl, substituted or unsubstituted phenyl, substituted or unsubstituted thienyl, substituted or unsubstituted furanyl, substituted or unsubstituted pyrrolyl, substituted or unsubstituted pyrimidinyl, substituted or unsubstituted pyridazinyl, substituted or unsubstituted pyrazolyl, and substituted or unsubstituted pyrazinyl; the substituent of said phenyl, thienyl, furanyl, pyrrolyl, pyrimidinyl, pyridazinyl, pyrazolyl, or pyrazinyl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

Further, the compound is as represented by formula (V): 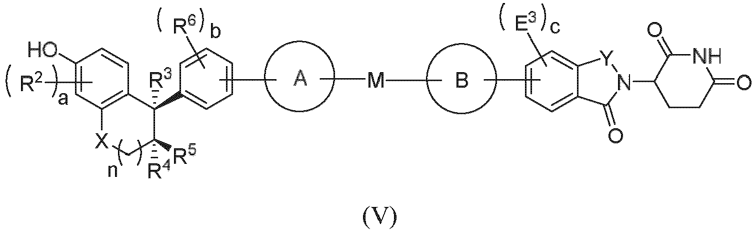 wherein,
R² represents a substituent at any position of the benzene ring, and a represents the number of the substituent R²;
each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
R³ and R⁴ are each independently selected from the group consisting of H and C₁-C₆ alkyl; alternatively, R³ and R⁴ are linked to form 3-6-membered cycloalkyl;
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
n is 0, 1, or 2;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.

Preferably,
R⁵ is selected from the group consisting of C₁-C₆ alkyl, 3-6-membered cycloalkyl, trifluoromethyl, trifluoroethyl, substituted or unsubstituted phenyl, substituted or unsubstituted thienyl, substituted or unsubstituted furanyl, substituted or unsubstituted pyrrolyl, substituted or unsubstituted pyrimidinyl, substituted or unsubstituted pyridazinyl, substituted or unsubstituted pyrazolyl, and substituted or unsubstituted pyrazinyl; the substituent of said phenyl, thienyl, furanyl, pyrrolyl, pyrimidinyl, pyridazinyl, pyrazolyl, or pyrazinyl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

Further, the compound is as represented by formula (VI): 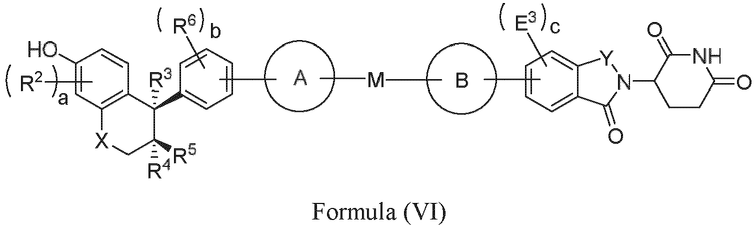 wherein
R² represents a substituent at any position of the benzene ring, and a represents the number of the substituent R²;
each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
R³ and R⁴ are each independently selected from the group consisting of H and C₁-C₆ alkyl; alternatively, R³ and R⁴ are linked to form 3-6-membered cycloalkyl;
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.

Preferably,
R⁵ is selected from the group consisting of C₁-C₆ alkyl, 3-6-membered cycloalkyl, trifluoromethyl, trifluoroethyl, substituted or unsubstituted phenyl, substituted or unsubstituted thienyl, substituted or unsubstituted furanyl, substituted or unsubstituted pyrrolyl, substituted or unsubstituted pyrimidinyl, substituted or unsubstituted pyridazinyl, substituted or unsubstituted pyrazolyl, and substituted or unsubstituted pyrazinyl; the substituent of said phenyl, thienyl, furanyl, pyrrolyl, pyrimidinyl, pyridazinyl, pyrazolyl, or pyrazinyl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

Further, the compound is as represented by formula (VII): 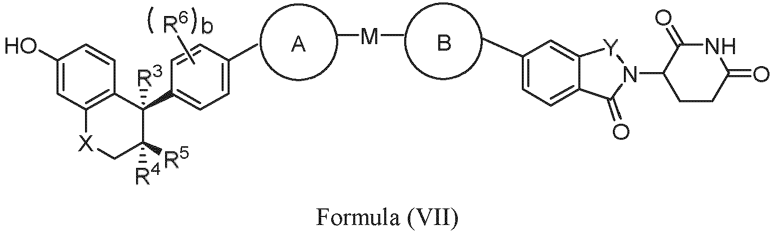 wherein
R³ and R⁴ are each independently selected from the group consisting of H and C₁-C₆ alkyl; alternatively, R³ and R⁴ are linked to form 3-6-membered cycloalkyl;
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.

Preferably,
R⁵ is selected from the group consisting of C₁-C₆ alkyl, 3-6-membered cycloalkyl, trifluoromethyl, trifluoroethyl, substituted or unsubstituted phenyl, substituted or unsubstituted thienyl, substituted or unsubstituted furanyl, substituted or unsubstituted pyrrolyl, substituted or unsubstituted pyrimidinyl, substituted or unsubstituted pyridazinyl, substituted or unsubstituted pyrazolyl, and substituted or unsubstituted pyrazinyl; the substituent of said phenyl, thienyl, furanyl, pyrrolyl, pyrimidinyl, pyridazinyl, pyrazolyl, or pyrazinyl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

Further, the compound is as represented by formula (VIII): 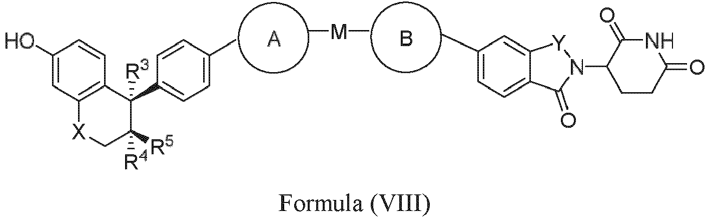 wherein,
R³ and R⁴ are each independently selected from the group consisting of H and C₁-C₆ alkyl; alternatively, R³ and R⁴ are linked to form 3-6-membered cycloalkyl;
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.

Preferably,
R⁵ is selected from the group consisting of C₁-C₆ alkyl, 3-6-membered cycloalkyl, trifluoromethyl, substituted or unsubstituted phenyl, substituted or unsubstituted thienyl, substituted or unsubstituted furanyl, substituted or unsubstituted pyrrolyl, substituted or unsubstituted pyrimidinyl, substituted or unsubstituted pyridazinyl, substituted or unsubstituted pyrazolyl, and substituted or unsubstituted pyrazinyl; the substituent of said phenyl, thienyl, furanyl, pyrrolyl, pyrimidinyl, pyridazinyl, pyrazolyl, or pyrazinyl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

Further, the compound is as represented by formula (IX): 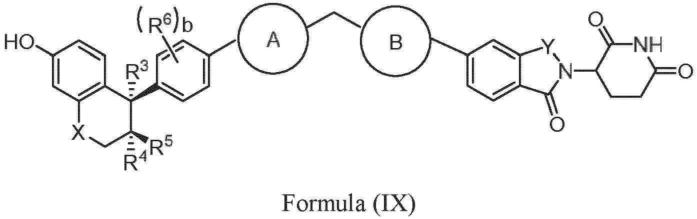 wherein,
R³ and R⁴ are each independently selected from the group consisting of H and C₁-C₆ alkyl; alternatively, R³ and R⁴ are linked to form 3-6-membered cycloalkyl;
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.

Preferably,
R⁵ is selected from the group consisting of C₁-C₆ alkyl, 3-6-membered cycloalkyl, trifluoromethyl, trifluoroethyl, substituted or unsubstituted phenyl, substituted or unsubstituted thienyl, substituted or unsubstituted furanyl, substituted or unsubstituted pyrrolyl, substituted or unsubstituted pyrimidinyl, substituted or unsubstituted pyridazinyl, substituted or unsubstituted pyrazolyl, and substituted or unsubstituted pyrazinyl; the substituent of said phenyl, thienyl, furanyl, pyrrolyl, pyrimidinyl, pyridazinyl, pyrazolyl, or pyrazinyl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

Further, the compound is as represented by formula (X): 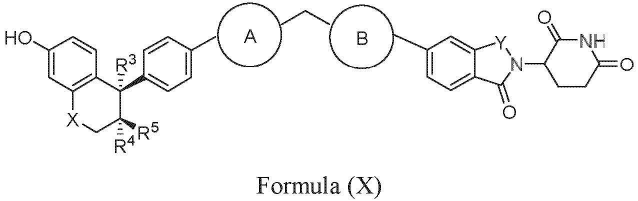 wherein,
R³ and R⁴ are each independently selected from the group consisting of H and C₁-C₆ alkyl; alternatively, R³ and R⁴ are linked to form 3-6-membered cycloalkyl;
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.

Preferably,
R⁵ is selected from the group consisting of C₁-C₆ alkyl, 3-6-membered cycloalkyl, trifluoromethyl, trifluoroethyl, substituted or unsubstituted phenyl, substituted or unsubstituted thienyl, substituted or unsubstituted furanyl, substituted or unsubstituted pyrrolyl, substituted or unsubstituted pyrimidinyl, substituted or unsubstituted pyridazinyl, substituted or unsubstituted pyrazolyl, and substituted or unsubstituted pyrazinyl; the substituent of said phenyl, thienyl, furanyl, pyrrolyl, pyrimidinyl, pyridazinyl, pyrazolyl, or pyrazinyl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

Further, the compound is as represented by formula (XI): 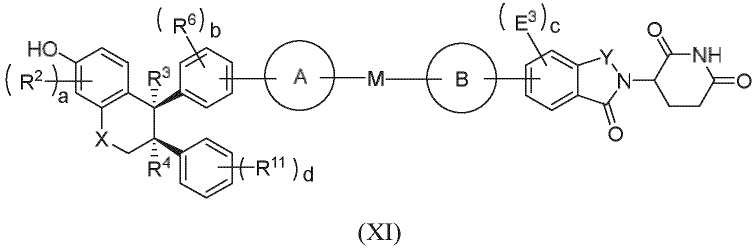 wherein,
R² represents a substituent at any position of the benzene ring, and a represents the number of the substituent R²;
each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
R³, R⁴ are each independently selected from the group consisting of H and C₁-C₆ alkyl; alternatively, R³ and R⁴ are linked to form 3-6-membered cycloalkyl;
R¹¹ represents a substituent at any position of the benzene ring, and d is the number of the substituent R¹¹;
each R¹¹ is independently selected from the group consisting of halogen, hydroxyl, amino, cyano, trifluoromethyl, C₁-C₆ alkyl, 3-6-membered cycloalkyl, and C₁-C₆ alkoxy;
d is 0, 1, 2, or 3;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.

Preferably,
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

Further, the compound is as represented by formula (XII): 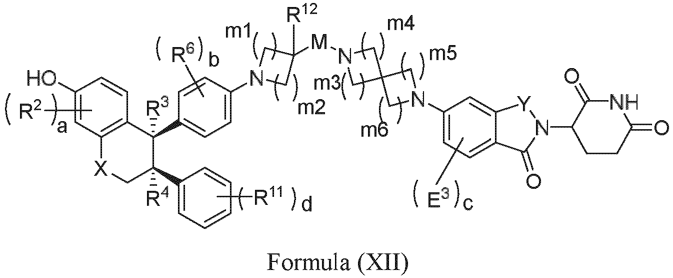 wherein,
R² represents a substituent at any position of the benzene ring, and a represents the number of the substituent R²;
each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
R³ and R⁴ are each independently selected from the group consisting of H and C₁-C₆ alkyl; alternatively, R³ and R⁴ are linked to form 3-6-membered cycloalkyl;
R¹¹ represents a substituent at any position of the benzene ring, and d is the number of the substituent R¹¹;
each R¹¹ is independently selected from the group consisting of halogen, hydroxyl, amino, cyano, trifluoromethyl, C₁-C₆ alkyl, 3-6-membered cycloalkyl, and C₁-C₆ alkoxy;
d is 0, 1, 2, or 3;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
R¹² is selected from the group consisting of H, halogen, C₁-C₆ alkyl, and hydroxyl;
m1, m2, m3, m4, m5, m6 are each independently selected from the group consisting of 0, 1, 2 or 3;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.

Preferably,
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl.

Further, the compound is as represented by formula (XIII): 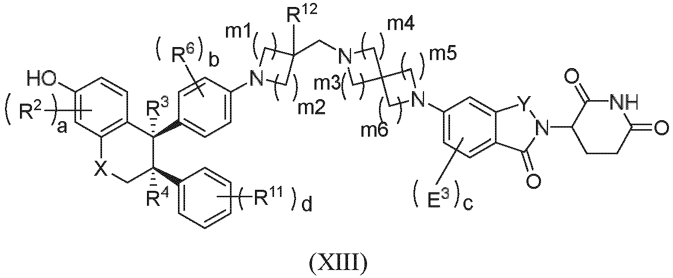 wherein,
R² represents a substituent at any position of the benzene ring, and a represents the number of the substituent R²;
each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
R³ and R⁴ are each independently selected from the group consisting of H and C₁-C₆ alkyl; alternatively, R³ and R⁴ are linked to form 3-6-membered cycloalkyl;
R¹¹ represents a substituent at any position of the benzene ring, and d is the number of the substituent R¹¹;
each R¹¹ is independently selected from the group consisting of halogen, hydroxyl, amino, cyano, trifluoromethyl, C₁-C₆ alkyl, 3-6-membered cycloalkyl, and C₁-C₆ alkoxy;
d is 0, 1, 2, or 3;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
R¹² is selected from the group consisting of H, halogen, C₁-C₆ alkyl, and hydroxyl;
m1, m2, m3, m4, m5, m6 are each independently selected from the group consisting of 0, 1, 2 or 3;
E³ represents a substituent at any position of the benzene ring, and c is the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; and the number of the heteroatom is 1, 2, or 3.

Preferably,
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl.

Further, the compound is as represented by formula (XIV): 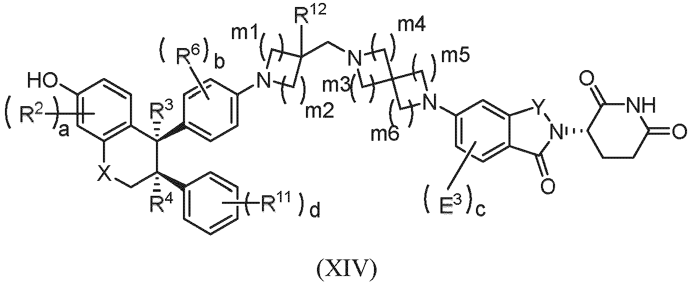 wherein,
R² represents a substituent at any position of the benzene ring, and a represents the number of the substituent R²;
each R² are each independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
R³ and R⁴ are each independently selected from the group consisting of H and C₁-C₆ alkyl; alternatively, R³ and R⁴ are linked to form 3-6-membered cycloalkyl;
R¹¹ represents a substituent at any position of the benzene ring, and d is the number of the substituent R¹¹;
each R¹¹ is independently selected from the group consisting of halogen, hydroxyl, amino, cyano, trifluoromethyl, C₁-C₆ alkyl, 3-6-membered cycloalkyl, and C₁-C₆ alkoxy;
d is 0, 1, 2, or 3;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
R¹² is selected from the group consisting of H, halogen, C₁-C₆ alkyl, and hydroxyl;
m1, m2, m3, m4, m5, m6 are each independently selected from the group consisting of 0, 1, 2 or 3;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.

Preferably,
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl.

Further, the compound is as represented by formula (XV): 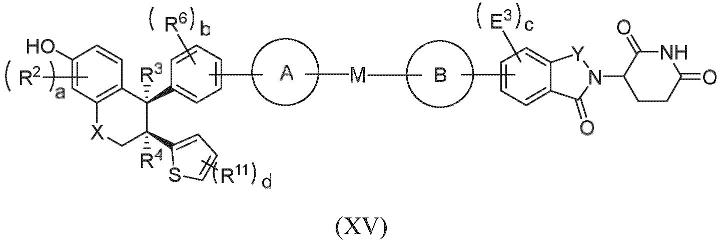 wherein,
R² represents a substituent at any position of the benzene ring, and a represents the number of the substituent R²;
each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
R³ and R⁴ are each independently selected from the group consisting of H and C₁-C₆ alkyl; alternatively, R³ and R⁴ are linked to form 3-6-membered cycloalkyl;
R¹¹ is a substituent at any position of the thiophene ring, and d is the number of the substituent R¹¹;
each R¹¹ is independently selected from the group consisting of halogen, hydroxyl, amino, cyano, trifluoromethyl, C₁-C₆ alkyl, 3-6-membered cycloalkyl, and C₁-C₆ alkoxy;
d is 0, 1, 2, or 3;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.

Preferably,
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

Further, the compound is as represented by formula (XVI): 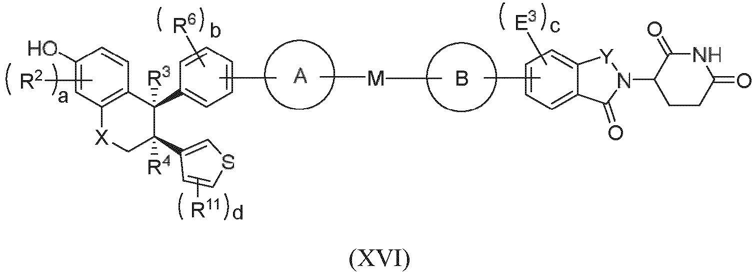 wherein,
R² represents a substituent at any position of the benzene ring, and a represents the number of the substituent R²;
each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
R³ and R⁴ are each independently selected from the group consisting of H and C₁-C₆ alkyl; alternatively, R³ and R⁴ are linked to form 3-6-membered cycloalkyl;
R¹¹ is a substituent at any position of the thiophene ring, and d is the number of the substituent R¹¹;
each R¹¹ is independently selected from the group consisting of halogen, hydroxyl, amino, cyano, trifluoromethyl, C₁-C₆ alkyl, 3-6-membered cycloalkyl, and C₁-C₆ alkoxy;
d is 0, 1, 2, or 3;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.

Preferably,
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

Further, the compound is as represented by formula (XVII): 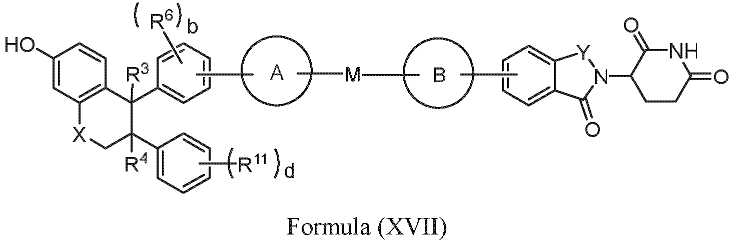 wherein,
R³ and R⁴ are each independently selected from the group consisting of H and C₁-C₆ alkyl; alternatively, R³ and R⁴ are linked to form 3-6-membered cycloalkyl;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R¹¹ represents a substituent at any position of the benzene ring, and d is the number of the substituent R¹¹; R⁶ represents a substituent at any position of the benzene ring, and b is the number of the substituent R⁶;
d is 0, 1, 2, or 3; b is 0, 1, 2, or 3; and at least one of d and b is not 0;
R¹¹ and R⁶ are each independently selected from the group consisting of halogen, hydroxyl, amino, cyano, trifluoromethyl, C₁-C₆ alkyl, 3-6-membered cycloalkyl, and C₁-C₆ alkoxy; and at least one of them is fluorine;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heterocycloaryl is N; the number of the heteroatom is 1, 2, or 3.

Preferably,
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

Further, the compound is as represented by formula (XVIII): 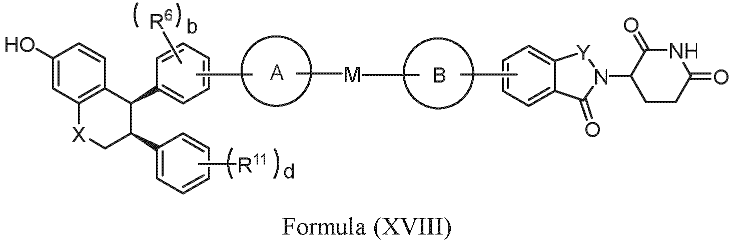 wherein,
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R¹¹ represents a substituent at any position of the benzene ring, and d is the number of the substituent R¹¹; R⁶ represents a substituent at any position of the benzene ring, and b is the number of the substituent R⁶;
d is 0, 1, 2, or 3; b is 0, 1, 2, or 3; and at least one of d and b is not 0;
R¹¹ and R⁶ are each independently selected from the group consisting of halogen, hydroxyl, amino, cyano, trifluoromethyl, C₁-C₆ alkyl, 3-6-membered cycloalkyl, and C₁-C₆ alkoxy; and at least one of them is fluorine;
ring A and ring B are each independently selected from the group consisting of 4-12-membered cycloalkyl and 4-12-membered heterocycloalkyl;
M is selected from the group consisting of absence and CR⁹R¹⁰;
R⁹ and R¹⁰ are each independently selected from the group consisting of H and C₁-C₆ alkyl;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heterocycloaryl is N; the number of the heteroatom is 1, 2, or 3.

Preferably,
X is selected from the group consisting of CH₂, CF₂ or O;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

Further, the compound is as represented by formula (XIX): 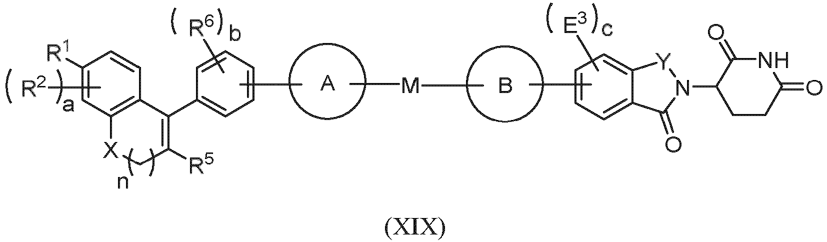 wherein,
R¹ is selected from the group consisting of H, C₁-C₆ alkyl, hydroxyl, amino or C₁-C₆ alkoxy, and dihydroxyboranyl;
R² represents a substituent at any position of the benzene ring, and a represents the number of the substituent R²;
each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
n is 0, 1, or 2;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.

Preferably,
R⁵ is selected from the group consisting of C₁-C₆ alkyl, 3-6-membered cycloalkyl, trifluoromethyl, trifluoroethyl, substituted or unsubstituted phenyl, substituted or unsubstituted thienyl, substituted or unsubstituted furanyl, substituted or unsubstituted pyrrolyl, substituted or unsubstituted pyrimidinyl, substituted or unsubstituted pyridazinyl, substituted or unsubstituted pyrazolyl, and substituted or unsubstituted pyrazinyl; the substituent of said phenyl, thienyl, furanyl, pyrrolyl, pyrimidinyl, pyridazinyl, pyrazolyl, or pyrazinyl is selected from the group consisting of halogen, hydroxyl, amino, cyano, trifluoromethyl, C₁-C₆ alkyl, 3-6-membered cycloalkyl, and C₁-C₆ alkoxy.

Further, the compound is as represented by formula (XX): 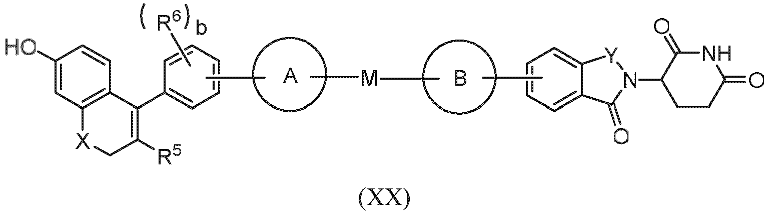 wherein,
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R⁶ represents a substituent at any position of the benzene ring, and b represents the number of the substituent R⁶;
R⁶ are each independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
Y is selected from the group consisting of absence, CR^{Y1}R^{Y2} C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.

Preferably,
R⁵ is selected from the group consisting of C₁-C₆ alkyl, 3-6-membered cycloalkyl, trifluoromethyl, trifluoroethyl, substituted or unsubstituted phenyl, substituted or unsubstituted thienyl, substituted or unsubstituted furanyl, substituted or unsubstituted pyrrolyl, substituted or unsubstituted pyrimidinyl, substituted or unsubstituted pyridazinyl, substituted or unsubstituted pyrazolyl, and substituted or unsubstituted pyrazinyl; the substituent of said phenyl, thienyl, furanyl, pyrrolyl, pyrimidinyl, pyridazinyl, pyrazolyl, or pyrazinyl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

Further, the compound is as represented by formula (XXI): 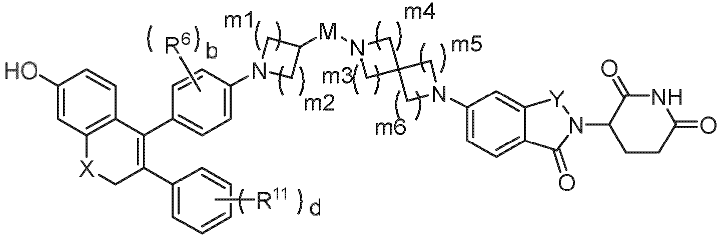 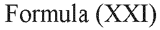 wherein,
R¹¹ represents a substituent at any position of the benzene ring, and d is the number of the substituent R¹¹;
each R¹¹ is independently selected from the group consisting of halogen, hydroxyl, amino, cyano, trifluoromethyl, C₁-C₆ alkyl, 3-6-membered cycloalkyl, and C₁-C₆ alkoxy;
d is 0, 1, 2, or 3;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
M is selected from the group consisting of absence, CR⁹R¹⁰ O, S, SO, SO₂, and NR⁹;
R⁹ and R¹⁰ are each independently selected from the group consisting of H and C₁-C₆ alkyl;
m1, m2, m3, m4, m5, and m6 are each independently selected from the group consisting of 0, 1, 2 or 3;
Y is selected from the group consisting of absence, CR^{Y1}R^{Y2} C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;

Preferably,
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl.

Further, said ring A and ring B are each independently selected from the group consisting of the following structures:

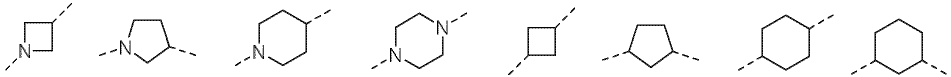

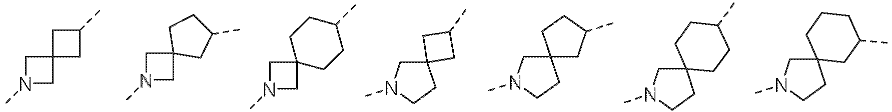

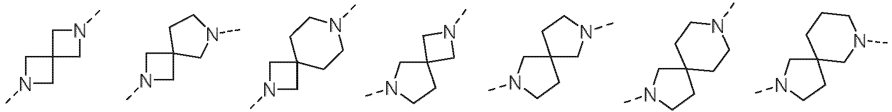

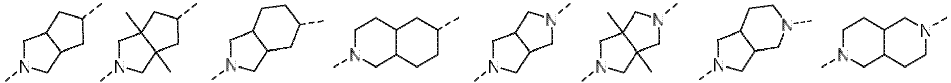

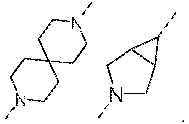

Further, said 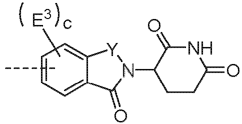 is selected from the group consisting of the following structures:

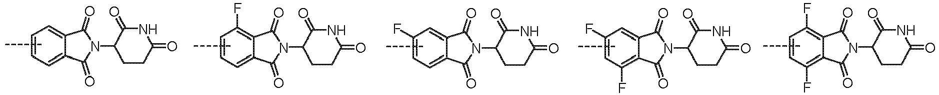

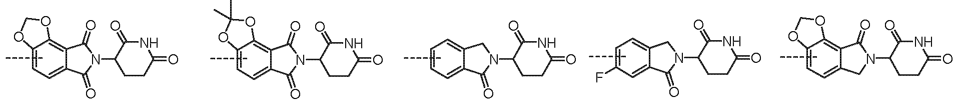

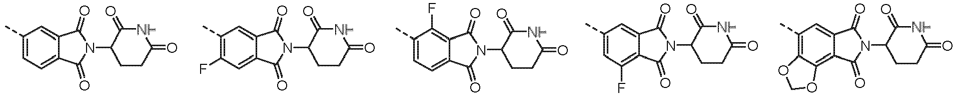

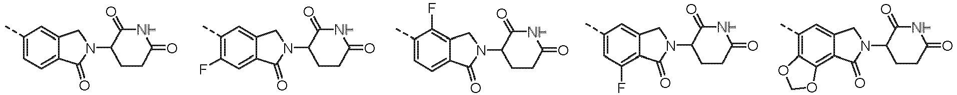

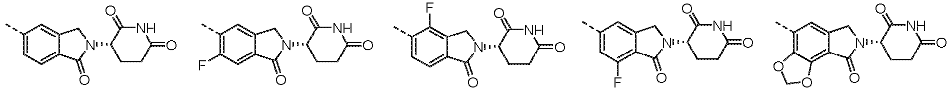

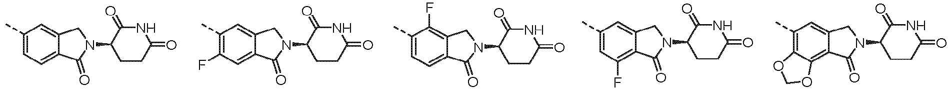

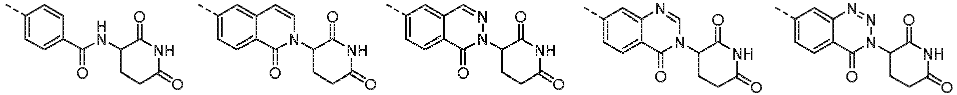

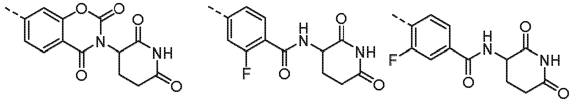

Further, the compound is selected from the group consisting of the following compounds:

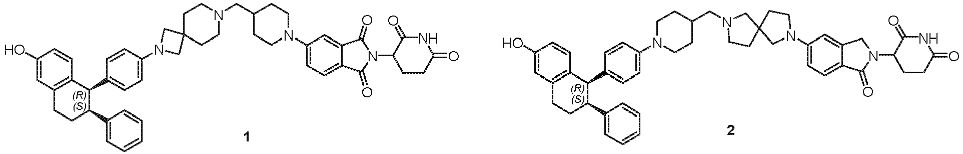

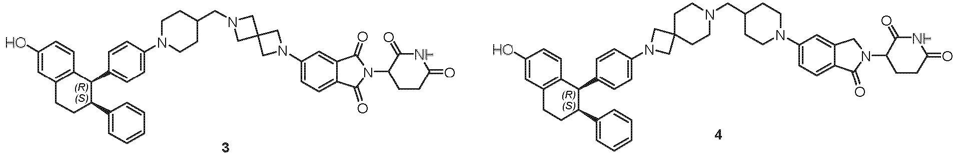

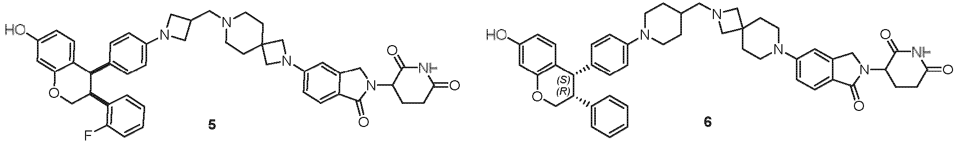

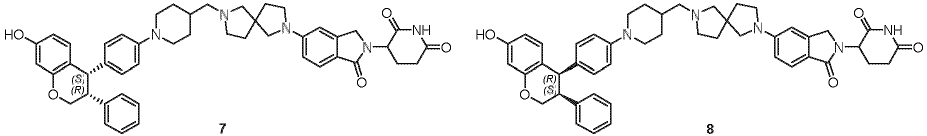

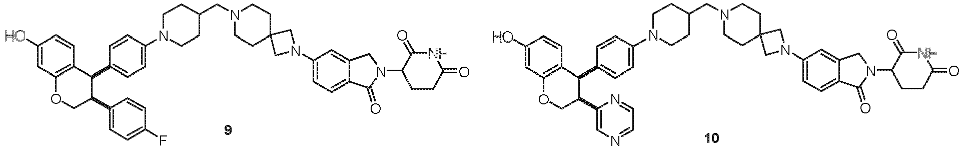

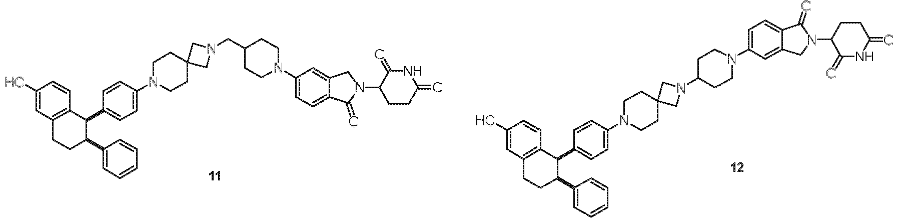

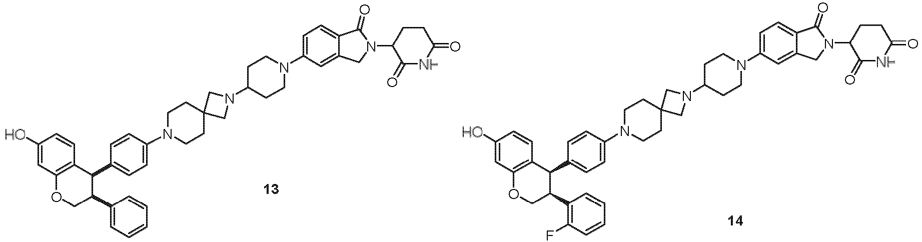

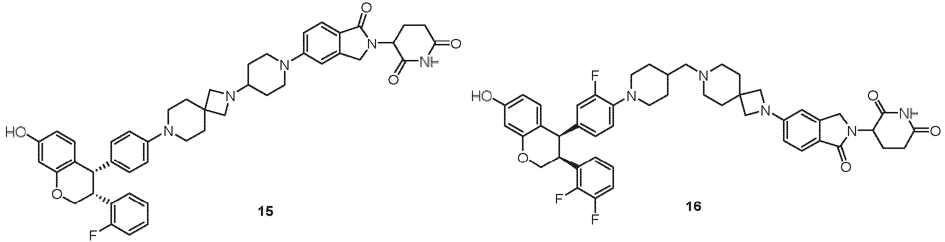

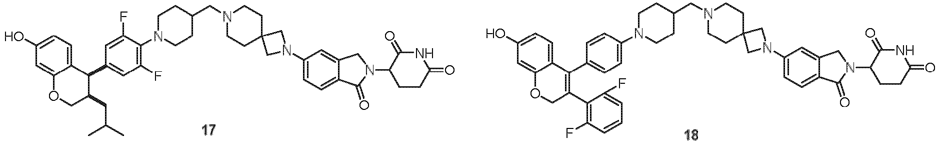

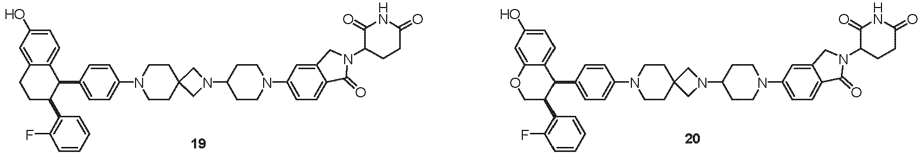

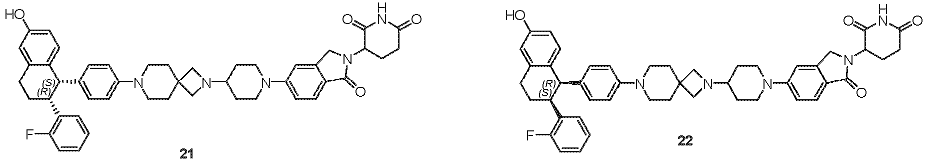

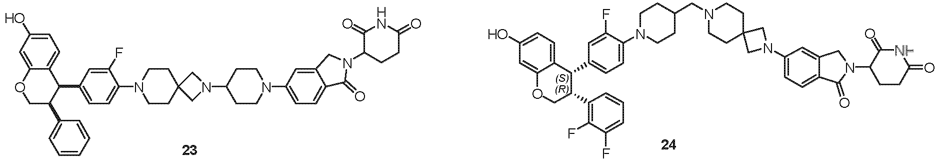

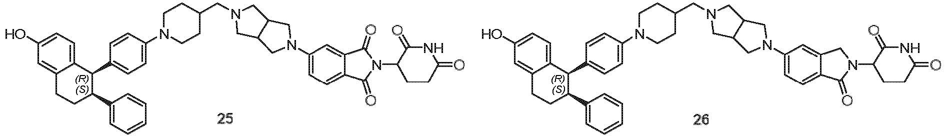

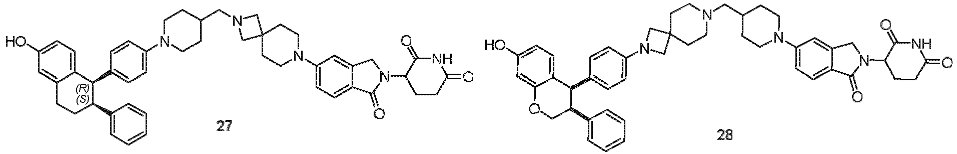

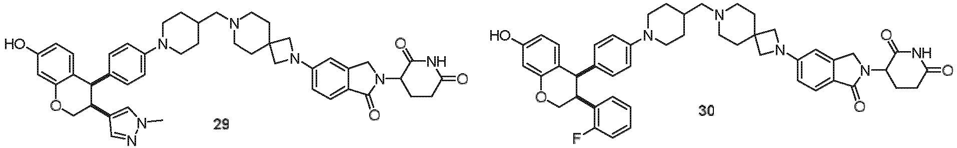

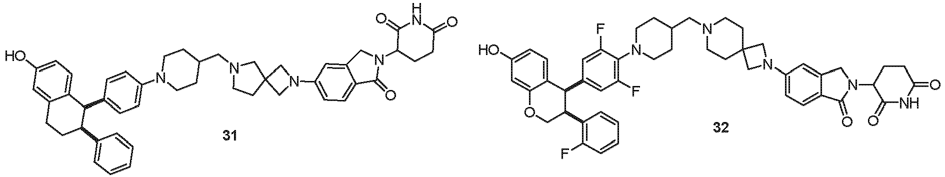

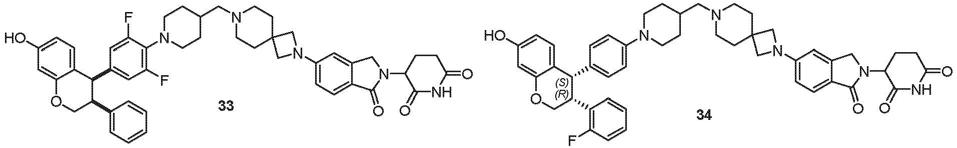

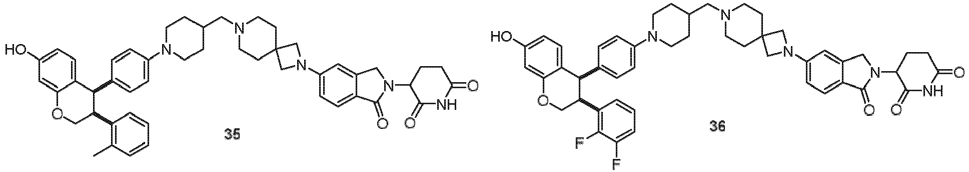

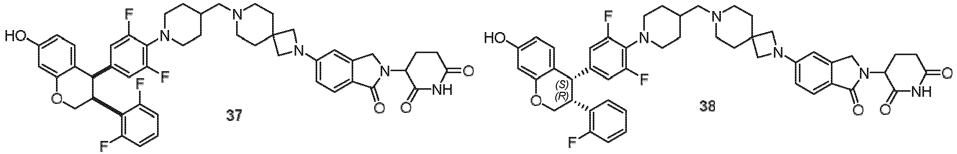

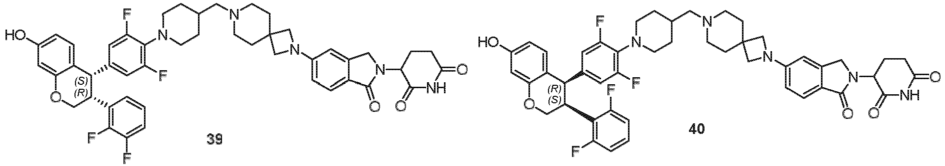

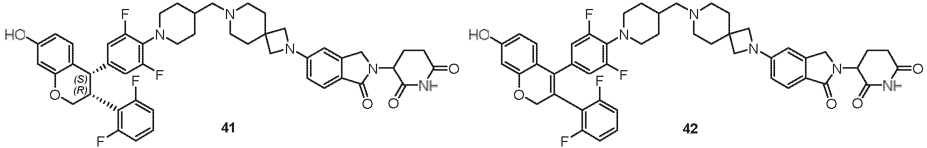

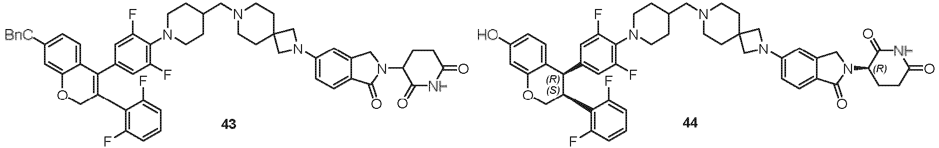

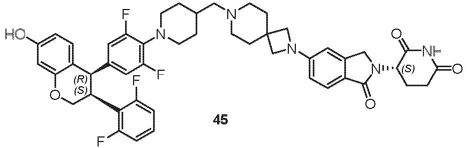

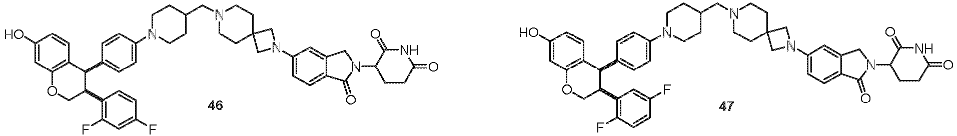

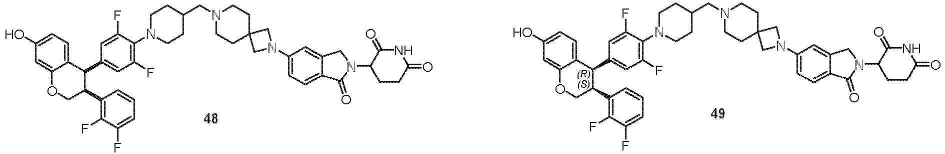

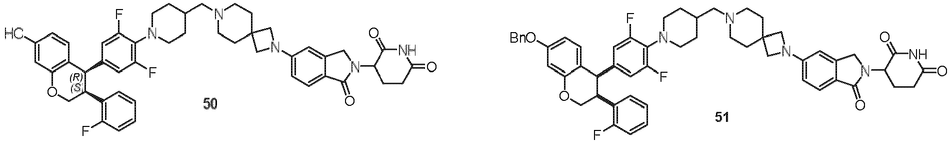

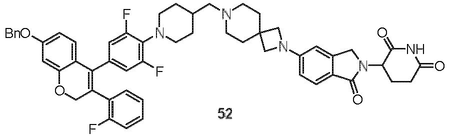

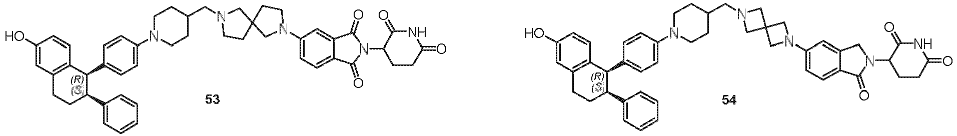

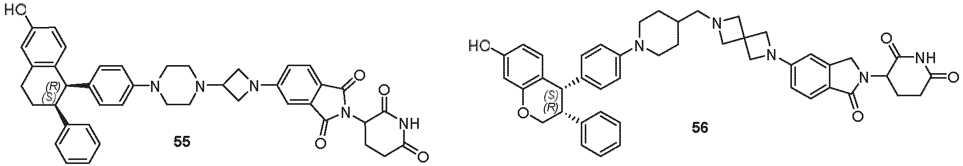

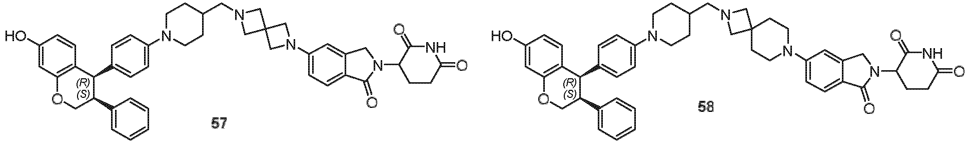

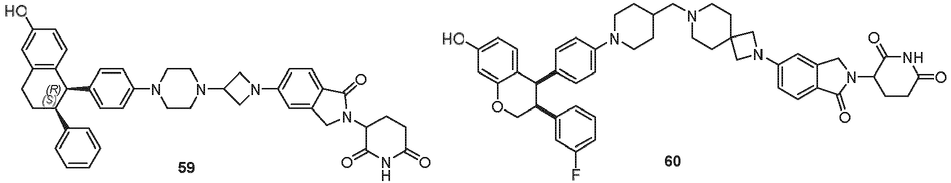

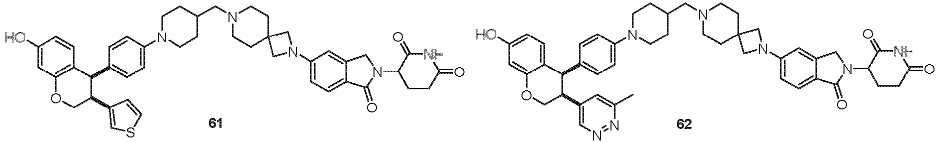

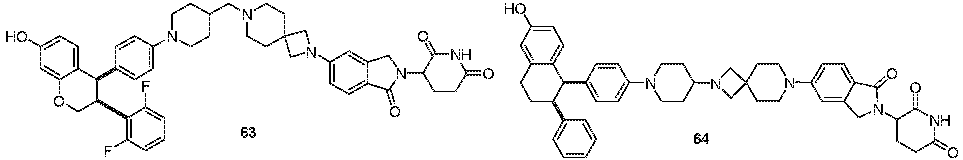

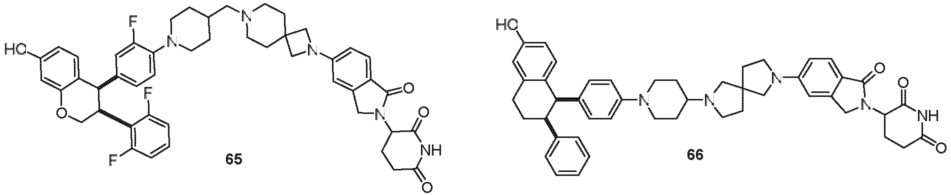

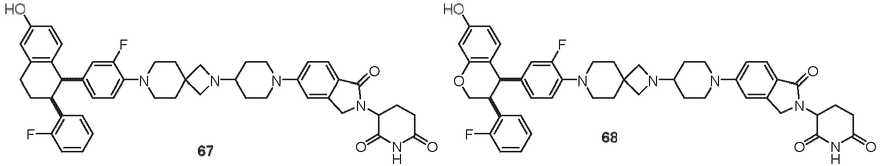

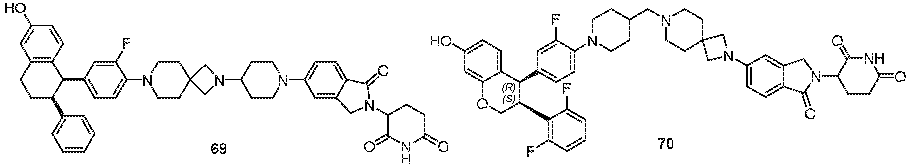

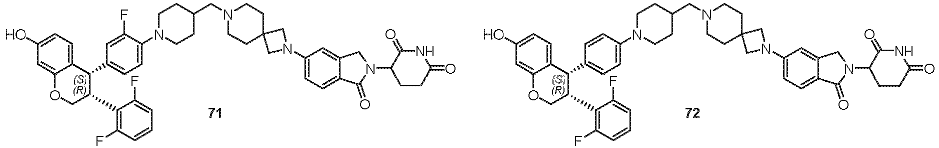

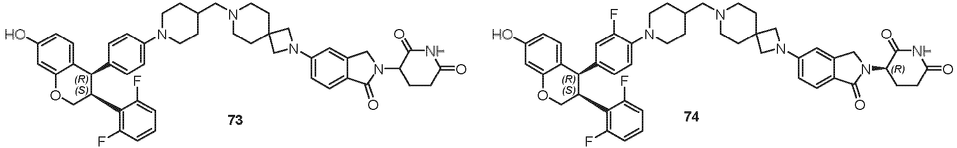

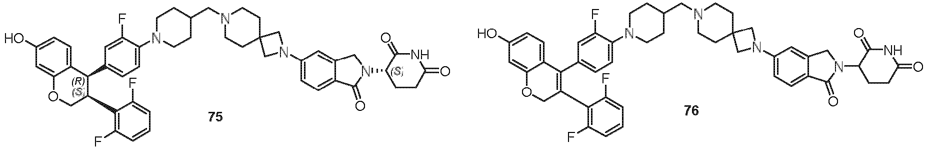

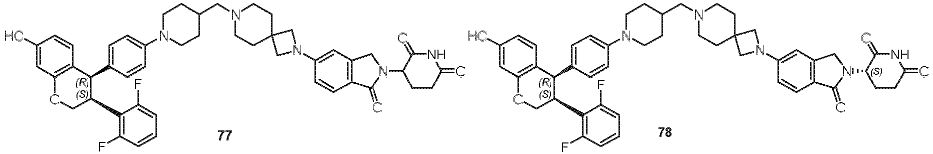

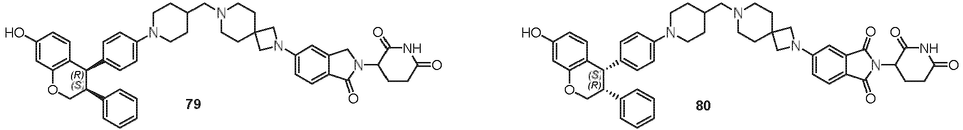

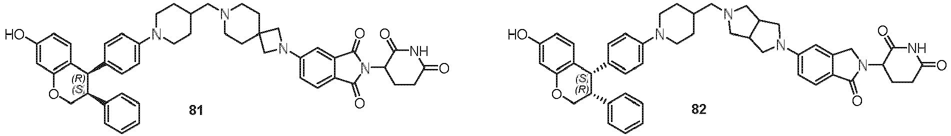

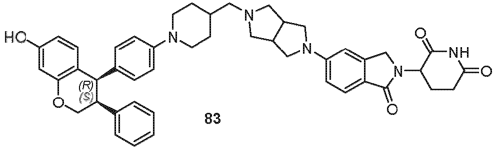

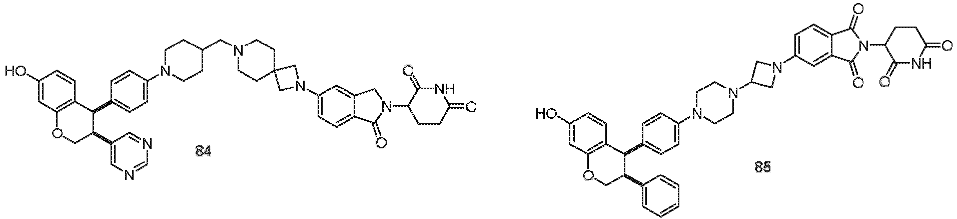

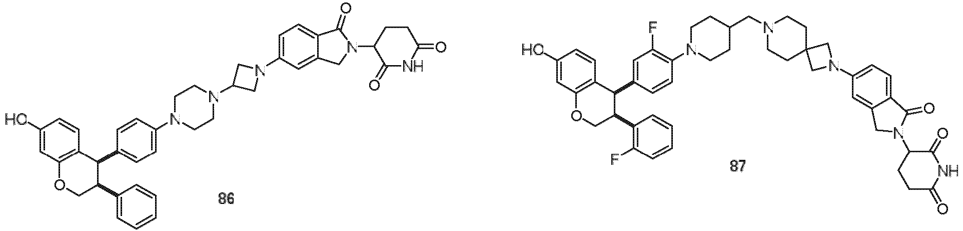

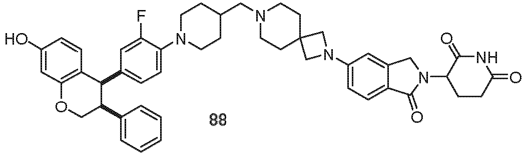

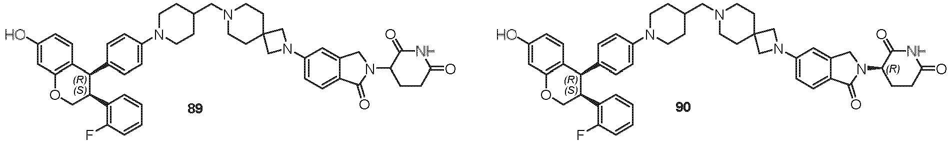

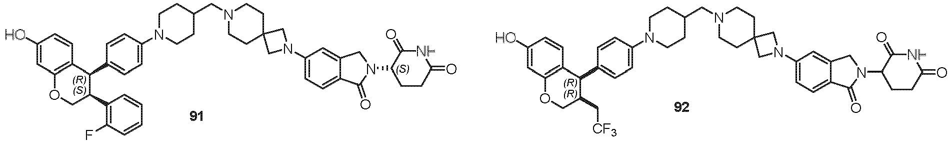

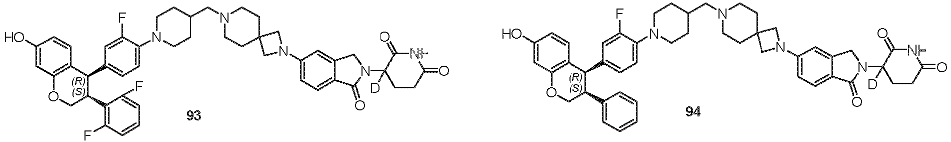

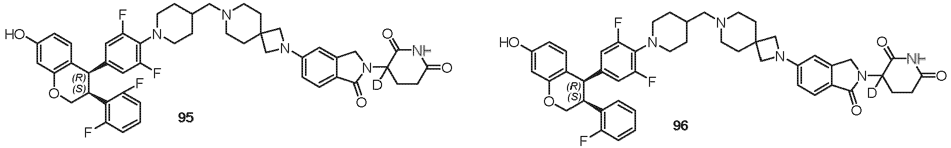

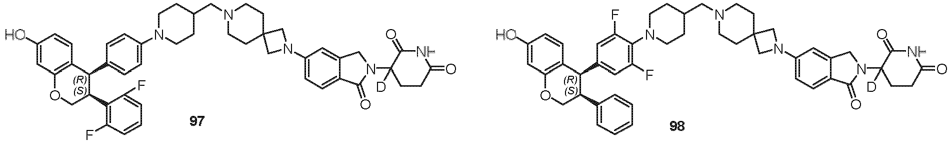

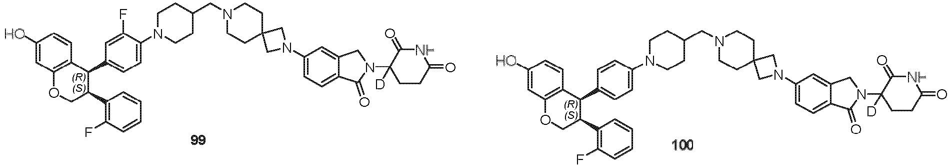

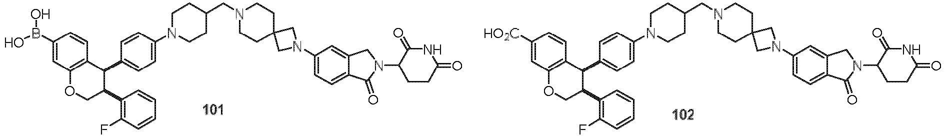

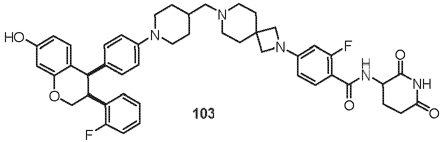

Further, the salt is a pharmaceutically acceptable salt; the pharmaceutically acceptable salt is the phosphate, D-camphorsulfonate, hydrochloride, hydrobromide, hydrofluorate, sulfate, nitrate, formate, acetate, propionate, oxalate, malonate, succinate, fumarate, maleate, lactate, malate, tartrate, citrate, picrate, mesylate, phenylmethanesulfonate, benzenesulfonate, aspartate or glutamate of the compound.

The present invention also provides the use of the above compound, or an optical isomer thereof, or a salt thereof, or a hydrate thereof, or a solvate thereof in the manufacture of estrogen receptor degraders.

The present invention also provides the use of the above compound, or an optical isomer thereof, or a salt thereof, or a hydrate thereof, or a solvate thereof in the manufacture of medicaments for treating estrogen receptor-related diseases.

The present invention also provides the use of the above compound, or an optical isomer thereof, or a salt thereof, or a hydrate thereof, or a solvate thereof in the manufacture of medicaments for the treatment and/or prevention of cancer.

The present invention also provides the use of the above compound, or an optical isomer thereof, or a salt thereof, or a hydrate thereof, or a solvate thereof in the manufacture of medicaments for the treatment and/or prevention of bone cancer, colorectal cancer, endometrial cancer, prostate cancer, ovarian cancer, uterine cancer, cervical cancer, lung cancer, and breast cancer.

The present invention also provides a medicament for the treatment of cancer, which is formed by using the above compound, or an optical isomer thereof, or a salt thereof, or a hydrate thereof, or a solvate thereof as active ingredients, in combination with pharmaceutically acceptable excipients.

The compounds and derivatives provided in the present invention can be named according to IUPAC (International Union of Pure and Applied Chemistry) or CAS (Chemical Abstracting Service, Columbus, OH) naming system.

For the definition of terms used in the present invention: unless defined otherwise, the initial definition provided for the group or term herein applies to the group or term of the whole specification; for the terms that are not specifically defined herein, they should have the same meaning as commonly understood by one of ordinary skill in the art to which the disclosure belongs.

"Substitution" means that the hydrogen in a molecule is substituted with other different atoms or molecules.

The minimum and maximum contents of carbon atoms in hydrocarbon groups are represented by prefixes, for example, the prefix Cₐ-C_{b} alkyl indicates any alkyl containing "a"-"b" carbon atoms. Therefore, for example, "C₁-C₈ alkyl" refers to an alkyl containing 1-8 carbon atoms; "C₁-C₈ alkoxy" refers to an alkoxy containing 1-8 carbon atoms.

"Alkyl" refers to a saturated hydrocarbon chain containing a specified number of carbon atoms. For example, C₁-C₈ alkyl refers to alkyls having 1-8 carbon atoms, i.e. 1, 2, 3, 4, 5, 6, 7, or 8 carbon atoms. The alkyl group can be linear or branched. Typical branched alkyls have one, two, or three branches. Alkyls include methyl, ethyl, propyl (n-propyl and isopropyl), butyl (n-butyl, isobutyl, and tert-butyl), pentyl (n-pentyl, isopentyl, and neopentyl), hexyl, and the same.

"Halogen" refers to fluorine, chlorine, bromine, or iodine.

In the present invention, a cycloalkyl refers to a saturated or partially saturated non-aromatic cyclic group consisting of carbons, without ring heteroatoms, which has a single ring or polycyclic rings (including fused, bridged, and spiro ring systems). Heterocycloalkyl refers to a saturated or partially saturated non-aromatic cyclic group containing at least one heteroatom; it includes a single ring or polycyclic rings (including fused, bridged, and spiro ring systems); among them, heteroatoms refer to N, O, and S. Examples of heterocyclic groups include, for example, piperidyl, piperazinyl, and morpholinyl.

In the present invention, aryl refers to an unsaturated aromatic group, which does not contain any ring heteroatom and has a single ring or multi-rings (including fused, bridged, and spiro ring system), such as phenyl, anthranyl, or naphthyl. Heteroaryl refers to an aromatic unsaturated ring containing at least one heteroatom; it has a single ring or multi-rings (including fused, bridged, and spiro ring system); among them, heteroatoms refer to N, O, and S. For example, pyridyl, pyrazinyl, pyridazinyl, pyrazolyl, furanyl, thienyl, oxazolyl, etc.

In the present invention, the structure of OBn is

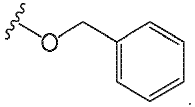

In the compound of formula (I) according to the present invention, provided that the dashed line in the benzene ring is a bond, the structure of the compound is 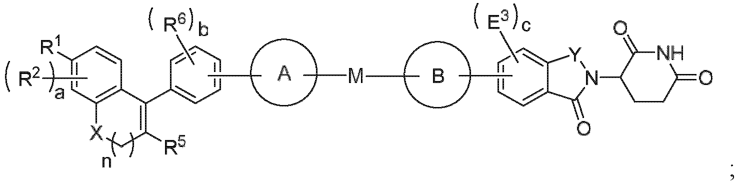 provided that the dashed line in the benzene ring is absent, the structure of the compound is

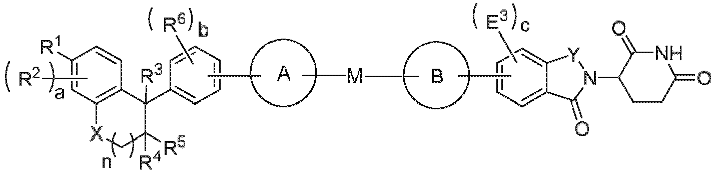

In the compound of formula (I) according to the present invention, provided that the dashed line between Y and W is absent, the structure of the compound is 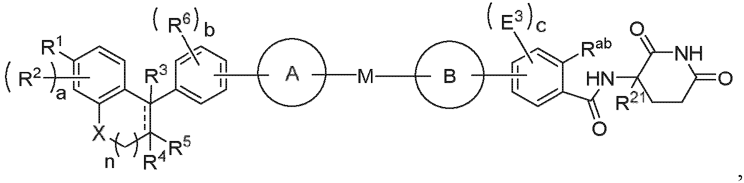 and R^{ab} is halogen; provided that the dashed line between Y and W is a bond, the structure of the compound is

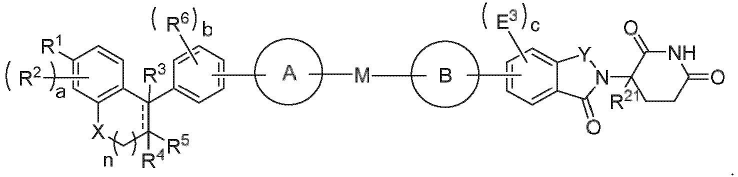

The present invention provides an aromatic compound, which can be used in the manufacture of ER degraders, as well as in the manufacture of medicaments for treating diseases related to estrogen receptors, such as medicaments for treating and/or preventing cancer, and said cancer can be breast cancer.

Obviously, based on the above content of the present invention, according to the common technical knowledge and the conventional means in the field, other various modifications, alternations, or changes can further be made, without department from the above basic technical spirits.

With reference to the following specific examples, the above content of the present invention is further illustrated. But it should not be construed that the scope of the above subject matter of the present invention is limited to the following examples. The techniques realized based on the above content of the present invention are all within the scope of the present invention.

### Description of Figures

Figure 1. The experimental curve of the compound in Example 22.
Figure 2. Western blotting of the compound in Example 81 demonstrating the result of ER degradation.

### Examples

The starting materials and equipment used in the specific examples of the present invention are all known products obtained by purchasing those commercially available.

### Example 1. 2-(2,6-dioxopiperidin-3-yl)-5-(4-(2-(4-((1R,2S)-6-hydroxyl-2-phenyl-1,2,3,4-tetrahydronaphthalen-1-yl)phenyl)-2,7-diazaspiro[3.5]nonan-7-yl)methyl)piperidin -1-yl)isoinodolin-1,3-dione

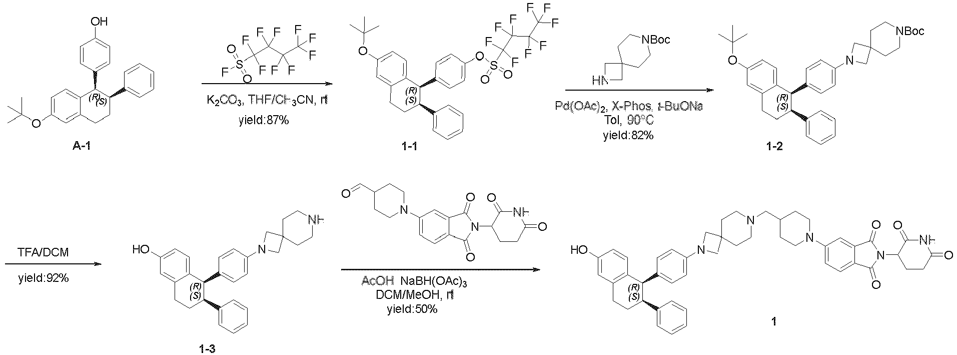

Starting material **A-1** was prepared using the literature method (WO2018102725).

### Step 1: Synthesis of 4-((1R,2S)-6-(tert-butoxy)-2-phenyl-1,2,3,4-tetrahydronaphthalen-1-yl)phenyl 1,1,2,2,3,4,4-perfluorobutane-1-sulfonate

4-((1*R*, 2*S*)-6-(tert-butoxy)-2-phenyl-1,2,3,4-tetrahydronaphthalen-1-yl)phenol (1.5 g, 4.0 mmol) was dissolved in the mixed solvent of THF (10 mL) and MeCN(10 mL), to which were added K₂CO₃ (837 mg, 6.0 mmol) and perfluorobutanesulfonyl fluoride (1.22 g, 4.0 mmol), and then the mixture was allowed to react overnight at room temperature. TLC detection indicated disappearance of starting materials. The reaction solution was concentrated, and the residue was subjected to column chromatography, to obtain 2.27 g of product, with a yield of 87%.

### Step 2: Synthesis of tert-butyl 2-(4-((1R, 2S)-6-(tert-butoxy)-2-phenyl-1,2,3,4-tetrahydronaphthalen-1-yl)phenyl)-2,7-diazaspiro[3.5]nonane-7-carboxylate

4-((1*R*,2*S*)-6-(tert-butoxy)-2-phenyl-1,2,3,4-tetrahydronaphthalen-1-yl)phenyl 1,1,2,2,3,4,4-perfluorobutane-1-sulfonate (200 mg, 0.33 mmol) and tert-butyl 2,7-diazaspiro[3.5]nonane-7-carboxylate (112 mg, 0.49 mmol) were dissolved in toluene (10 mL), to which were added Pd(OAc)₂ (11 mg, 0.05 mmol), X-Phos (32 mg, 0.07 mmol) and sodium tert-butoxide (95 mg, 0.99 mmol), and then the system was purged with Ar thrice. The reaction was allowed to react at 90 °C overnight. After completion of the reaction, the reaction solution was cooled to room temperature, and filtered via diatomaceous earth, followed by adding water. The resultant solution was extracted with ethyl acetate, washed with saturated NaCl solution, dried, and concentrated, followed by column chromatography to obtain 145 mg of product, with a yield of 82%. The product was confirmed as the target compound by LC-MS.

### Step 3: Synthesis of (5R,6S)-5-(4-(2,7-diazaspiro[3.5]nonane-2-yl)phenyl)-6-phenyl-5,6,7,8-tetrahydronaphthalen-2-ol

tert-butyl 2-(4-((1*R*,2*S*)-6-(tert-butoxy)-2-phenyl-1,2,3,4-tetrahydronaphthalen-1-yl) phenyl)-2,7-diazaspiro[3.5]nonane-7-carboxylate (145 mg, 0.25 mmol) was dissolved in DCM (6 mL), to which was added TFA (3 mL) in an ice bath, and then the reaction was allowed to react at room temperature for 1 h. The reaction was monitored by TLC until the raw materials disappeared. The reaction solution was adjusted to be neutral with saturated NaHCO₃ solution in an ice bath, and then extracted with DCM. The organic phase was washed with saturated NaCl solution, dried and concentrated, followed by column chromatograph, to obtain 97 mg of product, with a yield of 92%, which was directly used in the next step. LC/MS (ESI+) calcd for C₂₉H₃₂N₂O ( [M+H]⁺ ) *m*/*z* 425.3; found 425.3.

### Step 4: Synthesis of 2-(2,6-dioxopiperidin-3-yl)-5-(4-(2-(4-((1R, 2S)-6-hydroxyl-2-phenyl-1,2,3,4-tetrahydronaphthalen-1-yl)phenyl)-2,7-diazaspiro[3.5]nonan-7-yl)methyl)piperidin-1-yl) 1,3-dione

(5*R*,6*S*)-5-(4-(2,7-diazaspiro[3.5]nonane-2-yl)phenyl)-6-phenyl-5,6,7,8-tetrahydronaphthal en-2-ol (47 mg, 0.11 mmol) was dissolved in dichloromethane/methanol (2 mL, 10:1), to which was added 1-(2-(2,6-dioxopiperidin-3-yl)-1,3-dioxoisoinodolin-5-yl)piperidine-4-carbaldehyde (37 mg, 0.1 mmol), followed by addition of glacial acetic acid (18 mg, 0.3 mmol), and then the reaction was stirred for 0.5 h at 35 °C. The reaction solution was cooled to room temperature, and sodium triacetoxyborohydride (42 mg, 0.2 mmol) was added. The reaction was allowed to react overnight. The reaction was monitored by TLC until the raw materials disappeared, and then the reaction was quenched by adding a saturated NaHCO₃ solution. The resultant solution was extracted with dichloromethane. The organic phase was dried and concentrated, and the residue was isolated and purified by prep-TLC to obtain 39 mg of product, with a yield of 50%. ¹H NMR (400 MHz, DMSO-*d*₆) *δ* 11.08 (s, 1H), 9.11 (s, 1H), 7.64 (d, *J =* 8.5 Hz, 1H), 7.29 (d, *J* = *2.3* Hz, 1H), 7.21 (dd, *J =* 8.8, 2.3 Hz, 1H), 7.12 (dd, *J =* 10.0, 6.9 Hz, 3H), 6.82 (d, *J =* 7.1 Hz, 2H), 6.67 - 6.54 (m, 2H), 6.47 (dd, *J =* 8.3, 2.6 Hz, 1H), 6.15 (d, *J =* 8.0 Hz, 2H), 6.02 (d, *J* = 8.2 Hz, 2H), 5.06 (dd, *J =* 12.9, 5.4 Hz, 1H), 4.10 (d, *J =* 4.9 Hz, 1H), 4.03 (d, *J =* 12.9 Hz, 2H), 3.37 (s, 4H), 3.30 - 3.19 (m, 2H), 3.06 - 2.79 (m, 5H), 2.66 - 2.51 (m, 2H), 2.26 (s, 3H), 2.08 (d, *J =* 6.9 Hz, 3H), 2.04 - 1.92 (m, 2H), 1.76 (d, *J =* 12.5 Hz, 2H), 1.68 (q, *J =* 6.9, 5.5 Hz, 4H), 1.17 - 1.05 (m, 2H). LC/MS (ESI+) calcd for C₄₈H₅₁N₅O₅ ( [M+H]⁺ ) *m*/*z* 778.4; found 778.4.

### Example 2. 3-(5-(7-((1-(4-((1R,2S)-6-hydroxyl-2-phenyl-1,2,3,4-tetrahydronaphthalen-1-yl)phenyl)piperidin-4-yl)methyl)-2,7-diazaspiro[4.4]nonane-2-yl)-1-oxoisoinodolin-2-yl)piperidin-2,6-dione

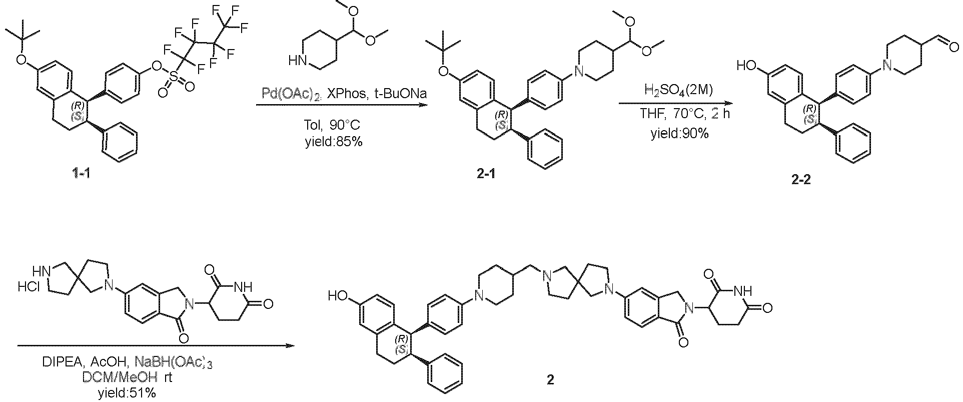

### Step 1: Synthesis of 1-(4-((1R,2S)-6-(tert-butoxy)-2-phenyl-1,2,3,4-tetrahydronaphthalen-1-yl)phenyl)-4-(dimethoxymethyl)piperidine

4-((1*R*,2*S*)-6-(tert-butoxy)-2-phenyl-1,2,3,4-tetrahydronaphthalen-1-yl)phenyl 1,1,2,2,3,4,4-perfluorobutane-1-sulfonate (1.2 g, 1.83 mmol) and 4-(dimethoxymethyl)piperidine (407 mg, 2.75 mmol) were dissolved toluene (30 mL), to which were added Pd(OAc)₂ (62 mg, 0.28 mmol), X-Phos (175 mg, 0.37 mmol), and sodium tert-butoxide (529 mg, 5.50 mmol), and then the system was purged with Ar thrice. The reaction was allowed to react at 90 °C overnight. After completion of the reaction, the reaction solution was cooled to room temperature, and filtered via diatomaceous earth, followed by adding water. The resultant solution was extracted with ethyl acetate. The organic phase was washed with saturated NaCl solution, dried, and concentrated, followed by column chromatography to obtain 798 mg of product, with a yield of 85%. The product was confirmed as the target compound by LC-MS.

### Step 2: Synthesis of 1-(4-((1R, 2S)-6-hydroxyl-2-phenyl-1,2,3,4-tetrahydronaphthalen-1-yl)phenyl)piperidine-4-carbaldehyde

1-(4-((1*R*,2*S*)-6-(tert-butoxy)-2-phenyl-1,2,3,4-tetrahydronaphthalen-1-yl)phenyl)-4-(dime thoxymethyl)piperidine (760 mg, 1.48 mmol) was dissolved in THF(40 mL), to which was added sulfuric acid (30 mL, 2 M), and the reaction was allowed to react for 30 min at 70 °C. The reaction was monitored by TLC until the raw materials disappeared. The reaction solution was cooled to room temperature, and adjusted to be pH 8 with saturated NaHCO₃ solution, and then extracted with ethyl acetate. The organic phase was washed with saturated NaCl solution, dried and concentrated, followed by column chromatograph, to obtain 547 mg of product, which was directly used in the next step.

### Step 3: Synthesis of 3-(5-(7-((1-(4-((1R,2S)-6-hydroxyl-2-phenyl-1,2,3,4-tetrahydronaphthalen-1-yl)phenyl)piperidin-4-yl)methyl)-2,7-diazaspiro[4.4]nonane-2-yl)-1-ox oisoinodolin-2-yl)piperidin-2,6-dione

3-(1-oxo-5-(2,7-diazaspiro[4.4]nonane-2-yl)isoindol-2-yl)piperidin-2,6-dione hydrochloride (20 mg, 0.05 mmol) was dissolved in dichloromethane/methanol (3 mL, 10:1), to which was added DIPEA (20 mg, 0.15 mmol), and then the reaction was stirred at room temperature for 5 min, to which was added 1-(4-((1*R*, 2*S*)-6-hydroxyl-2-phenyl-1,2,3,4-tetrahydronaphthalen-1-yl)phenyl)piperidine-4-carbaldehyde(20 mg, 0.05 mmol), followed by addition of glacial acetic acid (19 mg, 0.14 mmol). The reaction was stirred for 0.5 h at 35 °C. The reaction solution was cooled to room temperature, and sodium triacetoxyborohydride (30 mg, 0.14 mmol) was added. The reaction was allowed to react overnight. The reaction was monitored by TLC until the raw materials disappeared, and then the reaction was quenched by adding a saturated NaHCO₃ solution. The resultant solution was extracted with dichloromethane. The organic phase was dried and concentrated, and the residue was subjected to column chromatography to obtain 20 mg of product, with a yield of 51%. ¹H NMR (400 MHz, DMSO-*d*₆) *δ* 10.94 (s, 1H), 9.12 (s, 1H), 7.47 (d, *J =* 8.4 Hz, 1H), 7.19 - 7.04 (m, 3H), 6.85 - 6.78 (m, 2H), 6.65 - 6.56 (m, 4H), 6.54 - 6.43 (m, 3H), 6.18 (d, *J =* 8.5 Hz, 2H), 5.03 (dd, *J* = 13.3, 5.1 Hz, 1H), 4.28 (d, *J =* 16.7 Hz, 1H), 4.16 (dd, *J* = 16.8, 2.4 Hz, 1H), 4.11 (d, *J* = 4.9 Hz, 1H), 3.60 - 3.45 (m, 3H), 3.32 - 3.12 (m, 6H), 2.93 (dd, *J=* 16.1, 9.7 Hz, 3H), 2.69 - 2.51 (m, 3H), 2.45 - 2.38 (m, 2H), 2.33 (s, 1H), 2.26 (d, *J* = 7.1 Hz, 2H), 2.10 (dt, *J=* 12.4, 6.1 Hz, 1H), 1.95 (dt, *J* = 13.9, 6.2 Hz, 3H), 1.75 (q, *J =* 10.5, 8.8 Hz, 5H), 1.49 (td, *J =* 7.5, 3.5 Hz, 1H), 1.13 (tt, *J* = 12.1, 6.8 Hz, 2H). LC/MS (ESI+) calcd for C₄₈H₅₃N₅O₄ ( [M+H]⁺ ) *m*/*z*: 764.4; found 764.5.

### Example 3. 2-(2,6-dioxopiperidin-3-yl)-5-(6-((1-(4-((1R,2S)-6-hydroxyl-2-phenyl-1,2,3,4-tetrahydronaphthalen-1-yl)phenyl)piperidin-4-yl)methyl)-2,6-diazaspiro[3.3]hepta n-2-yl)isoinodolin-1,3-dione

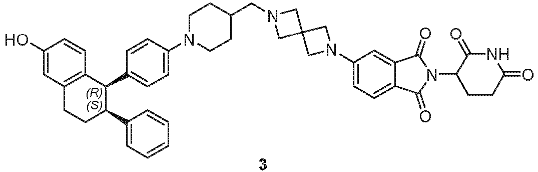

The target compound was prepared using a method similar to that in Example 2. ¹H NMR (400 MHz, DMSO-*d*₆) δ 11.08 (s, 1H), 9.12 (s, 1H), 7.64 (d, *J = 8.3* Hz, 1H), 7.13 (q, *J* = 7.9, 7.1 Hz, 3H), 6.85 - 6.73 (m, 3H), 6.67 - 6.58 (m, 3H), 6.55 - 6.43 (m, 3H), 6.19 (d, *J = 8.3* Hz, 2H), 5.05 (dd, *J* = 12.9, 5.4 Hz, 1H), 4.09 (s, 4H), 3.47 (d, *J* = 10.7 Hz, 2H), 3.29 (s, 4H), 3.01 - 2.79 (m, 4H), 2.63 - 2.54 (m, 1H), 2.49 - 2.37 (m, 3H), 2.28 (s, 2H), 2.10 (dd, *J* = 11.6, 5.5 Hz, 1H), 2.05 - 1.93 (m, 2H), 1.67 (d, *J* = 12.3 Hz, 3H), 1.14 (tt, *J* = 12.7, 6.6 Hz, 3H). LC/MS (ESI+) calcd for C₄₆H₄₇N₅O₅ ( [M+H]⁺ ) *m*/*z:* 750.4; found 750.4.

### Example 4. 3-(5-(4-(2-(4-((1R, 2S)-6-hydroxyl-2-phenyl-1,2,3,4-tetrahydronaphthalen-1-yl)phenyl)-2,7-diazaspiro[3.5]nonan-7-yl)methyl)piperidin-1-yl)-1-oxoisoinodolin-2-yl)pi peridin-2,6-dione

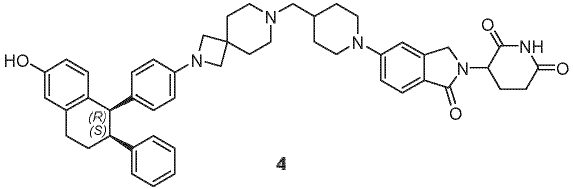

The target compound was prepared using a method similar to that in Example 1. ¹H NMR (400 MHz, DMSO-*d*₆) *δ* 10.96 (s, 1H), 9.12 (d, *J =* 30.4 Hz, 1H), 7.53 - 7.34 (m, 2H), 7.15 - 7.10 (m, 2H), 7.02 (d, *J =* 8.3 Hz, 2H), 6.82 (d, *J =* 7.2 Hz, 2H), 6.67 - 6.57 (m, 2H), 6.52 - 6.42 (m, 1H), 6.15 (d, *J =* 8.2 Hz, 2H), 6.02 (d, *J =* 8.1 Hz, 2H), 5.03 (dd, *J =* 13.3, 5.1 Hz, 1H), 4.30 (d, *J* = 16.7 Hz, 1H), 4.18 (d, *J* = 16.9 Hz, 1H), 4.10 (d, *J* = 4.9 Hz, 1H), 3.85 (d, *J* = 12.3 Hz, 2H), 3.37 (s, 7H), 2.89 (ddd, *J =* 18.2, 11.2, 5.4 Hz, 3H), 2.79 (t, *J =* 12.2 Hz, 2H), 2.60 - 2.54 (m, 1H), 2.31 (dtd, *J =* 31.3, 18.9, 16.1, 9.1 Hz, 4H), 2.09 (dd, *J =* 16.7, 6.5 Hz, 3H), 1.95 (dd, *J* = 12.0, 6.1 Hz, 1H), 1.75 (d, *J =* 11.2 Hz, 3H), 1.71 - 1.65 (m, 4H), 1.19 - 1.12 (m, 2H). LC/MS (ESI+) calcd for C₄₈H₅₃N₅O₄ ( [M+H]⁺ ) *m*/*z:* 764.4; found 764.4.

### Example 5. 3-(5-(7-((1-(4-(cis-3-(2-fluorophenyl)-7-hydroxypyran-4-yl)phenyl) azetidin-3-yl)methyl)-2,7-diazaspiro[3.5]nonane-2-yl)-1-oxoisoinodolin-2-yl)piperidin-2,6-dione

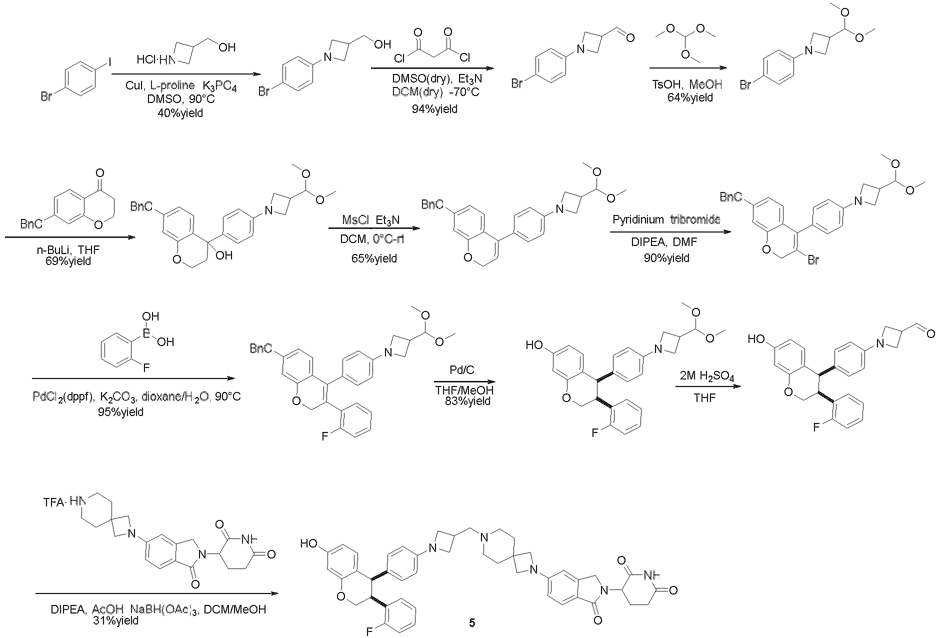

### Step 1: Synthesis of (1-(4-bromophenyl)azetidin-3-yl)methanol

Azetidin-3-ylmethanol hydrochloride (3.00 g, 24.3 mmol), p-bromoiodobenzene (5.72 g, 20.0 mmol), CuI (571 mg, 3.0 mmol), L-proline (392 mg, 3.0 mmol), and K₃PO₄(8.49 g, 40 mmol) were added into DMSO (80 mL), and the system was purged with nitrogen to have a nitrogen atmosphere. The reaction was allowed to react overnight at 90 °C. After cooling to room temperature, the reaction solution was filtered via diatomaceous earth, added with water, and then extracted with ethyl acetate. The organic layer was washed with saturated NaCl solution, dried, and concentrated, followed by column chromatography to obtain 1.93 g of product, with a yield of 40%. LC/MS (ESI+) calcd for C₁₀H₁₂BrNO ( [M+H]⁺ ) *m*/*z*: 242.0; found 242.1.

### Step 2: Synthesis of 1-(4-bromophenyl)azetidine-3-formaldehyde

Under Ar protection, to a dry three-necked flask, were added oxalyl chloride (388 mg, 3.05 mmol) and dry DCM (5 mL), to which was added the solution of DMSO (477 mg, 6.11 mmol) in DCM (2 mL) at -78 °C, and then the reaction was allowed to react 30 min at low temperature. The solution of (1-(4-bromophenyl)azetidin-3-yl)methanol (320 mg, 1.33 mmol) in DCM (3 mL) was added dropwise at -78 °C, and then allowed to react 30 min at this temperature, followed by adding triethylamine (698 mg, 6.92 mmol) dropwise. After addition, the reaction was slowly warmed to room temperature and allowed to further react for 2 h. The reaction was monitored by TLC until the raw material disappeared, and then quenched with water. The resultant solution was extracted with DCM, and the organic phase was concentrated, followed by column chromatography to obtain 300 mg of product, with a yield of 94%. LC/MS (ESI+) calcd for C₁₀H₁₀BrNO ( [M+19]⁺ ) *m*/*z:* 257.9; found 257.9.

### Step 3: Synthesis of 1-(4-bromophenyl)-3-(dimethoxymethyl)azetidine

1-(4-bromophenyl)azetidin-3-formaldehyde (300 mg, 1.25 mmol) was dissolved in MeOH (10 mL), to which were added trimethyl orthoformate (399 mg, 3.76 mmol) and p-toluenesulfonic acid (22 mg, 0.12 mmol), and then the reaction was allowed to react at room temperature overnight. The reaction was monitored by TLC until the reaction was complete. Water was added for quenching the reaction. The resultant solution was extracted with ethyl acetate. The organic phase was washed with saturated NaCl solution, dried and concentrated, followed by column chromatography, to obtain 193 mg of product, with a yield of 64%. LC/MS (ESI+) calcd for C₁₂H₁₆BrNO₂ ( [M+H]⁺ ) *m*/*z:* 286.0; found 285.9.

### Step 4: Synthesis of 7-(benzyloxy)-4-(4-(3-(dimethoxymethyl) azetidin-1-yl)phenyl) pyran-4-ol

Under nitrogen atmosphere, 1-(4-bromophenyl)-3-(dimethoxymethyl)azetidine (1.25 g, 4.38 mmol) was dissolved in dry THF (15 mL), and then cooled to below -70 °C, followed by adding n-BuLi (2.6 mL, 2.5 M, 6.57 mmol). During the whole addition process, the temperature of the reaction system was kept below -70 °C. After addition, the reaction was allowed to react for 1 h under the conditions of keeping the temperature. The solution of 7-(benzyloxy)pyran-4-one (1.01 g, 3.98 mmol) in dry THF (10 mL) was slowly added to the system, and the temperature of the reaction system was kept below -70 °C during the whole addition procedure. The mixture was reacted for 1h under the conditions of keeping the temperature. The reaction system was gradually warmed to around -40 °C, and TLC monitoring indicated the complete reaction of the raw material 7-(benzyloxy)pyran-4-one. Saturated ammonium chloride solution was added to quench the reaction, and the resultant solution was extracted with ethyl acetate. The organic phase was washed with saturated NaCl solution, dried and concentrated, followed by column chromatography, to obtain 1.4 g of product, with a yield of 69%. LC/MS (ESI+) calcd for C₂₈H₃₁NO₅ ( [M+H]⁺ ) *m*/*z:* 462.2; found 462.1.

### Step 5: Synthesis of 1-(4-(7-(benzyloxy)-2H-chromene-4-yl)phenyl)-3-(dimethoxymethyl) azetidine

7-(benzyloxy)-4-(4-(3-(dimethoxymethyl)azetidin-1-yl)phenyl)pyran-4-ol (1.4 g, 3.04 mmol) was dissolved in dichloromethane (15 mL), to which was added triethylamine (921 mg, 9.12 mmol), followed by addition of methanesulfonyl chloride (692 mg, 6.07 mmol) in an ice bath. After addition, the reaction was warmed to room temperature, and allowed to react for 2 h. TLC detection indicated disappearance of the starting material. Water was added to quench the reaction, and the resultant solution was extracted with dichloromethane. The organic phase was dried and concentrated, followed by column chromatography, to obtain 875 mg of product, with a yield of 65%. LC/MS (ESI+) calcd for C₂₈H₂₉NO₄ ( [M+H]⁺ ) *m*/*z:* 444.2; found 444.1.

### Step 6: Synthesis of 1-(4-(7-(benzyloxy)-3-bromo-2H-chromene-4-yl)phenyl)-3-(dimethoxymethyl)azetidine

1-(4-(7-(benzyloxy)-2H-chromene-4-yl)phenyl)-3-(dimethoxymethyl)azetidine (760 mg, 1.72 mmol) was dissolved in DMF (10 mL), to which was added DIPEA (666 mg, 5.16 mmol), and then pyridinium tribromide (604 mg, 1.89 mmol) was added in batches in an ice bath. After addition, the reaction was warmed to room temperature and stirred overnight. TLC detection indicated disappearance of the starting material. Water was added to quench the reaction, and the resultant solution was extracted with ethyl acetate. The organic phase was dried and concentrated, and the residue was separated by column chromatography, to obtain 800 mg of product, with a yield of 90%. LC/MS (ESI+) calcd for C₂₈H₂₈BrNO₄ ( [M+H]⁺ ) *m*/*z*: 522.1; found 522.1.

### Step 7: Synthesis of 1-(4-(7-(benzyloxy)-3-(2-fluorophenyl)-2H-chromene-4-yl)phenyl)-3-(dimethoxymethyl)azetidine

To a reaction flask, were added 1-(4-(7-(benzyloxy)-3-bromo-2H-chromene-4-yl) phenyl)-3-(dimethoxymethyl)azetidine (300 mg, 0.57 mmol), o-fluorophenylboronic acid (89 mg, 0.63 mmol), Pd(dppf)Cl₂(42 mg, 0.06 mmol), potassium carbonate (157 mg, 1.14 mmol), and dioxane/H₂O(12 mL/3 mL), and then the system was purged with nitrogen. The reaction was allowed to react at 90 °C overnight. The reaction was complete by detection with TLC. The reaction solution was cooled to room temperature, filtered via diatomaceous earth, and concentrated, followed by column chromatography to obtain 290 mg of product, with a yield of 95%. LC/MS (ESI+) calcd for C₃₄H₃₂FNO₄ ( [M+H]⁺ ) *m*/*z:* 538.2; found 538.2.

### Step 8. Synthesis of cis-4-(4-(3-(dimethoxymethyl)azetidin-1-yl)phenyl)-3-(2-fluorophenyl) pyran-7-ol

1-(4-(7-(benzyloxy)-3-(2-fluorophenyl)-2H-chromene-4-yl)phenyl)-3-(dimethoxymethyl)a zetidine (290 mg, 0.54 mmol) was dissolved in THF (10 mL), to which was added methanol (20 mL), followed by addition of 10% Pd/C (30 mg, 10%), and then the system was purged with hydrogen. The reaction was allowed to react overnight at room temperature. The reaction was complete by detection with TLC. The reaction solution was filtered via diatomaceous earth to remove Pd/C, and then concentrated, followed by column chromatography to obtain 200 mg of product, with a yield of 83%. LC/MS (ESI+) calcd for C₂₇H₂₈FNO₄ ( [M+H]⁺ ) *m*/*z*: 450.2; found 450.1.

### Step 9: Synthesis of 1-(4-(cis-3-(2-fluorophenyl)-7-hydroxychroman-4-yl)phenyl)azetidin-3-formaldehyde

*cis*-4-(4-(3-(dimethoxymethyl)azetidin-1-yl)phenyl)-3-(2-fluorophenyl)pyran-7-ol (165 mg, 0.37 mmol) was dissolved in THF (10 mL), to which was added sulfuric acid (3 mL, 2 M), and the reaction was allowed to react for 30 min at 70 °C. The reaction was monitored by TLC until the raw materials disappeared. The reaction solution was cooled to room temperature, and adjusted to be about pH 8 with saturated NaHCO₃ solution, and then extracted with ethyl acetate. The organic phase was washed with saturated NaCl solution, dried and concentrated, followed by column chromatograph, to obtain 150 mg of crude product, which was directly used in the next step. LC/MS (ESI+) calcd for C₂₅H₂₂FNO₃ ( [M+H]⁺ ) *m*/*z:* 404.2; found 404.1_{∘}

### Step 10: Synthesis of 3-(5-(7-((1-(4-(cis-3-(2-fluorophenyl)-7-hydroxypyran-4-yl)phenyl) azetidin-3-yl)methyl)-2,7-diazaspiro[3.5]nonane-2-yl)-1-oxoisoinodolin-2-yl)piperidin-2,6-dion e

2-(2,6-dioxopiperidin-3-yl)-5-(2,7-diazaspiro[3.5]nonane-2-yl)isoinodolin-1,3-dione trifluoroacetate (47 mg, 0.13 mmol) was dissolved in dichloromethane/methanol (5 mL, 10:1), to which was added DIPEA (40 mg, 0.3 mmol), and then stirred for 5 min at room temperature, followed by addition of 1-(4-(*cis*-3-(2-fluorophenyl)-7-hydroxychroman-4-yl)phenyl)azetidin-3-formaldehyde (52 mg, 0.13 mmol). Then, glacial acetic acid (23 mg, 0.39 mmol) was added. The reaction was stirred for 0.5 h at 35 °C. The reaction solution was cooled to room temperature, and sodium triacetoxyborohydride (82 mg, 0.39 mmol) was added. The reaction was allowed to react overnight. The reaction was monitored by TLC until the raw materials disappeared, and then quenched by adding saturated NaHCO₃ solution. The resultant solution was extracted with dichloromethane. The organic phase was dried and concentrated, and the residue was subjected to column chromatography to obtain 30 mg of product, with a yield of 31%.

¹H NMR (400 MHz, DMSO-*d*₆) *δ* 10.94 (s, 1H), 9.32 (s, 1H), 7.47 (d, *J* = 8.3 Hz, 1H), 7.26 - 7.19 (m, 1H), 7.16 - 7.10 (m, 1H), 6.92 - 6.86 (m, 1H), 6.65 (d, *J* = 8.2 Hz, 1H), 6.51 - 6.39 (m, 3H), 6.36 (d, *J* = 8.1 Hz, 2H), 6.32 - 6.26 (m, 2H), 6.09 (d, *J =* 8.1 Hz, 2H), 5.03 (dd, *J* = 13.3, 5.1 Hz, 1H), 4.39 - 4.26 (m, 2H), 4.23 - 4.11 (m, 3H), 3.78 (td, *J =* 7.4, 4.8 Hz, 2H), 3.70 (dd, *J* = 10.9, 4.5 Hz, 1H), 3.61 (s, 4H), 3.30 (s, 2H), 2.88 (ddt, *J =* 20.6, 14.8, 6.2 Hz, 2H), 2.62 - 2.54 (m, 1H), 2.40 - 2.18 (m, 5H), 1.96 (dd, *J =* 12.9, 7.1 Hz, 1H), 1.72 (t, *J =* 5.4 Hz, 4H), 1.23 (d, *J* = 3.7 Hz, 2H). LC/MS (ESI+) calcd for C₄₅H₄₆FN₅O₅ ( [M+H]⁺ ) *m*/*z*: 756.3; found 756.2.

### Example 6. 3-(5-(2-((1-(4-((3R, 4S)-7-hydroxyl-3-phenyltryptophane-4-yl)phenyl) piperidin-4-yl)methyl)-2,7-diazaspiro[3.5]nonan-7-yl)-1-oxoisoinodolin-2-yl)piperidin-2,6-dione

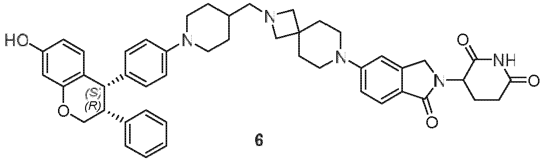

The target compound was prepared using a method similar to that in Example 5. ¹H NMR (400 MHz, DMSO-*d*₆) *δ* 10.96 (s, 1H), 9.34 (s, 1H), 7.50 (d, *J =* 8.4 Hz, 1H), 7.14 (p, *J =* 3.7 Hz, 3H), 7.11 - 7.01 (m, 2H), 6.76 (dd, *J =* 6.6, 3.0 Hz, 2H), 6.63 (dd, *J =* 14.0, 8.4 Hz, 3H), 6.38 (d, *J =* 8.4 Hz, 2H), 6.34 - 6.22 (m, 2H), 5.04 (dd, *J =* 13.3, 5.1 Hz, 1H), 4.38 - 4.31 (m, 1H), 4.29 (s, 1H), 4.20 (s, 1H), 4.17 (d, *J* = 4.7 Hz, 2H), 3.56 (t, *J* = 10.8 Hz, 6H), 3.29 (s, 4H), 3.16 - 3.01 (m, 3H), 2.63 - 2.55 (m, 1H), 2.52 (s, 2H), 2.47 (s, 1H), 2.42 - 2.31 (m, 1H), 2.01 (d, *J* = 7.4 Hz, 1H), 1.97 - 1.90 (m, 2H), 1.85 (s, 2H), 1.76 (d, *J =* 12.3 Hz, 3H), 1.23 (s, 2H). LC/MS (ESI+) calcd for C₄₇H₅₁N₅O₅ ( [M+H]⁺ ) *m*/*z:* 766.4; found 766.4_{∘}

### Example 7. 3-(5-(7-((1-(4-((3R,4S)-7-hydroxyl-3-phenyltryptophane-4-yl)phenyl) piperidin-4-yl)methyl)-2,7-diazaspiro[4.4]nonane-2-yl)-1-oxoisoinodolin-2-yl)piperidin-2,6 -dione

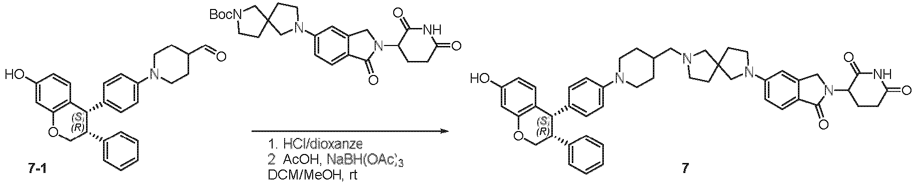

Starting material **7-1** was prepared by reference to the literature method (US10800770). The target compound was prepared using a method similar to that in Example 5. ¹H NMR (400 MHz, DMSO-*d*₆) *δ* 10.94 (s, 1H), 9.30 (s, 1H), 7.50 - 7.43 (m, 1H), 7.15 - 7.11 (m, 3H), 6.78 - 6.73 (m, 2H), 6.65 (d, *J = 8.3* Hz, 1H), 6.59 (d, *J* = 9.2 Hz, 4H), 6.36 (d, *J* = 8.2 Hz, 2H), 6.31 - 6.23 (m, 2H), 5.03 (dd, *J =* 13.3, 5.1 Hz, 1H), 4.34 - 4.25 (m, 2H), 4.22 - 4.13 (m, 3H), 3.51 (dt, *J* = 11.5, 6.5 Hz, 5H), 3.22 (d, *J* = 9.4 Hz, 3H), 2.89 (td, *J =* 13.1, 12.0, 6.2 Hz, 2H), 2.71 - 2.58 (m, 2H), 2.54 (d, *J =* 7.9 Hz, 2H), 2.48 - 2.40 (m, 2H), 2.31 (dd, *J =* 17.9, 5.6 Hz, 3H), 1.96 (dq, *J* = 9.8, 5.5 Hz, 3H), 1.76 (q, *J =* 8.0, 7.5 Hz, 4H), 1.23 (d, *J* = 3.6 Hz, 2H). LC/MS (ESI+) calcd for C₄₇H₅₁N₅O₅ ( [M+H]⁺ ) *m*/*z:* 766.4; found 766.4_{∘}

### Example 8. 3-(5-(7-((1-(4-((3S,4R)-7-hydroxyl-3-phenyltryptophane-4-yl)phenyl) piperidin-4-yl)methyl)-2,7-diazaspiro[4.4]nonane-2-yl)-1-oxoisoinodolin-2-yl)piperidin-2,6 -dione

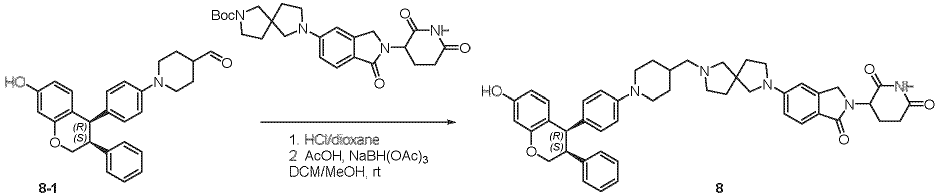

Starting material **8-1** was prepared by reference to the literature method. The target compound was prepared using a method similar to that in Example 5. ¹H NMR (400 MHz, DMSO-*d*₆) *δ* 11.13 - 10.66 (m, 1H), 9.31 (s, 1H), 7.48 (t, *J* = 8.4 Hz, 1H), 7.17 - 7.11 (m, 3H), 6.77 - 6.72 (m, 2H), 6.65 (d, *J* = 8.3 Hz, 1H), 6.59 (d, *J =* 9.3 Hz, 4H), 6.36 (d, *J =* 8.2 Hz, 2H), 6.31 - 6.24 (m, 2H), 5.03 (dd, *J =* 13.3, 5.1 Hz, 1H), 4.36 - 4.25 (m, 2H), 4.21 - 4.11 (m, 3H), 3.51 (dt, *J* = 11.8, 6.6 Hz, 5H), 3.22 (d, *J* = 9.5 Hz, 3H), 2.98 - 2.78 (m, 2H), 2.59 (td, *J =* 16.3, 6.0 Hz, 4H), 2.47 - 2.38 (m, 2H), 2.34 (dd, *J* = 13.2, 4.4 Hz, 1H), 2.26 (d, *J =* 7.2 Hz, 2H), 2.00 - 1.92 (m, 3H), 1.75 (q, *J* = 6.6 Hz, 4H), 1.23 (d, *J* = 3.6 Hz, 2H). LC/MS (ESI+) calcd for C₄₇H₅₁N₅O₅ ( [M+H]⁺ ) *m*/*z:* 766.4; found 766.4_{∘}

### Example 9. 3-(5-(7-((1-(4-(cis-3-(4-fluorophenyl)-7-hydroxyltryptophane-4- yl)phenyl) piperidin-4-yl)methyl)-2,7-diazaspiro[3.5]nonane-2-yl)-1-oxoisoinodolin-2-yl)piperidin-2,6 -dione

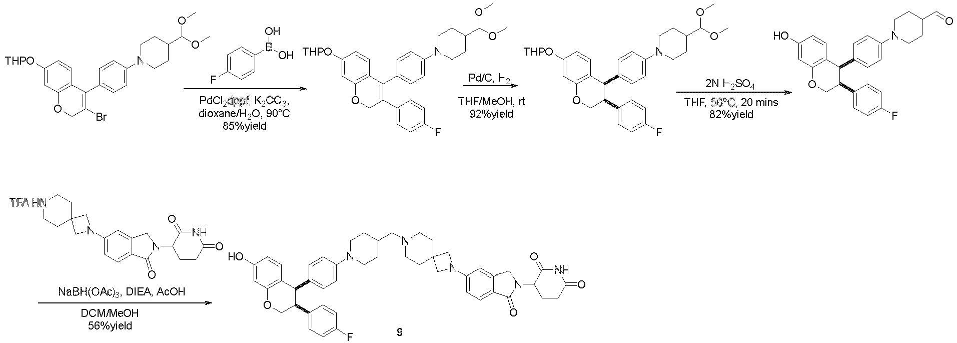

The target compound was prepared using a method similar to that in Example 5. ¹H NMR (400 MHz, DMSO-*d*₆) *δ* 10.95 (s, 1H), 9.32 (s, 1H), 7.50 (d, *J =* 8.3 Hz, 1H), 6.98 (t, *J =* 8.8 Hz, 2H), 6.79 (dd, *J =* 8.5, 5.6 Hz, 2H), 6.65 (d, *J =* 8.3 Hz, 3H), 6.53 - 6.45 (m, 2H), 6.39 (d, *J =* 8.2 Hz, 2H), 6.34 - 6.21 (m, 2H), 5.03 (dd, *J =* 13.3, 5.1 Hz, 1H), 4.35 - 4.25 (m, 2H), 4.22 - 4.12 (m, 3H), 3.70 (d, *J =* 13.5 Hz, 4H), 3.64 - 3.52 (m, 4H), 3.17 - 3.10 (m, 1H), 3.05 - 2.84 (m, 4H), 2.58 (d, *J =* 16.0 Hz, 3H), 2.41 - 2.27 (m, 2H), 1.98 (td, *J =* 15.0, 6.7 Hz, 5H), 1.78 (s, 3H), 1.25 - 1.24 (m, 2H). LC/MS (ESI+) calcd for C₄₇H₅₀FN₅O₅ ( [M+H]⁺ ) *m*/*z:* 784.4; found 784.4_{∘}

### Example 10. 3-(5-(7-((1-(4-(cis-7-hydroxyl-3-(pyrazin-2-yl)pyran-4-yl)phenyl) piperidin-4-yl)methyl)-2,7-diazaspiro[3.5]nonane-2-yl)-1-oxoisoinodolin-2-yl)piperidin-2,6 -dione

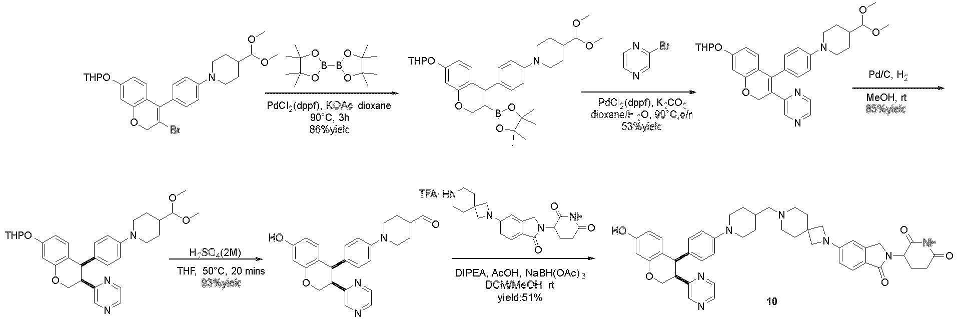

### Step 1: Synthesis of 4-(dimethoxymethyl)-1-(4-(7-((tetrahydro-2H-pyran-2-yl)oxy)-3-(4,4,5,5-tetramethyl-1,3,2-dioxaborolan-2-yl)-2H-toluene-4-yl)phenyl)piperidine

1-(4-(3-bromo-7-((tetrahydro-2H-pyran-2-yl)oxy)-2H-chromene-4-yl)phenyl)-4-(dimethox ymethyl)piperidine (543 mg, 1.00 mmol), bis(pinacolato)diboron (381 mg, 1.50 mmol), PdCl₂(dppf) (73 mg, 0.1 mmol), and potassium acetate (294 mg, 3.00 mmol) were added into 1,4-dioxane (15 mL), and then the system was purged with nitrogen. The reaction was allowed to react at 90 °C for 3 h. The reaction solution was cooled to room temperature, and water was added to quench the reaction. The resultant solution was extracted with ethyl acetate. The organic phase was washed with saturated NaCl solution, dried and concentrated, followed by column chromatography, to obtain 508 mg of product, with a yield of 86%. LC/MS (ESI+) calcd for C₃₄H₄₆BNO₇ ( [M+H]⁺ ) *m*/*z:* 592.3; found 592.4.

### Step 2: Synthesis of 2-(4-(4-(4-(4-(dimethoxymethyl)piperidin-1-yl)phenyl)-7-((tetrahydro-2H-pyran-2-yl)oxy)-2H-chromene-3-yl)pyrazine

4-(dimethoxymethyl)-1-(4-(7-((tetrahydro-2H-pyran-2-yl)oxy)-3-(4,4,5,5-tetramethyl-1,3, 2-dioxaborolan-2-yl)-2H-toluene-4-yl)phenyl)piperidine (300 mg, 0.51 mmol), 2-bromopyrazine (105 mg, 0.66 mmol), PdCl₂(dppf) (37 mg, 0.05 mmol), and K₂CO₃ (211 mg, 1.53 mmol) were added into the mixed solvent of 1,4-dioxane/H₂O (15 mL /4 mL), and then the system was purged with nitrogen. The reaction was allowed to react at 90 °C overnight. The reaction solution was cooled to room temperature, and water was added to quench the reaction. The resultant solution was extracted with ethyl acetate. The organic phase was washed with saturated NaCl solution, dried and concentrated, followed by column chromatography, to obtain 147 mg of product, with a yield of 53%. LC/MS (ESI+) calcd for C₃₂H₃₇N₃O₅ ( [M+H]⁺ ) *m*/*z*: 544.3; found 544.2.

### Step 3: Synthesis of 2-(cis-4-(4-(dimethoxymethyl)piperidin-1-yl)phenyl)-7-((tetrahydro-2H-pyran-2-yl)oxy)pyran-3-yl)pyrazine

2-(4-(4-(4-(4-(dimethoxymethyl)piperidin-1-yl)phenyl)-7-((tetrahydro-2H-pyran-2-yl)oxy) -2H-chromene-3-yl)pyrazine (147 g, 0.27 mmol) was dissolved in the mixed solvent of THF(10 mL) and MeOH (10 mL), to which was added Pd/C (20 mg), and then the system was purged with hydrogen thrice. The reaction was stirred overnight at room temperature. The reaction was complete by detection with TLC. The reaction solution was filtered via diatomaceous earth to remove Pd/C, and then concentrated, followed by separation over column chromatography to obtain 125 mg of product, with a yield of 85%.

### Step 4: Synthesis of 1-(4-(cis-7-hydroxyl-3-(pyrazin-2-yl)pyran-4-yl)phenyl) piperidine-4-carbaldehyde

2-(*cis*-4-(4-(dimethoxymethyl)piperidin-1-yl)phenyl)-7-((tetrahydro-2H-pyran-2-yl)oxy)py ran-3-yl)pyrazine (125 mg, 0.23 mmol) was dissolved in THF (15 mL), to which was added 2M H₂SO₄ (5 mL), and then the reaction was warmed to 50 °C and reacted for 20 min. TLC detection indicated disappearance of starting materials. The reaction solution was adjusted to alkaline with saturated NaHCO₃ solution in an ice bath, and then extracted with ethyl acetate, followed by drying and concentrating, to obtain 88 mg of product, with a yield of 93%.

### Step 5: Synthesis of 3-(5-(7-((1-(4-(cis-7-hydroxyl-3-(pyrazin-2-yl)pyran-4-yl)phenyl) piperidin-4-yl)methyl)-2,7-diazaspiro[3.5]nonane-2-yl)-1-oxoisoinodolin-2-yl)piperidin-2,6-dio ne

3-(1-oxo-5-(2,7-diazaspiro[3.5]nonane-2-yl)isoinodolin-2-yl)piperidin-2,6-dione trifluoroacetate (38 mg, 0.08 mmol) was dissolved in dichloromethane/methanol (5 mL, 10:1), to which was added DIPEA (37 mg, 0.29 mmol), and then the reaction was stirred for 10 min, followed by addition of 1-(4-(*cis*-7-hydroxyl-3-(pyrazin-2-yl)pyran-4-yl)phenyl)piperidine-4-carbaldehyde (30 mg, 0.07 mmol) and glacial acetic acid (18 mg, 0.3 mmol). The reaction was stirred for 0.5 h at 35 °C. The reaction solution was cooled to room temperature, and sodium triacetoxyborohydride (46 mg, 0.2 mmol) was added. The reaction was allowed to react overnight. The reaction was monitored by TLC until the raw materials disappeared, and then quenched by adding saturated NaHCO₃ solution. The resultant solution was extracted with dichloromethane. The organic phase was dried and concentrated, and the residue was subjected to column chromatography to obtain 28 mg of product, which was determined to be the target compound by LC-MS, with a yield of 51%. ¹H NMR (400 MHz, DMSO-*d*₆) *δ* 10.94 (s, 1H), 9.33 (s, 1H), 8.47 (d, *J* = 1.5 Hz, 1H), 8.40 (d, *J =* 2.5 Hz, 1H), 8.36 (t, *J =* 2.1 Hz, 1H), 7.47 (d, *J* = 8.3 Hz, 1H), 6.69 (d, *J =* 8.3 Hz, 1H), 6.57 (d, *J =* 8.5 Hz, 2H), 6.53 - 6.43 (m, 2H), 6.39 - 6.26 (m, 4H), 5.03 (dd, *J* = 13.3, 5.1 Hz, 1H), 4.47 - 4.34 (m, 3H), 4.33 - 4.14 (m, 2H), 3.80 (dt, *J* = 10.4, 4.7 Hz, 1H), 3.62 (s, 4H), 3.55 - 3.47 (m, 2H), 2.94 - 2.83 (m, 1H), 2.57 (dd, *J =* 17.5, 4.3 Hz, 1H), 2.48 - 2.43 (m, 1H), 2.41 - 2.22 (m, 4H), 2.15 (d, *J =* 24.0 Hz, 2H), 2.03 - 1.90 (m, 2H), 1.83 - 1.65 (m, 6H), 1.59 (s, 1H), 1.22 (s, 1H), 1.15 - 1.05 (m, 2H). LC/MS (ESI+) calcd for C₄₅H₄₉N₇O₅ ( [M+H]⁺ ) *m*/*z:* 768.4; found 768.3.

### Example 11. 3-(5-(4-(7-(4-(cis-6-hydroxyl-2-phenyl-1,2,3,4-tetrahydronaphthalen-1-yl) phenyl)-2,7-diazaspiro[3.5]nonane-2-yl)methyl)piperidin-1-yl)-1-oxoisoinodolin-2-yl)piper idin-2,6-dione

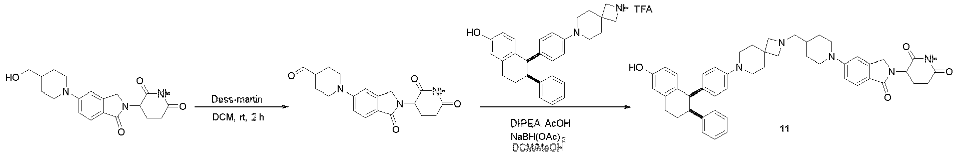

### Step 1: Synthesis of 1-(2-(2,6-dioxopiperidin-3-yl)-1-oxoisoinodolin-5-yl)piperidine-4-carbaldehyde

3-(5-(4-(hydroxymethyl)piperidin-1-yl)-1-oxoisoinodolin-2-yl)piperidin-2,6-dione (150 mg, 0.42 mmol) was dissolved in DCM (40 mL), to which was added Dess-martin (356 mg, 0.84 mmol) at 0 °C, and then the reaction was allowed to react for 2 h at room temperature. TLC detection indicated disappearance of starting materials. The reaction solution was cooled to room temperature, and water was added to quench the reaction. The resultant solution was extracted with ethyl acetate. The organic phase was dried, concentrated, and purified by prep-TLC, to obtain 60 mg of product, with a yield of 40%. LC/MS (ESI+) calcd for C₁₉H₂₁N₃O₄ ( [M+H]⁺ ) *m*/*z:* 356.2; found 356.1.

### Step 2: Synthesis of 3-(5-(4-(7-(4-(cis-6-hydroxyl-2-phenyl-1,2,3,4-tetrahydronaphthalen-1-yl)phenyl)-2,7-diazaspiro[3.5]nonane-2-yl)methyl)piperidin-1-yl)-1-oxoisoinodolin-2-yl)pipe ridin-2,6-dione

*cis*-5-(4-(2,7-diazaspiro[3.5]nonan-7-yl)phenyl)-6-phenyl-56,7,8-tetrahydronaphthalen-2-o l trifluoroacetate (30 mg, 0.07 mmol) was dissolved in DCM/MeOH (5 mL/1 mL ), to which was added DIPEA (27 mg, 0.2 mmol), and then the reaction was stirred for 5mins, followed by addition of 1-(2-(2,6-dioxopiperidin-3-yl)-1-oxoisoindol-5-yl)piperidine-4-carbaldehyde (25 mg, 0.07 mmol) and AcOH (27 mg, 0.2 mmol). The reaction was allowed to react for 30 min at room temperature, and NaBH(OAc)₃ (45 mg, 0.2 mmol) was added. The reaction was allowed to react overnight. The reaction was monitored by TLC until the raw materials disappeared, and then the resultant solution was extracted with dichloromethane. The organic phase was dried and concentrated, and the residue was subjected to column chromatography to obtain 13 mg of product, which was determined to be the target compound by LC-MS, with a yield of 25%. ¹H NMR (400 MHz, DMSO-*d*₆) *δ* 10.95 (s, 1H), 9.11 (s, 1H), 7.49 (d, *J =* 8.5 Hz, 1H), 7.18 - 7.07 (m, 3H), 7.02 (d, *J =* 8.6 Hz, 2H), 6.86 - 6.78 (m, 2H), 6.73 - 6.58 (m, 3H), 6.53 (d, *J =* 8.6 Hz, 2H), 6.47 (dd, *J =* 8.3, 2.6 Hz, 1H), 6.19 (d, *J =* 8.4 Hz, 1H), 5.04 (dd, *J =* 13.3, 5.1 Hz, 1H), 4.30 (d, *J* = 16.9 Hz, 1H), 4.18 (d, *J =* 16.8 Hz, 1H), 4.12 (d, *J =* 5.0 Hz, 1H), 3.84 (d, *J* = 12.7 Hz, 2H), 3.27 (s, 2H), 2.92 (s, 8H), 2.77 (t, *J =* 12.2 Hz, 2H), 2.60 (s, 2H), 2.33 (t, *J =* 1.9 Hz, 2H), 2.09 (dd, *J =* 12.2, 6.3 Hz, 1H), 1.97 (dd, *J =* 16.6, 9.9 Hz, 3H), 1.70 (s, 6H), 1.50 - 1.41 (m, 2H), 1.22 (s, 2H). LC/MS (ESI+) calcd for C₄₈H₅₃N₅O₄ ( [M+H]⁺ ) *m*/*z:* 764.4; found 764.3.

### Example 12. 3-(5-(4-(4-(cis-6-hydroxyl-2-phenyl-1,2,3,4-tetrahydronaphthalen-1-yl)phenyl)-2,7-diazaspiro[3.5]nonane-2-yl)piperidin-1-yl)-1-oxoisoinodolin-2-yl)pyridine -2,6-dione

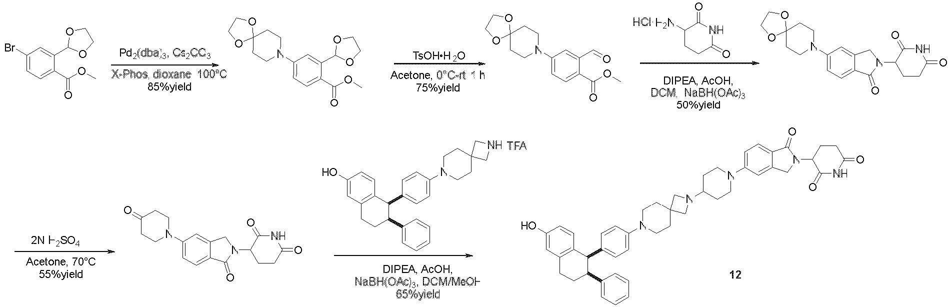

### Step 1: Synthesis of methyl 2-(1,3-dioxolane-2-yl)-4-(1,4-dioxa-8-azaspiro[4.5]decane-8-yl)benzoate

To a reaction flask, were added methyl 4-bromo-2-(1,3-dioxolane-2-yl)benzoate (1.0 g, 3.5 mmol), 1,4-oxa-8-azaspiro[4.5]decane (553 mg, 3.86 mmol), Pd₂(dba)₃(320 mg, 0.35 mmol), X-Phos (333 mg, 0.70 mmol), cesium carbonate (2.29 g, 7.02 mmol), and dioxane (30 mL), and then the system was purged with nitrogen. The reaction was allowed to react at 100 °C overnight. The raw material disappeared by TLC detection. The reaction solution was cooled to room temperature, and then filtered via diatomaceous earth. The filtrate was concentrated, followed by column chromatography, to obtain 1.04 g of product, with a yield of 85%. LC/MS (ESI+) calcd for C₁₈H₂₃NO₆ ( [M+H]⁺ ) *m*/*z:* 350.2; found 350.1.

### Step 2: Synthesis of methyl 2-formyl-4-(1,4-dioxa-8-azaspiro[4.5]decane-8-yl)benzoate

Methyl 2-(1,3-dioxolane-2-yl)-4-(1,4-dioxa-8-azaspiro[4.5]decane-8-yl)benzoate (1 g, 2.87 mmol) was dissolved in acetone (15 mL), to which was added p-toluenesulfonic acid monohydrate (654 mg, 3.44 mmol) in an ice bath, and then the reaction was allowed to react for 1.5 h at room temperature. TLC detection indicated disappearance of starting materials. Water was added to quench the reaction. The resultant solution was extracted with ethyl acetate. The organic phase was concentrated, and then subjected to column chromatography, to obtain 656 mg of product, with a yield of 75%. LC/MS (ESI+) calcd for C₁₆H₁₉NO₅ ( [M+H]⁺ ) *m*/*z*: 306.1; found 306.0.

### Step 3: Synthesis of 3-(1-oxo-5-(1,4-dioxa-8-azaspiro[4.5]decane-8-yl)isoinodolin-2-yl) piperidin-2,6-dione

To a one-necked flask, was added 3-aminopiperidin-2,6-dione (388 mg, 2.36 mmol), to which was added DCM (20 mL), followed by adding DIPEA (507mg, 3.92 mmol) dropwise. The reaction solution was stirred for 10 min, and then methyl 2-formyl-4-(1,4-dioxa-8-azaspiro[4.5]decane-8-yl)benzoate (600 mg, 1.96 mmol) and AcOH (1.18 g, 19.6 mmol) were added. The reaction was allowed to react for 6 h at 35 °C, and NaBH(OAc)₃ (2.49 g, 11.76 mmol) was added. The reaction was allowed to react overnight. The reaction was monitored by TLC until the raw materials disappeared, and then the resultant solution was extracted with dichloromethane. The organic phase was washed with saturated NaCl solution, dried and concentrated, and the residue was subjected to column chromatography to obtain 377 mg of product, with a yield of 50%. LC/MS (ESI+) calcd for C₂₀H₂₃N₃O₅ ( [M+H]⁺ ) *m*/*z:* 386.2; found 386.1.

### Step 4: Synthesis of 3-(1-oxo-5-(4-oxopiperidin-1-yl)isoinodolin-2-yl)piperidin-2,6-dione

3-(1-oxo-5-(1,4-dioxa-8-azaspiro[4.5]decane-8-yl)isoindol-2-yl)piperidin-2,6-dione (150 mg, 0.39 mmol) was dissolved in THF (10 mL), to which was added 2M H₂SO₄ (3 mL), and then the reaction was warmed to 50 °C and reacted for 20 min. TLC detection indicated disappearance of starting materials. The reaction solution was adjusted to alkaline with saturated NaHCO₃ solution in an ice bath, and then extracted with ethyl acetate, followed by drying and concentrating. The residue was separated and purified to obtain 73 mg of product, with a yield of 55%. LC/MS (ESI+) calcd for C₁₈H₁₉N₃O₄ ( [M+H]⁺ ) *m*/*z:* 342.1; found 342.0.

### Step 5: Synthesis of 3-(5-(4-(4-(cis-6-hydroxyl-2-phenyl-1,2,3,4-tetrahydronaphthalen-1-yl)phenyl)-2,7-diazaspiro[3.5]nonane-2-yl)piperidin-1-yl)-1-oxoisoinodolin-2-yl)pyridine-2,6 -dione

*cis*-5-(4-(2,7-diazaspiro[3.5]nonan-7-yl)phenyl)-6-phenyl-56,7,8-tetrahydronaphthalen-2-o l trifluoroacetate (30 mg, 0.07 mmol) was dissolved in dichloromethane/methanol (2 mL, 10:1), to which was added DIPEA (27 mg, 0.2 mmol), and then the reaction was stirred for 5min, followed by addition of 3-(1-oxo-5-(4-oxopiperidin-1-yl)isoinodolin-2-yl)piperidin-2,6-dione (24 mg, 0.07 mmol) and AcOH (27 mg, 0.2 mmol). The reaction was allowed to react for 30 min at room temperature, and NaBH(OAc)₃ (45 mg, 0.2 mmol) was added. The reaction was allowed to react overnight. The reaction was monitored by TLC until the raw materials disappeared, and then the resultant solution was extracted with dichloromethane. The organic phase was dried and concentrated, and the residue was subjected to column chromatography to obtain 34 mg of product, which was determined to be the target compound by LC-MS, with a yield of 65%. ¹H NMR (400 MHz, DMSO-*d*₆) *δ* 10.95 (s, 1H), 9.11 (s, 1H), 7.49 (d, *J =* 8.3 Hz, 1H), 7.18 - 7.07 (m, 3H), 7.03 (d, *J =* 9.6 Hz, 2H), 6.89 - 6.78 (m, 2H), 6.67 - 6.59 (m, 2H), 6.57 - 6.42 (m, 3H), 6.19 (d, *J =* 8.3 Hz, 2H), 5.04 (dd, *J =* 13.2, 5.1 Hz, 1H), 4.33 - 4.10 (m, 3H), 3.74 (s, 2H), 3.26 (s, 1H), 2.92 (s, 10H), 2.75 - 2.55 (m, 2H), 2.42 - 2.31 (m, 2H), 2.19 - 1.86 (m, 5H), 1.69 (s, 6H), 1.22 (s, 2H). LC/MS (ESI+) calcd for C₄₇H₅₁N₅O₄ ( [M+H]⁺ ) *m*/*z*: 750.4; found 750.3.

### Example 13. 3-(5-(4-(7-(4-(cis-7-hydroxyl-3-phenyltryptophane-4-yl)phenyl)-2,7-diazaspiro[3.5]nonane-2-yl)piperidin-1-yl)-1-oxoisoinodolin-2-yl)pyridine-2,6-dione

The target compound was prepared using a method similar to that in Example 5. ¹H NMR (400 MHz, DMSO-*d*₆) *δ* 10.95 (s, 1H), 9.31 (s, 1H), 7.49 (d, *J* = 8.4 Hz, 1H), 7.14 (p, *J* = 3.6 Hz, 3H), 7.03 (d, *J =* 8.4 Hz, 2H), 6.75 (dd, *J =* 6.6, 3.0 Hz, 2H), 6.65 (d, *J =* 8.3 Hz, 1H), 6.60 (d, *J* = 8.4 Hz, 2H), 6.37 (d, *J =* 8.4 Hz, 2H), 6.32 - 6.24 (m, 2H), 5.04 (dd, *J =* 13.3, 5.1 Hz, 1H), 4.38 - 4.26 (m, 2H), 4.23 - 4.12 (m, 3H), 3.76 - 3.69 (m, 2H), 3.53 - 3.48 (m, 2H), 2.98 - 2.85 (m, 10H), 2.58 (ddd, *J* = 17.1, 4.2, 2.3 Hz, 1H), 2.37 (dd, *J =* 13.2, 8.6 Hz, 1H), 2.24 (dd, *J* = 8.8, 4.5 Hz, 1H), 1.98 - 1.93 (m, 1H), 1.70 (q, *J* = 5.9, 5.1 Hz, 6H), 1.22 (s, 2H). LC/MS (ESI+) calcd for C₄₆H₄₉N₅O₅ ( [M+H]⁺ ) *m*/*z:* 752.4; found 752.3.

### Example 14. 3-(5-(4-(7-(4-((3S, 4R)-3-(2-fluorophenyl)-7-hydroxychroman-4-yl) phenyl)-2,7-diazaspiro[3.5]nonane-2-yl)piperidin-1-yl)-1-oxoisoinodolin-2-yl)pyridine-2,6-dione

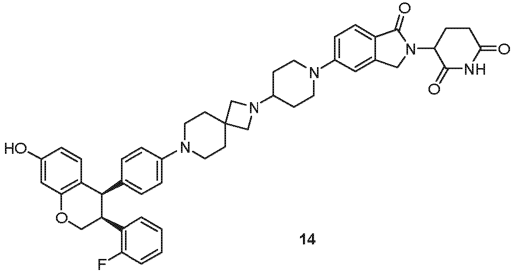

The target compound was prepared using a method similar to that in Example 5. ¹H NMR (400 MHz, DMSO-*d*₆) *δ* 10.95 (s, 1H), 9.36 (s, 1H), 7.49 (d, *J =* 8.4 Hz, 1H), 7.22 (tdd, *J =* 7.4, 5.4, 1.7 Hz, 1H), 7.13 (ddd, *J* = 9.9*,* 8.1, 1.3 Hz, 1H), 7.03 (d, *J =* 8.3 Hz, 2H), 6.88 (td, *J* = 7.5, 1.3 Hz, 1H), 6.66 (d, *J =* 8.3 Hz, 1H), 6.60 (d, *J =* 8.5 Hz, 2H), 6.46 - 6.34 (m, 3H), 6.34 - 6.24 (m, 2H), 5.04 (dd, *J =* 13.3, 5.1 Hz, 1H), 4.40 - 4.27 (m, 2H), 4.24 - 4.13 (m, 3H), 3.71 (td, *J* = 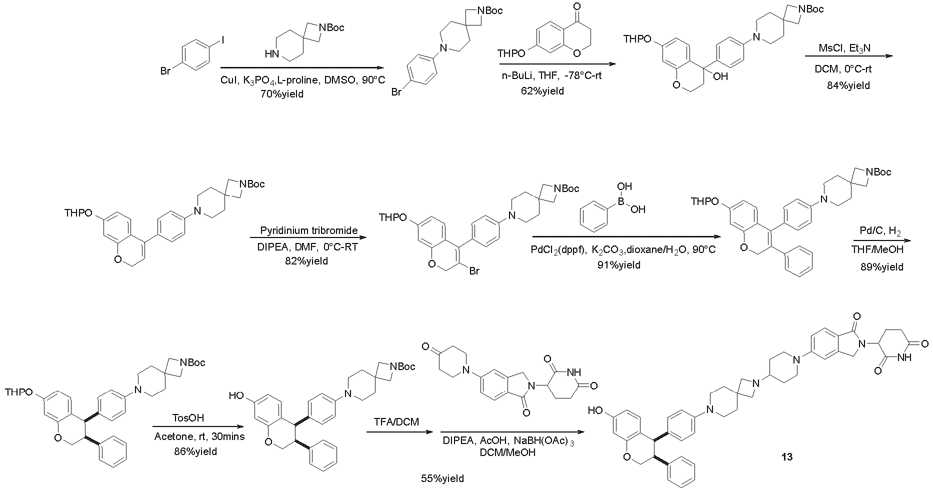 8.7, 8.2, 5.0 Hz, 4H), 3.00 - 2.86 (m, 10H), 2.62 - 2.54 (m, 1H), 2.37 (td, *J* = 13.3, 4.6 Hz, 1H), 2.25 (d, *J* = 8.6 Hz, 1H), 2.01 - 1.93 (m, 1H), 1.69 (q, *J* = 7.3, 4.9 Hz, 6H), 1.21 (d, *J* = 8.1 Hz, 2H). LC/MS (ESI+) calcd for C₄₆H₄₈FN₅O₅ ( [M+H]⁺ ) *m*/*z:* 770.4; found 770.2.

### Example 15. 3-(5-(4-(7-(4-((3R, 4S)-3-(2-fluorophenyl)-7-hydroxypyran-4-yl)phenyl)-2,7-diazaspiro[3.5]nonane-2-yl)piperidin-1-yl)-1-oxoisoinodolin-2-yl)pyridine-2,6-dione

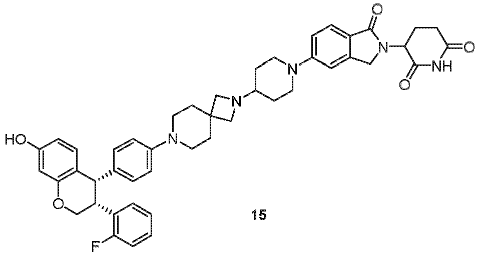

The target compound was prepared using a method similar to that in Example 5. ¹H NMR (400 MHz, DMSO-*d*₆) *δ* 10.95 (s, 1H), 9.33 (s, 1H), 7.49 (d, *J =* 8.4 Hz, 1H), 7.25 - 7.19 (m, 1H), 7.17 - 7.11 (m, 1H), 7.03 (d, *J* = 8.5 Hz, 2H), 6.90 - 6.86 (m, 1H), 6.66 (d, *J =* 8.3 Hz, 1H), 6.61 (d, *J =* 8.5 Hz, 2H), 6.41 (td, *J =* 8.5, 8.0, 6.1 Hz, 3H), 6.32 - 6.27 (m, 2H), 5.04 (dd, *J =* 13.3, 5.1 Hz, 1H), 4.38 - 4.28 (m, 2H), 4.23 - 4.16 (m, 3H), 3.90 - 3.57 (m, 4H), 2.98 - 2.89 (m, 10H), 2.61 - 2.55 (m, 1H), 2.37 (td, *J =* 13.0, 4.3 Hz, 1H), 2.30 - 2.23 (m, 1H), 1.99 - 1.93 (m, 1H), 1.69 (dd, *J* = 7.3, 3.9 Hz, 6H), 1.21 (d, *J* = 7.5 Hz, 2H). LC/MS (ESI+) calcd for C₄₆H₄₈FN₅O₅ ( [M+H]⁺ ) *m*/*z:* 770.4; found 770.2.

### Example 16. 3-(5-(7-((1-(4-((3S, 4R)-3-(2,3-difluorophenyl)-7-hydroxychroman-4-yl)-2-fluorophenyl)piperidin-4-yl)methyl)-2,7-diazaspiro[3.5]nonane-2-yl)-1-oxoisoinodolin-2-yl)piperidin-2,6-dione

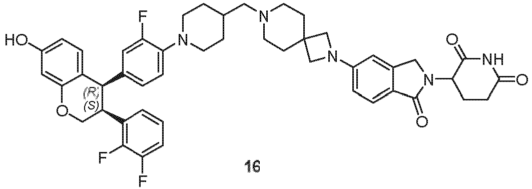

Using a method similar to that of Example 5, the racemic cis-3-(2,3-difluorophenyl)-4-(4-(4-(dimethoxymethyl)piperidin-1-yl)-3-fluorophenyl)chroman-7-ol was prepared, and then subjected to chiral SFC separation. The second fraction was collected to obtain 252 mg of product (3S,4R)-3-(2,3-difluorophenyl)-4-(4-(4-(dimethoxymethyl)piperidin-1-yl)-3-fluorophenyl)chroman-7-ol, with a yield of 47%. Chiral column: CHIRALPAK AY (30*250mm 10µm) (Daicel), mobile phase: A=CO₂, Co-Solvent B= EtOH (25%). The target compound was prepared using a method similar to that in Example 5. ¹H NMR (400 MHz, DMSO-*d*₆) *δ* 10.94 (s, 1H), 9.40 (s, 1H), 7.47 (d, *J =* 8.3 Hz, 1H), 7.27 (dtd, *J* = 9.7, 8.1, 1.5 Hz, 1H), 6.95 (dtt, *J* = 9.7, 5.4, 2.1 Hz, 1H), 6.75 (t, *J=* 8.8 Hz, 1H), 6.68 (d, *J* = 8.2 Hz, 1H), 6.53 - 6.44 (m, 2H), 6.40 - 6.22 (m, 5H), 5.03 (dd, *J =* 13.2, 5.1 Hz, 1H), 4.30 (ddt, *J* = 26.5, 12.7, 7.1 Hz, 4H), 4.16 (d, *J =* 16.9 Hz, 1H), 3.78 (ddd, *J* = 11.0, 5.5, 3.4 Hz, 1H), 3.61 (s, 4H), 3.21 (d, *J =* 10.9 Hz, 2H), 2.96 - 2.83 (m, 1H), 2.61 - 2.51 (m, 3H), 2.43 - 2.17 (m, 5H), 2.12 (d, *J =* 7.0 Hz, 2H), 1.97 - 1.90 (m, 1H), 1.79 - 1.68 (m, 6H), 1.59 (t, *J =* 10.4 Hz, 1H), 1.22 - 1.14 (m, 2H). LC/MS (ESI+) calcd for C₄₇H₄₈F₃N₅O₅ ( [M+H]⁺ ) *m*/*z:* 820.4; found 820.2.

### Example 17. 3-(5-(7-((1-(2,6-difluoro-4-(cis-7-hydroxyl-3-isobutylchroman-4-yl)phenyl)piperidin-4-yl)methyl)-2,7-diazaspiro[3.5]nonane-2-yl)-1-oxoisoinodolin-2-yl)pip eridin-2,6-dione

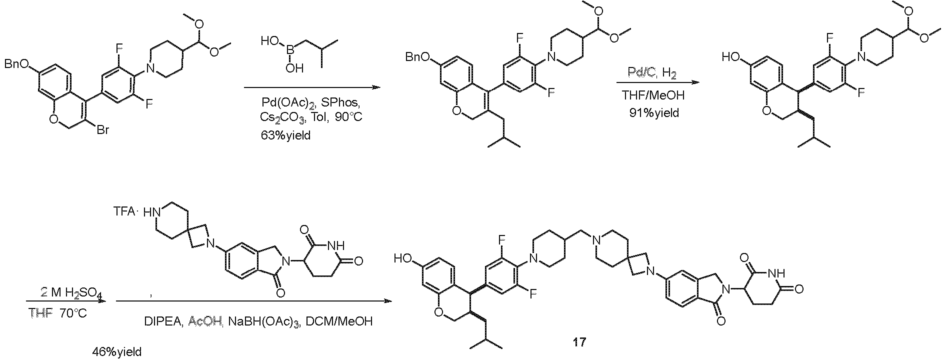

The target compound was prepared using a method similar to that in Example 5. ¹H NMR (400 MHz, DMSO-*d*₆) *δ* 10.95 (s, 1H), 9.31 (s, 1H), 7.47 (d, *J =* 8.3 Hz, 1H), 6.70 - 6.58 (m, 3H), 6.52 - 6.43 (m, 2H), 6.24 (d, *J* = 7.5 Hz, 2H), 5.03 (dd, *J* = 13.3, 5.1 Hz, 1H), 4.33 - 4.13 (m, 2H), 4.03 - 3.95 (m, 2H), 3.74 (t, *J=* 11.3 Hz, 1H), 3.62 (s, 4H), 3.12 (d, *J =* 11.2 Hz, 2H), 3.02 - 2.86 (m, 3H), 2.57 (dd, *J* = 17.8, 4.3 Hz, 1H), 2.42 - 2.26 (m, 4H), 2.14 (tt, *J* = 11.9, 7.8 Hz, 4H), 1.97 - 1.90 (m, 1H), 1.77 - 1.59 (m, 8H), 1.22 (d, *J* = 3.2 Hz, 2H), 1.17 (dd, *J* = 9.1*,* 5.2 Hz, 2H), 0.83 (dd, *J =* 6.5, 3.9 Hz, 6H). LC/MS (ESI+) calcd for C₄₅H₅₃F₂N₅O₅ ( [M+H]⁺ ) *m*/*z:* 782.4; found 782.2.

### Example 18. 3-(5-(7-((1-(4-(3-(2,6-difluorophenyl)-7-hydroxyl-2H-chromene-4-yl) phenyl)piperidin-4-yl)methyl)-2,7-diazaspiro[3.5]nonane-2-yl)-1-oxoisoinodolin-2-yl)piper idin-2,6-dione

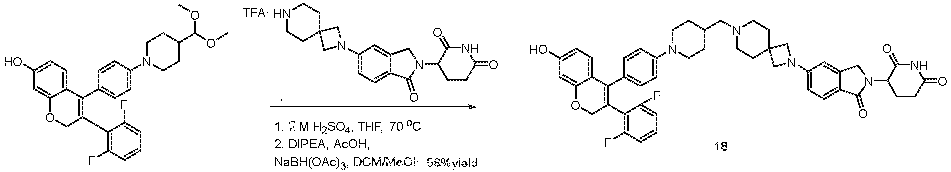

The target compound was prepared using a method similar to that in Example 5. ¹H NMR (400 MHz, DMSO-*d*₆) *δ* 10.95 (s, 1H), 9.82 (s, 1H), 7.48 (d, *J =* 8.2 Hz, 1H), 7.28 (tt, *J =* 8.3, 6.6 Hz, 1H), 6.97 (q, *J =* 8.0, 6.7 Hz, 2H), 6.88 - 6.72 (m, 4H), 6.64 (d, *J* = 8.3 Hz, 1H), 6.53 - 6.43 (m, 2H), 6.38 - 6.29 (m, 2H), 5.03 (dd, *J* = 13.3, 5.1 Hz, 1H), 4.79 (s, 2H), 4.33 - 4.14 (m, 2H), 3.65 (d, *J =* 11.1 Hz, 7H), 2.94 - 2.85 (m, 1H), 2.66 - 2.53 (m, 3H), 2.40 - 2.23 (m, 4H), 2.15 (d, *J* = 6.8 Hz, 2H), 1.97 - 1.92 (m, 1H), 1.85 - 1.54 (m, 7H), 1.21 (d, *J =* 4.4 Hz, 2H). LC/MS (ESI+) calcd for C₄₇H₄₇F₂N₅O₅ ( [M+H]⁺ ) *m*/*z:* 800.3; found 799.9.

### Example 19. 3-(5-(4-(7-(4-(cis-2-(2-fluorophenyl)-6-hydroxyl-1,2,3,4-tetrahydronaphthalen-1-yl)phenyl)-2,7-diazaspiro[3.5]nonane-2-yl)piperidin-1-yl)-1-oxois oinodolin-2-yl)piperidin-2,6-dione

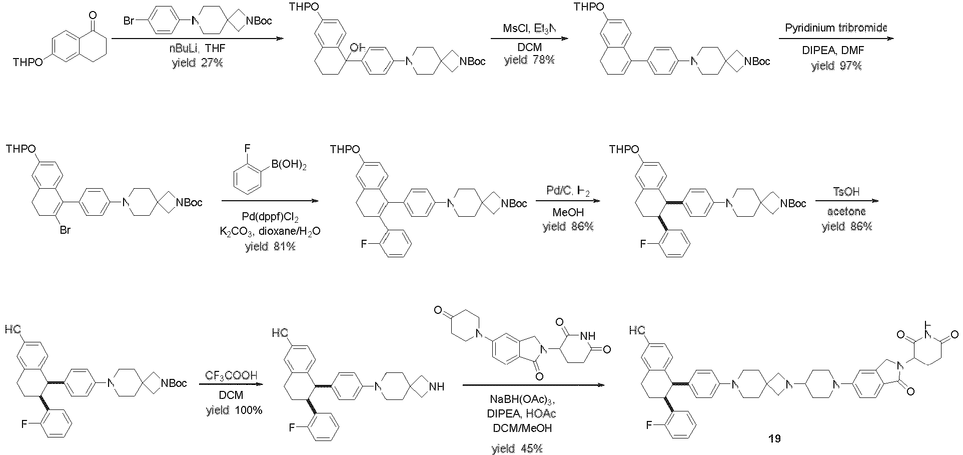

### Step 1: Synthesis of tert-butyl 7-(4-(1-hydroxyl-6-((tetrahydro-2H-pyran-2-yl)oxy)-1,2,3,4-tetrahydronaphthalen-1-yl)phenyl)-2,7-diazaspiro[3.5]nonane-2-carboxylate

Under nitrogen atmosphere, tert-butyl 7-(4-bromophenyl)-2,7-diazaspiro[3.5]nonane-2-carboxylate (2 g, 5.4 mmol) was dissolved in dry THF (40 mL), and then cooled to below -70 °C, followed by slowly adding n-BuLi (4.8 mL, 2.5 M, 12.3 mmol). During the whole addition process, the temperature of the reaction system was kept below -70 °C. After addition, the reaction was allowed to react for 1 h under the conditions of keeping the temperature. The solution of 6-((tetrahydro-*2H*-pyran-2-yl)oxy)-3,4-dihydronaphthalene-1(*2H*)-one (1.2 g, 4.9 mmol) in dry THF (40 mL) was slowly added to the system, and the temperature of the reaction system was kept below -70 °C during the whole addition procedure. The mixture was reacted for 1h under the conditions of keeping the temperature. The reaction system was gradually warmed to around -40 °C, and TLC monitoring indicated the complete reaction of the raw material 6-((tetrahydro-*2H*-pyran-2-yl)oxy)-3,4-dihydronaphthalene-1(*2H*)-one. Saturated ammonium chloride solution was added to quench the reaction, and the resultant solution was extracted with ethyl acetate. The organic phase was washed with saturated NaCl solution, dried and concentrated, followed by column chromatography, to obtain 713 mg of product, with a yield of 27%. LC/MS (ESI+) calcd for C₃₃H₄₄N₂O₅ ( [M+H]⁺ ) *m*/*z:* 549.3; found 549.2.

### Step 2: Synthesis of tert-butyl 7-(4-(6-((tetrahydro-2H-pyran-2-yl)oxy)-3,4-dihydronaphthalene-1-yl)phenyl)-2,7-diazaspiro[3.5]nonane-2-carboxylate

tert-butyl 7-(4-(1-hydroxyl-6-((tetrahydro-2*H*-pyran-2-yl)oxy)-1,2,3,4-tetrahydronaphthalen-1-yl)phenyl)-2,7-diazaspiro[3.5]nonane-2-carboxylate (713 mg, 1.3 mmol) was dissolved in dichloromethane (15 mL), to which was added triethylamine (394 mg, 3.9 mmol), followed by addition of methanesulfonyl chloride (0.2 mL, 2.6 mmol) in an ice bath. After addition, the reaction was warmed to room temperature, and allowed to react for 2 h. TLC detection indicated disappearance of the starting material. Water was added to quench the reaction, and the resultant solution was extracted with dichloromethane. The organic phase was dried and concentrated, followed by column chromatography, to obtain 539 mg of product, with a yield of 78%.

### Step 3: Synthesis of tert-butyl 7-(4-(2-bromo-6-((tetrahydro-2H-pyran-2-yl)oxy)-3,4-tetrahydronaphthalen-1-yl)phenyl)-2,7-diazaspiro[3.5]nonane-2-carboxylate

tert-butyl 7-(4-(6-((tetrahydro-2*H*-pyran-2-yl)oxy)-3,4-dihydronaphthalene-1-yl)phenyl)-2,7- diazaspiro[3.5]nonane-2-carboxylate (539 mg, 1.0 mmol) was dissolved in DMF (10 mL), to which was added DIPEA (0.33 mL, 2 mmol), followed by adding pyridinium tribromide (384 mg, 1.2 mmol) in batches in an ice bath. After addition, the reaction was warmed to room temperature and stirred overnight. TLC detection indicated disappearance of the starting material. Water was added to quench the reaction, and the resultant solution was extracted with ethyl acetate. The organic phase was dried and concentrated, and the residue was separated by column chromatography eluted with petroleum ether/ethyl acetate (3:1), to obtain 598 mg of product, with a yield of 97%.

### Step 4: Synthesis of tert-butyl 7-(4-(2-(2-fluorophenyl)-6-((tetrahydro-2H-pyran-2-yl)oxy)-3,4-tetrahydronaphthalen-1-yl)phenyl)-2,7-diazaspiro[3.5]nonane-2-carboxylate

To a reaction flask, were added tert-butyl 7-(4-(2-bromo-6-((tetrahydro-2*H*-pyran-2-yl)oxy)-3,4-tetrahydronaphthalen-1-yl)phenyl)-2,7-diazaspiro[3.5]nonane-2-carboxylate (598 mg, 0.98 mmol), 2-flurorphenylboronic acid (165 mg, 1.26 mmol), Pd(dppf)Cl₂ (72 mg, 0.098 mmol), potassium carbonate (270 mg, 1.96 mmol) and the solvent dioxane/water (20 mL/5 mL), and then the system was purged with nitrogen. The reaction was allowed to react at 90 °C overnight. The reaction was complete by detection with TLC. The reaction solution was cooled to room temperature, filtered via diatomaceous earth, and concentrated, followed by column chromatography to obtain 498 mg of product, with a yield of 81%. LC/MS (ESI+) calcd for C₃₉H₄₅FN₂O₄ ( [M+H]⁺ ) *m*/*z:* 625.3; found 625.3.

### Step 5: Synthesis of tert-butyl 7-(4-(cis-2-(2-fluorophenyl)-6-((tetrahydro-2H-pyran-2-yl) oxy)-1,2,3,4-tetrahydronaphthalen-1-yl)phenyl)-2,7-diazaspiro[3.5]nonane-2-carboxylate

tert-butyl 7-(4-(2-(2-fluorophenyl)-6-((tetrahydro-2*H*-pyran-2-yl)oxy)-3,4-tetrahydronaphthalen-1-yl)phenyl)-2,7-diazaspiro[3.5]nonane-2-carboxylate (498 mg, 0.8 mmol) was dissolved in THF(15 mL), to which were added methanol (15 mL) and 10% Pd/C (50 mg, 10%wt), and then the system was purged with hydrogen. The reaction was allowed to react overnight at room temperature. The reaction was complete by detection with TLC. The reaction solution was filtered via diatomaceous earth to remove Pd/C, and then concentrated, followed by column chromatography to obtain 429 mg of product, with a yield of 86%.

### Step 6: Synthesis of tert-butyl 7-(4-(cis-2-(2-fluorophenyl)-6-hydroxyl-1,2,3,4-tetrahydronaphthalen-1-yl)phenyl)-2,7-diazaspiro[3.5]nonane-2-carboxylate

tert-butyl 7-(4-(*cis*-2-(2-fluorophenyl)-6-((tetrahydro-2*H*-pyran-2-yl)oxy)-1,2,3,4-tetrahydronaphthalen-1-yl)phenyl)-2,7-diazaspiro[3.5]nonane-2-carboxylate was dissolved in acetone (20 mL), to which was added·p-toluenesulfonic acid monohydrate (156 mg, 0.82 mmol). After addition, the reaction was stirred for 0.5 h - 1.5 h at room temperature. TLC detection indicated disappearance of the starting material. Water was added to quench the reaction, and the resultant solution was extracted with ethyl acetate. The organic phase was dried and concentrated, and the residue was separated over column chromatography eluted with petroleum ether/ethyl acetate (5: 1), to obtain 320 mg of product, with a yield of 86%. LC/MS (ESI+) calcd for C₃₄H₃₉FN₂O₃ ( [M+H]⁺ ) *m*/*z:* 543.3; found 543.2.

### Step 7: Synthesis of cis-5-(4-(2,7-diazaspiro[3.5]nonane-7-yl)phenyl)-6-(2-fluorophenyl)-5,6,7,8-tetrahydronaphthalen-2-ol

tert-butyl 7-(4-(*cis*-2-(2-fluorophenyl)-6-hydroxyl-1,2,3,4-tetrahydronaphthalen-1-yl) phenyl)-2,7-diazaspiro[3.5]nonane-2-carboxylate (55 mg, 0.1 mmol) was dissolved in dichloromethane (4 mL), to which was added trifluoroacetic acid (1 mL) in an ice bath, and the reaction was allowed to react for 1 h at room temperature. The reaction was monitored by TLC until the raw materials disappeared. The reaction solution was adjusted to be about pH 8 with saturated NaHCO₃ solution in an ice bath, and then extracted with dichloromethane/methanol (10:1). The organic phase was concentrated to obtain 50 mg of crude product. LC/MS (ESI+) calcd for C₂₉H₃₁FN₂O ( [M+H]⁺ ) *m*/*z:* 443.2; found 443.1. Yield: 100%.

### Step 8. Synthesis of 3-(5-(4-(7-(4-(cis-2-(2-fluorophenyl)-6-hydroxyl-1,2,3,4-tetrahydronaphthalen-1-yl)phenyl)-2,7-diazaspiro[3.5]nonane-2-yl)piperidin-1-yl)-1-oxoisoinod olin-2-yl)piperidin-2,6-dione

Crude *cis*-5-(4-(2,7-diazaspiro[3.5]nonane-7-yl)phenyl)-6-(2-fluorophenyl)-5,6,7,8-tetrahydronaphthalen-2-ol (50 mg, 0.1 mmol) and 3-(1-oxo-5-(4-oxopiperidin-1-yl)isoinodolin-2-yl)piperidin-2,6-dione (45 mg, 0.1 mmol) were dissolved in dichloromethane/methanol (5 mL, 4:1), to which was added glacial acetic acid (20 mg, 0.3 mmol), and then the reaction was stirred for 0.5 h at 35 °C. The reaction solution was cooled to room temperature, and sodium triacetoxyborohydride (70 mg, 0.3 mmol) was added. The reaction was allowed to react overnight. The reaction was monitored by TLC until the raw materials disappeared, and then quenched by adding saturated NaHCO₃ solution. The resultant solution was extracted with dichloromethane. The organic phase was dried and concentrated, and the residue was subjected to column chromatography to obtain 39 mg of product, with a total yield of 45% for two-steps. ¹H NMR (400 MHz, DMSO-*d*₆) *δ* 10.95 (s, 1H), 9.15 (s, 1H), 7.49 (d, *J =* 8.4 Hz, 1H), 7.21 - 7.11 (m, 2H), 7.03 (d, *J =* 8.5 Hz, 2H), 6.87 (td, *J =* 7.4, 1.5 Hz, 1H), 6.64 (d, *J =* 8.4 Hz, 1H), 6.60 (d, *J =* 2.5 Hz, 1H), 6.54 (d, *J =* 8.5 Hz, 2H), 6.48 (dd, *J =* 8.3, 2.5 Hz, 1H), 6.41 (td, *J =* 7.8, 1.7 Hz, 1H), 6.23 (d, *J =* 8.4 Hz, 2H), 5.04 (dd, *J =* 13.3, 5.1 Hz, 1H), 4.31 (d, *J =* 16.8 Hz, 1H), 4.23 - 4.13 (m, 2H), 3.72 (d, *J* = 12.5 Hz, 2H), 3.55 - 3.51 (m, 1H), 2.93 (d, *J* = 6.6 Hz, 12H), 2.63 - 2.54 (m, 1H), 2.43 - 2.30 (m, 1H), 2.23 (t, *J =* 6.9 Hz, 1H), 2.11 (tt, *J =* 14.6, 7.2 Hz, 1H), 2.04 - 1.91 (m, 2H), 1.68 (s, 7H), 1.23 (s, 2H). LC/MS (ESI+) calcd for C₄₇H₅₀FN₅O₄ ( [M+H]⁺ ) *m*/*z:* 768.4; found 768.3.

### Example 20. 3-(5-(4-(7-(4-(cis-3-(2-fluorophenyl)-7-hydroxychroman-4-yl)phenyl)-2,7-diazaspiro[3.5]nonane-2-yl)piperidin-1-yl)-1-oxoisoinodolin-2-yl)piperidin-2,6-dione

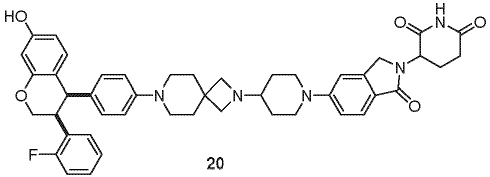

The target compound was prepared using a method similar to that in Example 19. ¹H NMR (400 MHz, DMSO-*d*₆) *δ* 10.95 (s, 1H), 9.33 (s, 1H), 7.49 (d, *J* = 8.5 Hz, 1H), 7.21 (t, *J* = 6.9 Hz, 1H), 7.14 (s, 1H), 7.03 (d, *J =* 9.7 Hz, 2H), 6.89 (t, *J =* 7.5 Hz, 1H), 6.67 (d, *J =* 8.3 Hz, 1H), 6.61 (d, *J =* 8.3 Hz, 2H), 6.40 (t, *J =* 7.4 Hz, 3H), 6.32 - 6.27 (m, 2H), 5.04 (dd, *J =* 13.2, 5.1 Hz, 1H), 4.38 - 4.28 (m, 2H), 4.20 (q, *J =* 12.0, 10.5 Hz, 3H), 3.72 (s, 4H), 2.95 (d, *J* = 6.7 Hz, 10H), 2.60 (s, 1H), 2.34 (d, *J* = 4.8 Hz, 2H), 1.98 (d, *J* = 10.1 Hz, 1H), 1.68 (d, *J =* 6.5 Hz, 6H), 1.24 - 1.22 (m, 2H). LC/MS (ESI+) calcd for C₄₆H₄₈FN₅O₅ ( [M+H]⁺ ) *m*/*z:* 770.3; found 770.2_{∘}

### Example 21. 3-(5-(4-(7-(4-((1S,2R)-2-(2-fluorophenyl)-6-hydroxyl-1,2,3,4-tetrahydronaphthalen-1-yl)phenyl)-2,7-diazaspiro[3.5]nonane-2-yl)piperidin-1-yl)-1-oxois oinodolin-2-yl)piperidin-2,6-dione

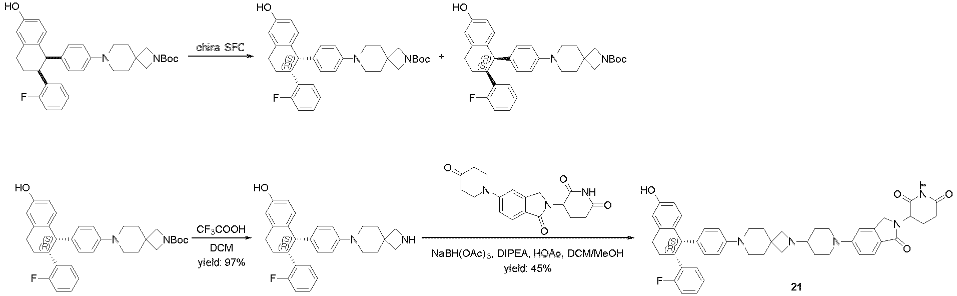

### Step 1: Synthesis of tert-butyl 7-(4-((1S,2R)-2-(2-fluorophenyl)-6-hydroxyl-1,2,3,4-tetrahydronaphthalen-1-yl)phenyl)-2,7-diazaspiro[3.5]nonane-2-carboxylate

Racemic tert-butyl 7-(4-(*cis*-2-(2-fluorophenyl)-6-hydroxyl-1,2,3,4-tetrahydronaphthalen-1-yl)phenyl)-2,7-diazaspiro[3.5]nonane-2-carboxylate (276 mg, 0.51 mmol) was separated by chiral SFC into two fractions, in which the first fraction was tert-butyl 7-(4-((1*S*,2*R*)-2-(2-fluorophenyl)-6-hydroxyl-1,2,3,4-tetrahydronaphthalen-1-yl)phenyl)-2,7-diazaspiro[3.5]nonane -2-carboxylate (129 mg, 0.24 mmol), 47%, while the second fraction was tert-butyl 7-(4-((1*R*,2*S*)-2-(2-fluorophenyl)-6-hydroxyl-1,2,3,4-tetrahydronaphthalen-1-yl) phenyl)-2,7-diazaspiro[3.5]nonane-2-carboxylate (137 mg, 0.25 mmol) with a yield of 50%. Column: CHIRALPAK AD (30*250 mm 5 µm) (Daicel), mobile phase: A = CO₂, co-solvent B=MeOH/ACN=1/1= 40%. For this scheme, the first fraction tert-butyl 7-(4-((1*S*,2*R*)-2-(2-fluorophenyl)-6-hydroxyl-1,2,3,4-tetrahydronaphthalen-1-yl)phenyl)-2,7-dia zaspiro[3.5]nonane-2-carboxylate was used in the next step.

### Step 2: Synthesis of (5S,6R)-5-(4-(2,7-diazaspiro[3.5]nonane-7-yl)phenyl)-6-(2-fluorophenyl)-5,6,7,8-tetrahydronaphthalen-2-ol

tert-butyl 7-(4-((1*S*,2*R*)-2-(2-fluorophenyl)-6-hydroxyl-1,2,3,4-tetrahydronaphthalen-1-yl) phenyl)-2,7-diazaspiro[3.5]nonane-2-carboxylate (129 mg, 0.29 mmol) was dissolved in DCM (6 mL), to which was added trifluoroacetic acid (1.5 mL) in an ice bath, and the reaction was allowed to react for 1 h at room temperature. The reaction was monitored by TLC until the raw materials disappeared. The reaction solution was adjusted to be about pH 8 with saturated NaHCO₃ solution in an ice bath, and then extracted with dichloromethane/methanol (10:1). The organic phase was concentrated to obtain 105 mg of crude product. LC/MS (ESI+) calcd for C₂₉H₃₁FN₂O ( [M+H]⁺ ) *m*/*z:* 443.2; found 443.1. Yield 97%.

### Step 3: Synthesis of 3-(5-(4-(7-(4-((1S,2R)-2-(2-fluorophenyl)-6-hydroxyl-1,2,3,4-tetrahydronaphthalen-1-yl)phenyl)-2,7-diazaspiro[3.5]nonane-2-yl)piperidin-1-yl)-1-oxoisoinod olin-2-yl)piperidin-2,6-dione

Crude (5*S*,6*R*)-5-(4-(2,7-diazaspiro[3.5]nonane-7-yl)phenyl)-6-(2-fluorophenyl)-5,6,7,8-tetrahydronaphthalen-2-ol (31 mg, 0.07 mmol) and 3-(1-oxo-5-(4-oxopiperidin-1-yl) isoinodolin-2-yl)piperidin-2,6-dione (29 mg, 0.08 mmol) were dissolved in dichloromethane/methanol (5 mL, 4:1), to which was added glacial acetic acid (20 mg, 0.3 mmol), and then the reaction was stirred for 0.5 h at 35 °C. The reaction solution was cooled to room temperature, and sodium triacetoxyborohydride (70 mg, 0.3 mmol) was added. The reaction was allowed to react overnight. The reaction was monitored by TLC until the raw materials disappeared, and then quenched by adding saturated NaHCO₃ solution. The resultant solution was extracted with dichloromethane. The organic phase was dried and concentrated, and the residue was subjected to column chromatography to obtain 26 mg of product, with a yield of 45%. ¹H NMR (400 MHz, DMSO-*d*₆) *δ* 10.95 (s, 1H), 9.14 (s, 1H), 7.49 (d, *J =* 8.5 Hz, 1H), 7.26 - 7.09 (m, 2H), 7.03 (d, *J =* 8.7 Hz, 2H), 6.86 (td, *J =* 7.3, 1.5 Hz, 1H), 6.64 (d, *J =* 8.4 Hz, 1H), 6.60 (d, *J =* 2.5 Hz, 1H), 6.54 (d, *J =* 8.5 Hz, 2H), 6.48 (dd, *J =* 8.3, 2.6 Hz, 1H), 6.44 - 6.38 (m, 1H), 6.23 (d, *J* = 8.3 Hz, 2H), 5.04 (dd, *J =* 13.3, 5.1 Hz, 1H), 4.31 (d, *J* = 16.9 Hz, 1H), 4.24 - 4.13 (m, 2H), 3.73 (d, *J =* 12.3 Hz, 2H), 3.53 (dd, *J =* 12.8, 4.4 Hz, 1H), 2.92 (d, *J* = 10.8 Hz, 12H), 2.63 - 2.54 (m, 1H), 2.43 - 2.32 (m, 1H), 2.32 - 2.21 (m, 1H), 2.20 - 2.05 (m, 1H), 1.97 (ddd, *J* = 20.9, 15.4, 8.5 Hz, 2H), 1.71 - 1.66 (m, 7H), 1.23 (s, 2H). LC/MS (ESI+) calcd for C₄₇H₅₀FN₅O₄ ( [M+H]⁺ ) *m*/*z:* 768.3; found 768.3.

### Example 22. 3-(5-(4-(7-(4-((1R,2S)-2-(2-fluorophenyl)-6-hydroxyl-1,2,3,4-tetrahydronaphthalen-1-yl)phenyl)-2,7-diazaspiro[3.5]nonane-2-yl)piperidin-1-yl)-1-oxois oinodolin-2-yl)piperidin-2,6-dione

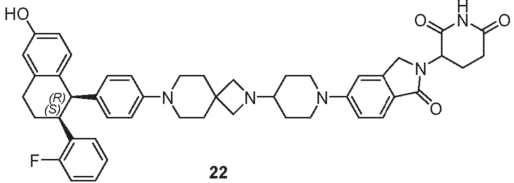

The second fraction tert-butyl 7-(4-((1*R*,2*S*)-2-(2-fluorophenyl)-6-hydroxyl-1,2,3,4-tetrahydronaphthalen-1-yl)phenyl)-2,7-diazaspiro[3.5]nonane-2-carboxylate was used in the reaction, and the target compound was prepared using a method similar to that in Example 21. ¹H NMR (400 MHz, DMSO-*d*₆) *δ* 10.95 (s, 1H), 9.14 (s, 1H), 7.49 (d, *J =* 8.5 Hz, 1H), 7.24 - 7.10 (m, 2H), 7.03 (d, *J* = 8.7 Hz, 2H), 6.86 (td, *J=* 7.4, 1.5 Hz, 1H), 6.64 (d, *J=* 8.4 Hz, 1H), 6.60 (d, *J* = 2.6 Hz, 1H), 6.54 (d, *J =* 8.5 Hz, 2H), 6.48 (dd, *J =* 8.3, 2.6 Hz, 1H), 6.41 (td, *J* = 7.7, 1.7 Hz, 1H), 6.23 (d, *J =* 8.3 Hz, 2H), 5.04 (dd, *J =* 13.3, 5.1 Hz, 1H), 4.31 (d, *J =* 17.0 Hz, 1H), 4.23 - 4.12 (m, 2H), 3.72 (t, *J* = 8.5 Hz, 2H), 3.52 (dt, *J =* 13.1, 3.7 Hz, 1H), 2.93 (t, *J* = 9.7 Hz, 12H), 2.70 - 2.54 (m, 1H), 2.43 - 2.33 (m, 1H), 2.32 - 2.20 (m, 1H), 2.19 - 2.05 (m, 1H), 2.03 - 1.89 (m, 2H), 1.68 (s, 7H), 1.23 (d, *J* = 3.5 Hz, 2H). LC/MS (ESI+) calcd for C₄₇H₅₀FN₅O₄ ( [M+H]⁺ ) *m*/*z:* 768.3; found 768.3.

### Example 23. 3-(5-(4-(7-(4-(cis-3-(2-fluorophenyl)-7-hydroxychroman-4-yl)phenyl)-2,7-diazaspiro[3.5]nonane-2-yl)piperidin-1-yl)-1-oxoisoinodolin-2-yl)piperidin-2,6-dione

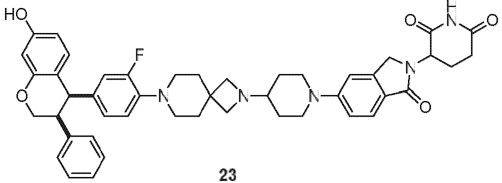

The target compound was prepared using a method similar to that in Example 19. ¹H NMR (400 MHz, DMSO-*d*₆) *δ* 10.96 (s, 1H), 9.37 (s, 1H), 7.49 (d, *J =* 8.4 Hz, 1H), 7.20 - 7.15 (m, 3H), 7.03 (d, *J =* 8.6 Hz, 2H), 6.78 (dd, *J =* 6.6, 3.1 Hz, 2H), 6.69 (dd, *J =* 13.0, 8.6 Hz, 2H), 6.34 - 6.26 (m, 3H), 6.14 (dd, *J =* 14.2, 2.0 Hz, 1H), 5.04 (dd, *J =* 13.3, 5.1 Hz, 1H), 4.37 - 4.27 (m, 2H), 4.27 - 4.16 (m, 3H), 3.72 (dd, *J =* 10.9, 6.8 Hz, 2H), 3.55 (dt, *J =* 11.4, 4.6 Hz, 2H), 2.93 (d, *J =* 15.5 Hz, 6H), 2.79 (s, 4H), 2.64 - 2.55 (m, 1H), 2.43 - 2.32 (m, 1H), 2.26 (s, 1H), 2.00 - 1.91 (m, 1H), 1.74 (t, *J =* 5.6 Hz, 6H), 1.23 (d, *J =* 3.5 Hz, 2H). LC/MS (ESI+) calcd for C₄₆H₄₈FN₅O₅ ( [M+H]⁺ ) *m*/*z:* 770.3; found 770.2.

### Example 24. 3-(5-(7-((1-(4-((3R,4S)-3-(2,3-difluorophenyl)-7-hydroxychroman-4-yl)-2-fluorophenyl)piperidin-4-yl)methyl)-2,7-diazaspiro[3.5]nonane-2-yl)-1-oxoisoinodo lin-2-yl)piperidin-2,6-dione

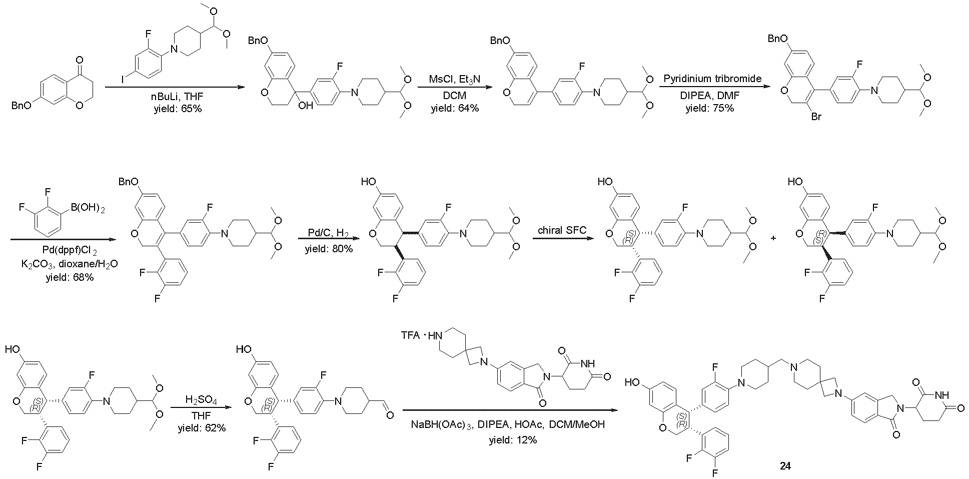

### Step 1: Synthesis of 7-(benzyloxy)-4-(4-(4-(dimethoxymethyl)piperidin-1-yl)-3-fluorophenyl) chroman-4-ol

Under nitrogen atmosphere, 4-(dimethoxymethyl)-1-(2-fluoro-4-iodophenyl)piperidine (15 g, 39.6 mmol) was dissolved in dry THF (90 mL), and then cooled to below -70 °C, followed by slowly adding n-BuLi (18.7 mL, 2.5 M, 46.77 mmol). During the whole addition process, the temperature of the reaction system was kept below -70 °C. After addition, the reaction was allowed to react for 1 h under the conditions of keeping the temperature. The solution of 7-(benzyloxy)chroman-4-one (9.14 g, 35.98 mmol) in dry THF (50 mL) was slowly added to the system, and the temperature of the reaction system was kept below -70 °C during the whole addition procedure. The mixture was reacted for 1h under the conditions of keeping the temperature. The reaction system was gradually warmed to around -40 °C, and TLC monitoring indicated the complete reaction of the raw material 7-(benzyloxy)chroman-4-one. Saturated ammonium chloride solution was added to quench the reaction, and the resultant solution was extracted with ethyl acetate. The organic phase was washed with saturated NaCl solution, dried and concentrated, followed by column chromatography, to obtain 13 g of product. LC/MS (ESI+) calcd for C₃₀H₃₄FNO₅ ( [M+H]⁺ ) *m*/*z:* 508.2; found 508.2. Yield 65%.

### Step 2: Synthesis of 1-(4-(7-(benzyloxy)-2H-chromene-4-yl)-2-fluorophenyl)-4-(dimethoxymethyl)piperidine

7-(benzyloxy)-4-(4-(4-(dimethoxymethyl)piperidin-1-yl)-3-fluorophenyl)chroman-4-ol (13 g, 25.56 mmol) was dissolved in dichloromethane (100 mL), to which was added triethylamine (7.77 g, 76.9 mmol), followed by adding methanesulfonyl chloride (5.85 g, 51.3 mmol) in an ice bath. After addition, the reaction was warmed to room temperature, and allowed to react for 2 h. TLC detection indicated disappearance of the starting material. Water was added to quench the reaction, and the resultant solution was extracted with dichloromethane. The organic phase was dried and concentrated, followed by column chromatography, to obtain 8 g of product. LC/MS (ESI+) calcd for C₃₀H₃₂FNO₄ ( [M+H]⁺ ) *m*/*z:* 490.2; found 490.2. Yield 64%.

### Step 3: Synthesis of 1-(4-(7-(benzyloxy)-3-bromo-2H-chromene-4-yl)-2-fluorophenyl)-4-(dimethoxymethyl)piperidine

1-(4-(7-(benzyloxy)-2*H*-chromene-4-yl)-2-fluorophenyl)-4-(dimethoxymethyl)piperidine (8 g, 16.35 mmol) was dissolved in DMF (40 mL), to which was added DIPEA (6.33 g, 49.08 mmol), and then pyridinium tribromide (5.76 g, 17.99 mmol) was added in portions in an ice bath. After addition, the reaction was warmed to room temperature and stirred overnight. TLC detection indicated disappearance of the starting material. Water was added to quench the reaction, and the resultant solution was extracted with ethyl acetate. The organic phase was dried and concentrated, and the residue was triturated in petroleum ether/ethyl acetate (63:1), to obtain 7 g of product, with a yield of 75%.

### Step 4: Synthesis of 1-(4-(7-(benzyloxy)-3-(2,3-difluorophenyl)-2H-chromene-4-yl)-2-fluorophenyl)-4-(dimethoxymethyl)piperidine

To a reaction flask, were added 1-(4-(7-(benzyloxy)-3-bromo-2*H*-chromene-4-yl)-2-fluorophenyl)-4-(dimethoxymethyl)piperidine (1.0 g, 1.76 mmol), 2,3-diflurorphenylboronic acid (335 mg, 2.12 mmol), Pd(dppf)Cl₂ (130 mg, 0.18 mmol), potassium carbonate (488 mg, 3.54 mmol) and dioxane (30 mL), and then the system was purged with nitrogen. The reaction was allowed to react at 80 °C overnight. The reaction was complete by detection with TLC. The reaction solution was cooled to room temperature, filtered via diatomaceous earth, and concentrated, followed by column chromatography to obtain 715 mg of product. LC/MS (ESI+) calcd for C₃₆H₃₄F₃NO₄ ( [M+H]⁺ ) *m*/*z:* 602.2; found 602.2. Yield 68%.

### Step 5: Synthesis of cis-3-(2,3-difluorophenyl)-4-(4-(4-(dimethoxymethyl)piperidin-1-yl)-3-fluorophenyl)chroman-7-ol

1-(4-(7-(benzyloxy)-3-(2,3-difluorophenyl)-2*H*-chromene-4-yl)-2-fluorophenyl)-4-(dimeth oxymethyl)piperidine (700 mg, 1.2 mmol) was dissolved in THF (2 mL), to which were successively added methanol (20 mL) and 10% Pd/C (70 mg, 10%), and then the system was purged with hydrogen. The reaction was allowed to react overnight at room temperature. The reaction was complete by detection with TLC. The reaction solution was filtered via diatomaceous earth to remove Pd/C, and then concentrated, followed by column chromatography to obtain 480 mg of product. LC/MS (ESI+) calcd for C₂₉H₃₀F₃NO₄( [M+H]⁺ ) *m*/*z:* 514.2; found 514.1. Yield 80%.

### Step 6: Synthesis of (3R,4S)-3-(2,3-difluorophenyl)-4-(4-(4-(dimethoxymethyl)piperidin-1-yl)-3-fluorophenyl)chroman-7-ol

Racemic *cis*-3-(2,3-difluorophenyl)-4-(4-(4-(dimethoxymethyl)piperidin-1-yl)-3-fluorophenyl)chroman-7-ol (491 mg, 0.96 mmol) was separated into two fractions by chiral SFC, in which the first fraction was (3*R*,4*S*)-3-(2,3-difluorophenyl)-4-(4-(4-(dimethoxymethyl) piperidin-1-yl)-3-fluorophenyl)chroman-7-ol (239 mg, 0.47 mmol), with a yield of 49%, while the second fraction was (*3S*,*4R*)-3-(2,3-difluorophenyl)-4-(4-(4-(dimethoxymethyl)piperidin-1-yl)-3-fluorophenyl)chroman-7-ol (252 mg, 0.49 mmol), with a yield of 51%. Column: CHIRALPAK AY (30*250mm 10µm) (Daicel), mobile phase: A=CO₂, co-solvent B=EtOH= 25%. In this scheme, the first fraction (3*R*, *4S*)-3-(2,3-difluorophenyl)-4-(4-(4-(dimethoxymethyl)piperidin-1-yl)-3-fluorophenyl)chroman-7-ol was used in the next step.

### Step 7: Synthesis of 1-(4-((3R, 4S)-3-(2,3-difluorophenyl)-7-hydroxychroman-4-yl)-2-fluorophenyl)piperidine-4-carbaldehyde

(*3R*,*4S*)-3-(2,3-difluorophenyl)-4-(4-(4-(dimethoxymethyl)piperidin-1-yl)-3-fluorophenyl) chroman-7-ol (100 mg, 0.19 mmol) was dissolved in THF (9 mL), to which was added sulfuric acid (3 mL, 2 M), and the reaction was allowed to react for 0.5 h at 70 °C. The reaction was monitored by TLC until the raw materials disappeared. The reaction solution was cooled to room temperature, and adjusted to be about pH 8 with saturated NaHCO₃ solution. The resultant solution was extracted with ethyl acetate. The organic phase was washed with saturated NaCl solution, dried, and concentrated. The residue was subjected to column chromatography, to obtain 98 mg of crude product. LC/MS (ESI+) calcd for C₂₇H₂₄F₃NO₃ ( [M+H]⁺ ) *m*/*z:* 468.1; found 468.1. The crude product was directly used in the next step.

### Step 8. Synthesis of 3-(5-(7-((1-(4-((3R,4S)-3-(2,3-difluorophenyl)-7-hydroxychroman-4-yl)-2-fluorophenyl)piperidin-4-yl)methyl)-2,7-diazaspiro[3.5]nonane-2-yl)-1-oxoisoinodolin-2-yl)piperidin-2,6-dione

3-(1-oxo-5-(piperazine-1-yl)isoinodolin-2-yl)piperidin-2,6-dione hydrochloride (89 mg, 0.24 mmol) was dissolved in dichloromethane/methanol (12 mL, 5:1), to which was added DIPEA (84 mg, 0.65 mmol), and then the reaction was stirred at room temperature for 5 min, followed by addition of 1-(4-((3*R*, *4S*)-3-(2,3-difluorophenyl)-7-hydroxychroman-4-yl)-2-fluorophenyl)piperidine-4-carbaldehyde (98 mg, 0.22 mmol) and glacial acetic acid (40 mg, 0.66 mmol). The reaction was stirred for 0.5 h at 35 °C, and then cooled to room temperature. Sodium triacetoxyborohydride (140 mg, 0.66 mmol) was added. The reaction was allowed to react overnight. The reaction was monitored by TLC until the raw materials disappeared, and then quenched by adding saturated NaHCO₃ solution. The resultant solution was extracted with dichloromethane. The organic phase was dried and concentrated, and the residue was subjected to column chromatography to obtain 20 mg of product, with a yield of 12%. ¹H NMR (400 MHz, DMSO-*d*₆) *δ* 10.94 (s, 1H), 9.40 (s, 1H), 7.48 (d, *J =* 8.3 Hz, 1H), 7.27 (q, *J =* 8.8 Hz, 1H), 6.95 (q, *J* = 7.7 Hz, 1H), 6.75 (t, *J =* 8.8 Hz, 1H), 6.68 (d, *J* = 8.2 Hz, 1H), 6.51 (s, 1H), 6.47 (dd, *J =* 8.3, 2.0 Hz, 1H), 6.38 (td, *J* = 8.7*,* 8.2, 4.0 Hz, 2H), 6.35 - 6.29 (m, 2H), 6.26 (dd, *J* = 14.1, 2.0 Hz, 1H), 5.03 (dd, *J =* 13.3, 5.1 Hz, 1H), 4.37 (t, *J =* 10.8 Hz, 1H), 4.33 - 4.22 (m, 3H), 4.16 (d, *J =* 16.9 Hz, 1H), 3.83 - 3.74 (m, 1H), 3.62 (s, 4H), 3.21 (d, *J =* 11.0 Hz, 2H), 2.90 (ddd, *J* = 17.9, 13.4, 5.4 Hz, 1H), 2.69 - 2.52 (m, 3H), 2.46 - 2.19 (m, 5H), 2.13 (d, *J* = 7.1 Hz, 2H), 1.96 - 1.88 (m, 1H), 1.74 (t, *J=* 6.1 Hz, 6H), 1.59 (s, 1H), 1.23 (s, 2H). LC/MS (ESI+) calcd for C₄₇H₄₈F₃N₅O₅ ( [M+H]⁺ ) *m*/*z:* 820.3; found 820.3.

### Example 25: 2-(2,6-dioxopiperidin-3-yl)-5-(5-((1-(4-((1R,2S)-6-hydroxyl-2-phenyl-1,2,3,4-tetrahydronaphthalen-1-yl)phenyl)piperidin-4-yl)methyl)hexahydropyrrolo[3,4-c]p yrrole-2(1H)-yl)isoinodolin-1,3-dione

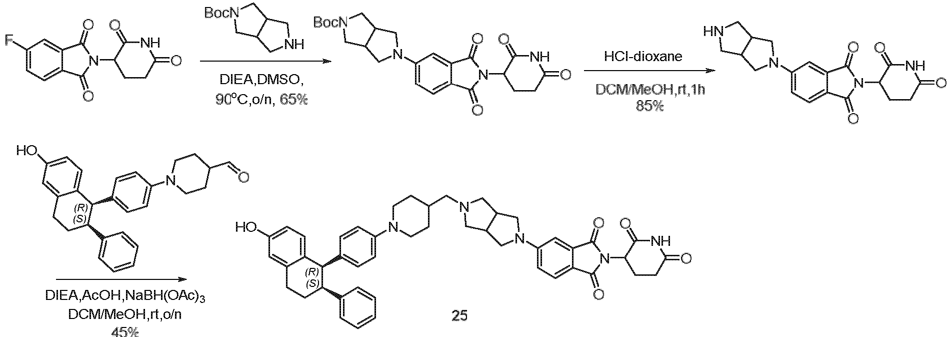

### Step 1: Synthesis of tert-butyl 5-(2-(2,6-dioxopiperidin-3-yl)-1,3-dioxoisoinodolin-5-yl) hexahydropyrrolo[3,4-c]pyrrole-2(1H)-carboxylate

Compound 2-(2,6-dioxopiperidin-3-yl)-5-fluoroisoinodolin-1,3-dione (552 mg, 2.00 mmol) and tert-butyl hexahydropyrrolo[3,4-*c*]pyrrole-2(1*H*)-carboxylate (530 mg, 2.50 mmol) were added into 8 mL of DMSO, to which was added DIPEA (516 mg, 4.00 mmol), and then the reaction was stirred overnight at 90 °C. The reaction was cooled to room temperature, and then added with a small amount of water. The resultant reaction was extracted three times with EA. The organic phases were combined, washed three times with saturated NaCl solution, dried over anhydrous Na₂SO₄, and rotatory evaporated to dry. The residue was purified by column chromatography to obtain the target compound (608 mg, 1.30 mmol), with a yield of 65%. LC/MS (ESI+) calcd for C₂₄H₂₈N₄O₆ (M + H⁺) *m*/*z,* 469.2; found, 469.2.

### Step 2: Synthesis of 2-(2,6-dioxopiperidin-3-yl)-5-(hexahydropyrrolo[3,4-c]pyrrole-2(1H)-yl)isoinodolin-1,3-dione

Compound tert-butyl 5-(2-(2,6-dioxopiperidin-3-yl)-1,3-dioxoisoinodolin-5-yl) hexahydropyrrolo[3,4-*c*]pyrrole-2(1*H*)-carboxylate (50 mg, 0.10 mmol) was added into 4 mL of DCM and 1 mL of MeOH, to which was then added 2 mL of 4 M HCl-dioxane solution, and the reaction was stirred for 1 h at room temperatue. The reaction solution was concentrated to obtain the target compound (32 mg, 0.08 mmol), with a yield of 85%. LC/MS (ESI+) calcd for C₁₉H₂₀N₄O₄ (M + H⁺) *m*/*z,* 369.1; found, 369.1.

### Step 3: Synthesis of 2-(2,6-dioxopiperidin-3-yl)-5-(5-((1-(4-((1R,2S)-6-hydroxyl-2-phenyl-1,2,3,4-tetrahydronaphthalen-1-yl)phenyl)piperidin-4-yl)methyl)hexahydropyrrolo[3, 4-c]pyrrole-2(1H)-yl)isoinodolin-1,3-dione

Compound 2-(2,6-dioxopiperidin-3-yl)-5-(hexahydropyrrolo[3,4-c]pyrrole-2(1*H*)-yl) isoinodolin-1,3-dione (30 mg, 0.08 mmol) and 1-(4-((1*R*,2*S*)-6-hydroxyl-2-phenyl-1,2,3,4-tetrahydronaphthalen-1-yl)phenyl)piperidine-4-carbaldehyde (32 mg, 0.08 mmol) were added into 6 mL of DCM and 2 mL of MeOH, to which were successively added DIEA (13 mg, 0.10 mmol) and AcOH (13 mg, 0.20 mmol), and the reaction was stirred for 1 h at room temperatue, followed by addition of NaBH(OAc)₃ (65 mg, 0.30 mmol). The reaction was allowed to react overnight at room temperature, and then added with water. The resultant reaction was extracted twice with DCM. The organic phases were combined, washed with saturated NaCl solution, dried over anhydrous Na₂SO₄, and rotatory evaporated to dry. The residue was purified by column chromatography to obtain the target compound (27 mg, 0.03 mmol), with a yield of 45%. LC/MS (ESI+) calcd for C₄₇H₄₉N₅O₅ (M + H⁺) *m*/*z,* 764.3; found, 764.3. ¹H NMR (400 MHz, DMSO-*d*₆) *δ* 11.08 (s, 1H), 9.10 (s, 1H), 7.64 (d, *J =* 8.5 Hz, 1H), 7.17 - 7.06 (m, 3H), 6.95 (d, *J =* 2.2 Hz, 1H), 6.88 - 6.77 (m, 3H), 6.67 - 6.55 (m, 2H), 6.48 (dd, *J =* 12.2, 7.3 Hz, 3H), 6.17 (d, *J =* 8.3 Hz, 2H), 5.06 (dd, *J =* 12.8, 5.4 Hz, 1H), 4.11 (d, *J =* 4.9 Hz, 1H), 3.67 (s, 2H), 3.47 (d, *J =* 11.8 Hz, 2H), 3.25 (d, *J =* 9.6 Hz, 3H), 2.99 - 2.80 (m, 5H), 2.56 (t, *J =* 8.9 Hz, 3H), 2.46 (d, *J =* 11.4 Hz, 4H), 2.23 (d, *J =* 7.2 Hz, 2H), 2.13 -1.95 (m, 3H), 1.69 (s, 3H), 1.48 (s, 2H).

### Example 26: 3-(5-(5-((1-(4-((1R,2S)-6-hydroxyl-2-phenyl-1,2,3,4-tetrahydronaphthalen-1-yl)phenyl)piperidin-4-yl)methyl)hexahydropyrrolo[3,4-c]pyrrole-2(1H)-yl)-1-oxoisoinodolin-2-yl)piperidin-2,6-dione

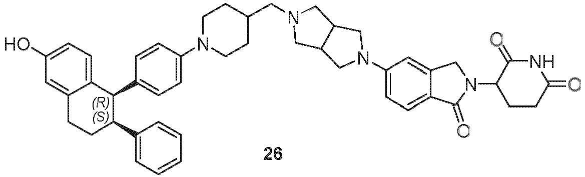

The target compound was prepared using a method similar to that in Example 25. LC/MS (ESI+) calcd for C₄₇H₅₁N₅O₄ (M + H⁺) *m*/*z,* 750.4; found, 750.4. ¹H NMR (400 MHz, DMSO-*d*₆) *δ* 10.94 (s, 1H), 9.11 (s, 1H), 7.47 (dd, *J =* 12.7, 8.7 Hz, 1H), 7.12 (q, *J =* 8.4, 7.3 Hz, 3H), 6.86 - 6.77 (m, 2H), 6.70 (d, *J =* 7.3 Hz, 2H), 6.65 - 6.56 (m, 2H), 6.54 - 6.42 (m, 3H), 6.17 (d, *J* = 8.3 Hz, 2H), 5.03 (dd, *J =* 13.3, 5.2 Hz, 1H), 4.31 (d, *J =* 16.9 Hz, 1H), 4.22 - 4.06 (m, 2H), 3.62 - 3.42 (m, 5H), 3.12 (d, *J =* 10.6 Hz, 2H), 2.92 (m , 5H), 2.63 - 2.52 (m, 3H), 2.46 - 2.30 (m, 4H), 2.25 (m, 2H), 2.13 (m, 4H), 1.70 (m, 3H), 1.50 (s, 2H).

### Example 27: 3-(5-(2-((1-(4-((1R,2S)-6-hydroxyl-2-phenyl-1,2,3,4-tetrahydronaphthalen-1-yl)phenyl)piperidin-4-yl)methyl)-2,7-diazaspiro[3.5]nonan-7-yl)-1 -oxoisoinodolin-2-yl)piperidin-2,6-dione

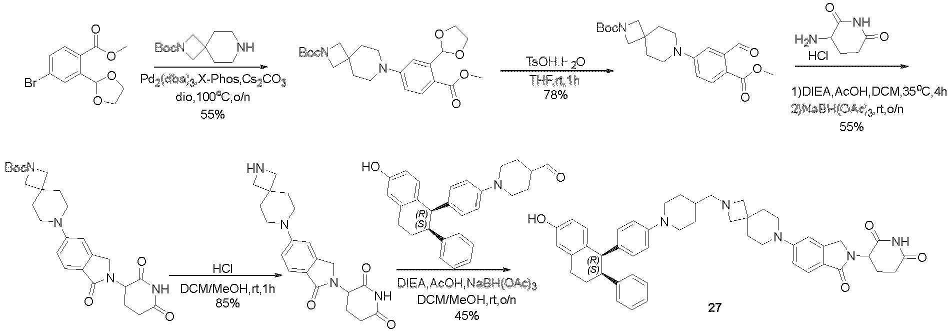

### Step 1: Synthesis of tert-butyl 7-(3-(1,3-dioxolane-2-yl)-4-(methoxycarbonyl)phenyl)-2,7-diazaspiro[3.5]nonane-2-carboxylate

Compound tert-butyl 2,7-diazaspiro[3.5]nonane-2-carboxylate (700 mg, 3.10 mmol), methyl 4-bromo-2-(1,3-dioxolane-2-yl)benzoate (850 mg, 3.00 mmol), Pd₂(dba)₃ (274 mg, 0.30 mmol), X-Phos (285 mg, 0.60 mmol), and Cs₂CO₃ (1950 mg, 6.00 mmol) were dissolved in 15 mL of 1,4-dioxane, and the reaction was stirred overnight at 100 °C under N₂ protection. The reaction solution was added with water, and then extracted twice with EA. The organic phases were combined, washed with saturated NaCl solution, dried over anhydrous Na₂SO₄, and rotatory evaporated to dry. The residue was purified by column chromatography and dired to obtain the target compound (712 mg, 1.65 mmol), with a yield of 55%. LC/MS (ESI+) calcd for C₂₃H₃₂N₂O₆ (M + H⁺) *m*/*z,* 433.2; found, 433.2.

### Step 2: Synthesis of tert-butyl 7-(3-formyl-4-(methoxycarbonyl)phenyl)-2,7-diazaspiro[3.5]nonane-2-carboxylate

Compound tert-butyl 7-(3-(1,3-dioxolane-2-yl)-4-(methoxycarbonyl)phenyl)-2,7-diazaspiro[3.5]nonane-2-carboxylate (712 mg, 1.65 mmol) and TsOH.H₂O (380 mg, 2.00 mmol) were dissolved in 10 mL of THF, and the reaction was stirred for 1h at room temperature. The reaction solution was added with water, and then extracted twice with EA. The organic phases were combined, washed with saturated NaCl solution, dried over anhydrous Na₂SO₄, and rotatory evaporated to dry. The residue was purified by column chromatography and dired to obtain the target compound (499 mg, 1.28 mmol), with a yield of 78%. LC/MS (ESI+) calcd for C₂₁H₂₈N₂O₅ (M + H⁺) *m*/*z,* 389.2; found, 389.2.

### Step 3: Synthesis of tert-butyl 7-(2-(2,6-dioxopiperidin-3-yl)-1-oxoisoinodolin-5-yl)-2,7-diazaspiro[3.5]nonane-2-carboxylate

Compound tert-butyl 7-(3-formyl-4-(methoxycarbonyl)phenyl)-2,7-diazaspiro[3.5]nonane-2-carboxylate (499 mg, 1.28 mmol) and 3-aminopiperidin-2,6-dione (249 mg, 1.53 mmol) were dissolved in 20 mL of DCM, to which were added DIEA (496 mg, 3.84 mmol) and AcOH (768 mg, 12.80 mmol), and then stirred at 35 °C for 4 h. The reaction solution was cooled to room temperature, and added with NaBH(OAc)₃ (815 mg, 3.84 mmol). The resultant solution was stirred overnight at room temperature, and added with NH₄Cl solution, followed by extracting with EA for three times. The organic phases were combined, washed with saturated NaCl solution, dried over anhydrous Na₂SO₄, and rotatory evaporated to dry. The residue was purified by column chromatography and dired to obtain the target compound (329 mg, 0.70 mmol), with a yield of 55%. LC/MS (ESI+) calcd for C₂₅H₃₂N₄O₅ (M + H⁺) *m*/*z,* 469.2; found, 469.2.

### Step 4: Synthesis of 3-(1-oxo-5-(2,7-diazaspiro[3.5]nonan-7-yl)isoinodolin-2-yl)piperidin-2,6-dione

The target compound was prepared using a method similar to that in Example 11. LC/MS (ESI+) calcd for C₂₀H₂₄N₄O₃ (M + H⁺) *m*/*z,* 369.2; found, 369.2.

### Step 5: Synthesis of 3-(5-(2-((1-(4-((1R,2S)-6-hydroxyl-2-phenyl-1,2,3,4-tetrahydronaphthalen-1-yl)phenyl)piperidin-4-yl)methyl)-2,7-diazaspiro[3.5]nonan-7-yl)-1-oxoi soinodolin-2-yl)piperidin-2,6-dione

The target compound was prepared using a method similar to that in Example 11. LC/MS (ESI+) calcd for C₄₈H₅₃N₅O₄ (M + H⁺) *m*/*z,* 764.4; found, 764.4. ¹H NMR (400 MHz, DMSO-*d*₆) *δ* 10.95 (s, 1H), 9.15 (s, 1H), 7.49 (d, *J =* 8.4 Hz, 1H), 7.19 - 6.98 (m, 5H), 6.88 - 6.76 (m, 2H), 6.65 - 6.57 (m, 2H), 6.56 - 6.44 (m, 3H), 6.19 (d, *J =* 8.3 Hz, 2H), 5.03 (dd, *J =* 13.3, 5.1 Hz, 1H), 4.31 (d, *J =* 16.9 Hz, 1H), 4.22 - 4.05 (m, 2H), 3.69 - 3.48 (m, 7H), 3.04 (d, *J* = 6.9 Hz, 3H), 2.90 (ddd, *J =* 18.8, 15.1, 5.9 Hz, 4H), 2.78 (s, 2H), 2.63 - 2.54 (m, 1H), 2.47 - 2.29 (m, 2H), 2.17 - 1.92 (m, 3H), 1.83 (t, *J =* 5.3 Hz, 3H), 1.72 (d, *J =* 11.7 Hz, 3H), 1.56 (s, 1H).

### Example 28: 3-(5-(4-((2-(4-)((3,4-cis)-7-hydroxyl-3-phenylchroman-4-yl)phenyl)-2,7-diazaspiro[3.5]nonan-7-yl)methyl)piperidin-1-yl)-1-oxoisoinodolin-2-yl)piperidin-2,6-dion e

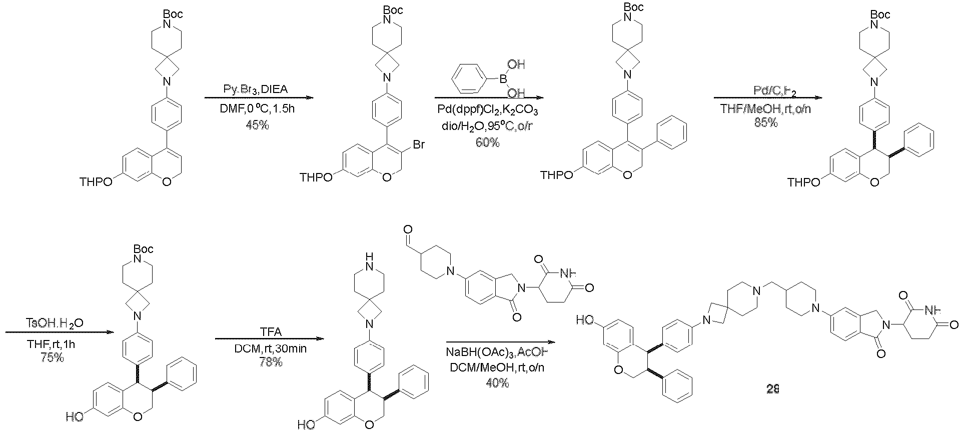

### Step 1: Synthesis of tert-butyl 2-(4-(3-bromo-7-((tetrahydro-2H-pyran-2-yl)oxy)-2H-chromene-4-yl)phenyl)-2,7-diazaspiro[3.5]nonane-7-carboxylate

Compound tert-butyl 2-(4-(7-((tetrahydro-2*H*-pyran-2-yl)oxy)-2*H*-chromene-4-yl)phenyl)-2,7-diazaspiro[3.5]nonane-7-carboxylate (266 mg, 0.50 mmol) and DIEA (200 mg, 1.50 mmol) were dissolved in 4 mL of DMF, to which was added pyridinium tribromide (239 mg, 0.75 mmol) in an ice bath, and then stirred for 1.5 h in an ice bath. The reaction solution was added with water, and then extracted three times with EA. The organic phases were combined, washed with saturated NaCl solution, dried over anhydrous Na₂SO₄, and rotatory evaporated to dry. The residue was purified by column chromatography and dired to obtain the target compound (137 mg, 0.22 mmol), with a yield of 45%. LC/MS (ESI+) calcd for C₃₂H₃₉BrN₂O₅ (M + H⁺) *m*/*z*, 611.2; found, 611.2.

### Step 2: Synthesis of tert-butyl 2-(4-(3-phenyl-7-((tetrahydro-2H-pyran-2-yl)oxy)-2H-chromene-4-yl)phenyl)-2,7-diazaspiro[3.5]nonane-7-carboxylate

Compound tert-butyl 2-(4-(3-bromo-7-((tetrahydro-2*H*-pyran-2-yl)oxy)-2*H*-chromene-4-yl) phenyl)-2,7-diazaspiro[3.5]nonane-7-carboxylate (136 mg, 0.22 mmol), phenylboronic acid (41 mg, 0.33 mmol), Pd(dppf)Cl₂ (15 mg, 0.02 mmol), and K₂CO₃ (82 mg, 0.60 mmol) were dissolved in 8 mL of 1,4-dioxane and 3 mL of H₂O, and then stirred overnight at 95 °C. The reaction solution cooled to room temperature, added with water, and then extracted three times with EA. The organic phases were combined, washed with saturated NaCl solution, dried over anhydrous Na₂SO₄, and rotatory evaporated to dry. The residue was purified by column chromatography and dired to obtain the target compound (80 mg, 0.13 mmol), with a yield of 60%. LC/MS (ESI+) calcd for C₃₈H₄₄N₂O₅ (M + H⁺) *m*/*z,* 609.3; found, 609.3.

### Step 3: Synthesis of tert-butyl 2-(4-((3S,4R)-3-phenyl-7-((tetrahydro-2H-pyran-2-yl)oxy) chroman-4-yl)phenyl)-2,7-diazaspiro[3.5]nonane-7-carboxylate

Compound tert-butyl 2-(4-(3-phenyl-7-((tetrahydro-2*H*-pyran-2-yl)oxy)-2*H*-chromene-4-yl)phenyl)-2,7-diazaspiro[3.5]nonane-7-carboxylate (80 mg, 0.13 mmol) was dissolved in 10 mL of THF and 10 mL of MeOH, to which was added 20 mg Pd/C under Ar protection, and then the reaction was allowed to react overnight under hydrogen atmosphere. The reaction solution was filtered via diatomaceous earth, and then rotatory evaporated to dry, to obtain the target compound (67 mg, 0.11 mmol), with a yield of 85%. LC/MS (ESI+) calcd for C₃₈H₄₆N₂O₅ (M + H⁺) *m*/*z,* 611.3; found, 611.3.

### Step 4: Synthesis of tert-butyl 2-(4-((3S,4R)-7-hydroxyl-3-phenylchroman-4-yl)phenyl)-2,7-diazaspiro[3.5]nonane-7-carboxylate

Compound tert-butyl 2-(4-((3*S*,4*R*)-3-phenyl-7-((tetrahydro-2*H*-pyran-2-yl)oxy)chroman-4-yl)phenyl)-2,7-diazaspiro[3.5]nonane-7-carboxylate (67 mg, 0.11 mmol) was dissolved in 5 mL of THF, to which was added TsOH.H₂O (30 mg, 0.16 mmol), and then the mixture was allowed to react for 1 h at room temperature. The reaction solution was diluted with EA, and then sequentially washed once with NaHCO₃ solution and NaCl solution, dried over anhydrous Na₂SO₄, and rotatory evaporated to dry, to obtain the target compound (43 mg, 0.08 mmol), with a yield of 75%. LC/MS (ESI+) calcd for C₃₃H₃₈N₂O₄ (M + H⁺) *m*/*z,* 527.3; found, 527.3.

### Step 5: Synthesis of (3S,4R)-4-(4-(2,7-diazaspiro[3.5]nonane-2-yl)phenyl)-3-phenylchroman-7-ol

Compound tert-butyl 2-(4-((3*S*,4*R*)-7-hydroxyl-3-phenylchroman-4-yl)phenyl)-2,7-diazaspiro[3.5]nonane-7-carboxylate (43 mg, 0.08 mmol) was dissolved in 8 mL of DCM, to which was added 2 mL of TFA in an ice bath, and then the ice bath was removed. The reaction was allowed to react for 30 min at room temperatue and concentrated. The residue was diluted in DCM, and adjusted to be alkaline with NaHCO₃ solution. The resultant solution was extracted with DCM for three times. The organic phase was combined, washed once with NaCl solution, dried over anhydrous Na₂SO₄, and rotatory evaporated to dry, to obtain the target compound (26 mg, 0.06 mmol), with a yield of 78%. LC/MS (ESI+) calcd for C₂₈H₃₀N₂O₂ (M + H⁺) *m*/*z,* 427.2; found, 427.2.

### Step 6: Synthesis of 3-(5-(4-((2-(4-)((3,4-cis)-7-hydroxyl-3-phenylchroman-4-yl)phenyl)-2,7-diazaspiro[3.5]nonan-7-yl)methyl)piperidin-1-yl)-1-oxoisoinodolin-2-yl)piperidin-2,6-dion e

The target compound was prepared using a method similar to that in Example 25. LC/MS (ESI+) calcd for C₄₇H₅₁N₅O₅ (M + H⁺) *m*/*z,* 766.4; found, 766.4. ¹H NMR (400 MHz, DMSO-*d*₆) *δ* 10.94 (s, 1H), 9.21 (s, 1H), 7.48 (d, *J* = 8.7 Hz, 1H), 7.41 - 7.25 (m, 6H), 7.01 (d, *J* = 7.9 Hz, 2H), 6.92 (dd, *J =* 8.4, 2.1 Hz, 1H), 6.76 (d, *J =* 2.1 Hz, 1H), 6.55 (dd, *J =* 19.9, 8.3 Hz, 2H), 6.24 - 6.15 (m, 2H), 5.03 (dd, *J =* 13.3, 5.1 Hz, 1H), 4.22 - 3.95 (m, 5H), 3.83 (d, *J =* 12.3 Hz, 2H), 3.11 (s, 5H), 2.96 - 2.69 (m, 5H), 2.62 - 2.53 (m, 1H), 2.35 (qd, *J* = 13.1, 4.5 Hz, 2H), 1.98 - 1.92 (m, 2H), 1.71 (d, *J =* 12.6 Hz, 4H), 1.55 (d, *J* = 5.7 Hz, 5H), 1.22 (s, 2H).

### Example 29: 3-(5-(7-((1-(4-((3,4-cis)-7-hydroxyl-3-(1-methyl-1H-pyrazole-4-yl) chroman-4-phenyl)piperidin-4-methyl)-2,7-diazaspiro[3.5]nonane-2-yl)-1-oxoisoinodolin-2 -ylpiperidin-2,6-dione

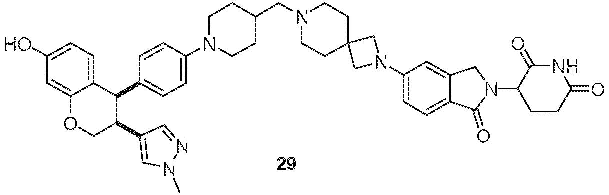

The target compound was prepared using a method similar to that in Example 10. LC/MS (ESI+) calcd for C₄₅H₅₁N₇O₅ (M + H⁺) *m*/*z,* 770.4; found, 770.4. ¹H NMR (400 MHz, DMSO-*d*₆) δ 10.94 (s, 1H), 9.26 (s, 1H), 7.71 - 7.31 (m, 1H), 7.02 (s, 1H), 6.75 - 6.66 (m, 3H), 6.59 (dd, *J* = 17.6, 8.3 Hz, 3H), 6.52 - 6.44 (m, 2H), 6.29 - 6.20 (m, 2H), 5.03 (dd, *J* = 13.2, 5.1 Hz, 1H), 4.29 (d, *J* = 17.0 Hz, 1H), 4.20 - 4.00 (m, 4H), 3.67 (s, 3H), 3.64 - 3.53 (m, 6H), 3.38 (d, *J* = 5.9 Hz, 2H), 2.97 - 2.77 (m, 1H), 2.57 (d, *J =* 15.0 Hz, 2H), 2.41 - 2.20 (m, 5H), 2.12 (d, *J* = 6.7 Hz, 2H), 1.95 (s, 2H), 1.75 (d, *J* = 8.8 Hz, 6H), 1.62 (s, 2H), 1.45 (s,1H).

### Example 30: 3-(5-(7-((1-(4-((3,4-cis)-3-(2-fluorophenyl)-7-hydroxychroman-4-yl) phenyl)piperidin-4-methyl)-2,7-diazaspiro[3.5]nonane-2-yl)-1-oxoisoinodolin-2-ylpiperidin -2,6-dione

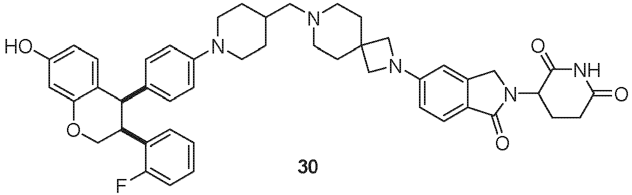

The target compound was prepared using a method similar to that in Example 10. LC/MS (ESI+) calcd for C₄₇H₅₀FN₅O₅ (M + H⁺) *m*/*z*, 784.4; found, 784.4. ¹H NMR (400 MHz, DMSO-*d*₆) *δ* 10.94 (s, 1H), 9.34 (s, 1H), 7.47 (d, *J* = 8.3 Hz, 1H), 7.22 (q, *J =* 7.1 Hz, 1H), 7.14 (dd, *J* = 10.7, 8.1 Hz, 1H), 6.89 (t, *J* = 7.5 Hz, 1H), 6.67 (d, *J =* 8.3 Hz, 1H), 6.60 (d, *J =* 8.4 Hz, 2H), 6.53 - 6.36 (m, 5H), 6.34 - 6.24 (m, 2H), 5.03 (dd, *J =* 13.3, 5.1 Hz, 1H), 4.43 - 4.07 (m, 5H), 3.70 (dt, *J* = 11.5, 4.2 Hz, 1H), 3.61 (s, 4H), 3.57 - 3.48 (m, 2H), 2.89 (td, *J* = 13.2, 6.7 Hz, 1H), 2.62 - 2.54 (m, 1H), 2.47 (s, 1H), 2.41 - 2.16 (m, 5H), 2.10 (d, *J =* 7.3 Hz, 2H), 1.90 (s, 3H), 1.73 (t, *J =* 5.3 Hz, 6H), 1.59 (s, 1H).

### Example 31: 3-(5-(6-((1-(4-((1,2-cis)-6-hydroxyl-2-phenyl-1,2,3,4-tetrahydronaphthalen-1-yl)phenyl)piperidin-4-yl)methyl)-2,6-diazaspiro [3.4] octane-2-yl)-1-oxoisoinodolin-2-yl)piperidin-2,6-one

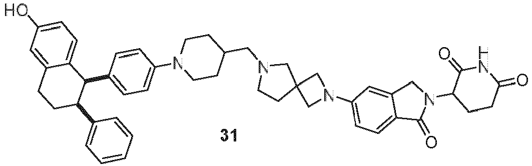

The target compound was prepared using a method similar to that in Example 10. LC/MS (ESI+) calcd for C₄₇H₅₁N₅O₄ (M + H⁺) *m*/*z,* 750.4; found, 750.4. ¹H NMR (400 MHz, DMSO-*d*₆) *δ* 10.96 (s, 1H), 9.15 (s, 1H), 7.50 (d, *J =* 8.3 Hz, 1H), 7.20 - 7.05 (m, 3H), 6.87 - 6.77 (m, 2H), 6.66 - 6.58 (m, 2H), 6.57- 6.45 (m, 5H), 6.20 (d, *J =* 8.4 Hz, 2H), 5.04 (dd, *J* = 13.3, 5.1 Hz, 1H), 4.30 (d, *J* = 17.0 Hz, 1H), 4.24 - 4.08 (m, 2H), 3.89 (d, *J =* 22.9 Hz, 4H), 3.60 (t, *J =* 6.5 Hz, 1H), 3.56 - 3.46 (m, 2H), 3.32 - 3.21 (m, 3H), 3.12 (d, *J =* 7.5 Hz, 2H), 3.03 - 2.78 (m, 5H), 2.65 - 2.52 (m, 2H), 2.35 (dd, *J=* 13.3, 4.5 Hz, 1H), 2.20 - 2.03 (m, 2H), 2.02 - 1.92 (m, 2H), 1.80 (d, *J* = 12.1 Hz, 2H), 1.70 (d, *J=* 9.8 Hz, 2H).

### Example 32: 3-(5-(7-((1-(2,6-difluoro-4-((3,4-cis)-3-(2-fluorophenyl)-7-hydroxybenzodihydropyran-4-yl)phenyl)piperidin-4-yl)methyl)-2,7-diazaspiro[3.5]nonane -2-yl)-1-oxoisoinodolin-2-yl)piperidin-2,6-dione

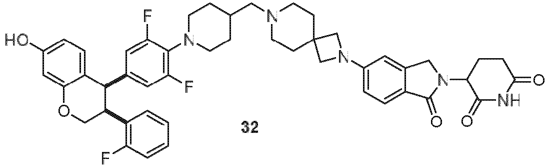

The target compound was prepared using a method similar to that in Example 10. LC/MS (ESI+) calcd for C₄₇H₄₈F₃N₅O₅ (M + H⁺) *m*/*z,* 820.3; found, 820.3. ¹H NMR (400 MHz, DMSO-*d*₆) δ 10.95 (s, 1H), 9.45 (s, 1H), 7.48 (d, *J =* 8.3 Hz, 1H), 7.32 - 7.22 (m, 1H), 7.21 - 7.10 (m, 1H), 6.97 (td, *J* = 7.5, 1.3 Hz, 1H), 6.70 (d, *J =* 8.0 Hz, 1H), 6.60 (d, *J=* 1.7 Hz, 1H), 6.53 - 6.43 (m, 2H), 6.33 (d, *J* = 7.7 Hz, 2H), 6.13 (d, *J =* 10.7 Hz, 2H), 5.03 (dd, *J* = 13.3, 5.1 Hz, 1H), 4.38 - 4.28 (m, 2H), 4.27 (s, 1H), 4.18 (s, 1H), 3.76 (dd, *J =* 8.7, 3.9 Hz, 1H), 3.62 (s, 4H), 3.02 (d, *J =* 11.1 Hz, 3H), 2.96 - 2.80 (m, 1H), 2.44 - 2.23 (m, 4H), 2.12 (s, 2H), 1.91 (s, 2H), 1.74 (s, 6H), 1.59 (s, 1H), 1.22 (s, 2H).

### Example 33: 3-(5-(7-((1-(2,6-difluoro-4-((3,4-cis)-3-(2-fluorophenyl)-7-hydroxychroman-4-yl)phenyl)piperidin-4-yl)methyl)-2,7-diazaspiro[3.5]nonane-2-yl)-1-ox oisoinodolin-2-yl)piperidin-2,6-dione

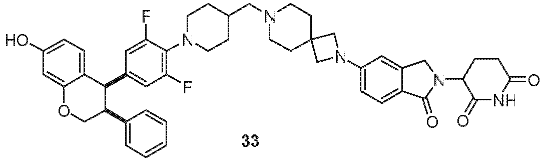

The target compound was prepared using a method similar to that in Example 10. LC/MS (ESI+) calcd for C₄₇H₄₈F₃N₅O₅ (M + H⁺) *m*/*z* 820.3; found 820.3. ¹H NMR (400 MHz, DMSO-*d*₆) *δ* 10.95 (s, 1H), 9.42 (s, 1H), 7.48 (d, *J =* 8.3 Hz, 1H), 7.19 (p, *J* = 3.7 Hz, 3H), 6.84 (dd, *J* = 6.7*,* 2.9 Hz, 2H), 6.69 (d, *J =* 8.0 Hz, 1H), 6.56 - 6.42 (m, 2H), 6.35 - 6.25 (m, 2H), 6.08 (d, *J* = 10.7 Hz, 2H), 5.03 (dd, *J =* 13.3, 5.1 Hz, 1H), 4.35 - 4.26 (m, 3H), 4.16 (d, *J* = 17.0 Hz, 1H), 3.67 - 3.52 (m, 5H), 3.02 (d, *J =* 11.2 Hz, 2H), 2.91 (dt, *J =* 17.3, 9.1 Hz, 3H), 2.63 - 2.53 (m, 1H), 2.41 - 2.23 (m, 4H), 2.13 (s, 2H), 1.98 (dd, *J =* 14.6, 6.7 Hz, 1H), 1.81 - 1.64 (m, 6H), 1.60 (s, 1H), 1.23 (d, *J* = 3.4 Hz, 2H).

### Example 34: 3-(5-(7-((1-(4-((3R,4S)-3-(2-fluorophenyl)-7-hydroxychroman-4-yl) phenyl)piperidin-4-yl)methyl)-2,7-diazaspiro[3.5]nonane-2-yl)-1-oxoisoindol-2-yl)piperidi n-2,6-dione

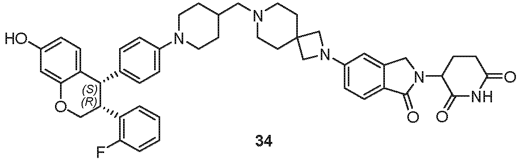

The target compound was prepared using 1-(4-((3*R*,4*S*)-3-(2-fluorophenyl)-7-hydroxychroman-4-yl)phenyl)piperidine-4-carbaldehyde as starting material and the method similar to that of Example 10. LC/MS (ESI+) calcd for C₄₇H₅₀FN₅O₅ (M + H⁺) *m*/*z,* 784.3; found, 784.3. ¹H NMR (400 MHz, DMSO-*d*₆) *δ* 10.95 (s, 1H), 9.34 (s, 1H), 7.47 (d, *J =* 8.3 Hz, 1H), 7.27 - 7.19 (m, 1H), 7.17 - 7.10 (m, 1H), 6.92 - 6.86 (m, 1H), 6.67 (dd, *J =* 8.3, 2.2 Hz, 1H), 6.60 (d, *J =* 8.5 Hz, 2H), 6.52 - 6.36 (m, 5H), 6.33 - 6.25 (m, 2H), 5.03 (dd, *J =* 13.3, 5.1 Hz, 1H), 4.41 - 4.11 (m, 5H), 3.71 (dt, *J* = 11.2, 4.5 Hz, 1H), 3.62 (s, 4H), 3.53 (d, *J=* 11.9 Hz, 2H), 2.96 - 2.79 (m, 1H), 2.63 - 2.52 (m, 2H), 2.42 - 2.19 (m, 5H), 2.11 (d, *J =* 7.3 Hz, 2H), 2.03 - 1.90 (m, 2H), 1.79 - 1.54 (m, 7H), 1.23 (d, *J=* 3.4 Hz, 2H).

### Example 35: 3-(5-(7-((1-(4-(cis-7-hydroxyl-3-(o-methylphenyl)chroman-4-yl) phenyl)piperidin-4-yl)methyl)-2,7-diazaspiro[3.5]nonane-2-yl)-1-oxoisoinodolin-2-yl)piper idin-2,6-dione

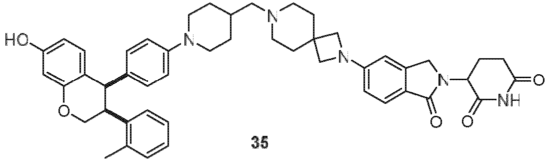

The target compound was prepared using a method similar to that in Example 10. LC/MS (ESI+) calcd for C₄₈H₅₃N₅O₅ (M + H⁺) *m*/*z,* 780.4; found, 780.4. ¹H NMR (400 MHz, DMSO-*d*₆) *δ* 10.94 (s, 1H), 9.31 (s, 1H), 7.47 (d, *J= 8.3* Hz, 1H), 7.25 - 7.14 (m, 1H), 7.03 (t, *J* = 7.2 Hz, 1H), 6.78 (t, *J* = 7.6 Hz, 1H), 6.67 (d, *J =* 8.2 Hz, 1H), 6.58 (d, *J=* 8.5 Hz, 2H), 6.52 - 6.43 (m, 2H), 6.37 - 6.21 (m, 4H), 5.99 (d, *J =* 7.7 Hz, 1H), 5.03 (dd, *J =* 13.2, 5.1 Hz, 1H), 4.41 - 4.16 (m, 4H), 4.15 - 4.05 (m, 1H), 3.56 (d, *J =* 44.5 Hz, 6H), 2.96 - 2.79 (m, 1H), 2.62 - 2.53 (m, 1H), 2.40 (s, 3H), 2.37 - 2.20 (m, 4H), 2.10 (d, *J =* 7.1 Hz, 2H), 1.96 (ddd, *J =* 18.8, 14.1, 7.8 Hz, 3H), 1.73 (s, 6H), 1.59 (s, 1H), 1.22 (s, 2H).

### Example 36: 3-(5-(7-((1-(4-(cis-3-(2,3-difluorophenyl)-7-hydroxychroman-4-yl)phenyl)piperidin-4-yl)methyl)-2,7-diazaspiro[3.5]nonane-2-yl)-1-oxoisoindol-2-yl)pipe ridin-2,6-dione

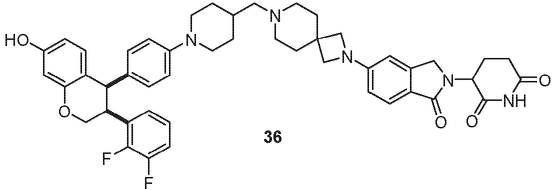

The target compound was prepared using a method similar to that in Example 10. LC/MS (ESI+) calcd for C₄₇HF₂N₅O₅ (M + H⁺) m/z, 802.3; found, 802.3. ¹H NMR (400 MHz, DMSO-*d*₆) *δ* 10.95 (s, 1H), 9.36 (s, 1H), 7.48 (d, *J =* 8.3 Hz, 1H), 7.25 (dt, *J =* 11.2, 7.8 Hz, 1H), 6.96 - 6.88 (m, 1H), 6.70 - 6.61 (m, 3H), 6.53 - 6.39 (m, 4H), 6.35 - 6.27 (m, 3H), 5.03 (dd, *J* = 13.3, 5.1 Hz, 1H), 4.45 - 4.08 (m, 6H), 3.79 - 3.71 (m, 1H), 3.62 (s, 3H), 3.59 - 3.50 (m, 2H), 2.90 (s, 1H), 2.69 - 2.53 (m, 2H), 2.41 - 2.19 (m, 5H), 2.12 (s, 2H), 1.97 (ddt, *J =* 18.0, 12.4, 7.2 Hz, 3H), 1.72 (d, *J* = 15.9 Hz, 7H), 1.17 (s, 2H).

### Example 37: 3-(5-(7-((1-(4-(cis-3-(2,6-difluorophenyl)-7-hydroxychroman-4-yl)-2,6-difluorophenyl(piperidin-4-yl)methyl)-2,7-diazaspiro[3.5]nonane-2-yl)-1-oxoisoinodolin-2-yl)piperidin-2,6-one

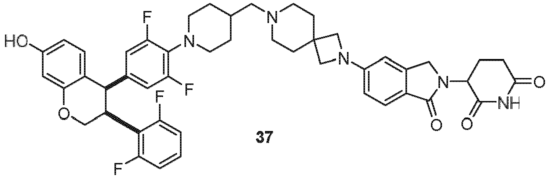

The target compound was prepared using a method similar to that in Example 10. LC/MS (ESI+) calcd for C₄₇H₄₇F₄N₅O₅ (M + H⁺) *m*/*z,* 838.3; found,838.3. ¹H NMR (400 MHz, DMSO-*d*₆) δ 10.94 (s, 1H), 9.47 (s, 1H), 7.47 (d, *J* = 8.3 Hz, 1H), 7.36 (tt, *J* = 8.3*,* 6.4 Hz, 1H), 6.99 (t, *J =* 9.2 Hz, 2H), 6.71 (d, *J =* 8.3 Hz, 1H), 6.52 - 6.44 (m, 2H), 6.34 (d, *J =* 7.5 Hz, 2H), 6.18 (d, *J* = 10.8 Hz, 2H), 5.03 (dd, *J=* 13.3, 5.1 Hz, 1H), 4.45 (d, *J=* 11.6 Hz, 1H), 4.34 - 4.23 (m, 2H), 4.22 - 4.12 (m, 2H), 3.83 (ddd, *J =* 11.9, 5.3, 3.0 Hz, 1H), 3.62 (s, 4H), 3.04 (d, *J =* 11.1 Hz, 2H), 2.91 (d, *J =* 13.4 Hz, 3H), 2.62 - 2.53 (m, 1H), 2.41 - 2.23 (m, 4H), 2.12 (d, *J =* 6.5 Hz, 2H), 1.97 (d, *J =* 12.8 Hz, 2H), 1.79 - 1.67 (m, 6H), 1.60 (s, 1H), 1.22 (d, *J =* 3.4 Hz, 2H).

### Example 38: 3-(5-(7-((1-(2,6-difluoro-4-((3R,4S)-3-(2-fluorophenyl)-7-hydroxychroman-4-yl)phenyl)piperidin-4-yl)methyl)-2,7-diazaspiro[3.5]nonane-2-yl)-1-ox oisoindol-2-yl)piperidin-2,6-dione

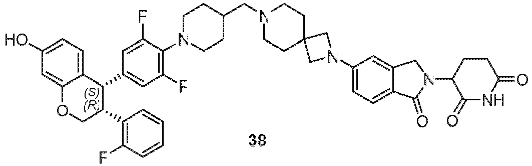

The target compound was prepared using a method similar to that in Example 34. LC/MS (ESI+) calcd for C₄₇H₄₈F₃N₅O₅ (M + H⁺) *m*/*z* 820.3; found 820.3. ¹H NMR (400 MHz, DMSO-*d*₆) *δ* 10.95 (s, 1H), 9.45 (s, 1H), 7.48 (d, *J =* 8.2 Hz, 1H), 7.33 - 7.23 (m, 1H), 7.17 (ddd, *J* = 9.8, 8.4, 1.3 Hz, 1H), 6.97 (td, *J* = 7.6, 1.3 Hz, 1H), 6.70 (d, *J =* 8.0 Hz, 1H), 6.60 (td, *J =* 7.8, 1.7 Hz, 1H), 6.53 - 6.42 (m, 2H), 6.33 (d, *J =* 7.8 Hz, 2H), 6.13 (d, *J =* 10.6 Hz, 2H), 5.03 (dd, *J* = 13.2, 5.1 Hz, 1H), 4.42 - 4.22 (m, 4H), 4.17 (d, *J =* 16.9 Hz, 1H), 3.76 (dt, *J=* 10.2, 4.4 Hz, 1H), 3.63 (s, 4H), 3.03 (d, *J* = 11.1 Hz, 2H), 2.90 (q, *J =* 12.1, 9.8 Hz, 3H), 2.63 - 2.53 (m, 1H), 2.42 - 2.18 (m, 4H), 2.13 (d, *J =* 14.2 Hz, 2H), 1.98 - 1.92 (m, 1H), 1.70 (d, *J* = 19.0 Hz, 7H), 1.18 (d, *J* = 7.1 Hz, 2H).

### Example 39: 3-(5-(7-((1-(4-((3R,4S)-3-(2,3-difluorophenyl)-7-hydroxychroman-4-yl)-2,6-difluorophenyl)piperidin-4-yl)methyl)-2,7-diazaspiro[3.5]nonane-2-yl)-1-oxoisoin odolin-2-yl)piperidin-2,6-one

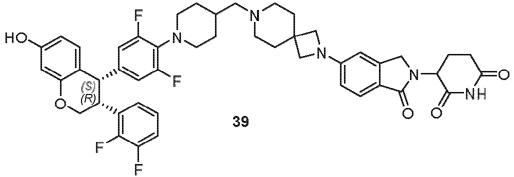

The target compound was prepared using a method similar to that in Example 34. LC/MS (ESI+) calcd for C₄₇H₄₇F₄N₅O₅ (M + H⁺) *m*/*z* 838.3; found 838.3. ¹H NMR (400 MHz, DMSO-*d*₆) *δ* 10.94 (s, 1H), 9.46 (s, 1H), 7.47 (d, *J* = 8.3 Hz, 1H), 7.29 (dt, *J* = 8.2, 1.6 Hz, 1H), 7.04 - 6.90 (m, 1H), 6.75 - 6.62 (m, 1H), 6.57 - 6.41 (m, 3H), 6.33 (d, *J* = 7.4 Hz, 2H), 6.20 (d, *J= 10.7* Hz, 2H), 5.03 (dd, *J=* 13.3, 5.1 Hz, 1H), 4.42 - 4.29 (m, 2H), 4.25 (d, *J=* 10.7 Hz, 1H), 4.16 (d, *J* = 17.0 Hz, 1H), 3.86 - 3.72 (m, 1H), 3.62 (s, 4H), 3.09 - 2.81 (m, 6H), 2.59 (d, *J* = 3.5 Hz, 1H), 2.44 - 2.20 (m, 4H), 2.10 (d, *J =* 27.1 Hz, 2H), 1.99 (s, 1H), 1.83 - 1.48 (m, 7H), 1.24 (d, *J* = 5.6 Hz, 2H).

### Example 40: 3-(5-(7-((1-(4-((3S,4R)-3-(2,6-difluorophenyl)-7-hydroxychroman-4-yl)-2,6-difluorophenyl(piperidin-4-yl)methyl)-2,7-diazaspiro[3.5]nonane-2-yl)-1-oxoisoinodoli n-2-yl)piperidin-2,6-one

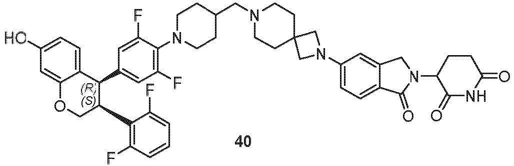

The target compound was prepared using a method similar to that in Example 16. LC/MS (ESI+) calcd for C₄₇H₄₇F₄N₅O₅ (M + H⁺) *m*/*z* 838.3; found 838.3. ¹H NMR (400 MHz, DMSO-*d*₆) *δ* 10.95 (s, 1H), 9.51 (s, 1H), 7.48 (d, *J= 8.3* Hz, 1H), 7.41 - 7.29 (m, 1H), 6.99 (t, *J* = 9.2 Hz, 2H), 6.75 - 6.67 (m, 1H), 6.52 - 6.44 (m, 2H), 6.35 (d, *J* = 7.1 Hz, 2H), 6.18 (d, *J* = 10.7 Hz, 2H), 5.03 (dd, *J =* 13.3, 5.1 Hz, 1H), 4.46 (s, 1H), 4.34 - 4.11 (m, 4H), 3.83 (dq, *J* = 8.7, 2.7 Hz, 1H), 3.61 (d, *J* = 26.4 Hz, 6H), 3.14 - 2.84 (m, 8H), 2.62 - 2.53 (m, 1H), 2.34 (dd, *J* = 13.2, 4.6 Hz, 2H), 1.98 - 1.92 (m, 1H), 1.78 (d, *J* = 43.9 Hz, 7H), 1.22 - 1.10 (m, 2H).

### Example 41: 3-(5-(7-((1-(4-((3R,4S)-3-(2,6-difluorophenyl)-7-hydroxychroman-4-yl)-2,6-difluorophenyl(piperidin-4-yl)methyl)-2,7-diazaspiro[3.5]nonane-2-yl)-1-oxoisoinodoli n-2-yl)piperidin-2,6-one

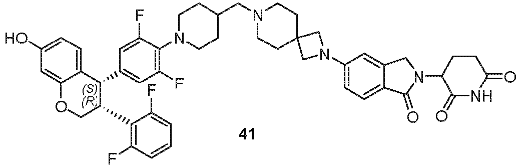

The target compound was prepared using a method similar to that in Example 34. LC/MS (ESI+) calcd for C₄₇H₄₇F₄N₅O₅ (M + H⁺) *m*/*z* 838.3; found 838.3. ¹H NMR (400 MHz, DMSO-*d*₆) *δ* 10.94 (s, 1H), 9.47 (s, 1H), 7.47 (d, *J =* 8.3 Hz, 1H), 7.36 (tt, *J =* 8.3, 6.4 Hz, 1H), 6.99 (t, *J =* 9.2 Hz, 2H), 6.71 (d, *J =* 8.3 Hz, 1H), 6.52 - 6.44 (m, 2H), 6.34 (d, *J =* 7.5 Hz, 2H), 6.18 (d, *J* = 10.8 Hz, 2H), 5.03 (dd, *J* = 13.3, 5.1 Hz, 1H), 4.45 (d, *J* = 11.6 Hz, 1H), 4.34 - 4.23 (m, 2H), 4.22 - 4.12 (m, 2H), 3.83 (ddd, *J* = 11.9, 5.3, 3.0 Hz, 1H), 3.62 (s, 4H), 3.04 (d, *J* = 11.1 Hz, 2H), 2.91 (d, *J =* 13.4 Hz, 3H), 2.62 - 2.53 (m, 1H), 2.41 - 2.23 (m, 4H), 2.12 (d, *J* = 6.5 Hz, 2H), 1.97 (d, *J =* 12.8 Hz, 2H), 1.79 - 1.67 (m, 6H), 1.60 (s, 1H), 1.22 (d, *J =* 3.4 Hz, 2H).

### Example 42: 3-(5-(7-((1-(4-(3-(2,6-difluorophenyl)-7-hydroxyl-2H-chromene-4-yl)-2,6-difluorophenyl(piperidin-4-yl)methyl)-2,7-diazaspiro[3.5]nonane-2-yl)-1-oxoisoinodolin-2-yl)piperidin-2,6-dione

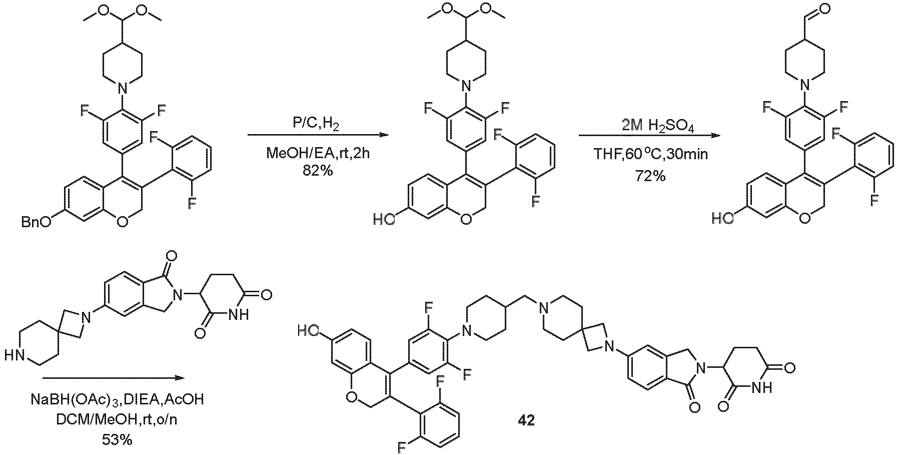

### Step 1: Synthesis of 3-(2,6-difluorophenyl)-4-(4-(4-dimethoxymethyl)piperidin-1-yl) -3,5-difluorophenyl)-2H-chromen-7-ol

Compound 1-(4-(7-(benzyloxy)-3-(2,6-difluorophenyl)-2*H*-chromene-4-yl)-2,6-difluorophenyl]-4-(dimethoxymethyl)piperidine (200 mg, 0.32 mmol) was dissolved in 10 mL EA and 10 mL MeOH, to which was added Pd/C (50 mg, 10%), and then the mixture was allowed to react for 2h under hydrogen atmosphere. The reaction solution was filtered via diatomaceous earth, and the filter cake was rinsed with DCM/MeOH = 10:1. The filtrate was concentrated to obtain the target compound (138 mg, 0.26 mmol), with a yield of 82%. LC/MS (ESI+) calcd for C₂₉H₂₇F₄NO₄ (M + H⁺) *m*/*z,* 530.2; found, 530.2.

### Step 2: Synthesis of 1-(4-(3-(2,6-difluorophenyl)-7-hydroxyl-2H-chromene-4-yl)-2,6-difluorophenyl]piperidine-4-carbaldehyde

The target compound was prepared using a method similar to that in Example 23. LC/MS (ESI+) calcd for C₂₇H₂₁F₄NO₃ (M + H⁺) m/z, 484.1; found,484.1.

### Step 3: Synthesis of 3-(5-(7-((1-(4-(3-(2,6-difluorophenyl)-7-hydroxyl-2H-chromene-4-yl)-2,6-difluorophenyl(piperidin-4-yl)methyl)-2,7-diazaspiro[3.5]nonane-2-yl)-1-o xoisoinodolin-2-yl)piperidin-2,6-dione

The target compound was prepared using a method similar to that in Example 23. LC/MS (ESI+) calcd for C₄₇H₄₅F₄N₅O₅ (M + H⁺) *m*/*z* 836.3; found 836.3. ¹H NMR (400 MHz, DMSO-*d*₆) *δ* 10.95 (s, 1H), 9.93 (s, 1H), 7.50 (d, *J* = 8.2 Hz, 1H), 7.39 - 7.29 (m, 1H), 7.04 (t, *J* = 7.9 Hz, 2H), 6.69 - 6.58 (m, 3H), 6.54 - 6.44 (m, 2H), 6.40 - 6.34 (m, 2H), 5.04 (dd, *J* = 13.2, 5.1 Hz, 1H), 4.82 (s, 2H), 4.31 (d, *J* = 17.0 Hz, 1H), 4.18 (d, *J* = 16.9 Hz, 1H), 3.85 - 3.52 (m, 6H), 3.13 (q, *J =* 9.8, 7.2 Hz, 4H), 3.05 - 2.84 (m, 5H), 2.60 (d, *J* = 3.6 Hz, 2H), 2.43 - 2.27 (m, 2H), 2.04 - 1.92 (m, 4H), 1.78 (d, *J* = 12.6 Hz, 2H), 1.24 - 1.19 (m, 2H).

### Example 43: 3-(5-(7-((1-(4-(7-(benzyloxy)-3-(2,6-difluorophenyl)-2H-chroman-4-yl)-2,6-difluorophenyl(piperidin-4-yl)methyl)-2,7-diazaspiro[3.5]nonane-2-yl)-1-oxoisoino dolin-2-yl)piperidin-2,6-one

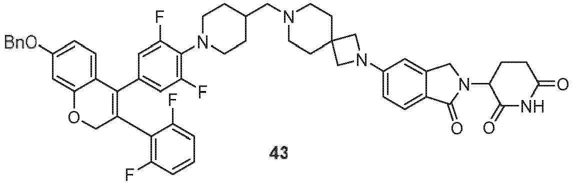

The target compound was prepared using a method similar to that in Example 42. LC/MS (ESI+) calcd for C₅₄H₅₁F₄N₅O₅ (M + H⁺) *m*/*z* 926.3; found 926.3. ¹H NMR (400 MHz, DMSO-*d*₆) *δ* 10.95 (s, 1H), 7.47 - 7.30 (m, 7H), 7.05 (t, *J* = 8.0 Hz, 2H), 6.72 (d, *J* = 8.5 Hz, 1H), 6.63 (ddd, *J* = 19.7, 7.3, 3.0 Hz, 4H), 6.54 - 6.44 (m, 2H), 5.12 (s, 2H), 5.04 (dd, *J* = 13.2, 5.1 Hz, 1H), 4.30 (d, *J* = 17.0 Hz, 1H), 4.17 (d, *J* = 17.0 Hz, 1H), 3.82 - 3.55 (m, 7H), 3.13 (q, *J* = 7.9, 7.4 Hz, 5H), 3.04 - 2.84 (m, 5H), 2.63 - 2.54 (m, 1H), 2.35 (tt, *J =* 14.0, 6.9 Hz, 2H), 1.97 (td, *J =* 14.3, 11.7, 6.7 Hz, 4H), 1.75 (d, *J* = 12.4 Hz, 3H), 1.22 (s, 2H).

### Example 44: (R)-3-(5-(7-((1-(4-((3S,4R)-3-(2,6-difluorophenyl)-7-hydroxychroman-4-yl)-2,6-difluorophenyl(piperidin-4-yl)methyl)-2,7-diazaspiro[3.5]nonane-2-yl)-1-oxoisoino dolin-2-yl)piperidin-2,6-one

### Example 45: (S)-3-(5-(7-((1-(4-((3S,4R)-3-(2,6-difluorophenyl)-7-hydroxychroman4-yl)-2,6-difluorophenyl(piperidin-4-yl)methyl)-2,7-diazaspiro[3.5]nonane-2-yl)-1-oxoisoino dolin-2-yl)piperidin-2,6-one

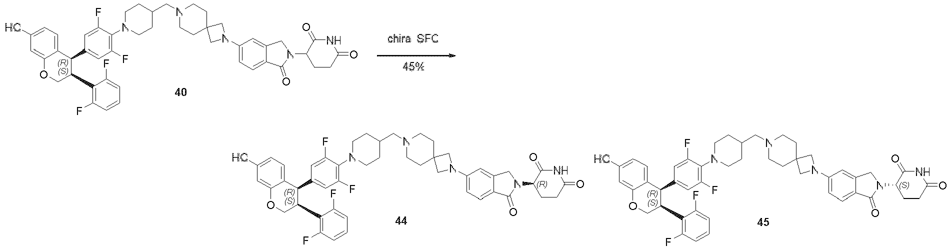

In this example, compound **40** was separated by chiral SFC, to obtain the target compound. Chiral column: CHIRALPAK AS (30*250 mm, 5µm) (Daicel), mobile phase: A=CO₂, Co-Solvent B= IPA/ACN=1/1 (0.1% 7M NH₃ in MeOH).

The first fraction was Example 44. LC/MS (ESI+) calcd for C₄₇H₄₇F₄N₅O₅ (M + H⁺) *m*/*z* 838.3; found 838.3. ¹H NMR (400 MHz, DMSO-*d*₆) *δ* 10.95 (s, 1H), 9.47 (s, 1H), 7.47 (d, *J* = 8.3 Hz, 1H), 7.41 - 7.27 (m, 1H), 6.99 (t, *J* = 9.2 Hz, 2H), 6.71 (d, *J* = 8.2 Hz, 1H), 6.53 - 6.42 (m, 2H), 6.34 (d, *J =* 7.6 Hz, 2H), 6.18 (d, *J =* 10.8 Hz, 2H), 5.03 (dd, *J* = 13.3, 5.1 Hz, 1H), 4.46 (s, 1H), 4.34 - 4.24 (m, 2H), 4.22 - 4.10 (m, 2H), 3.89 - 3.77 (m, 1H), 3.62 (s, 4H), 3.09 - 2.82 (m, 5H), 2.54 (s, 1H), 2.41 - 2.20 (m, 4H), 2.12 (d, *J =* 6.8 Hz, 2H), 1.93 (dd, *J =* 12.0, 6.3 Hz, 1H), 1.71 (d, *J =* 20.5 Hz, 7H), 1.23 (s, 2H).

The second fraction was Example 45. LC/MS (ESI+) calcd for C₄₇H₄₇F₄N₅O₅ (M + H⁺) m/z 838.3; found 838.3. ¹H NMR (400 MHz, DMSO-*d*₆) *δ* 10.95 (s, 1H), 9.47 (s, 1H), 7.47 (d, *J = 8.3* Hz, 1H), 7.41 - 7.27 (m, 1H), 6.99 (t, *J =* 9.2 Hz, 2H), 6.71 (d, *J* = 8.2 Hz, 1H), 6.53 - 6.42 (m, 2H), 6.34 (d, *J =* 7.6 Hz, 2H), 6.18 (d, *J =* 10.8 Hz, 2H), 5.03 (dd, *J* = 13.3, 5.1 Hz, 1H), 4.46 (s, 1H), 4.34 - 4.24 (m, 2H), 4.22 - 4.10 (m, 2H), 3.89 - 3.77 (m, 1H), 3.62 (s, 4H), 3.09 - 2.82 (m, 5H), 2.54 (s, 1H), 2.41 - 2.20 (m, 4H), 2.12 (d, *J =* 6.8 Hz, 2H), 1.93 (dd, *J* = 12.0, 6.3 Hz, 1H), 1.71 (d, *J* = 20.5 Hz, 7H), 1.23 (s, 2H).

### Example 46: 3-(5-(7-((1-(4-(cis-3-(2,4-difluorophenyl)-7-hydroxychroman-4-yl)phenyl) piperidin-4-yl)methyl)-2,7-diazaspiro[3.5]nonane-2-yl)-1-oxoisoinodolin-2-yl)piperidin-2,6 -dione

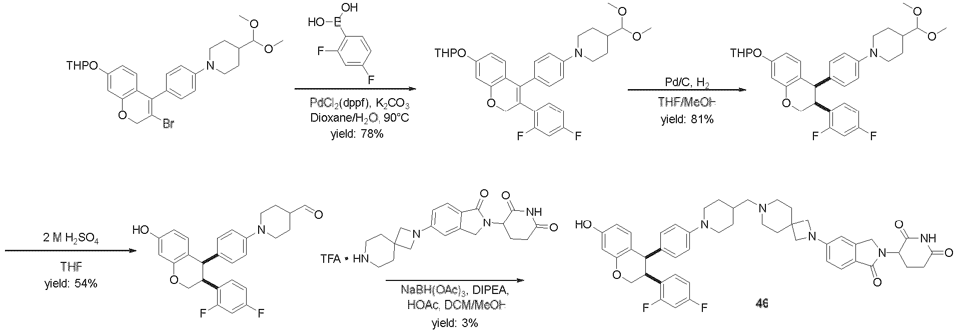

### Step 1: Synthesis of 1-(4-(3-(2,4-difluorophenyl)-7-((tetrahydro-2H-pyran-2-yl)oxy)-2H-chromene-4-yl)phenyl)-4-(dimethoxymethyl)piperidine

To a reaction flask, were added 1-(4-(3-bromo-7-((tetrahydro-2H-pyran-2-yl)oxy)-2*H*-chromene-4-yl)phenyl)-4-(dimethoxymethyl)piperidine (120mg, 0.22 mmol), 2,4-diflurorphenylboronic acid (70 mg, 0.44 mmol), Pd(dppf)Cl₂ (16 mg, 0.02 mmol), potassium carbonate (76 mg, 0.66 mmol), and dioxane (8 mL), and then the system was purged with nitrogen. The reaction was allowed to react overnight at 95 °C. TLC detection indicated completion of the reaction. The reaction solution was cooled to room temperature, and filtered via diatomaceous earth. The filtrate was concentrated, and the residue was subjected to column chromatography, to obtain 100 mg of product, which was determined to be the target compound by LC-MS, with a yield of 78%. LC/MS (ESI⁺) calcd for C₃₄H₃₇F₂NO₅ (M+H⁺) *m*/*z*, 578.2; found, 578.2.

### Step 2: Synthesis of 1-(4-(cis-3-(2,4-difluorophenyl)-7-((tetrahydro-2H-pyran-2-yl)oxy) chroman-4-yl)phenyl)-4-(dimethoxymethyl)piperidine

1-(4-(3-(2,4-difluorophenyl)-7-((tetrahydro-2*H*-pyran-2-yl)oxy)-2*H*-chromene-4-yl)phenyl )-4-(dimethoxymethyl)piperidine (100 mg, 0.17 mmol) was dissolved in THF (25 mL), to which was added methanol (25mL), followed by addition of 10% Pd/C (20 mg, 20%), and then the system was purged with hydrogen. The reaction was allowed to react overnight at room temperature. TLC detection indicated completion of the reaction. The reaction solution was filtered via diatomaceous earth to remove Pd/C. The filtrate was concentrated, and the residue was subjected to column chromatography, to obtain 80 mg of product, which was determined to be the target compound by LC-MS, with a yield of 81%. LC/MS (ESI+) calcd for C₃₄H₃₉F₂NO₅ (M + H⁺) *m*/*z*, 580.2; found, 580.2.

### Step 3: Synthesis of 1-(4-(cis-3-(2,4-difluorophenyl)-7-hydroxychroman-4-yl)phenyl) piperidine-4-carbaldehyde

1-(4-(*cis*-3-(2,4-difluorophenyl)-7-((tetrahydro-2*H*-pyran-2-yl)oxy)chroman-4-yl)phenyl)-4-(dimethoxymethyl)piperidine (80 mg, 0.14 mmol) was dissolved in THF (4 mL), to which was added sulfuric acid (1 mL, 2 M), and then the mixture was allowed to react for 0.5 h at 60 °C. MS indicated the disappearance of starting material. The reaction solution was cooled to room temperature, and adjusted to be about pH 8 with saturated NaHCO₃ solution. The resultant solution was extracted with ethyl acetate. The organic phase was washed with saturated NaCl solution, dried, and concentrated. The residue was subjected to column chromatography, to obtain 42 mg of crude product, which was directly used in the next step, with a yield of 54%. LC/MS (ESI+) calcd for C₂₇H₂₅F₂NO₃ (M + H⁺) *m*/*z*, 450.5; found, 450.5.

### Step 4: Synthesis of 3-(5-(7-((1-(4-(cis-3-(2,4-difluorophenyl)-7-hydroxychroman -4-yl)phenyl)piperidin-4-yl)methyl)-2,7-diazaspiro[3.5]nonane-2-yl)-1-oxoisoinodolin-2-yl)pip eridin-2,6-dione

3-(1-oxo-5-(2,7-diazaspiro[3.5]nonane-2-yl)isoinodolin-2-yl)piperidin-2,6-dione trifluoroacetate (50 mg, 0.1 mmol) was dissolved in dichloromethane/methanol (2 mL, 10: 1), to which was added DIPEA (40 mg, 0.3 mmol), and then stirred at room temperature for 5 min, followed by addition of 1-(4-(*cis*-3-(2,4-difluorophenyl)-7-hydroxychroman-4-yl)phenyl) piperidine-4-carbaldehyde (40 mg, 0.09 mmol). Then, glacial acetic acid (22 mg, 0.3 mmol) was added, and the reaction was stirred for 0.5 h at room temperature, followed by addition of sodium triacetoxyborohydride (42 mg, 0.2 mmol). The mixture was allowed to react overnight. The reaction was monitored by TLC until the raw materials disappeared, and then quenched by adding saturated NaHCO₃ solution. The resultant solution was extracted with dichloromethane. The organic phase was dried and concentrated, and the residue was subjected to column chromatography to obtain 2 mg of product, which was determined to be the target compound by LC-MS, with a yield of 3%. LC/MS (ESI+) calcd for C₄₇H₄₉F₂N₅O₅ (M + H⁺) *m*/*z*, 802.3; found, 802.3.

### Example 47: 3-(5-(7-((1-(4-(cis-3-(2,5-difluorophenyl)-7-hydroxychroman-4-yl)phenyl) piperidin-4-yl)methyl)-2,7-diazaspiro[3.5]nonane-2-yl)-1-oxoisoinodolin-2-yl)piperidin-2,6 -dione

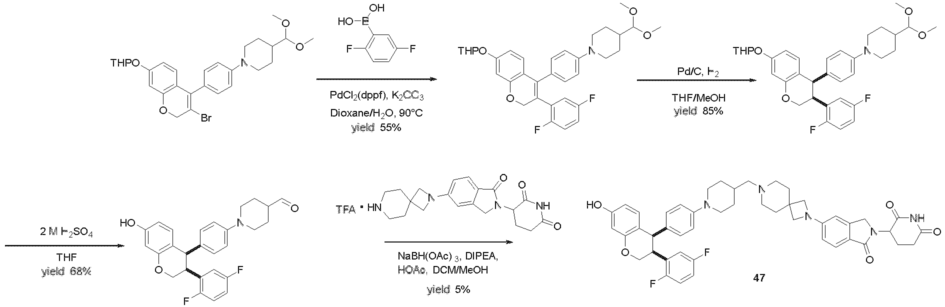

### Step 1: Synthesis of 1-(4-(3-(2,5-difluorophenyl)-7-((tetrahydro-2H-pyran-2-yl)oxy)-2H-chromene-4-yl)-2-fluorophenyl)-4-(dimethoxymethyl)piperidine

The target compound was prepared using a method similar to that in Example 10. LC/MS (ESI+) calcd for C₃₄H₃₇F₂NO₅ (M + H⁺) *m*/*z*, 578.2; found, 578.2.

### Step 2: Synthesis of 1-(4-(3-(2,5-difluorophenyl)-7-((tetrahydro-2H-pyran-2-yl)oxy) chroman-4-yl)phenyl)-4-(dimethoxymethyl)piperidine

The target compound was prepared using a method similar to that in Example 10. LC/MS (ESI+) calcd for C₃₄H₃₉F₂NO₅ (M + H⁺) m/z, 580.2; found, 580.2.

### Step 3: Synthesis of 1-(4-(3-(2,5-difluorophenyl)-7-hydroxychroman-4-yl)phenyl) piperidine-4-carbaldehyde

The target compound was prepared using a method similar to that in Example 10. LC/MS (ESI+) calcd for C₂₇H₂₅F₂NO₃ (M + H⁺) m/z, 450.5; found, 450.5.

### Step 4: Synthesis of 3-(5-(7-((1-(4-(cis-3-(2,5-difluorophenyl)-7-hydroxychroman-4-yl) phenyl)piperidin-4-yl)methyl)-2,7-diazaspiro[3.5]nonane-2-yl)-1-oxoisoinodolin-2-yl)piperidin -2,6-dione

The target compound was prepared using a method similar to that in Example 10. LC/MS (ESI+) calcd for C₄₇H₄₉F₂N₅O₅ (M + H⁺) *m*/*z*, 802.3; found, 802.3.

### Example 48: 3-(5-(7-((1-(2,6-difluoro-4-(cis-3-(2,3-difluorophenyl)-7-hydroxychroman-4-yl)phenyl)piperidin-4-yl)methyl)-2,7-diazaspiro[3.5]nonane-2-yl)-1-ox oisoinodolin-2-yl)piperidin-2,6-dione

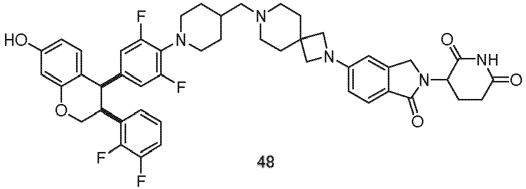

The target compound was prepared using a method similar to that in Example 10. LC/MS (ESI+) calcd for C₄₇H₄₇F₄N₅O₅ (M + H⁺) *m*/*z*, 838.3; found, 838.3. ¹H NMR (400 MHz, DMSO-*d*₆) δ 10.94 (s, 1H), 9.46 (s, 1H), 7.47 (d, *J =* 8.3 Hz, 1H), 7.35-7.24 (m, 1H), 7.03-6.93 (m, 1H), 6.73-6.66 (m, 1H), 6.53-6.42 (m, 3H), 6.33 (d, *J =* 7.3 Hz, 2H), 6.20 (d, *J* = 10.7 Hz, 2H), 5.03 (dd, *J =* 13.3, 5.1 Hz, 1H), 4.42-4.20 (m, 4H), 4.16 (d, *J =* 16.9 Hz, 1H), 3.80 (ddd, *J* = 9.7, 5.6, 3.4 Hz, 1H), 3.62 (s, 4H), 3.03 (d, *J* = 11.3 Hz, 2H), 2.89 (td, *J* = 13.4, 5.5 Hz, 3H), 2.62-2.53 (m, 1H), 2.38-2.28 (m, 3H), 2.13 (d, *J* = 6.7 Hz, 2H), 1.97 (d, *J* = 13.4 Hz, 1H), 1.77-1.65 (m, 6H), 1.60 (s, 1H), 1.22 (s, 2H).

### Example 49: 3-(5-(7-((1-(4-((3S, 4R)-3-(2,3-difluorophenyl)-7-hydroxychroman-4-yl)-2,6-difluorophenyl)piperidin-4-yl)methyl)-2,7-diazaspiro[3.5]nonane-2-yl)-1-oxoisoin odolin-2-yl)piperidin-2,6-one

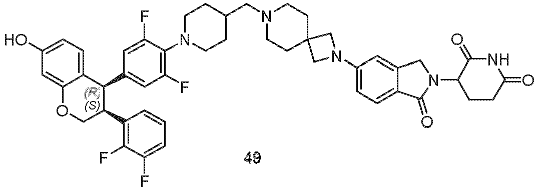

The target compound was prepared using a method similar to that in Example 16. LC/MS (ESI+) calcd for C₄₇H₄₇F₄N₅O₅ (M + H⁺) *m*/*z*, 838.3; found, 838.3. ¹H NMR (400 MHz, DMSO-*d*₆) *δ* 10.94 (s, 1H), 9.46 (s, 1H), 7.47 (d, *J* = 8.3 Hz, 1H), 7.35-7.24 (m, 1H), 7.03-6.93 (m, 1H), 6.73-6.66 (m, 1H), 6.53-6.42 (m, 3H), 6.33 (d, *J =* 7.3 Hz, 2H), 6.20 (d, *J* = 10.7 Hz, 2H), 5.03 (dd, *J =* 13.3, 5.1 Hz, 1H), 4.42-4.20 (m, 4H), 4.16 (d, *J* = 16.9 Hz, 1H), 3.80 (ddd, *J* = 9.7, 5.6, 3.4 Hz, 1H), 3.62 (s, 4H), 3.03 (d, *J* = 11.3 Hz, 2H), 2.89 (td, *J* = 13.4, 5.5 Hz, 3H), 2.62-2.53 (m, 1H), 2.38-2.28 (m, 3H), 2.13 (d, *J* = 6.7 Hz, 2H), 1.97 (d, *J* = 13.4 Hz, 1H), 1.77-1.65 (m, 6H), 1.60 (s, 1H), 1.22 (s, 2H).

### Example 50: 3-(5-(7-((1-(4-((3S,4R)-3-(2-fluorophenyl)-7-hydroxychroman-4-yl)-2,6-difluorophenyl)piperidin-4-yl)methyl)-2,7-diazaspiro[3.5]nonane-2-yl)-1-oxoisoinodolin-2-yl)piperidin-2,6-one

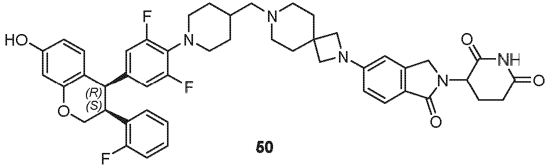

The target compound was prepared using a method similar to that in Example 16. LC/MS (ESI+) calcd for C₄₇H₄₈F₃N₅O₅ (M + H⁺) *m*/*z*, 820.3.; found, 820.3. ¹H NMR (400 MHz, DMSO-*d*₆) *δ* 10.95 (s, 1H), 9.45 (s, 1H), 7.48 (d, *J =* 8.2 Hz, 1H), 7.33 - 7.23 (m, 1H), 7.17 (ddd, *J =* 9.8, 8.4, 1.3 Hz, 1H), 6.97 (td, *J* = 7.6, 1.3 Hz, 1H), 6.70 (d, *J* = 8.0 Hz, 1H), 6.60 (td, *J* = 7.8, 1.7 Hz, 1H), 6.53 - 6.42 (m, 2H), 6.33 (d, *J* = 7.8 Hz, 2H), 6.13 (d, *J* = 10.6 Hz, 2H), 5.03 (dd, *J =* 13.2, 5.1 Hz, 1H), 4.42 - 4.22 (m, 4H), 4.17 (d, *J* = 16.9 Hz, 1H), 3.76 (dt, *J* = 10.2, 4.4 Hz, 1H), 3.63 (s, 4H), 3.03 (d, *J* = 11.1 Hz, 2H), 2.90 (q, *J* = 12.1, 9.8 Hz, 3H), 2.63 - 2.53 (m, 1H), 2.42 - 2.18 (m, 4H), 2.13 (d, *J =* 14.2 Hz, 2H), 1.98 - 1.92 (m, 1H), 1.70 (d, *J =* 19.0 Hz, 7H), 1.18 (d, *J* = 7.1 Hz, 2H).

### Example 51: 3-(5-(7-((1-(4-(cis-7-(benzyloxy)-3-(2-fluorophenyl)chroman-4-yl)-2,6-difluorophenyl)piperidin-4-yl)methyl)-2,7-diazaspiro[3.5]nonane-2-yl)-1-oxoisoinodoli n-2-yl)piperidin-2,6-one

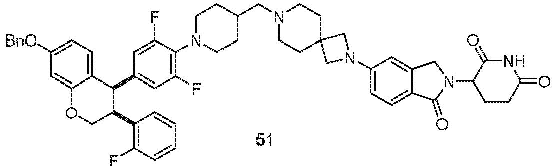

The target compound was prepared using a method similar to that in Example 10. LC/MS (ESI+) calcd for C₅₄H₅₄F₃N₅O₅ (M + H⁺) *m*/*z*, 910.4; found, 910.4. ¹H NMR (400 MHz, DMSO-*d*₆) *δ* 10.95 (s, 1H), 7.51-7.41 (m, 4H), 7.41-7.31 (m, 3H), 7.27 (dtd, *J* = 7.3, 5.9, 5.0, 1.7 Hz, 1H), 7.22-7.14 (m, 1H), 7.14-7.02 (m, 1H), 6.71-6.65 (m, 1H), 6.65-6.59 (m, 2H), 6.59-6.54 (m, 1H), 6.52-6.43 (m, 2H), 5.10 (d, *J* = 8.4 Hz, 2H), 5.03 (dd, *J* = 13.2, 5.1 Hz, 1H), 4.46-4.21 (m, 4H), 4.16 (d, *J* = 16.9 Hz, 1H), 3.78 (dt, *J* = 10.6, 4.6 Hz, 1H), 3.63 (s, 4H), 3.02 (d, *J* = 11.4 Hz, 2H), 2.94 (d, *J=* 5.4 Hz, 3H), 2.92-2.84 (m, 2H), 2.62-2.53 (m, 1H), 2.34 (dd, *J* = 18.2, 9.5 Hz, 3H), 2.12 (s, 2H), 2.03-1.92 (m, 2H), 1.72 (d, *J =* 13.5 Hz, 6H), 1.60 (s, 1H) 1.33 (s, 2H).

### Example 52: 3-(5-(7-((1-(4-(cis-7-(benzyloxy)-3-(2-fluorophenyl)chroman-4-yl)-2,6-difluorophenyl)piperidin-4-yl)methyl)-2,7-diazaspiro[3.5]nonane-2-yl)-1-oxoisoinodolin-2-yl)piperidin-2,6-one

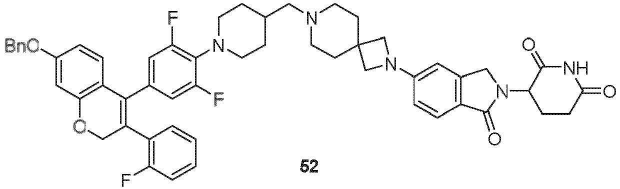

The target compound was prepared using a method similar to that in Example 42. LC/MS (ESI+) calcd for C₅₄H₅₂F₃N₅O₅ (M + H⁺) *m*/*z*, 908.3; found, 908.3. ¹H NMR (400 MHz, DMSO-*d*₆) *δ* 10.95 (s, 1H), 7.50-7.41 (m, 4H), 7.40-7.32 (m, 3H), 7.28 (dddd, *J =* 12.5, 7.9, 5.5, 3.0 Hz, 1H), 7.20-7.11 (m, 1H), 7.08 (td, *J* = 7.3, 5.0 Hz, 2H), 6.69 (d, *J* = 9.0 Hz, 3H), 6.63 (d, *J* = 2.5 Hz, 1H), 6.59 (dd, *J* = 8.6, 2.5 Hz, 1H), 6.53-6.43 (m, 2H), 5.11 (s, 2H), 5.03 (dd, *J* = 13.3, 5.1 Hz, 1H), 4.29 (d, *J =* 17.0 Hz, 1H), 4.16 (d, *J* = 16.9 Hz, 1H), 3.62 (s, 4H), 3.14 (d, *J* = 11.3 Hz, 2H), 3.03-2.83 (m, 3H), 2.63-2.53 (m, 1H), 2.35-2.24 (m, 3H), 2.16 (d, *J* = 15.0 Hz, 2H), 2.02-1.91 (m, 2H), 1.72 (d, *J =* 17.4 Hz, 6H), 1.64 (s, 1H), 1.22 (s, 2H).

### Example 53. 2-(2,6-dioxopiperidin-3-yl)-5-(7-((1-(4-((1R,2S)-6-hydroxyl-2- phenyl-1,2,3,4-tetrahydronaphthalen-1-yl)phenyl)piperidin-4-yl)methyl)-2,7-diazaspiro[4.4]nonan e-2-yl)isoinodolin-1,3-dione

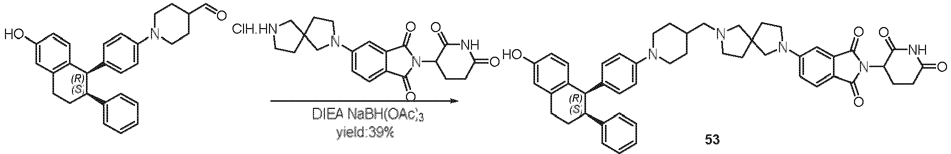

2-(2,6-dioxopiperidin-3-yl)-5-(2,7-diazaspiro[4.4]nonane-2-yl)isoinodolin-1,3-dione hydrochloride (36 mg, 0.075 mmol) was dissolved in 5 mL mixed solvent of dichloromethane/methanol = 5:1, to which was added *N,N*-diisopropylethylamine (13 mg, 0.1 mmol), and then stirred at room temperature for 10 min, followed by addition of 1-(4-((1*R*,2*S*)-6-hydroxyl-2-phenyl-1,2,3,4-tetrahydronaphthalen-1-yl)phenyl)piperidine-4-carb aldehyde (20 mg, 0.05 mmol) and glacial acetic acid (6 mg, 0.1 mmol). The reaction was stirred at room temperature for 10 min, and then sodium triacetoxyborohydride (21 mg, 0.1 mmol) was added. The reaction solution was stirred for 3 h at room temperature and washed with water. The resultant solution was extracted with dichloromethane. The organic phase was dried and concentrated under reduced pressure, and the residue was separated and purified by TLC, to obtain the product 2-(2,6-dioxopiperidin-3-yl)-5-(7-((1-(4-((1*R*,2*S*)-6-hydroxyl-2-phenyl-1,2,3,4-tetrahydronaphthalen-1-yl)phenyl)piperidin-4-yl)methyl)-2,7-diazaspiro[4.4]nonane-2-y l)isoinodolin-1,3-dione (15 mg), with a yield of 39%. LC/MS (ESI+) calcd for C₄₈H₅₁N₅O₅ ([M+H]⁺) *m*/*z* 778.4, found 778.4; ¹H NMR (400 MHz, DMSO-*d*₆) *δ* 7.63 (d, *J* = 8.4 Hz, 1H), 7.13 (qd, *J* = 7.4, 6.1, 2.9 Hz, 3H), 6.88 (d, *J* = 2.2 Hz, 1H), 6.81 (ddd, *J* = 12.5, 8.3, 2.1 Hz, 3H), 6.63 (d, *J* = 8.4 Hz, 1H), 6.59 (d, *J* = 2.6 Hz, 1H), 6.51 (d, *J* = 8.8 Hz, 2H), 6.47 (dd, *J* = 8.2, 2.6 Hz, 1H), 6.18 (d, *J* = 8.4 Hz, 2H), 5.05 (dd, *J* = 12.9, 5.4 Hz, 1H), 4.11 (d, *J* = 5.0 Hz, 1H), 3.26 (s, 2H), 3.01 - 2.84 (m, 4H), 2.65 - 2.56 (m, 2H), 2.56 - 2.51 (m, 2H), 2.47 - 2.38 (m, 3H), 2.28 - 2.21 (m, 2H), 2.15 - 2.07 (m, 1H), 2.06 - 1.88 (m, 4H), 1.79 - 1.67 (m, 5H), 1.49 (s, 1H), 1.23 (d, *J =* 3.5 Hz, 1H), 1.13 (d, *J =* 12.2 Hz, 2H).

### Example 54. 2-(2,6-dioxopiperidin-3-yl)-5-(6-((1-(4-((1R,2S)-6-hydroxyl-2-phenyl-1,2,3,4-tetrahydronaphthalen-1-yl)phenyl)piperidin-4-yl)methyl)-2,6-diazaspiro[3.3]hepta n-2-yl)isoinodolin-1,3-dione

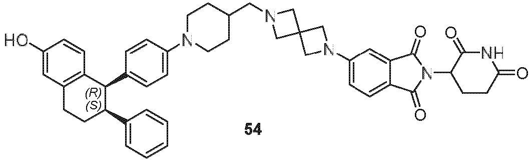

The target compound was prepared using a method similar to that in Example 1. LC/MS (ESI+) calcd for C₄₈H₅₁N₅O₅ ([M+H]⁺) *m*/*z* 750.4, found 750.4. ¹H NMR (400 MHz, DMSO-*d*₆) *δ* 10.94 (s, 1H), 9.12 (s, 1H), 7.48 (d, *J* = 8.3 Hz, 1H), 7.17 - 7.08 (m, 3H), 6.86 - 6.79 (m, 2H), 6.64 (d, *J* = 8.4 Hz, 1H), 6.59 (d, *J* = 2.5 Hz, 1H), 6.55 - 6.49 (m, 3H), 6.47 (ddd, *J* = 7.4, 4.8, 2.3 Hz, 2H), 6.19 (d, *J* = 8.3 Hz, 2H), 5.02 (dd, *J=* 13.3, 5.1 Hz, 1H), 4.29 (d, *J* = 17.0 Hz, 1H), 4.16 (d, *J* = 17.0 Hz, 1H), 4.12 (d, *J* = 5.0 Hz, 1H), 3.95 (d, *J* = 4.7 Hz, 4H), 3.50 - 3.45 (m, 2H), 3.28 (s, 4H), 3.01 - 2.83 (m, 3H), 2.62 - 2.53 (m, 1H), 2.48 - 2.38 (m, 2H), 2.34 (dd, *J* = 13.2, 4.5 Hz, 1H), 2.24 (d, *J =* 6.7 Hz, 2H), 2.14 - 2.06 (m, 1H), 1.99 - 1.90 (m, 1H), 1.67 (d, *J* = 12.1 Hz, 3H), 1.32 (d, *J =* 9.5 Hz, 1H).

### Example 55. 2-(2,6-dioxopiperidin-3-yl)-5-(3-(4-(4-((1R,2S)-6-hydroxyl-2-phenyl-1,2,3,4-tetrahydronaphthalen-1-yl)phenyl)piperazine-1-yl)azetidin-1-yl)isoinodolin-1,3-dio ne

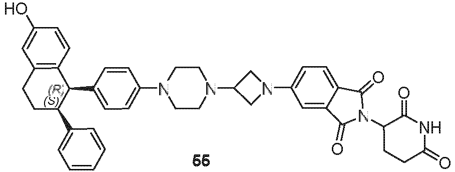

The target compound was prepared using a method similar to that in Example 1. LC/MS (ESI+) calcd for C₄₂H₄₁N₅O₅ ([M+H]⁺) *m*/*z* 696.3, found 696.3; ¹H NMR (400 MHz, DMSO-*d*₆) *δ* 11.03 (s, 1H), 9.12 (s, 1H), 7.64 (d, *J* = 8.3 Hz, 1H), 7.19 - 7.08 (m, 3H), 6.88 - 6.81 (m, 2H), 6.79 (d, *J* = 2.1 Hz, 1H), 6.68 - 6.61 (m, 2H), 6.60 (d, *J =* 2.5 Hz, 1H), 6.55 (d, *J =* 8.6 Hz, 2H), 6.47 (dd, *J* = 8.3, 2.6 Hz, 1H), 6.21 (d, *J* = 8.5 Hz, 2H), 5.05 (dd, *J* = 12.9, 5.4 Hz, 1H), 4.18 - 4.05 (m, 3H), 3.87 (dd, *J* = 8.9, 4.9 Hz, 2H), 3.31 - 3.23 (m, 2H), 3.01 (t, *J* = 4.9 Hz, 4H), 2.98 - 2.81 (m, 3H), 2.62 - 2.55 (m, 1H), 2.54 (d, *J* = 3.0 Hz, 1H), 2.46 (d, *J* = 4.9 Hz, 4H), 2.18 - 2.05 (m, 1H), 2.05 - 1.96 (m, 1H), 1.70 (s, 1H).

### Example 56. 3-(5-(6-((1-(4-((3R,4S)-7-hydroxyl-3-phenyl-chroman-4-yl)phenyl) piperidin-4-yl)methyl)-2,6-diazaspiro[3.3]heptane-2-yl)-1-oxoisoinodolin-2-yl)piperidin-2, 6-one

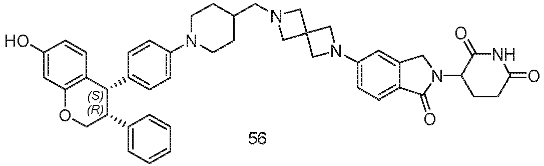

The target compound was prepared using a method similar to that in Example 24. LC/MS (ESI+) calcd for C₄₅H₄₇N₅O₅ ([M+H]⁺) *m*/*z* 738.4, found 738.4. ¹H NMR (400 MHz, DMSO-*d*₆) *δ* 10.94 (s, 1H), 9.32 (s, 1H), 7.48 (d, *J* = 8.3 Hz, 1H), 7.14 (p, *J* = 3.7 Hz, 3H), 6.75 (dd, *J* = 6.6, 3.0 Hz, 2H), 6.65 (d, *J* = 8.3 Hz, 1H), 6.59 (d, *J* = 8.5 Hz, 2H), 6.51 (d, *J =* 1.9 Hz, 1H), 6.47 (dd, *J* = 8.3, 2.0 Hz, 1H), 6.37 (d, *J* = 8.4 Hz, 2H), 6.30 (d, *J* = 2.5 Hz, 1H), 6.27 (dd, *J* = 8.2, 2.4 Hz, 1H), 5.02 (dd, *J* = 13.3, 5.1 Hz, 1H), 4.36 - 4.25 (m, 2H), 4.16 (dd, *J* = 11.0, 6.1 Hz, 3H), 3.98 (s, 4H), 3.54 - 3.49 (m, 6H), 3.03 (s, 1H), 2.96 - 2.85 (m, 2H), 2.57 (d, *J* = 23.6 Hz, 2H), 2.45 (s, 2H), 2.40 - 2.28 (m, 2H), 2.00 - 1.92 (m, 1H), 1.68 (d, *J* = 12.5 Hz, 2H), 1.39 (s, 1H), 1.22 (m, *J* = 3.2 Hz, 2H).

### Example 57. 3-(5-(6-((1-(4-((3S,4R)-7-hydroxyl-3-phenyl-chroman-4-yl)phenyl) piperidin-4-yl)methyl)-2,6-diazaspiro[3.3]heptan-2-yl)-1-oxoisoinodolin-2-yl)piperidin-2,6-one

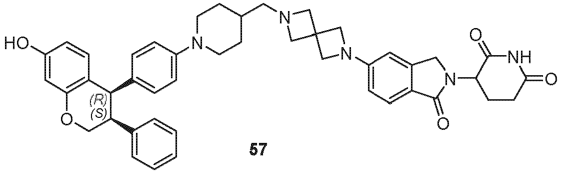

The target compound was prepared using a method similar to that in Example 16. LC/MS (ESI+) calcd for C₄₅H₄₇N₅O₅ ([M+H]⁺) *m*/*z* 738.4, found 738.4. ¹H NMR (400 MHz, DMSO-*d*₆) *δ* 10.94 (s, 1H), 9.29 (s, 1H), 7.48 (d, *J* = 8.3 Hz, 1H), 7.14 (p, *J* = 3.5 Hz, 3H), 6.75 (dd, *J* = 6.6, 3.0 Hz, 2H), 6.66 (d, *J* = 8.3 Hz, 1H), 6.59 (d, *J* = 8.7 Hz, 2H), 6.51 (d, *J =* 1.9 Hz, 1H), 6.46 (dd, *J* = 8.3, 2.0 Hz, 1H), 6.37 (d, *J* = 8.5 Hz, 2H), 6.30 (d, *J* = 2.4 Hz, 1H), 6.27 (dd, *J* = 8.2, 2.4 Hz, 1H), 5.03 (dd, *J* = 13.3, 5.1 Hz, 1H), 4.39 - 4.25 (m, 2H), 4.16 (dd, *J* = 11.1, 6.1 Hz, 3H), 3.96 (s, 4H), 3.57 - 3.46 (m, 3H), 3.28 (s, 4H), 2.89 (ddd, *J =* 17.5, 13.7, 5.7 Hz, 1H), 2.64 - 2.54 (m, 1H), 2.46 (d, *J* = 11.3 Hz, 1H), 2.34 (dd, *J* = 13.1, 4.5 Hz, 1H), 2.25 (d, *J* = 6.7 Hz, 2H), 1.99 - 1.90 (m, 1H), 1.69 (d, *J* = 12.4 Hz, 2H), 1.34 (s, 1H), 1.23 (s, 1H), 1.20 - 1.08 (m, 2H).

### Example 58. 3-(5-(2-((1-(4-((3S,4R)-7-hydroxyl-3-phenyl-chroman-4-yl)phenyl) piperidin-4-yl)methyl)-2,7-diazaspiro[3.5]nonan-7-yl)-1-oxoisoinodolin-2-yl)piperidin-2,6-dione

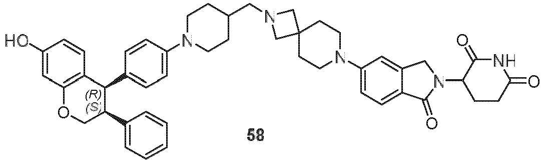

The target compound was prepared using a method similar to that in Example 16. LC/MS (ESI+) calcd for C₄₇H₅₁N₅O₅ ([M+H]⁺) *m*/*z* 766.4, found 766.4. ¹H NMR (400 MHz, DMSO-*d*₆) *δ* 10.96 (s, 1H), 9.31 (s, 1H), 7.49 (d, *J* = 8.4 Hz, 1H), 7.18 - 7.10 (m, 3H), 7.04 (d, *J* = 9.0 Hz, 2H), 6.75 (dd, *J* = 6.6, 3.0 Hz, 2H), 6.65 (d, *J* = 8.3 Hz, 1H), 6.59 (d, *J* = 8.8 Hz, 2H), 6.36 (d, *J* = 8.5 Hz, 2H), 6.30 (d, *J* = 2.4 Hz, 1H), 6.27 (dd, *J* = 8.2, 2.4 Hz, 1H), 5.03 (dd, *J =* 13.3, 5.1 Hz, 1H), 4.37 - 4.26 (m, 2H), 4.23 - 4.12 (m, 3H), 3.52 (d, *J =* 3.8 Hz, 2H), 3.28 - 3.24 (m, 4H), 2.94 (s, 4H), 2.91 - 2.84 (m, 1H), 2.62 - 2.53 (m, 1H), 2.45 (d, *J* = 12.2 Hz, 1H), 2.39 - 2.32 (m, 1H), 2.28 (d, *J* = 6.8 Hz, 2H), 1.99 - 1.91 (m, 1H), 1.78 - 1.62 (m, 6H), 1.33 (s, 1H), 1.23 (d, *J=* 3.7 Hz, 2H), 1.20 - 1.08 (m, 2H).

### Example 59. 3-(5-(3-(4-(4-((1R,2S)-6-hydroxyl-2-phenyl-1,2,3,4-tetrahydronaphthalen-1-yl)phenyl)piperazine-1-yl)azetidin-1-yl)-1-oxoisoindol-2-yl)piperi din-2,6-dione

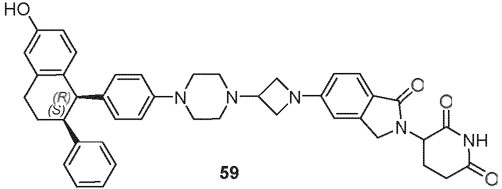

The target compound was prepared using a method similar to that in Example 19. LC/MS (ESI+) calcd for C₄₂H₄₃N₅O₄ ([M+H]⁺) *m*/*z* 682.3, found 682.3. ¹H NMR (400 MHz, DMSO-*d*₆) *δ* 10.94 (s, 1H), 9.12 (s, 1H), 7.49 (d, *J =* 8.3 Hz, 1H), 7.18 - 7.09 (m, 3H), 6.87 - 6.81 (m, 2H), 6.63 (d, *J* = 8.4 Hz, 1H), 6.60 (d, *J* = 2.5 Hz, 1H), 6.58 - 6.51 (m, 3H), 6.48 (ddd, *J* = 8.3, 4.5, 2.3 Hz, 2H), 6.21 (d, *J* = 8.5 Hz, 2H), 5.03 (dd, *J* = 13.3, 5.1 Hz, 1H), 4.30 (d, *J* = 16.9 Hz, 1H), 4.17 (d, *J* = 16.9 Hz, 1H), 4.13 (d, *J* = 4.9 Hz, 1H), 4.01 (t, *J* = 7.4 Hz, 2H), 3.73 (d, *J* = 6.8 Hz, 2H), 3.27 (s, 2H), 3.00 (s, 4H), 2.98 - 2.82 (m, 3H), 2.58 (d, *J* = 14.3 Hz, 1H), 2.43 (d, *J* = 9.6 Hz, 4H), 2.34 (dd, *J* = 13.7, 4.3 Hz, 1H), 2.09 (dd, *J* = 12.4, 6.3 Hz, 1H), 2.00 - 1.93 (m, 1H), 1.70 (d, *J* = 12.1 Hz, 1H).

### Example 60. 3-(5-(7-((1-(4-((3,4-cis)-3-(3-fluorophenyl)-7-hydroxyl-chroman-4-yl) phenyl)piperidin-4-yl)methyl)-2,7-diazaspiro[3.5]nonane-2-yl)-1-oxoisoindol-2-yl)piperidi n-2,6-dione

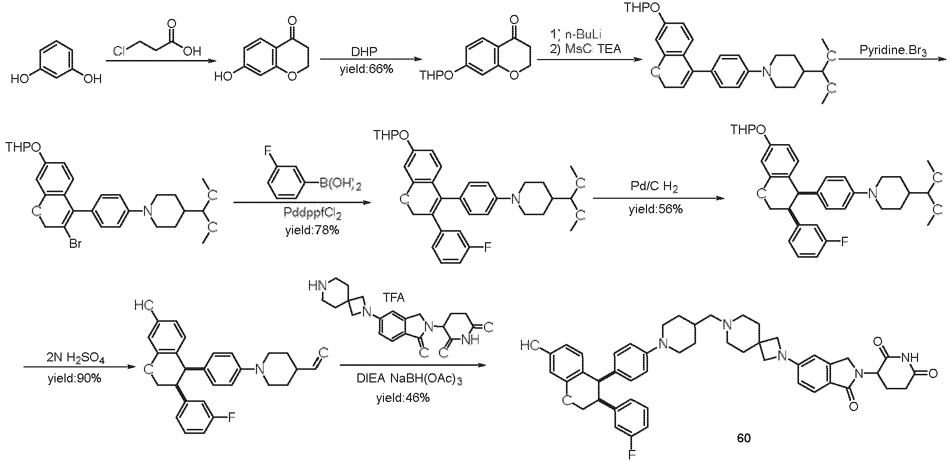

### Step 1: Synthesis of 7-hydroxychroman-4-one

1,3-benzenediol (80 g, 0.73 mmol) and 3-chloropropionic acid (86.7 g, 0.80 mmol) were added into a round-bottom flask, to which was added 68 mL of trifluoromethanesulfonic acid, and then stirred for 1 h at 80 °C. The reaction solution was cooled to room temperature, and then poured into 200 mL of ice water, followed by extracting with 300 mL of dichloromethane. The aqueous layer was further extracted with dichloromethane for three times. The organic layers were combined and concentrated under reduced pressure to dry. To the residue, was slowly added 25% NaOH aqueous solution (800 mL) in an ice-water bath. After addition, the mixture was allowed to react for 1 h under the conditions of keeping the temperature. The reaction solution was adjusted to be about pH 4 with 6N HCl solution, and then extracted with 600 mL of ethyl acetate. The aqueous layer was further extracted twice with ethyl acetate. The organic layers were combined, dried over anhydrous Na₂SO₄, and concentrated to dry under reduced pressure, to obtain the product 7-hydroxychroman-4-one (90 g), which was directly used in the next step.

### Step 2: Synthesis of 7-((tetrahydro-2H-pyran-2-yl)oxy)chroman-4-one

The crude product 7-hydroxychroman-4-one (90 g, 0.55 mol) obtained in the previous step was dissolved in 700 mL of dichloromethane, to which were added DHP (134 g, 1.59 mol) and PPTS (20 g, 0.08 mol), and then stirred overnight at room temperature, followed by addition of solid potassium carbonate (13.8 g, 0.1 mol). The reaction was stirred at room temperature for 0.5 h, and filtered. The filtrate was concentrated to dry under reduced pressure. The residue was separated and purified by column chromatography, to obtain the product 7-((tetrahydro-2H-pyran-2-yl)oxy)chroman-4-one (90 g), with a yield of 66%.

### Step 3: Synthesis of 4-(dimethoxymethyl)-1-(4-(7-((tetrahydro-2H-pyran-2-yl)oxy)-2H-chromene-4-yl)phenyl)piperidine

1-(4-bromophenyl)-4-(dimethoxymethyl)piperidine (8.0 g, 25.5 mmol) was dissolved in 80 mL of anhydrous tetrahydrofuran, and cooled to -78°C in a dry ice-acetone bath, to which was slowly added 1.6Nn-BuLi solution (21 mL). After addition, the temperature of the system was kept for 1.5 h. 7-((tetrahydro-2H-pyran-2-yl)oxy)chroman-4-one (6.34 g, 25.5 mmol) was dissolved in anhydrous tetrahydrofuran, and then added to the reaction system dropwise. After addition, the temperature of the system was kept for 2 h. Then, the reaction was slowly warmed to about 0 °C, and quenched by adding water. The resultant solution was washed once with saturated NaCl solution, dried over anhydrous Na₂SO₄, and concentrated to dry under reduced pressure. The residue was separated and purified by column chromatography, to obtain the product (6.48 g), which was dissolved in 100 mL of dichloromethane, followed by addition of triethylamine (4.0 g, 39.7 mmol). Methanesulfonyl chloride (1.82 g, 15.9 mmol) was added in an ice-water bath, and then the ice-water bath was removed. The reaction was allowed to react at room temperature for 1 h. TLC detection indicated completion of the reaction. The reaction solution was washed with water, dried over anhydrous Na₂SO₄, and concentrated to dry under reduced pressure. The residue was separated and purified by column chromatography, to obtain the product 4-(dimethoxymethyl)-1-(4-(7-((tetrahydro-2H-pyran-2-yl)oxy)-2H-chromene-4-yl) phenyl)piperidine (4.87 g), with a yield of 41%. LC/MS (ESI+) calcd for C₂₈H₃₅NO₅ ([M+H]⁺) *m*/*z* 466.2, found 466.2.

### Step 4: Synthesis of 1-(4-(3-bromo-7-((tetrahydro-2H-pyran-2-yl)oxy)-2H-chromene-4-yl) phenyl)-4-(dimethoxymethyl)piperidine

4-(dimethoxymethyl)-1-(4-(7-((tetrahydro-2H-pyran-2-yl)oxy)-2H-toluene-4-yl)phenyl) piperidine (2.33 g, 5.0 mmol) was dissolved in 15 mL of DMF, to which was added N,N-diisopropylethylamine (1.29 g, 10.0 mmol), and then tribromopyridine (1.76 g, 5.5 mmol) was added in an ice-water bath. The reaction was allowed to react for 20 min under the conditions of not changing the temperature, followed by addition of 35 mL water. The resultant solution was extracted with 50 mL of ethyl acetate. The aqueous layer was further extracted once with 40 mL of ethyl acetate. The organic layers were combined, washed once with saturated NaCl solution, dried over anhydrous Na₂SO₄, and concentrated to dry under reduced pressure. The residue was separated and purified by column chromatography, to obtain the product 1-(4-(3-bromo-7-((tetrahydro-2H-pyran-2-yl)oxy)-2H-chromene-4-yl)phenyl)-4-(dimethoxymethyl)piperidine (2.36 g), with a yield of 87%.

### Step 5: Synthesis of 4-(dimethoxymethyl)-1-(4-(3-(3-fluorophenyl)-7-((tetrahydro-2H-pyran-2-yl)oxy)-2H-chromene-4-yl)phenyl)piperidine

1-(4-(3-bromo-7-((tetrahydro-2H-pyran-2-yl)oxy)-2H-chromene-4-yl)phenyl)-4-(dimethox ymethyl)piperidine (200 mg, 0.37 mmol) and m-flurorphenylboronic acid (77 mg, 0.55 mmol) were added into a 50 mL round-bottom flask, to which was added 6 mL of 1,4-dioxane to dissolve, followed by addition of 2 mL water, potassium carbonate (128 mg, 1.0 mmol), and PddppfCl₂ (29 mg, 0.04 mmol), and then the mixture was allowed to react overnight at 95 °C under Argon protection. The reaction solution was added with water (35 mL) and extracted with 50 mL of ethyl acetate. The aqueous layer was extracted once with 40 mL of ethyl acetate. The organic layers were combined, washed once with saturated NaCl solution, dried over anhydrous Na₂SO₄, and concentrated to dry under reduced pressure. The residue was separated and purified by column chromatography, to obtain the product 4-(dimethoxymethyl)-1-(4-(3-(3-fluorophenyl)-7-((tetrahydro-2H-pyran-2-yl)oxy)-2H-chromene-4-yl)phenyl)piperidine (160 mg), with a yield of 76%. LC/MS (ESI+) calcd for C₃₄H₃₈FNO₅([M+H]⁺) *m*/*z* 560.3, found 560.3.

### Step 6: Synthesis of 4-(dimethoxymethyl)-1-(4-(3-(3-fluorophenyl)-7-((tetrahydro-2H-pyran-2-yl)oxy)chroman-4-yl)phenyl)piperidine

4-(dimethoxymethyl)-1-(4-(3-(3-fluorophenyl)-7-((tetrahydro-2H-pyran-2-yl)oxy)-2H-chr omene-4-yl)phenyl)piperidine (160 mg, 0.28 mmol) was dissolved in 20 mL mixed solvent of tetrahydrofuran/methanol = 1:1, to which was added 20 mg Pd/C, and then the system was purged with hydrogen for three times. The reaction was allowed to react overnight, and filtered. The filtrate was concentrated to dry, and the residue was separated and purified by TLC, to obtain the product 4-(dimethoxymethyl)-1-(4-(3-(3-fluorophenyl)-7-((tetrahydro-2H-pyran-2-yl)oxy)chroman-4-yl)phenyl)piperidine (90 mg). LC/MS (ESI+) calcd for C₃₄H₄₀FNO₅ ([M+H]⁺) *m*/*z* 562.3, found 562.3.

### Step 7: Synthesis of 1-(4-(3-(3-fluorophenyl)-7-hydroxyl-chroman-4-yl)phenyl)piperidine-4-carbaldehyde

4-(dimethoxymethyl)-1-(4-(3-(3-fluorophenyl)-7-((tetrahydro-2H-pyran-2-yl)oxy)chroman -4-yl)phenyl)piperidine (90 mg, 0.16 mmol) was dissolved in 5 mL of tetrahydrofuran, to which was added 2 mL of 2N H₂SO₄, and then the reaction was allowed to react for 1 h at 60°C. The reaction solution was adjusted to pH 7 with saturated NaHCO₃ solution in an ice-water bath, and then extracted with 15 mL of ethyl acetate. The water layer was further extracted once with 10 mL of ethyl acetate. The organic phase was dried over anhydrous Na₂SO₄, and concentrated to dry, to obtain the product 1-(4-(3-(3-fluorophenyl)-7-hydroxyl-chroman-4-yl)phenyl) piperidine-4-carbaldehyde (60 mg), with a yield of 90%.

### Step 8. Synthesis of 3-(5-(7-((1-(4-((3,4-cis)-3-(3-fluorophenyl)-7-hydroxyl-chroman-4-yl)phenyl)piperidin-4-yl)methyl)-2,7-diazaspiro[3.5]nonane-2-yl)-1-oxoisoindol-2-yl)piperidi n-2,6-dione

3-(1-oxo-5-(2,7-diazaspiro[3.5]nonane-2-yl)isoindol-2-yl)piperidin-2,6-dione trifluoroacetate (42 mg, 0.087mmol) was dissolved in 10 mL mixed solvent of dichloromethane/methanol (5:1), to which was added N,N-diisopropylethylamine (34 mg, 0.26 mmol), and then stirred for 10 min at room temperature, followed by addition of 1-(4-(3-(3-fluorophenyl)-7-hydroxyl-chroman-4-yl)phenyl)piperidine-4-carbaldehyde (30 mg, 0.069 mmol) and glacial acetic acid (20 mg, 0.34 mmol). The reaction was stirred for 10 min at room temperature, and then sodium triacetoxyborohydride (55 mg, 0.26 mmol) was added, followed by stirring at room temperature for 3 h. The reaction solution was washed with water, extracted with dichloromethane, and concentrated to dry under reduced pressure. The residue was separated and purified by TLC, to obtain the product 3-(5-(7-((1-(4-(3-(3-fluorophenyl)-7-hydroxyl-chroman-4-yl)phenyl)piperidin-4-yl)methyl)-2,7-diazaspiro[3.5]nonane-2-yl)-1-oxo isoindol-2-yl)piperidin-2,6-dione (25 mg), with a yield of 46%. LC/MS (ESI+) calcd for C₄₇H₅₀FN₅O₅ ([M+H]⁺) *m*/*z* 784.3, found 784.4; ¹H NMR (400 MHz, DMSO-*d*₆) *δ* 10.94 (s, 1H), 9.30 (s, 1H), 7.47 (d, *J* = 8.3 Hz, 1H), 7.19 (td, *J* = 7.9, 6.3 Hz, 1H), 7.01 - 6.94 (m, 1H), 6.70 - 6.59 (m, 4H), 6.57 - 6.44 (m, 3H), 6.40 (d, *J =* 8.4 Hz, 2H), 6.33 - 6.25 (m, 2H), 5.03 (dd, *J* = 13.2, 5.1 Hz, 1H), 4.30 (dd, *J =* 13.7, 10.8 Hz, 2H), 4.19 (d, *J* = 5.3 Hz, 2H), 4.14 (s, 1H), 3.62 (s, 4H), 3.55 (d, *J =* 11.4 Hz, 3H), 2.96 - 2.83 (m, 1H), 2.57 (d, *J =* 18.2 Hz, 2H), 2.38 - 2.21 (m, 4H), 2.10 (d, *J* = 7.1 Hz, 2H), 1.98 - 1.91 (m, 1H), 1.74 (s, 6H), 1.60 (s, 1H), 1.23 (d, *J* = 3.8 Hz, 2H), 1.16 - 1.07 (m, 2H).

### Example 61. 3-(5-(7-((1-(4-(7-hydroxyl-(3,4-cis)-3-(thiophene-3-yl)-chroman-4-yl) phenyl)piperidin-4-yl)methyl)-2,7-diazaspiro[3.5]nonane-2-yl)-1-oxoisoindol-2-yl)piperidi n-2,6-dione

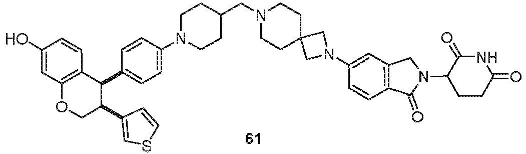

The target compound was prepared using a method similar to that in Example 60. LC/MS (ESI+) calcd for C₄₅H₄₉N₅O₅S ([M+H]⁺) *m*/*z* 772.3, found 772.3. ¹H NMR (400 MHz, DMSO-*d*₆) *δ* 10.94 (s, 1H), 9.28 (s, 1H), 7.47 (d, *J* = 8.3 Hz, 1H), 7.36 (dd, *J* = 4.9, 2.9 Hz, 1H), 6.75 (d, *J* = 2.9 Hz, 1H), 6.71 (dd, *J* = 5.0, 1.2 Hz, 1H), 6.64 (dd, *J* = 8.5, 4.7 Hz, 3H), 6.47 (dd, *J=* 19.1, 9.1 Hz, 4H), 6.29 (d, *J* = 2.4 Hz, 1H), 6.26 (dd, *J* = 8.2, 2.4 Hz, 1H), 5.03 (dd, *J* = 13.2, 5.1 Hz, 1H), 4.35 - 4.22 (m, 2H), 4.23 - 4.12 (m, 3H), 3.62 (s, 4H), 3.57 (dd, *J* = 15.0, 9.0 Hz, 3H), 2.97 - 2.83 (m, 1H), 2.62 - 2.52 (m, 2H), 2.38 - 2.25 (m, 4H), 2.11 (d, *J* = 7.2 Hz, 2H), 1.94 (d, *J* = 13.7 Hz, 1H), 1.74 (s, 6H), 1.60 (s, 1H), 1.23 (d, *J* = 3.8 Hz, 2H), 1.14 (m, *J* = 11.8 Hz, 2H).

### Example 62. 3-(5-(7-(1-(4-(7-hydroxyl-3-(6-methylpyridazine-4-yl)-chroman-4-yl) phenyl)piperidin-4-yl)methyl)-2,7-diazaspiro[3.5]nonane-2-yl)-1-oxoisoinodolin-2-yl)piper idin-2,6-dione

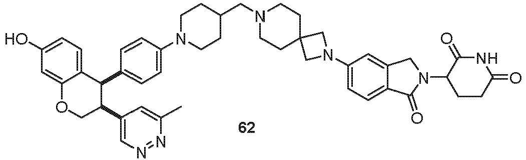

The target compound was prepared using a method similar to that in Example 60. LC/MS (ESI+) calcd for C₄₆H₅₁N₇O₅ ([M+H]⁺) *m*/*z* 782.4, found 782.4; ¹H NMR (400 MHz, DMSO-*d*₆) *δ* 10.94 (s, 1H), 9.37 (s, 1H), 8.55 (d, *J* = 2.1 Hz, 1H), 7.47 (d, *J* = 8.2 Hz, 1H), 6.77 (d, *J* = 2.1 Hz, 1H), 6.67 (dd, *J* = 8.5*,* 4.5 Hz, 3H), 6.51 (s, 1H), 6.47 (dd, *J =* 8.3, 2.0 Hz, 1H), 6.44 (d, *J* = 8.4 Hz, 2H), 6.33 (d, *J* = 2.4 Hz, 1H), 6.30 (dd, *J* = 8.2, 2.5 Hz, 1H), 5.03 (dd, *J =* 13.3, 5.1 Hz, 1H), 4.39 - 4.21 (m, 4H), 4.16 (d, *J* = 16.9 Hz, 1H), 3.62 (s, 4H), 3.59 - 3.52 (m, 3H), 2.95 - 2.85 (m, 1H), 2.56 (d, *J* = 17.7 Hz, 2H), 2.42 (s, 3H), 2.34 (dd, *J* = 13.3, 4.4 Hz, 4H), 2.12 (s, 2H), 1.94 (d, *J* = 11.0 Hz, 1H), 1.75 (s, 6H), 1.63 (s, 1H), 1.23 (d, *J=* 3.6 Hz, 2H), 1.17 - 1.09 (m, 2H).

### Example 63. 3-(5-(7-((1-(4-(3-(2,6-difluorophenyl)-7-hydroxyl-chroman-4-yl) phenyl)piperidin-4-yl)methyl)-2,7-diazaspiro[3.5]nonane-2-yl)-1-oxoisoinodolin-2-yl)piper idin-2,6-dione

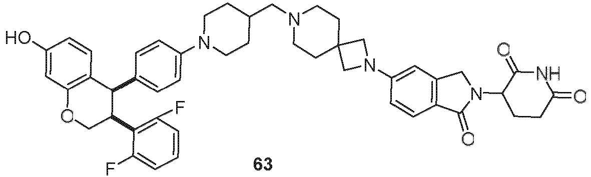

The target compound was prepared using a method similar to that in Example 60. LC/MS (ESI+) calcd for C₄₇H₄₉F₂N₅O₅ ([M+H]⁺) *m*/*z* 802.4, found 802.4; ¹H NMR (400 MHz, DMSO-*d*₆) *δ* 10.94 (s, 1H), 9.36 (s, 1H), 7.51 (d, *J* = 7.8 Hz, 1H), 7.31 (t, *J =* 7.0 Hz, 1H), 6.94 (t, *J* = 9.4 Hz, 2H), 6.66 (d, *J* = 9.2 Hz, 3H), 6.57 - 6.38 (m, 4H), 6.31 (d, *J* = 7.0 Hz, 2H), 5.03 (dd, *J* = 13.3, 5.1 Hz, 1H), 4.53 (s, 1H), 4.31 (d, *J* = 16.6 Hz, 2H), 4.17 (d, *J* = 16.8 Hz, 1H), 4.10 (d, *J=* 5.0 Hz, 1H), 3.73 (d, *J* = 29.7 Hz, 3H), 3.67 - 3.52 (m, 4H), 3.45 (s, 1H), 3.31 (dd, *J =* 7.4, 4.3 Hz, 1H), 3.14 (dd, *J =* 7.4, 4.3 Hz, 1H), 2.92 (dd, *J =* 31.7, 18.6 Hz, 4H), 2.58 (d, *J* = 16.4 Hz, 2H), 2.34 (d, *J=* 11.3 Hz, 2H), 2.17 - 1.86 (m, 6H), 1.77 (s, 2H), 1.28 (m, 2H).

### Example 64. 3-(5-(2-(1-(4-((1,2-cis)-6-hydroxyl-2-phenyl-1,2,3,4-tetrahydronaphthalen-1-yl)phenyl)piperidin-4-yl)-2,7-diazaspiro[3.5]nonan-7-yl)-1-oxoisoi nodolin-2-yl)piperidin-2,6-dione

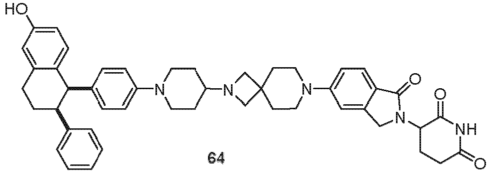

The target compound was prepared using a method similar to that in Example 19. LC/MS (ESI+) calcd for C₄₇H₅₁N₅O₄ ([M+H]⁺) *m*/*z* 750.4, found 750.4; ¹H NMR (400 MHz, DMSO-*d*₆) *δ* 10.95 (s, 1H), 9.11 (s, 1H), 7.49 (d, *J= 8.4* Hz, 1H), 7.13 (dq, *J* = 6.7, 5.7, 5.0 Hz, 3H), 7.04 (d, *J* = 10.5 Hz, 2H), 6.86 - 6.80 (m, 2H), 6.63 (d, *J* = 8.4 Hz, 1H), 6.60 (d, *J* = 2.5 Hz, 1H), 6.53 (d, *J =* 8.5 Hz, 2H), 6.47 (dd, *J* = 8.1, 2.6 Hz, 1H), 6.19 (d, *J* = 8.4 Hz, 2H), 5.04 (dd, *J =* 13.3, 5.1 Hz, 1H), 4.31 (d, *J =* 17.0 Hz, 1H), 4.18 (d, *J* = 16.9 Hz, 1H), 4.12 (d, *J* = 5.0 Hz, 1H), 3.43 (s, 2H), 3.28 (s, 6H), 2.93 (dq, *J* = 26.6, 14.0 Hz, 6H), 2.58 (d, *J =* 13.8 Hz, 3H), 2.42 - 2.29 (m, 2H), 2.14 - 2.06 (m, 1H), 2.01 - 1.91 (m, 2H), 1.74 (s, 8H).

### Example 65. 3-(5-(7-((1-(4-(3-(2,6-difluorophenyl)-7-hydroxyl-chroman-4-yl)-2-fluorophenyl)piperidin-4-yl)methyl)-2,7-diazaspiro[3.5]nonane-2-yl)-1-oxoisoinodolin-2-yl)piperidin-2,6-dione

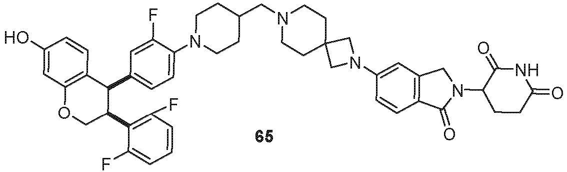

The target compound was prepared using a method similar to that in Example 60. LC/MS (ESI+) calcd for C₄₇H₄₈F₃N₅O₅ ([M+H]⁺) *m*/*z* 820.2, found 820.2; ¹H NMR (400 MHz, DMSO-*d*₆) *δ* 10.95 (s, 1H), 9.46 (d, *J* = 1.5 Hz, 1H), 7.51 (d, *J* = 8.3 Hz, 1H), 7.34 (p, *J* = 7.4 Hz, 1H), 6.96 (t, *J* = 9.4 Hz, 2H), 6.78 (t, *J* = 8.8 Hz, 1H), 6.72 - 6.66 (m, 1H), 6.51 (s, 1H), 6.48 (d, *J* = 8.3 Hz, 1H), 6.37 (d, *J* = 8.4 Hz, 1H), 6.35 - 6.30 (m, 2H), 6.25 (d, *J* = 14.0 Hz, 1H), 5.03 (dd, *J* = 13.2, 5.2 Hz, 1H), 4.50 (d, *J* = 11.9 Hz, 1H), 4.36 - 4.25 (m, 2H), 4.18 (d, *J* = 17.6 Hz, 2H), 3.81 (d, *J* = 11.1 Hz, 1H), 3.72 (d, *J* = 27.4 Hz, 4H), 3.65 - 3.53 (m, 4H), 3.43 (s, 2H), 3.23 (s, 2H), 3.14 (d, *J* = 7.4 Hz, 1H), 3.00 (s, 2H), 2.92 (d, *J=* 18.3 Hz, 2H), 2.58 (d, *J=* 13.7 Hz, 2H), 2.39 - 2.30 (m, 1H), 2.14 (s, 1H), 1.99 - 1.83 (m, 4H), 1.30 (m, 2H).

### Example 66. 3-(5-(7-(1-(4-(6-hydroxyl-2-phenyl-1,2,3,4-tetrahydronaphthalen-1-yl) phenyl)piperidin-4-yl)-2,7-diazaspiro[4.4]nonane-2-yl)-1-oxoisoinodolin-2-yl)piperidin-2,6 -dione

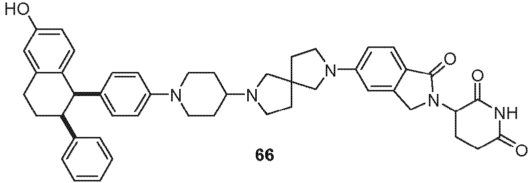

The target compound was prepared using a method similar to that in Example 19. LC/MS (ESI+) calcd for C₄₇H₅₁N₅O₄ ([M+H]⁺) *m*/*z* 750.4, found 750.4; ¹H NMR (400 MHz, DMSO-*d*₆) *δ* 10.95 (s, 1H), 9.16 (s, 1H), 7.50 (d, *J* = 8.8 Hz, 1H), 7.13 (dd, *J* = 12.0, 7.1 Hz, 3H), 6.86 - 6.80 (m, 2H), 6.68 - 6.52 (m, 6H), 6.48 (dd, *J* = 8.3*,* 2.5 Hz, 1H), 6.21 (d, *J* = 8.4 Hz, 2H), 5.03 (dd, *J* = 13.2, 5.1 Hz, 1H), 4.31 (d, *J* = 16.9 Hz, 1H), 4.18 (d, *J* = 16.9 Hz, 1H), 4.12 (t, *J = 5.3* Hz, 2H), 3.60 (d, *J* = 6.8 Hz, 5H), 3.33 - 3.25 (m, 4H), 3.16 (d, *J* = 5.2 Hz, 2H), 3.12 (d, *J* = 7.6 Hz, 2H), 3.03 - 2.83 (m, 4H), 2.58 (d, *J=* 17.6 Hz, 2H), 2.35 (dd, *J* = 13.0, 4.3 Hz, 1H), 2.15 - 1.89 (m, 8H), 1.71 (s, 2H).

### Example 67. 3-(5-(4-(7-(2-fluoro-4-((1,2-cis)-2-(2-fluorophenyl)-6-hydroxyl-1,2,3,4-tetrahydronaphthalen-1-yl)phenyl)-2,7-diazaspiro[3.5]nonane-2-yl)piperidin-1-yl)-1-oxois oinodolin-2-yl)pyridine-2,6-dione

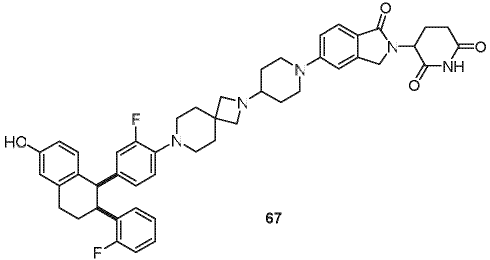

The target compound was prepared using a method similar to that in Example 19. LC/MS (ESI+) calcd for C₄₇H₄₉F₂N₅O₄([M+H]⁺) *m*/*z* 786.4, found 786.4; ¹H NMR (400 MHz, DMSO-*d*₆) *δ* 10.95 (s, 1H), 9.21 (s, 1H), 7.49 (d, *J =* 8.5 Hz, 1H), 7.25 - 7.12 (m, 2H), 7.03 (d, *J =* 8.6 Hz, 2H), 6.89 (td, *J* = 7.4, 1.5 Hz, 1H), 6.71 - 6.59 (m, 3H), 6.53 - 6.41 (m, 2H), 6.11 (dd, *J* = 8.3, 2.0 Hz, 1H), 6.02 (dd, *J* = 14.2, 2.0 Hz, 1H), 5.04 (dd, *J* = 13.3, 5.1 Hz, 1H), 4.30 (d, *J* = 16.9 Hz, 1H), 4.24 - 4.14 (m, 2H), 3.72 (d, *J* = 12.2 Hz, 2H), 3.61 - 3.51 (m, 1H), 3.01 - 2.84 (m, 8H), 2.75 (d, *J* = 6.3 Hz, 4H), 2.62 - 2.53 (m, 1H), 2.37 (td, *J* = 13.2, 4.6 Hz, 1H), 2.27 (d, *J =* 18.5 Hz, 1H), 2.09 (dd, *J* = 12.7, 6.3 Hz, 1H), 2.00 - 1.88 (m, 2H), 1.71 (d, *J* = 5.6 Hz, 7H), 1.23 (m, 2H).

### Example 68. 3-(5-(4-(7-(2-fluoro-4-((3,4-cis)-3-(2-fluorophenyl)-7-hydroxyl-chroman-4-yl)phenyl)-2,7-diazaspiro[3.5]nonane-2-yl)piperidin-1-yl)-1-oxoisoinodolin-2-yl)pyridine -2,6-dione

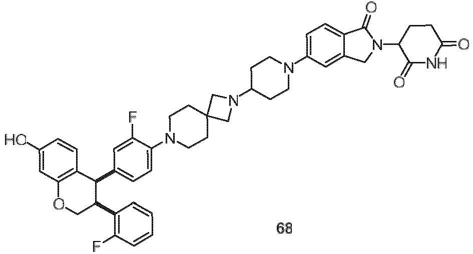

The target compound was prepared using a method similar to that in Example 19. LC/MS (ESI+) calcd for C₄₆H₄₇F₂N₅O₅ ([M+H]⁺) *m*/*z* 788.3, found 788.3. ¹H NMR (400 MHz, DMSO-*d*₆) *δ* 10.96 (s, 1H), 9.47 (s, 1H), 7.52 (d, *J= 8.4* Hz, 1H), 7.24 (q, *J* = 7.3 Hz, 1H), 7.20 - 7.13 (m, 1H), 7.13 - 7.04 (m, 2H), 6.92 (t, *J=* 7.5 Hz, 1H), 6.74 (t, *J=* 8.8 Hz, 1H), 6.68 (d, *J* = 8.3 Hz, 1H), 6.50 (t, *J* = 7.3 Hz, 1H), 6.40 - 6.27 (m, 3H), 6.20 (dd, *J* = 14.0, 2.0 Hz, 1H), 5.04 (dd, *J* = 13.2, 5.1 Hz, 1H), 4.27 (ddd, *J* = 34.2, 23.5, 13.3 Hz, 5H), 3.99 (d, *J* = 12.4 Hz, 2H), 3.83 (s, 2H), 3.74 (dd, *J* = 10.3, 5.1 Hz, 1H), 3.58 (p, *J* = 6.7 Hz, 4H), 3.10 (q, *J* = 7.4 Hz, 4H), 2.83 (dd, *J =* 25.8, 8.9 Hz, 5H), 2.58 (d, *J=* 16.5 Hz, 1H), 2.44 - 2.30 (m, 1H), 1.97 (d, *J* = 12.4 Hz, 4H), 1.83 (s, 1H), 1.56 (d, *J* = 11.9 Hz, 1H).

### Example 69. 3-(5-(4-(7-(2-fluoro-4-((1,2-cis)-6-hydroxyl-2-phenyl-1,2,3,4-tetrahydronaphthalen-1-yl)phenyl)-2,7-diazaspiro[3.5]nonane-2-yl)piperidin-1-yl)-1-oxois oinodolin-2-yl)pyridine-2,6-dione

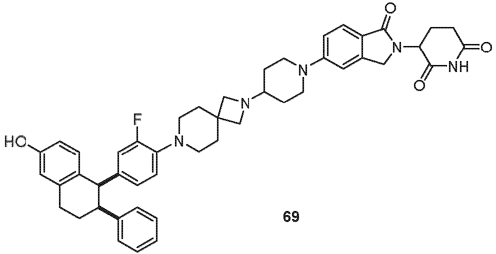

The target compound was prepared using a method similar to that in Example 19. LC/MS (ESI+) calcd for C₄₇H₅₀FN₅O₄ ([M+H]⁺) *m*/*z* 768.4, found 768.4. ¹H NMR (400 MHz, DMSO-*d*₆) *δ* 10.96 (s, 1H), 9.21 (s, 1H), 7.51 (d, *J* = 8.5 Hz, 1H), 7.16 (qd, *J* = 7.5, 6.4, 3.7 Hz, 3H), 7.07 (d, *J* = 11.1 Hz, 2H), 6.90 - 6.82 (m, 2H), 6.70 - 6.59 (m, 3H), 6.50 (dd, *J* = 8.3, 2.6 Hz, 1H), 6.09 (dd, *J* = 8.4, 1.9 Hz, 1H), 5.97 (dd, *J* = 14.3, 1.9 Hz, 1H), 5.04 (dd, *J* = 13.3, 5.1 Hz, 1H), 4.32 (d, *J* = 16.9 Hz, 1H), 4.24 - 4.14 (m, 2H), 3.91 (s, 2H), 3.57 (s, 2H), 3.09 (s, 2H), 3.03 - 2.66 (m, 10H), 2.63 - 2.54 (m, 1H), 2.37 (tt, *J* = 13.2, 6.5 Hz, 1H), 2.04 (dt, *J =* 13.0, 6.2 Hz, 1H), 1.95 (dd, *J =* 9.2, 4.1 Hz, 1H), 1.87 (d, *J* = 27.8 Hz, 6H), 1.78 - 1.63 (m, 2H), 1.42 (s, 2H).

### Example 70. 3-(5-(7-((1-(4-((3S,4R)-3-(2,6-difluorophenyl)-7-hydroxychroman-4-yl)-2-fluorophenyl)piperidin-4-yl)methyl)-2,7-diazaspiro[3.5]nonane-2-yl)-1-oxoisoinodoli n-2-yl)piperidin-2,6-dione

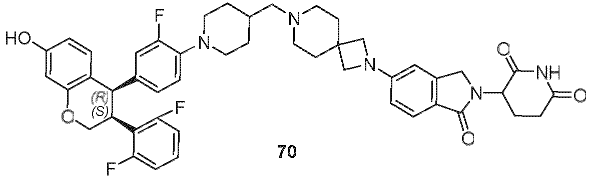

The target compound was prepared using a method similar to that in Example 60. LC/MS (ESI+) calcd for C₄₇H₄₈F₃N₅O₅ ([M+H]⁺) *m*/*z* 820.2, found 820.2. ¹H NMR (400 MHz, DMSO-*d*₆) *δ* 10.95 (s, 1H), 9.47 (s, 1H), 7.48 (d, *J* = 8.2 Hz, 1H), 7.33 (tt, *J* = 8.3, 6.4 Hz, 1H), 6.96 (t, *J* = 9.2 Hz, 2H), 6.75 (t, *J=* 8.8 Hz, 1H), 6.71 - 6.66 (m, 1H), 6.50 (d, *J=* 1.9 Hz, 1H), 6.46 (dd, *J= 8.4,* 1.9 Hz, 1H), 6.38 - 6.30 (m, 3H), 6.24 (dd, *J=* 14.1, 2.0 Hz, 1H), 5.03 (dd, *J* = 13.2, 5.1 Hz, 1H), 4.55 - 4.44 (m, 1H), 4.30 (d, *J* = 6.8 Hz, 1H), 4.27 (s, 1H), 4.19 - 4.14 (m, 2H), 3.81 (ddd, *J* = 11.7, 5.2, 3.1 Hz, 1H), 3.64 (s, 4H), 3.58 (q, *J* = 6.6 Hz, 1H), 3.35 (d, *J* = 12.3 Hz, 1H), 3.27 - 3.19 (m, 2H), 3.10 (q, *J* = 7.3 Hz, 1H), 2.95 - 2.84 (m, 1H), 2.56 (dd, *J* = 22.3, 7.2 Hz, 3H), 2.50 (p, *J* = 1.8 Hz, 2H), 2.34 (qd, *J* = 13.0, 4.5 Hz, 2H), 1.98 - 1.92 (m, 1H), 1.91 (s, 1H), 1.76 (d, *J =* 11.4 Hz, 6H), 1.24 - 1.22 (m, 2H).

### Example 71. 3-(5-(7-((1-(4-((3R,4S)-3-(2,6-difluorophenyl)-7-hydroxychroman-4-yl) -2-fluorophenyl)piperidin-4-yl)methyl)-2,7-diazaspiro[3.5]nonane-2-yl)-1-oxoisoinodolin-2 -yl)piperidin-2,6-dione

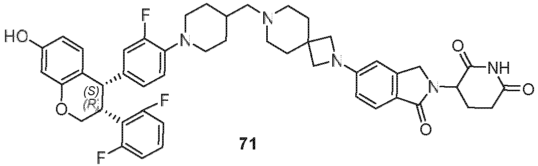

The target compound was prepared using a method similar to that in Example 60. LC/MS (ESI+) calcd for C₄₇H₄₈F₃N₅O₅ ([M+H]⁺) *m*/*z* 820.2, found 820.2. ¹H NMR (400 MHz, DMSO-*d*₆) *δ* 10.95 (s, 1H), 9.41 (s, 1H), 7.48 (d, *J =* 8.3 Hz, 1H), 7.38 - 7.29 (m, 1H), 6.97 (t, *J* = 9.3 Hz, 2H), 6.76 (t, *J* = 8.8 Hz, 1H), 6.70 (d, *J* = 9.0 Hz, 1H), 6.51 (s, 1H), 6.47 (dd, *J* = 8.3, 2.0 Hz, 1H), 6.35 (d, *J* = 9.0 Hz, 1H), 6.32 (dt, *J* = 4.7, 2.2 Hz, 2H), 6.27 - 6.20 (m, 1H), 5.03 (dd, *J =* 13.2, 5.1 Hz, 1H), 4.49 (t, *J* = 10.7 Hz, 1H), 4.29 (d, *J* = 16.6 Hz, 2H), 4.22 - 4.11 (m, 2H), 3.81 (dt, *J* = 11.6, 4.6 Hz, 1H), 3.61 (d, *J* = 9.4 Hz, 4H), 3.32 (s, 1H), 3.22 (d, *J* = 10.8 Hz, 2H), 2.96 - 2.84 (m, 1H), 2.57 (d, *J* = 22.1 Hz, 2H), 2.43 - 2.19 (m, 5H), 2.15 (d, *J=* 20.2 Hz, 2H), 1.95 (dd, *J=* 16.3, 9.4 Hz, 1H), 1.75 (t, *J* = 3.3 Hz, 6H), 1.60 (s, 1H), 1.23 (m, *J* = 3.9 Hz, 2H).

### Example 72. 3-(5-(7-((1-(4-((3R,4S)-3-(2,6-difluorophenyl)-7-hydroxychroman-4-yl)phenyl)piperidin-4-yl)methyl)-2,7-diazaspiro[3.5]nonane-2-yl)-1-oxoisoindol-2-yl)pipe ridin-2,6-dione

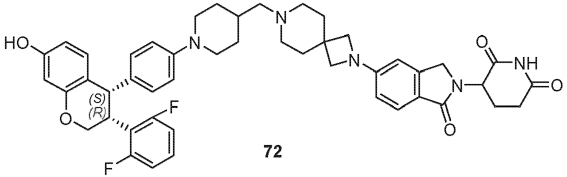

The target compound was prepared using a method similar to that in Example 60. LC/MS (ESI+) calcd for C₄₇H₄₉F₂N₅O₅ ([M+H]⁺) *m*/*z* 802.3, found 802.3. ¹H NMR (400 MHz, DMSO-*d*₆) *δ* 10.95 (s, 1H), 9.36 (s, 1H), 7.48 (d, *J* = 8.3 Hz, 1H), 7.31 (tt, *J* = 8.3, 6.4 Hz, 1H), 6.94 (t, *J* = 9.3 Hz, 2H), 6.69 - 6.60 (m, 3H), 6.51 (d, *J* = 1.9 Hz, 1H), 6.47 (dd, *J=* 8.3, 2.0 Hz, 1H), 6.43 (d, *J* = 8.4 Hz, 2H), 6.31 (s, 1H), 6.31 - 6.28 (m, 1H), 5.03 (dd, *J =* 13.2, 5.1 Hz, 1H), 4.59 - 4.48 (m, 1H), 4.29 (d, *J* = 17.2 Hz, 2H), 4.16 (d, *J* = 16.9 Hz, 1H), 4.10 (s, 1H), 3.78 (ddd, *J =* 11.8, 5.2, 3.1 Hz, 1H), 3.63 (s, 4H), 3.55 (d, *J* = 11.6 Hz, 2H), 3.17 (d, *J* = 4.7 Hz, 1H), 2.90 (ddd, *J* = 17.9, 13.5, 5.4 Hz, 1H), 2.63 - 2.52 (m, 2H), 2.49 (s, 1H), 2.41 - 2.21 (m, 4H), 2.12 (s, 2H), 1.99 - 1.91 (m, 1H), 1.73 (d, *J* = 15.5 Hz, 6H), 1.62 (s, 1H), 1.20 - 1.08 (m, 2H).

### Example 73. 3-(5-(7-((1-(4-((3S,4R)-3-(2,6-difluorophenyl)-7-hydroxychroman-4-yl) phenyl)piperidin-4-yl)methyl)-2,7-diazaspiro[3.5]nonane-2-yl)-1-oxoisoindol-2-yl)piperidi n-2,6-dione

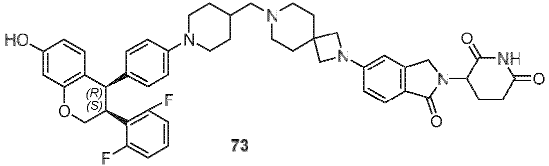

The target compound was prepared using a method similar to that in Example 60. LC/MS (ESI+) calcd for C₄₇H₄₉F₂N₅O₅ ([M+H]⁺) *m*/*z* 802.3, found 802.3. ¹H NMR (400 MHz, DMSO-*d*₆) δ 10.95 (s, 1H), 9.36 (s, 1H), 7.47 (d, *J* = 8.2 Hz, 1H), 7.31 (s, 1H), 6.94 (s, 2H), 6.65 (dd, *J =* 16.7, 8.4 Hz, 3H), 6.51 (s, 1H), 6.47 (d, *J =* 8.4 Hz, 1H), 6.42 (d, *J =* 8.2 Hz, 2H), 6.30 (d, *J* = 6.0 Hz, 2H), 5.03 (d, *J* = 13.4 Hz, 1H), 4.53 (s, 1H), 4.27 (s, 2H), 4.22 - 4.07 (m, 2H), 3.78 (s, 1H), 3.62 (s, 4H), 3.56 (s, 2H), 2.88 (d, *J* = 15.6 Hz, 1H), 2.59 (s, 2H), 2.32 (s, 4H), 2.11 (s, 2H), 1.96 (s, 2H), 1.74 (s, 6H), 1.61 (s, 1H), 1.46 (s, 1H), 1.13 (m, *J* = 9.6 Hz, 2H).

### Example 74. (R)-3-(5-(7-((1-(4-((3S,4R)-3-(2,6-difluorophenyl)-7-hydroxychroman-4-yl)-2-fluorophenyl)piperidin-4-yl)methyl)-2,7-diazaspiro[3.5]nonane-2-yl)-1-oxoisoinodoli n-2-yl)piperidin-2,6-dione

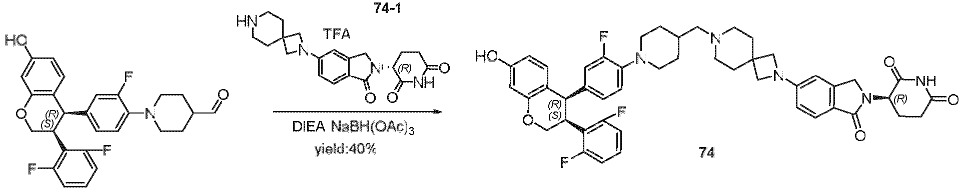

Intermediate **74-1** was used as the starting material, and the target compound was prepared using a method similar to that in Example 78. LC/MS (ESI+) calcd for C₄₇H₄₈F₃N₅O₅ ([M+H]⁺) *m*/*z* 820.2, found 820.2. ¹H NMR (400 MHz, DMSO-*d*₆) *δ* 10.95 (s, 1H), 9.60 (s, 1H), 7.47 (d, *J* = 8.3 Hz, 1H), 7.33 (tt, *J* = 8.4, 6.5 Hz, 1H), 7.03 - 6.92 (m, 2H), 6.79 - 6.72 (m, 1H), 6.69 (d, *J* = 9.0 Hz, 1H), 6.50 (d, *J* = 1.9 Hz, 1H), 6.47 (dd, *J* = 8.4, 2.0 Hz, 1H), 6.35 (d, *J* = 7.7 Hz, 1H), 6.34 - 6.30 (m, 2H), 6.23 (dd, *J* = 14.0, 2.0 Hz, 1H), 5.03 (dd, *J* = 13.3, 5.1 Hz, 1H), 4.54 - 4.44 (m, 1H), 4.35 - 4.24 (m, 2H), 4.21 - 4.11 (m, 2H), 3.81 (ddd, *J =* 11.7, 5.3, 3.0 Hz, 1H), 3.62 (s, 4H), 3.22 (d, *J* = 9.4 Hz, 2H), 2.89 (ddd, *J* = 17.2, 13.5, 5.4 Hz, 1H), 2.64 - 2.52 (m, 2H), 2.41 - 2.23 (m, 4H), 2.12 (d, *J* = 7.0 Hz, 2H), 1.98 - 1.91 (m, 1H), 1.89 (s, 2H), 1.81 - 1.68 (m, 6H), 1.59 (s, 1H), 1.26 - 1.18 (m, 2H).

### Example 75. (S)-3-(5-(7-((1-(4-((3S,4R)-3-(2,6-difluorophenyl)-7-hydroxychroman-4-yl)-2-fluorophenyl)piperidin-4-yl)methyl)-2,7-diazaspiro[3.5]nonane-2-yl)-1-oxoisoinodo lin-2-yl)piperidin-2,6-dione

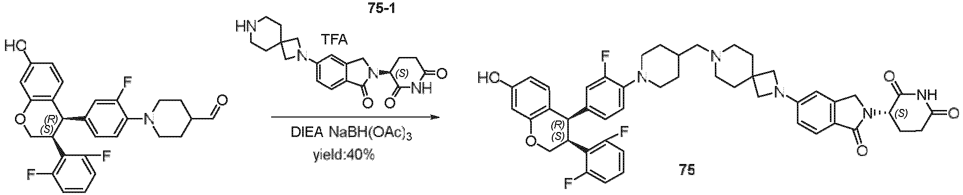

Intermediate **75-1** was used as the starting material, and then the target compound was prepared using a method similar to that in Example 78. LC/MS (ESI+) calcd for C₄₇H₄₈F₃N₅O₅ ([M+H]⁺) *m*/*z* 820.2, found 820.2. ¹H NMR (400 MHz, DMSO-*d*₆) *δ* 10.95 (s, 1H), 7.47 (d, *J* = 8.3 Hz, 1H), 7.33 (tt, *J = 8.3,* 6.5 Hz, 1H), 6.97 (q, *J* = 9.2, 7.5 Hz, 2H), 6.76 (t, *J* = 8.7 Hz, 1H), 6.72 - 6.66 (m, 1H), 6.50 (d, *J* = 2.0 Hz, 1H), 6.47 (dd, *J* = 8.3, 2.0 Hz, 1H), 6.38 - 6.34 (m, 1H), 6.34 - 6.30 (m, 2H), 6.23 (dd, *J* = 14.2, 2.0 Hz, 1H), 5.03 (dd, *J* = 13.3, 5.1 Hz, 1H), 4.48 (d, *J* = 11.8 Hz, 1H), 4.35 - 4.25 (m, 2H), 4.21 - 4.11 (m, 2H), 3.81 (dd, *J* = 10.7, 4.7 Hz, 1H), 3.62 (s, 4H), 3.23 (s, 2H), 2.94 - 2.85 (m, 1H), 2.62 - 2.52 (m, 2H), 2.41 - 2.24 (m, 4H), 2.12 (d, *J* = 7.1 Hz, 2H), 1.99 - 1.91 (m, 1H), 1.85 (s, 3H), 1.78 - 1.69 (m, 5H), 1.59 (s, 1H), 1.18 (m, 2H).

### Example 76. 3-(5-(7-((1-(4-(3-(2,6-difluorophenyl)-7-hydroxyl-2H-chromene-4-yl)-2-fluorophenyl)piperidin-4-yl)methyl)-2,7-diazaspiro[3.5]nonane-2-yl)-1-oxoisoinodolin-2-yl)piperidin-2,6-dione

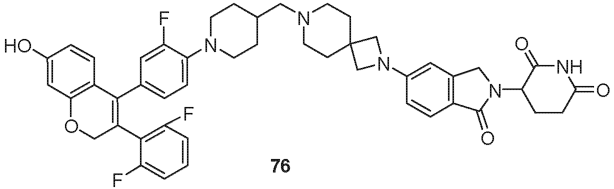

The target compound was prepared using a method similar to that in Example 42. LC/MS (ESI+) calcd for C₄₇H₄₆F₃N₅O₅ ([M+H]⁺) *m*/*z* 817.3, found 817.3. ¹H NMR (400 MHz, DMSO-*d*₆) *δ* 10.95 (s, 1H), 9.84 (s, 1H), 7.47 (d, *J* = 8.3 Hz, 1H), 7.31 (tt, *J* = 8.4, 6.6 Hz, 1H), 7.00 (t, *J* = 8.1 Hz, 2H), 6.89 (t, *J* = 8.8 Hz, 1H), 6.76 - 6.66 (m, 2H), 6.60 (dt, *J* = 8.7*,* 1.3 Hz, 1H), 6.51 (d, *J=* 1.8 Hz, 1H), 6.47 (dd, *J* = 8.4, 2.0 Hz, 1H), 6.39 - 6.32 (m, 2H), 5.03 (dd, *J* = 13.3, 5.1 Hz, 1H), 4.81 (s, 2H), 4.29 (d, *J* = 17.0 Hz, 1H), 4.16 (d, *J* = 16.9 Hz, 1H), 3.62 (s, 4H), 3.30 (s, 2H), 2.95 - 2.83 (m, 1H), 2.65 - 2.54 (m, 3H), 2.34 (td, *J* = 13.3, 8.6 Hz, 4H), 2.13 (d, *J =* 7.2 Hz, 2H), 1.99 - 1.91 (m, 1H), 1.74 (s, 6H), 1.62 (s, 1H), 1.27 - 1.15 (m, 3H).

### Example 77. 3-(5-(7-((1-(4-((3S,4R)-3-(2,6-difluorophenyl)-7-hydroxychroman-4-yl) phenyl)piperidin-4-yl)methyl)-2,7-diazaspiro[3.5]nonane-2-yl)-1-oxoisoinodolin-2-yl)piper idin-2,6-dione

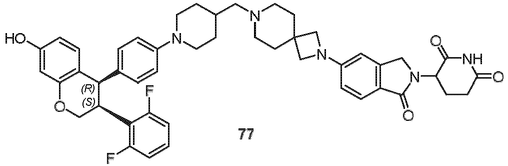

The target compound was prepared using a method similar to that in Example 60. LC/MS: calcd for C₄₇H₄₉F₂N₅O₅ ([M+H]⁺) *m*/*z* 802.37, found 802; ¹H NMR (400 MHz, DMSO-*d*₆) *δ* 10.95 (s, 1H), 9.37 (s, 1H), 7.48 (d, *J* = 8.2 Hz, 1H), 7.31 (tt, *J* = 8.4, 6.4 Hz, 1H), 6.94 (t, *J* = 9.3 Hz, 2H), 6.70 - 6.59 (m, 3H), 6.50 (d, *J* = 2.0 Hz, 1H), 6.47 (dd, *J* = 8.3, 2.0 Hz, 1H), 6.42 (d, *J* = 8.3 Hz, 2H), 6.32 (s, 1H), 6.31 - 6.29 (m, 1H), 5.03 (dd, *J* = 13.3, 5.1 Hz, 1H), 4.59 - 4.47 (m, 1H), 4.30 (d, *J* = 9.6 Hz, 1H), 4.27 (s, 1H), 4.16 (d, *J* = 16.9 Hz, 1H), 4.09 (d, *J* = 5.1 Hz, 1H), 3.78 (ddd, *J* = 11.7, 5.2, 3.1 Hz, 1H), 3.63 (s, 4H), 3.55 (d, *J* = 12.0 Hz, 3H), 3.11 (s, 1H), 2.95 - 2.84 (m, 1H), 2.61 - 2.52 (m, 2H), 2.34 (qd, *J* = 13.9, 13.5, 4.8 Hz, 4H), 2.09 (d, *J* = 17.1 Hz, 2H), 1.98 - 1.91 (m, 1H), 1.74 (s, 6H), 1.62 (s, 1H), 1.15 (h, *J* = 6.8 Hz, 2H).

### Example 78. (S)-3-(6-(7-((1-(4-((3S,4R)-3-(2,6-difluorophenyl)-7-hydroxychroman-4-yl)phenyl)piperidin-4-yl)methyl)-2,7-diazaspiro[3.5|nonane-2-yl)-1-oxoisoinodolin-2-yl)p iperidin-2,6-dione

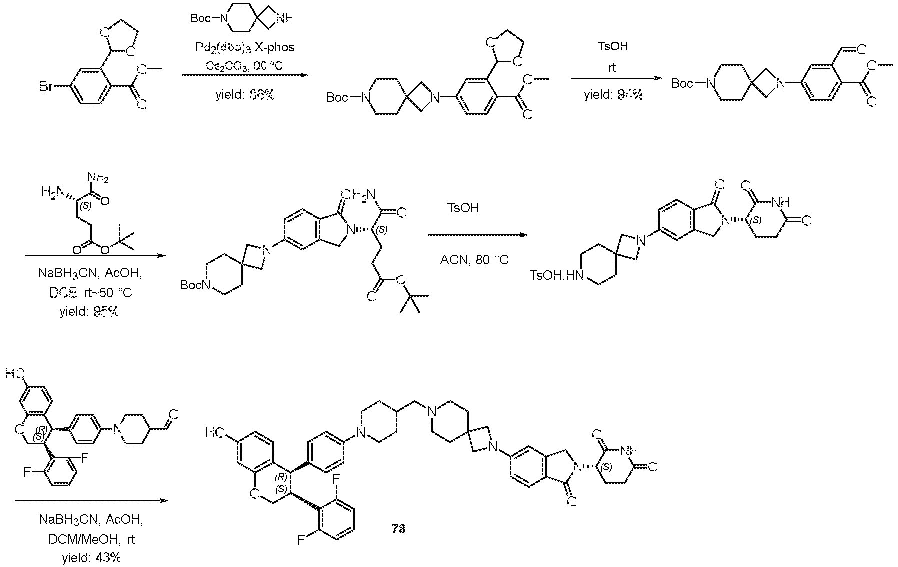

### Step 1: Synthesis of tert-butyl 2-(3-(1,3-dioxolane-2-yl)-4-(methoxycarbonyl)phenyl)-2,7-diazaspiro[3.5]nonane-7-carboxylate

Methyl 4-bromo-2-(1,3-dioxolane-2-yl)benzoate (10 g, 17 mmol) and tert-butyl 2,7-diazaspiro[3.5]nonane-7-carboxylate (4.35 g, 19.2 mmol) were dissolved in dioxane (100 mL), to which were added cesium carbonate (11.3 g, 35 mmol), X-phos (0.83 g, 1.75mmol), and Pd₂(dba)3 (0.8 g, 0.847 mmol), and the system was purged with nitrogen. The reaction was allowed to react overnight at 90 °C under nitrogen protection. TLC detection indicated completion of the reaction. The reaction solution was filtered via diatomaceous earth, and then washed with water. The resultant solution was extracted with ethyl acetate, washed with saturated NaCl solution, and purified by column chromatography eluted with petroleum ether/ethyl acetate to obtain the product (13 g), which was determined to be the target compound by LCMS. Chemical Formula: C₂₃H₃₂N₂O₆. LC/MS: calcd for 432.23 ([M+H]⁺) *m*/*z* 433.23, found 423.

### Step 2: Synthesis of tert-butyl 2-(3-formyl-4-(methoxycarbonyl)phenyl)-2,7-diazaspiro[3.5]nonane-7-carboxylate

tert-butyl 2-(3-(1,3-dioxolane-2-yl)-4-(methoxycarbonyl)phenyl)-2,7-diazaspiro[3.5]nonane-7-carboxylate (13 g, 30 mmol) was dissolved in acetone (130 ml), to which was added TSOH.H₂O (7.44 g, 39 mmol), and then the mixture was allowed to react at room temperature. TLC detection indicated completion of the reaction, and then the reaction solution was adjusted to alkaline with saturated NaHCO₃ aqueous solution. The resultant solution was extracted with ethyl acetate. The organic phase was washed with saturated NaCl solution, dried over anhydrous Na₂SO₄, and then concentrated, to obtain the product (11.5 g), with a yield of 94%.

### Step 3: Synthesis of tert-butyl (S)-2-(2-(1-amino-5-(tert-butoxy)-1,5-dioxopentane-2-yl)-1-oxoisoinodolin-5-yl)-2,7-diazaspiro[3.5]nonene-7-carboxylate

tert-butyl 2-(3-formyl-4-(methoxycarbonyl)phenyl)-2,7-diazaspiro[3.5]nonane-7-carboxylate (3.55 g, 9.14 mmol) and tert-butyl (S)-4,5-diamino-5-oxopentanoate (1.85 g, 9.14 mmol) were dissolved in dichloroethane (50 mL), to which was added acetic acid (548 mg, 9.14 mmol), and then the mixture was stirred for 50 min at room temperature, followed by addition of sodium cyanoborohydride (1.14 g, 18.3mmol). The reaction was stirred for 50 min at room temperature, and then warmed to 50 °C and reacted overnight. LCMS detection indicated completion of the reaction and the target compound. The reaction was quenched by adding water, and then extracted with dichloromethane. The organic phase was washed with saturated NaCl solution, dried over anhydrous Na₂SO₄, and then purified by column chromatography eluted with dichloromethane/methanol, to obtain the product (4.5 g), which was determined to be the target compound by LCMS, with a yield of 95%. Chemical Formula: C₂₉H₄₂N₄O₆. LC/MS: calcd for 542.31 ([M+H]⁺) *m*/*z* 543.32, found 543.

### Step 4: Synthesis of (S)-3-(1-oxo-5-(2,7-diazaspiro[3.5]nonane-2-yl)isoindol-2-yl) piperidin-2,6-dione-4-methylbenzenesulfonate

tert-butyl (*S*)-2-(2-(1-amino-5-(tert-butoxy)-1,5-dioxopentane-2-yl)-1-oxoisoindol-5-yl)-2,7-diazaspiro[3.5]nonene-7-carboxylate (5.00 g, 9.22 mmol) was dissolved in acetonitrile (50 ml), to which was added TSOH.H₂O (4.39 g, 23 mmol), and then the mixture was reacted at 80 °C overnight. LCMS indicated the target product. The reaction system was directly concentrated, and the residue was triturated in ethyl acetate at 70 °C and kept overnight. The solution was filtered to obtain the product (3.5 g). Chemical Formula: C₂₇H₃₂N₄O₆S. LC/MS: calcd for 368.18 ([M+H]⁺) *m*/*z* 369.18, found 369.

### Step 5: Synthesis of (S)-3-(6-(7-((1-(4-((3S,4R)-3-(2,6-difluorophenyl)-7-hydroxychroman-4-yl)phenyl)piperidin-4-yl)methyl)-2,7-diazaspiro[3.5]nonane-2-yl)-1-oxoisoi nodolin-2-yl)piperidin-2,6-dione

(S)-3-(1-oxo-5-(2,7-diazaspiro[3.5]nonane-2-yl)isoindol-2-yl)piperidin-2,6-dione-4-methy lbenzenesulfonate (100 mg, 0.29 mmol) was dissolved in dichloromethane/methanol (10 mL, 10:1), to which was added NaOAc (63 mg, 0.67 mmol), and then stirred at room temperature for 5 min, followed by addition of 1-(4-((3S,4R)-3-(2,6-difluorophenyl)-7-hydroxyltryptophane-4-yl)phenyl)piperidine-4-carbaldehyde (100 mg, 0.22mmol) and glacial acetic acid (40 mg, 0.67 mmol). The reaction was stirred for 0.5 h at room temperature, and then sodium triacetoxyborohydride (42 mg, 0.67 mmol) was added. The reaction was allowed to react overnight. TLC detection indicated disappearance of starting material. The reaction was quenched by adding saturated NaHCO₃ aqueous solution, and then extracted with dichloromethane. The organic phase was dried, concentrated, and then purified by prep-TLC developed with dichloromethane/methanol, to obtain the target compound (65 mg), with a yield of 43%. Chemical Formula: C₄₇H₄₉F₂N₅O₅. LC/MS: calcd for C₄₇H₄₉F₂N₅O₅ ([M+H]⁺) *m*/*z* 802.38, found 802; ¹H NMR (400 MHz, DMSO-*d*₆) δ 10.95 (s, 1H), 9.35 (s, 1H), 7.47 (d, *J* = 8.4 Hz, 1H), 7.31 (s, 1H), 6.94 (s, 2H), 6.65 (dd, *J* = 16.3, 8.3 Hz, 3H), 6.58 - 6.38 (m, 4H), 6.31 (s, 2H), 5.03 (d, *J =* 13.3 Hz, 1H), 4.53 (s, 1H), 4.29 (s, 1H), 4.27 (s, 1H), 4.16 (d, *J =* 16.8 Hz, 1H), 4.09 (s, 1H), 3.78 (s, 1H), 3.62 (s, 4H), 3.55 (s, 2H), 2.89 (s, 1H), 2.62 (d, *J* = 35.4 Hz, 3H), 2.32 (s, 4H), 2.10 (s, 2H), 1.96 (s, 2H), 1.74 (s, 5H), 1.61 (s, 2H), 1.13 (d, *J* = 12.7 Hz, 2H).

### Example 79. 3-(5-(7-((1-(4-((3S,4R)-7-hydroxyl-3-phenylchroman-4-yl)phenyl) piperidin-4-yl)methyl)-2,7-diazaspiro[3.5]nonane-2-yl)-1-oxoisoinodolin-2-yl)piperidin-2,6 -dione

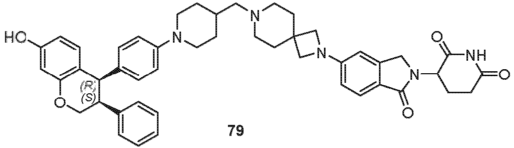

The target compound was prepared using a method similar to that in Example 60. ¹H NMR (400 MHz, DMSO-*d*₆) *δ* 10.98 (s, 1H), 9.28 (s, 1H), 7.54 (d, *J =* 8.3 Hz, 1H), 7.14 (p, *J* = 3.2, 2.7 Hz, 3H), 6.81 - 6.71 (m, 3H), 6.67 (dd, *J =* 8.2, 5.1 Hz, 2H), 6.51 - 6.49 (m, 2H), 6.35 (d, *J* = 8.6 Hz, 2H), 6.31 (d, *J* = 2.4 Hz, 1H), 6.27 (dd, *J* = 8.2, 2.5 Hz, 1H), 5.04 (dd, *J* = 12.9, 5.4 Hz, 1H), 4.33 (t, *J* = 11.1 Hz, 1H), 4.17 (t, *J* = 6.9 Hz, 2H), 4.15 - 4.13 (m, 2H),3.74 (s, 4H), 3.63 - 3.45 (m, 5H), 2.98 - 2.74 (m, 1H), 2.65 - 2.53 (m, 2H), 2.48 - 2.46 (m, 1H), 2.30 - 2.28 (m, 4H), 2.10 (d, *J* = 7.1 Hz, 2H), 2.02 - 1.99 (m, 1H), 1.78 - 1.73 (m, 7H), 1.28 - 1.23 (m, 2H). LC/MS (ESI+) calcd for C₄₇H₅₁N₅O₅ ( [M+H]⁺ ) *m*/*z*: 766.4; found 766.4.

### Example 80. 2-(2,6-dioxopiperidin-3-yl)-5-(7-((1-(4-((3R,4S)-7-hydroxyl-3-phenylchroman-4-yl)phenyl)piperidin-4-yl)methyl)-2,7-diazaspiro[3.5]nonane-2-yl)isoinod olin-1,3-dione

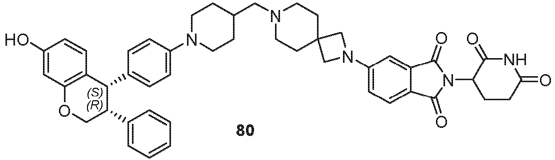

The target compound was prepared using a method similar to that in Example 60. ¹H NMR (400 MHz, DMSO-*d*₆) *δ* 11.12 (s, 1H), 9.31 (s, 1H), 7.63 (d, *J* = 8.3 Hz, 1H), 7.14 (p, *J* = 3.2, 2.7 Hz, 3H), 6.81 - 6.71 (m, 3H), 6.65 (dd, *J =* 8.2, 5.1 Hz, 2H), 6.62 - 6.56 (m, 2H), 6.37 (d, *J* = 8.6 Hz, 2H), 6.30 (d, *J* = 2.4 Hz, 1H), 6.27 (dd, *J* = 8.2, 2.5 Hz, 1H), 5.05 (dd, *J =* 12.9, 5.4 Hz, 1H), 4.33 (t, *J* = 11.1 Hz, 1H), 4.17 (t, *J* = 6.9 Hz, 2H), 3.73 (s, 4H), 3.63 - 3.45 (m, 4H), 2.98 - 2.74 (m, 2H), 2.65 - 2.53 (m, 3H), 2.48 - 2.46 (m, 1H), 2.30 - 2.28 (m, 3H), 2.10 (d, *J* = 7.1 Hz, 2H), 2.00 (dq, *J* = 11.0, 5.6, 4.5 Hz, 1H), 1.78 - 1.73 (m, 6H), 1.28 - 1.23 (m, 2H). LC/MS (ESI+) calcd for C₄₇H₄₉N₅O₆ ( [M+H]⁺ ) *m*/*z:* 780.4; found 780.4.

### Example 81. 2-(2,6-dioxopiperidin-3-yl)-5-(7-((1-(4-((3S,4R)-7-hydroxyl-3-phenylchroman-4-yl)phenyl)piperidin-4-yl)methyl)-2,7-diazaspiro[3.5]nonane-2-yl)isoinod olin-1,3-dione

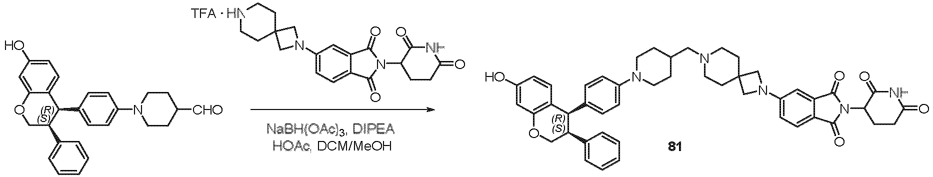

The target compound was prepared using a method similar to that in Example 1. ¹H NMR (400 MHz, DMSO-*d*₆) *δ* 11.07 (s, 1H), 9.32 (s, 1H), 7.68 - 7.57 (m, 1H), 7.14 (p, *J* = 3.4, 3.0 Hz, 3H), 6.80 - 6.71 (m, 3H), 6.67 - 6.62 (m, 2H), 6.60 (d, *J* = 8.5 Hz, 2H), 6.37 (d, *J= 8.6* Hz, 2H), 6.31 (d, *J* = 2.4 Hz, 1H), 6.27 (dd, *J* = 8.2, 2.4 Hz, 1H), 5.05 (dd, *J* = 12.9, 5.4 Hz, 1H), 4.32 (t, *J* = 11.1 Hz, 1H), 4.18 (dd, *J* = 11.5, 4.2 Hz, 2H), 3.73 (s, 4H), 3.60 - 3.49 (m, 4H), 2.96 - 2.78 (m, 2H), 2.63 - 2.57 (m, 1H), 2.54 (d, *J* = 8.3 Hz, 2H), 2.47 (d, *J* = 2.9 Hz, 1H), 2.42 - 2.20 (m, 3H), 2.11 (d, *J* = 7.1 Hz, 2H), 2.04 - 1.93 (m, 1H), 1.80 - 1.64 (m, 6H), 1.27 - 1.22 (m, 2H). LC/MS (ESI+) calcd for C₄₇H₄₉N₅O₆ ( [M+H]⁺ ) *m*/*z:* 780.4; found 780.4.

### Example 82. 3-(5-(5-((1-(4-((3R,4S)-7-hydroxyl-3-phenylchroman-4-yl)phenyl) piperidin-4-yl)methyl)hexahydropyrrolo[3,4-c]pyrrole-2(1H)-yl)-1-oxoisoinodolin-2-yl)pip eridin-2,6-dione

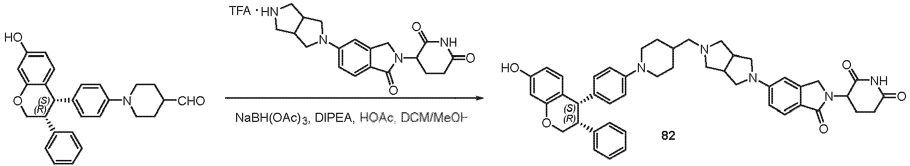

The target compound was prepared using a method similar to that in Example 60. ¹H NMR (400 MHz, DMSO-*d*₆) *δ* 10.89 (s, 1H), 9.32 (s, 1H), 7.48 (dd, *J =* 10.2, 3.4 Hz, 1H), 7.13 (p, *J* = 3.4, 3.0 Hz, 3H), 6.74 (dd, *J* = 6.6, 3.0 Hz, 2H), 6.70 (d, *J =* 7.4 Hz, 2H), 6.64 (d, *J =* 8.3 Hz, 1H), 6.58 (d, *J* = 8.8 Hz, 2H), 6.35 (d, *J* = 8.4 Hz, 2H), 6.30 (d, *J* = 2.4 Hz, 1H), 6.26 (dd, *J* = 8.2, 2.4 Hz, 1H), 5.03 (dd, *J=* 13.3, 5.1 Hz, 1H), 4.37 - 4.24 (m, 2H), 4.17 (dd, *J=* 17.0, 5.8 Hz, 3H), 3.62 - 3.45 (m, 4H), 3.16 - 3.04 (m, 3H), 2.93 - 2.89 (m, 3H), 2.80 (q, *J* = 7.2 Hz, 1H), 2.60 - 2.55 (m, 2H), 2.45 - 2.43 (m, 2H), 2.39 - 2.32 (m, 1H), 2.23 (d, *J* = 7.2 Hz, 2H), 2.07 -1.89 (m, 3H), 1.71 (d, *J* = 12.6 Hz, 2H), 1.23 (d, *J =* 3.7 Hz, 3H). LC/MS (ESI+) calcd for C₄₆H₄₉N₅O₅ ( [M+H]⁺ ) *m*/*z*: 752.4; found 752.4.

### Example 83. 3-(5-(5-((1-(4-((3S, 4R)-7-hydroxyl-3-phenylchroman-4-yl)phenyl) piperidin-4-yl)methyl)hexahydropyrrolo[3,4-c]pyrrole-2(1H)-yl)-1-oxoisoinodolin-2-yl)pip eridin-2,6-dione

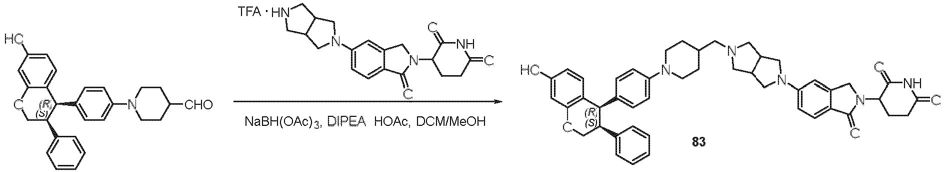

The target compound was prepared using a method similar to that in Example 78. ¹H NMR (400 MHz, DMSO-*d*₆) *δ* 10.93 (s, 1H), 9.31 (s, 1H), 7.48 (dd, *J =* 10.2, 3.4 Hz, 1H), 7.13 (p, *J* = 3.4, 3.0 Hz, 3H), 6.74 (dd, *J* = 6.6, 3.0 Hz, 2H), 6.70 (d, *J =* 7.4 Hz, 2H), 6.65 (d, *J =* 8.3 Hz, 1H), 6.58 (d, *J* = 8.8 Hz, 2H), 6.35 (d, *J* = 8.4 Hz, 2H), 6.30 (d, *J* = 2.4 Hz, 1H), 6.27 - 6.24 (m, 1H), 5.03 (dd, *J =* 13.3, 5.1 Hz, 1H), 4.39 - 4.24 (m, 2H), 4.17 (dd, *J=* 17.0, 5.8 Hz, 3H), 3.62 - 3.45 (m, 4H), 3.13 - 3.09 (m, 3H), 2.93 - 2.89 (m, 3H), 2.80 (q, *J* = 7.2 Hz, 1H), 2.65 - 2.52 (m, 2H), 2.45 - 2.43 (m, 2H), 2.39 - 2.32 (m, 1H), 2.23 (d, *J* = 7.2 Hz, 2H), 2.10 - 2.08 (m, 1H), 2.03 - 1.90 (m, 2H), 1.71 (d, *J* = 12.6 Hz, 2H), 1.23 (d, *J =* 3.7 Hz, 3H). LC/MS (ESI+) calcd for C₄₆H₄₉N₅O₅ ( [M+H]⁺ ) *m*/*z*: 752.4; found 752.4.

### Example 84. 3-(5-(7-((1-(4-(cis-7-hydroxyl-3-(pyrimidine-5-yl)chroman-4-yl)phenyl) piperidin-4-yl)methyl)-2,7-diazaspiro[3.5]nonane-2-yl)-1-oxoisoinodolin-2-yl)piperidin-2,6 -dione

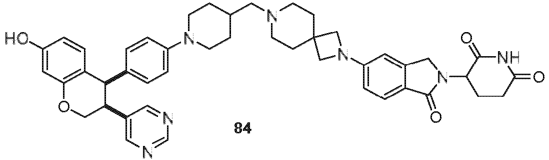

The target compound was prepared using a method similar to that in Example 60. ¹H NMR (400 MHz, DMSO-*d*₆) *δ* 10.89 (s, 1H), 9.37 (s, 1H), 8.95 (s, 1H), 8.13 (s, 2H), 7.47 (d, *J* = 8.3 Hz, 1H), 6.67 (dd, *J =* 8.4, 4.0 Hz, 3H), 6.47 (dd, *J* = 15.3, 8.7 Hz, 4H), 6.35 - 6.26 (m, 2H), 5.03 (dd, *J =* 13.3, 5.1 Hz, 1H), 4.38 (t, *J* = 10.9 Hz, 1H), 4.34 - 4.12 (m, 4H), 3.62 - 3.57 (m, 7H),2.91 - 2.87 (m, 2H), 2.75 (q, *J =* 7.2 Hz, 1H), 2.64 - 2.53 (m, 3H), 2.44 - 2.21 (m, 6H), 2.11 (d, *J =* 7.2 Hz, 3H), 1.96 - 1.92 (m, 2H), 1.79 - 1.67 (m, 2H), 1.27 - 1.23 (m, 2H). LC/MS (ESI+) calcd for C₄₅H₄₉N₇O₅ ( [M+H]⁺ ) *m*/*z*: 768.4; found 768.4.

### Example 85. 2-(2,6-dioxopiperidin-3-yl)-5-(3-(4-(4-(cis-7-hydroxyl-3-phenylchroman-4-yl)phenyl)piperazine-1-yl)azetidin-1-yl)isoinodolin-1,3-dione

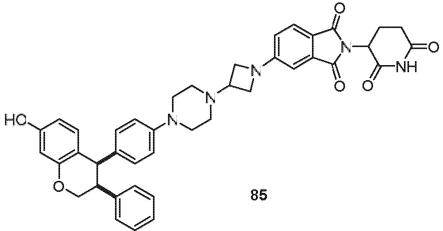

The target compound was prepared using a method similar to that in Example 19. ¹H NMR (400 MHz, DMSO-*d*₆) *δ* 11.06 (s, 1H), 9.29 (s, 1H), 7.65 (d, *J* = 8.3 Hz, 1H), 7.14 (dp, *J* = 5.7, 2.1 Hz, 3H), 6.85 - 6.71 (m, 3H), 6.64 (dd, *J* = 11.0, 8.5 Hz, 4H), 6.39 (d, *J* = 8.4 Hz, 2H), 6.35 - 6.20 (m, 2H), 5.06 (dd, *J* = 12.9, 5.4 Hz, 1H), 4.33 (t, *J* = 11.2 Hz, 1H), 4.24 - 4.14 (m, 2H), 4.15 - 4.07 (m, 2H), 3.88 (dd, *J* = 8.9, 4.9 Hz, 2H), 3.51 (dt, *J =* 11.7, 4.3 Hz, 1H), 3.17 - 2.95 (m, 4H), 2.87 (ddd, *J* = 17.4, 13.9, 5.5 Hz, 1H), 2.63 - 2.52 (m, 2H), 2.46 (t, *J* = 4.7 Hz, 4H), 2.04 - 1.96 (m, 1H), 1.29 - 1.20 (m, 1H). LC/MS (ESI+) calcd for C₄₁H₃₉N₅O₆ ( [M+H]⁺ ) *m*/*z:* 698.3; found 698.3.

### Example 86. 3-(5-(3-(4-(4-(cis-7-hydroxyl-3-phenylchroman-4-yl)phenyl)piperazine-1-yl)azetidin-1-yl)-1-oxoisoinodolin-2-yl)piperidin-2,6-dione

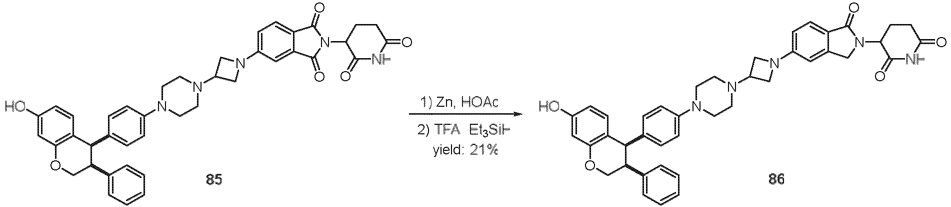

2-(2,6-dioxopiperidin-3-yl)-5-(3-(4-(4-(cis-7-hydroxyl-3-phenylchroman-4-yl)phenyl)pipe razine-1-yl)azetidin-1-yl)isoinodolin-1,3-dione (70 mg, 0.05 mmol) was dissolved in 5 mL of glacial acetic acid, to which was added zinc powder (64 mg, 1 mmol), and then the mixture was allowed to react for 2 h at 60 °C. TLC detection indicated disappearance of starting material. The reaction was cooled to room temperature, and filtered via diatomaceous earth. The filtrate was concentrated to remove glacial acetic acid. The reaction was adjusted to weakly alkaline with saturated NaHCO₃ solution, and extracted several times with 5 mL of ethyl acetate. The organic phases were combined, washed with saturated NaCl solution, dried, and concentrated. The residue was dissolved in 1 mL of dichloromethane, and then 0.2 mL of trifluoroacetic acid and triethylsilane (12 mg, 0.1 mmol) were successively added. The reaction was stirred at room temperature overnight. LC-MS detection indicated the disappearance of hydroxyl compounds, and then the reaction was terminated. The reaction solution was directly concentrated to remove dichloromethane and most of the trifluoroacetic acid, and then saturated NaHCO₃ solution was added to make the system weakly alkaline. The resultant solution was extracted several times with 5 mL of ethyl acetate. The organic phases were dried and combined, and concentrated, followed by separation and purification by column chromatography, to obtain 7 mg of target compound, with a yield of 21%. ¹H NMR (400 MHz, DMSO-*d*₆) *δ* 10.94 (s, 1H), 9.30 (s, 1H), 7.49 (d, *J* = 8.2 Hz, 1H), 7.23 - 7.09 (m, 3H), 6.76 (d, *J* = 6.0 Hz, 2H), 6.64 (dd, *J* = 11.7, 8.3 Hz, 3H), 6.53 (s, 1H), 6.49 (d, *J* = 8.4 Hz, 1H), 6.39 (d, *J* = 8.3 Hz, 2H), 6.34 - 6.21 (m, 2H), 5.03 (dd, *J* = 13.3, 5.1 Hz, 1H), 4.35 - 4.25 (m, 2H), 4.19 (q, *J* = 9.3, 8.5 Hz, 3H), 4.01 (t, *J* = 7.5 Hz, 2H), 3.74 - 3.71 (m, 2H), 3.04 - 3.03 (m, 4H), 2.94 - 2.85 (m, 1H), 2.67 - 2.60 (m, 2H), 2.46 - 2.45 (m, 3H), 2.01 - 1.89 (m, 1H). LC/MS (ESI+) calcd for C₄₁H₄₁N₅O₅( [M+H]⁺ ) *m*/*z*: 684.3; found 684.3.

### Example 87. 3-(5-(7-((1-(2-fluoro-4-(cis-3-(2-fluorophenyl)-7-hydroxybenzopyran-4-yl) phenyl)piperidin-4-yl)methyl)-2,7-diazaspiro[3.5]nonane-2-yl)-1-oxoisoinodolin-2-yl)piper idin-2,6-dione

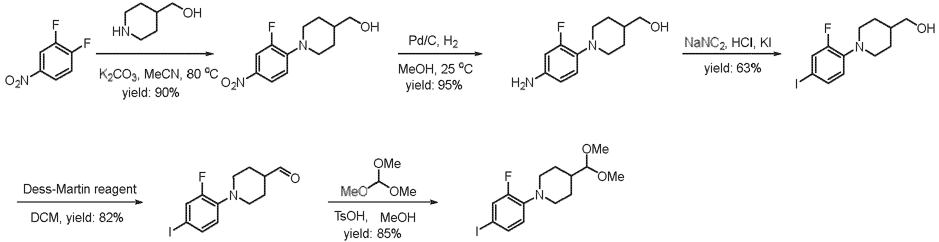

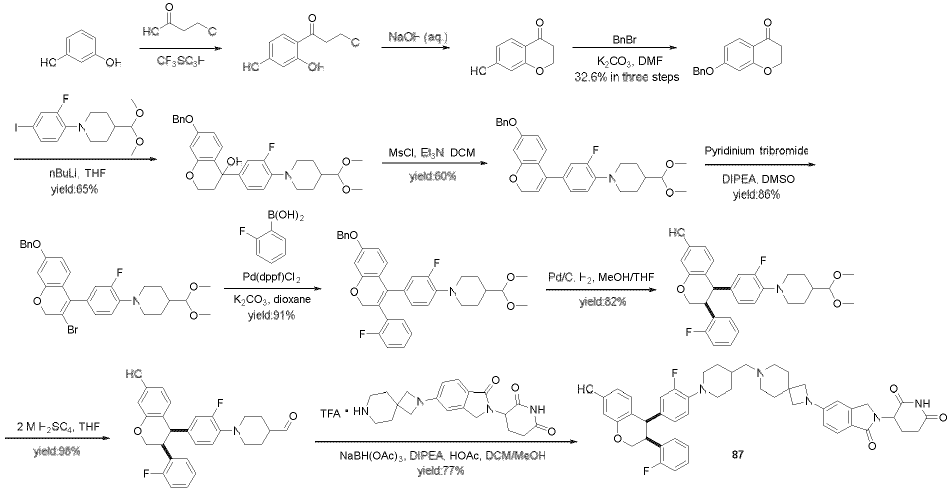

### Step 1: Synthesis of (1-(2-fluoro-4-nitrophenyl)piperidin-4-yl)methanol

3,4-difluoronitrophene (16 g, 100 mmol) and piperidinemethanol (12.1 g, 105 mmol) were dissolved in acetonitrile (200 mL), to which was added potassium carbonate (27.6 g, 200 mmol), and then heated overnight at 80 °C. TLC detection indicated disappearance of starting material 3,4-difluoronitrophene. The reaction system was cooled to room temperature, poured into water, and solids precipitated out. After filtration and drying, 21.3 g of product was obtained, which was identified as the target compound by LC-MS. The yield was 90%.

### Step 2: Synthesis of (1-(4-amino-2-fluorophenyl)piperidin-4-yl)methanol

(1-(2-fluoro-4-nitrophenyl)piperidin-4-yl)methanol (21.3 g, 95 mmol) was dissolved in methanol (260 mL), to which was added 10% Pd/C (1.1 g, 5%), and then the system was purged with hydrogen. The reaction was allowed to react overnight at room temperature, and monitored by TLC until the raw materials disappeared. The reaction solution was filtered via diatomaceous earth to remove Pd/C. The filtrate was concentrated to obtain 20.2 g of product, which was identified as the target compound by LC-MS, and directly used in the next step. The yield was 95%.

### Step 3: Synthesis of (1-(2-fluoro-4-iodophenyl)piperidin-4-yl)methanol

(1-(4-amino-2-fluorophenyl)piperidin-4-yl)methanol (20.2 g, 90 mmol) was dissolved in acetonitrile (100 mL), and the reaction was cooled to 0 °C, followed by addition of concentrated hydrochloric acid (12 M, 22.5 mL). Subsequently, the solution of sodium nitrite (7.5 g, 108 mmol) in water (10 mL) was slowly added dropwise at 0 °C. After addition, the reaction was stirred at low temperature for 0.5 h. Then, the solution of KI (15 g, 90 mmol) in water (25 mL) was added at 0 °C. The reaction was warmed to 15 °C and stirred overnight until the starting materials disappeared. The reaction was quenched with saturated NaHSO₃ solution, and then extracted with ethyl acetate. The organic phase was washed with saturated NaCl solution, dried, and concentrated, followed by column chromatography to obtain 19 g of product, which was identified as the target compound by LC-MS. The yield was 63%.

### Step 4: Synthesis of 1-(2-fluoro-4-iodophenyl)piperidine-4-carbaldehyde

(1-(2-fluoro-4-iodophenyl)piperidin-4-yl)methanol (19 g, 56.7 mmol) was dissolved in dichloromethane (300 mL), to which was added NaHCO₃ (9.5 g, 113.5 mmol), and then the reaction was cooled to 0 °C, followed by slowly adding Dess-Martin periodinane (48 g, 113.5 mmol) in portions. After addition, the reaction was allowed to react for 1 h, and TLC detection indicated completion of the reaction. The reaction solution was filtered via diatomaceous earth. The filtrate was concentrated, and the residue was subjected to column chromatography, to obtain 15.5 g of product, which was identified as the target compound by LC-MS. The yield was 82%.

### Step 5: Synthesis of 4-(dimethoxymethyl)-1-(2-fluoro-4-iodophenyl)piperidine

1-(2-fluoro-4-iodophenyl)piperidine-4-carbaldehyde (15.5 g, 46.6 mmol) was dissolved in methanol (100 mL), to which were successively added trimethyl orthoformate (7.4 g, 70 mmol) and p-toluenesulfonic acid (1.2 g, 7 mmol), and then the mixture was allowed to react overnight at 65 °C. The reaction was detected by TLC until the raw material disappeared. The reaction solution was cooled to room temperature, adjusted to pH 9 with saturated NaHCO₃ solution, and then concentrated to remove the organic solvent in the system. The residue was extracted with ethyl acetate. The organic phase was washed with saturated NaCl solution, concentrated and dried, followed by column chromatography, to obtain 15 g of product, which was identified as the target compound by LC-MS. The yield was 85%.

### Step 6: Synthesis of 3-chloro-1-(2,4-dihydroxylphenyl)propane-1-one

1,3-benzenediol (11 g, 100 mmol) and 1-chloropropionic acid (11.9 g, 110 mmol) were dissolved in trifluoromethanesulfonic acid (52.5 g, 350 mmol), and then reacted for 2h at 80 °C. The reaction was detected by TLC until the raw material 1,3-benzenediol disappeared. The reaction solution was cooled to room temperature, added with water, and then extracted with dichloromethane/methanol (10/1). The organic phase was concentrated, to obtain 23 g of crude product, which was directly used in the next step.

### Step 7: Synthesis of 7-hydroxychroman-4-one

The crude product obtained in the previous step was placed in an ice bath and cooled to 0 °C, to which was added NaOH (28 g, 700 mmol) aqueous solution (80 mL). The reaction temperature was maintained at 0°C, and the reaction was stirred for 0.5 h. The reaction was detected by TLC until the raw material disappeared, and then the pH of the system was adjusted to around 5 with 6N hydrochloric acid, followed by extracting with ethyl acetate. The organic phase was dried and concentrated, to obtain 10.8 g of crude product, which is directly used in the next step.

### Step 8. Synthesis of 7-(benzyloxy)chromane-4-one

The crude product obtained in the previous step was dissolved in DMF (60 mL), to which were successively added potassium carbonate (27.6 g, 200 mmol) and benzyl bromide (17.1 g, 100 mmol), and then the mixture was allowed to react overnight at 40 °C. The reaction was detected by TLC until the raw material disappeared, and then quenched by adding water. The resultant solution was extracted with ethyl acetate. The organic phase was washed with saturated NaCl solution, dried and concentrated. The residue was subjected to column chromatography, to obtain 8.2 g of product, which was identified as the target compound by LC-MS. The total yield was 32.6% for the above three steps.

### Step 9: Synthesis of 7-(benzyloxy)-4-(4-(4-(dimethoxymethyl)piperidin-1-yl)-3-fluorophenyl)chroman-4-ol

Under nitrogen atmosphere, 4-(dimethoxymethyl)-1-(2-fluoro-4-iodophenyl)piperidine (12.5 g, 33 mmol) was dissolved in dry THF(180 mL), and then cooled to below -70 °C, followed by slowly adding n-BuLi (14.4 mL, 2.5 M, 36 mmol). During the whole addition process, the temperature of the reaction system was kept below -70 °C. After addition, the reaction was allowed to react for 1 h under the conditions of keeping the temperature. The solution of 7-(benzyloxy)chromane-4-one (7.6 g, 30 mmol) in dry THF (40 mL) was slowly added to the system dropwise, and the temperature of the reaction system was kept below -70 °C during the whole addition procedure. The mixture was reacted for 1h under the conditions of keeping the temperature. The reaction system was gradually warmed to around -40 °C, and TLC monitoring indicated the complete reaction of the raw material 7-(benzyloxy)chroman-4-one. Saturated ammonium chloride solution was added to quench the reaction, and the resultant solution was extracted with ethyl acetate. The organic phase was washed with saturated NaCl solution, dried and concentrated, followed by column chromatography, to obtain 9.9 g of product, which was identified as the target compound by LC-MS. The yield was 65%.

### Step 10: Synthesis of 1-(4-(7-(benzyloxy)-2H-chromene-4-yl)-2-fluorophenyl)-4-(dimethoxymethyl)piperidine

7-(benzyloxy)-4-(4-(4-(dimethoxymethyl)piperidin-1-yl)-3-fluorophenyl)chroman-4-ol (9.9 g, 19.5 mmol) was dissolved in dichloromethane (100 mL), to which was added triethylamine (8 g, 80 mmol), followed by addition of methanesulfonyl chloride (4.5 g, 40 mmol) in an ice bath. After addition, the reaction was warmed to room temperature, and allowed to react for 2 h. TLC detection indicated disappearance of the starting material. Water was added to quench the reaction, and the resultant solution was extracted with dichloromethane. The organic phase was dried and concentrated, followed by column chromatography, to obtain 5.6 g of product, which was identified as the target compound by LC-MS. The yield was 60%.

### Step 11: Synthesis of 1-(4-(7-(benzyloxy)-3-bromo-2H-chromene-4-yl)-2-fluorophenyl)-4-(dimethoxymethyl)piperidine

1-(4-(7-(benzyloxy)-2*H*-chromene-4-yl)-2-fluorophenyl)-4-(dimethoxymethyl)piperidine (5.6 g, 11.7 mmol) was dissolved in DMF (40 mL), to which was added DIPEA (4.6 g, 35 mmol), and then pyridinium tribromide (5.6 g, 17.5 mmol) was added in portions in an ice bath. After addition, the reaction was warmed to room temperature and stirred overnight. TLC detection indicated disappearance of the starting material. Water was added to quench the reaction, and the resultant solution was extracted with ethyl acetate. The organic phase was dried and concentrated, and the residue was triturated in petroleum ether/ethyl acetate (6:1), to obtain 5.6 g of product, which was identified as the target compound by LC-MS. The yield was 86%.

### Step 12: Synthesis of 1-(4-(7-(benzyloxy)-3-(2-fluorophenyl)-2H-chromene-4-yl)-2-fluorophenyl)-4-(dimethoxymethyl)piperidine

To a reaction flask, were added 1-(4-(7-(benzyloxy)-3-bromo-2H-chromene-4-yl)-2-fluorophenyl)-4-(dimethoxymethyl)piperidine (5.6 g, 10 mmol), 2-flurorphenylboronic acid (3.2 g, 20 mmol), Pd(dppf)Cl₂ (0.85 g, 1 mmol), potassium carbonate (15 g, 70 mmol), and dioxane (50 mL), and then the system was purged with nitrogen. The reaction was allowed to react at 80 °C overnight. The reaction was complete by detection with TLC. The reaction solution was cooled to room temperature, filtered via diatomaceous earth, and concentrated, followed by column chromatography to obtain 4.3 g of product, which was identified as the target compound by LC-MS. Yield: 91%.

### Step 13: Synthesis of cis-4-(4-(4-(dimethoxymethyl)piperidin-1-yl)-3-fluorophenyl)-3-(2-fluorophenyl)chroman-7-ol

1-(4-(7-(benzyloxy)-3-(2-fluorophenyl)-2*H*-chromene-4-yl)-2-fluorophenyl)-4-(dimethoxy methyl)piperidine (4.3 g, 7.2 mmol) was dissolved in THF (25 mL), to which were successively added methanol (250 mL) and 10% Pd/C (800 mg, 20%), and then the system was purged with hydrogen. The reaction was allowed to react overnight at room temperature. The reaction was complete by detection with TLC. The reaction solution was filtered via diatomaceous earth to remove Pd/C, and then concentrated, followed by column chromatography to obtain 3 g of product, which was identified as the target compound by LC-MS. Yield: 82%.

### Step 14: Synthesis of 1-(2-fluoro-4-(cis-3-(2-fluorophenyl)-7-hydroxychromen-4-yl) phenyl)piperidine-4-carbaldehyde

*cis*-4-(4-(4-(dimethoxymethyl)piperidin-1-yl)-3-fluorophenyl)-3-(2-fluorophenyl)chroman -7-ol (51 mg, 0.1 mmol) was dissolved in THF (2 mL), to which was added sulfuric acid (1 mL, 2 M), and the reaction was allowed to react for 0.5 h at 70 °C. The reaction was monitored by TLC until the raw materials disappeared. The reaction solution was cooled to room temperature, and adjusted to about pH 8 with saturated NaHCO₃ solution. The resultant solution was extracted with ethyl acetate. The organic phase was washed with saturated NaCl solution, dried, and concentrated. The residue was subjected to column chromatography, to obtain 56 mg of crude product, which was directly used in the next step.

### Step 15: Synthesis of 3-(5-(7-((1-(2-fluoro-4-(cis-3-(2-fluorophenyl)-7-hydroxybenzopyran-4-yl)phenyl)piperidin-4-yl)methyl)-2,7-diazaspiro[3.5]nonane-2-yl)-1-oxoi soinodolin-2-yl)piperidin-2,6-dione

3-(1-oxo-5-(2,7-diazaspiro[3.5]nonane-2-yl)isoinodolin-2-yl)piperidin-2,6-dione trifluoroacetate (56 mg, 1.1 mmol) was dissolved in dichloromethane/methanol (2 mL, 10:1), to which was added DIPEA (40 mg, 0.3 mmol), and then the reaction was stirred at room temperature for 5 min, to which was added 1-(2-fluoro-4-(*cis*-3-(2-fluorophenyl)-7-hydroxychromen-4-yl)phenyl)piperidine-4-carbaldehyde (51 mg, 0.11 mmol), followed by addition of glacial acetic acid (18 mg, 0.3 mmol). The reaction was stirred for 0.5 h at 35 °C. The reaction solution was cooled to room temperature, and sodium triacetoxyborohydride (42 mg, 0.2 mmol) was added. The reaction was allowed to react overnight. The reaction was monitored by TLC until the raw materials disappeared, and then the reaction was quenched by adding saturated NaHCO₃ solution. The resultant solution was extracted with dichloromethane. The organic phase was dried and concentrated, and the residue was subjected to column chromatography to obtain 67 mg of product, which was identified as the target compound by LC-MS. Yield: 77%. ¹H NMR (400 MHz, DMSO-*d*₆) *δ* 10.94 (s, 1H), 9.41 (s, 1H), 7.47 (d, *J* = 8.3 Hz, 1H), 7.32 - 7.13 (m, 4H), 6.92 (t, *J* = 7.6 Hz, 1H), 6.78 - 6.65 (m, 2H), 6.49 (q, *J* = 8.5, 7.8 Hz, 2H), 6.35 - 6.28 (m, 2H), 6.19 (d, *J* = 14.0 Hz, 1H), 5.03 (dd, *J =* 13.2, 5.0 Hz, 1H), 4.48 - 4.06 (m, 5H), 3.76 - 3.73 (m, 1H), 3.63 (s, 4H), 3.50 - 3.42 (m, 6H), 3.20 (d, *J* = 11.1 Hz, 2H), 2.88 (d, *J* = 13.2 Hz, 1H), 2.57 (d, *J* = 19.3 Hz, 2H), 2.29 - 2.25 (m, 6H), 2.07 - 1.88 (m, 2H), 1.75 - 1.69 (m, 2H), 1.24 - 1.22 (m, 2H). LC/MS (ESI+) calcd for C₄₇H₄₉F₂N₅O₅ ( [M+H]⁺ ) *m*/*z:* 802.4; found 802.4.

### Example 88. 3-(5-(7-((1-(2-fluoro-4-(cis-7-hydroxyl-3-phenylchroman-4-yl)phenyl) piperidin-4-yl)methyl)-2,7-diazaspiro[3.5]nonane-2-yl)-1-oxoisoinodolin-2-yl)piperidin-2,6 -dione

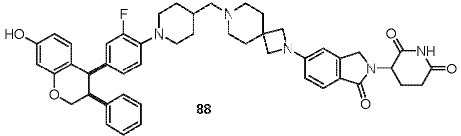

The target compound was prepared using a method similar to that in Example 60. ¹H NMR (400 MHz, DMSO-*d*₆) *δ* 10.94 (s, 1H), 9.37 (s, 1H), 7.48 (d, *J =* 8.3 Hz, 1H), 7.26 - 7.06 (m, 3H), 6.79 (dd, *J =* 6.6, 3.0 Hz, 2H), 6.73 (t, *J* = 8.8 Hz, 1H), 6.67 (d, *J =* 8.2 Hz, 1H), 6.50 (s, 1H), 6.47 (dd, *J* = 8.3, 2.0 Hz, 1H), 6.38 - 6.23 (m, 3H), 6.14 (dd, *J* = 14.2, 2.0 Hz, 1H), 5.03 (dd, *J =* 13.3, 5.1 Hz, 1H), 4.43 - 4.10 (m, 5H), 3.84 - 3.51 (m, 7H), 3.25 - 3.00 (m, 6H), 2.89 (td, *J =* 13.4, 6.9 Hz, 1H), 2.67 - 2.54 (m, 2H), 2.34 (td, *J* = 13.6, 9.1 Hz, 3H), 1.95 (d, *J* = 6.1 Hz, 1H), 1.77 - 1.75 (m, 6H), 1.29 - 1.22 (m, 2H). LC/MS (ESI+) calcd for C₄₇H₅₀FN₅O₅ ( [M+H]⁺ ) *m*/*z:* 784.4; found 784.4.

### Example 89. 3-(5-(7-((1-(4-((3S, 4R)-3-(2-fluorophenyl)-7-hydroxychroman-4-yl) phenyl)piperidin-4-yl)methyl)-2,7-diazaspiro[3.5]nonane-2-yl)-1-oxoisoinodolin-2-yl)piper idin-2,6-dione

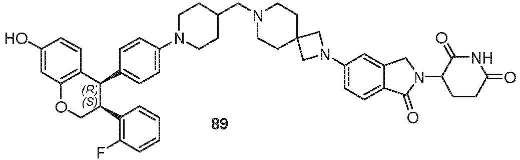

The target compound was prepared using a method similar to that in Example 78. ¹H NMR (400 MHz, DMSO-*d*₆) *δ* 10.95 (s, 1H), 9.36 (s, 1H), 7.48 (d, *J* = 8.3 Hz, 1H), 7.26 - 7.18 (m, 1H), 7.14 (ddd, *J* = 9.7, 8.2, 1.3 Hz, 1H), 6.89 (td, *J* = 7.5, 1.3 Hz, 1H), 6.67 (d, *J* = 8.3 Hz, 1H), 6.60 (d, *J* = 8.4 Hz, 2H), 6.50 (d, *J* = 1.9 Hz, 1H), 6.47 (dd, *J* = 8.3, 2.0 Hz, 1H), 6.41 (td, *J* = 8.4, 8.0, 6.1 Hz, 3H), 6.32 (d, *J* = 2.4 Hz, 1H), 6.29 (dd, *J* = 8.3, 2.4 Hz, 1H), 5.03 (dd, *J* = 13.3, 5.1 Hz, 1H), 4.36 (t, *J* = 11.0 Hz, 1H), 4.29 (d, *J* = 16.9 Hz, 1H), 4.25 - 4.13 (m, 3H), 3.74 - 3.68 (m, 1H), 3.63 (s, 4H), 3.55 - 3.52 (m, 3H), 3.36 - 3.34 (m, 1H), 2.90 (ddd, *J* = 17.5, 13.3, 5.3 Hz, 1H), 2.63 - 2.52 (m, 2H), 2.48 - 2.46 (m, 2H), 2.34 (qd, *J* = 13.2, 4.5 Hz, 4H), 2.12 - 2.10 (m, 1H), 2.00 - 1.98 (m, 1H), 1.75 - 1.72 (m, 6H), 1.27 - 1.22 (m, 2H). LC/MS (ESI+) calcd for C₄₇H₅₀FN₅O₅ ( [M+H]⁺ ) *m*/*z:* 784.4; found 784.4.

### Example 90. (R)-3-(5-(7-((1-(4-((3S, 4R)-3-(2-fluorophenyl)-7-hydroxychroman-4-yl) phenyl)piperidin-4-yl)methyl)-2,7-diazaspiro[3.5]nonane-2-yl)-1-oxoisoinodolin-2-yl)piper idin-2,6-dione

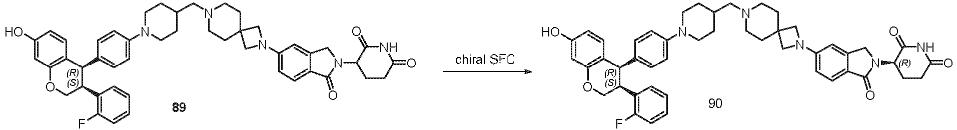

Compound **89** was separated by chiral SFC to obtain the target product. Column: CHIRALPAK AS (30*250 mm 5µm) (Daicel), mobile phase: A=CO₂, Co-Solvent B= IPA/ACN=1/1 (0.1% 7M NH₃ in MeOH), the first fraction was collected, which was the desired product. ¹H NMR (400 MHz, DMSO-*d*₆) *δ* 10.94 (s, 1H), 9.33 (s, 1H), 7.46 (dd, *J* = 12.5, 8.3 Hz, 1H), 7.32 - 7.18 (m, 1H), 7.14 (ddd, *J* = 9.6*,* 8.3, 1.4 Hz, 1H), 6.89 (td, *J =* 7.5, 1.3 Hz, 1H), 6.67 (d, *J* = 8.3 Hz, 1H), 6.60 (d, *J* = 8.5 Hz, 2H), 6.53 - 6.49 (m, 1H), 6.47 (dd, *J* = 8.4, 2.0 Hz, 1H), 6.45 - 6.41 (m, 1H), 6.39 (d, *J* = 8.5 Hz, 2H), 6.31 (d, *J* = 2.3 Hz, 1H), 6.28 (dd, *J* = 8.2, 2.4 Hz, 1H), 5.03 (dd, *J =* 13.3, 5.1 Hz, 1H), 4.36 (t, *J =* 11.0 Hz, 1H), 4.29 (d, *J* = 16.9 Hz, 1H), 4.25 - 4.11 (m, 3H), 3.70 (dt, *J =* 11.4, 4.4 Hz, 1H), 3.61 (s, 4H), 3.52 (d, *J =* 15.7 Hz, 3H), 3.32 - 3.25 (m, 1H), 2.96 - 2.84 (m, 1H), 2.70 - 2.52 (m, 2H), 2.46 - 2.48 (m, 2H), 2.39 - 2.18 (m, 2H), 2.12 - 2.09 (m, 3H), 1.97 - 1.90 (m, 1H), 1.71 - 1.73 (m, 6H), 1.27 - 1.22 (m, 2H). LC/MS (ESI+) calcd for C₄₇H₅₀FN₅O₅( [M+H]⁺ ) *m*/*z:* 784.4; found 784.4.

### Example 91. (S)-3-(5-(7-((1-(4-((3S, 4R)-3-(2-fluorophenyl)-7-hydroxychroman-4-yl) phenyl)piperidin-4-yl)methyl)-2,7-diazaspiro[3.5]nonane-2-yl)-1-oxoisoinodolin-2-yl)piper idin-2,6-dione

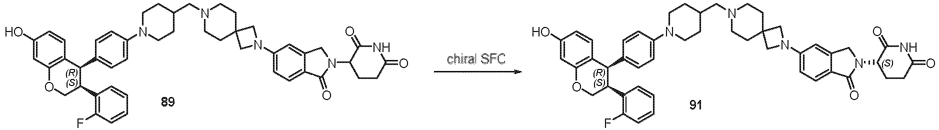

Compound **89** was separated by chiral SFC to obtain the target product. Column: CHIRALPAK AS(30*250mm 5µm) (Daicel), mobile phase: A=CO₂, Co-Solvent B= IPA/ACN=1/1(0.1% 7M NH₃ In MeOH), the second fraction was collected, which was the desired product. ¹H NMR (400 MHz, DMSO-*d*₆) *δ* 10.94 (s, 1H), 9.33 (s, 1H), 7.46 (dd, *J* = 12.5, 8.3 Hz, 1H), 7.32 - 7.18 (m, 1H), 7.14 (ddd, *J* = 9.6, 8.3, 1.4 Hz, 1H), 6.89 (td, *J* = 7.5, 1.3 Hz, 1H), 6.67 (d, *J* = 8.3 Hz, 1H), 6.60 (d, *J* = 8.5 Hz, 2H), 6.53 - 6.49 (m, 1H), 6.47 (dd, *J* = 8.4, 2.0 Hz, 1H), 6.45 - 6.41 (m, 1H), 6.39 (d, *J =* 8.5 Hz, 2H), 6.31 (d, *J =* 2.3 Hz, 1H), 6.28 (dd, *J =* 8.2, 2.4 Hz, 1H), 5.03 (dd, *J* = 13.3, 5.1 Hz, 1H), 4.36 (t, *J* = 11.0 Hz, 1H), 4.29 (d, *J* = 16.9 Hz, 1H), 4.25 - 4.11 (m, 3H), 3.70 (dt, *J* = 11.4, 4.4 Hz, 1H), 3.61 (s, 4H), 3.52 (d, *J* = 15.7 Hz, 3H), 3.32 - 3.25 (m, 1H), 2.96 - 2.84 (m, 1H), 2.70 - 2.52 (m, 2H), 2.46 - 2.48 (m, 1H), 2.39 - 2.18 (m, 3H), 2.10 (d, *J* = 7.0 Hz, 3H), 1.97 - 1.90 (m, 1H), 1.71 - 1.73 (m, 6H), 1.27 - 1.22 (m, 2H). LC/MS (ESI+) calcd for C₄₇H₅₀FN₅O₅ ( [M+H]⁺ ) *m*/*z:* 784.4; found 784.4.

### Example 92. 3-(5-(7-((1-(4-(cis-7-hydroxyl-3-(2,2,2-trifluoroethyl)chroman-4-yl) phenyl)piperidin-4-yl)methyl)-2,7-diazaspiro[3.5]nonane-2-yl)-1-oxoisoinodolin-2-yl)piper idin-2,6-dione

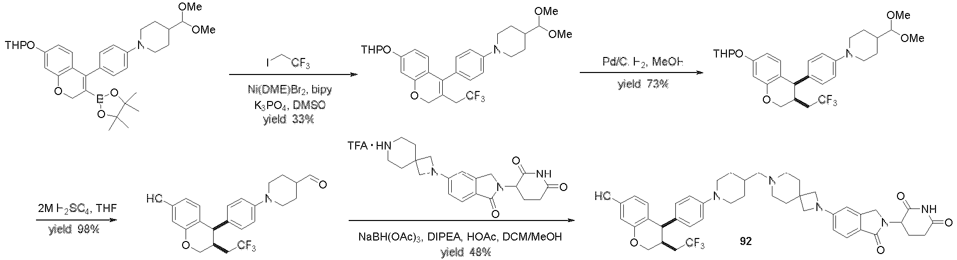

### Step 1: Synthesis of 4-(dimethoxymethyl)-1-(4-(7-((tetrahydro-2H-pyran-2-yl)oxy)-3-(2,2,2-trifluoroethyl)-2H-chromene-4-yl)phenyl)piperidine

To the high-pressure sealing tube, were added 4-(dimethoxymethyl)-1-(4-(7-((tetrahydro-2H-pyran-2-yl)oxy)-3-(4,4,5,5-tetramethyl-1,3,2-dioxaborolan-2-yl)-2H-chromene-4-yl)phenyl) piperidine (591 mg, 1 mmol), nickel(II) bromide ethylene glycol dimethyl ether complex (31 mg, 0.1 mmol), bipyridine (31 mg, 0.2 mmol), potassium phosphate (640 mg, 3 mmol) and dimethyl sulfoxide (5 mL), and the system was purged with nitrogen. 2-iodo-1,1,1-trifluoroethane (630 mg, 3 mmol) was added to the system under nitrogen atmosphere, and then the reaction was allowed to react overnight at 80 °C under sealed conditions. The reaction was monitored by TLC until the raw materials disappeared, and then cooled to room temperature. The reaction solution was filtered via diatomaceous earth. The filtrate was concentrated, and the residue was subjected to column chromatography, to obtain 180 mg of product, which was identified as the target compound by LC-MS. Yield: 33%.

### Step 2: Synthesis of 4-(dimethoxymethyl)-1-(4-(cis-7-((tetrahydro-2H-pyran-2-yl)oxy)-3-(2,2,2-trifluoroethyl)chroman-4-yl)phenyl)piperidine

4-(dimethoxymethyl)-1-(4-(7-((tetrahydro-2H-pyran-2-yl)oxy)-3-(2,2,2-trifluoroethyl)-2H-chromene-4-yl)phenyl)piperidine (180 mg, 0.33 mmol) was dissolved in THF (2 mL), to which were successively added methanol (25 mL) and 10% Pd/C (20 mg, 10%), and then the system was purged with hydrogen. The reaction was allowed to react overnight at room temperature. The reaction was complete by detection with TLC. The reaction solution was filtered via diatomaceous earth to remove Pd/C, and then concentrated, followed by column chromatography to obtain 3 g of product, which was identified as the target compound by LC-MS. Yield: 73%.

### Step 3: Synthesis of 1-(4-(cis-7-hydroxyl-3-(2,2,2-trifluoroethyl)chroman-4-yl)phenyl) piperidine-4-carbaldehyde

4-(dimethoxymethyl)-1-(4-(*cis*-7-((tetrahydro-2*H*-pyran-2-yl)oxy)-3-(2,2,2-trifluoroethyl)c hroman-4-yl)phenyl)piperidine (130 mg, 0.23 mmol) was dissolved in THF (2 mL), to which was added sulfuric acid (1 mL, 2 M), and the reaction was allowed to react for 0.5 h at 70 °C. The reaction was monitored by TLC until the raw materials disappeared. The reaction solution was cooled to room temperature, and adjusted to about pH 8 with saturated NaHCO₃ solution. The resultant solution was extracted with ethyl acetate. The organic phase was washed with saturated NaCl solution, dried, and concentrated. The residue was subjected to column chromatography, to obtain 130 mg of crude product, which was directly used in the next step.

### Step 4: Synthesis of 3-(5-(7-((1-(4-(cis-7-hydroxyl-3-(2,2,2-trifluoroethyl)chroman-4-yl) phenyl)piperidin-4-yl)methyl)-2,7-diazaspiro[3.5]nonane-2-yl)-1-oxoisoinodolin-2-yl)piperidin -2,6-dione

Crude product 3-(1-oxo-5-(2,7-diazaspiro[3.5]nonane-2-yl)isoinodolin-2-yl)piperidin-2,6-dione trifluoroacetate (51 mg, 0.11 mmol) was dissolved in dichloromethane/methanol (2 mL, 10:1), to which was added DIPEA (40 mg, 0.3 mmol), and then the reaction was stirred at room temperature for 5 min, to which was added 1-(4-(cis-7-hydroxyl-3-(2,2,2-trifluoroethyl) chroman-4-yl)phenyl)piperidine-4-carbaldehyde (42 mg, 0.1 mmol), followed by addition of glacial acetic acid (18 mg, 0.3 mmol). The reaction was stirred for 0.5 h at 35 °C. The reaction solution was cooled to room temperature, and sodium triacetoxyborohydride (42 mg, 0.2 mmol) was added. The reaction was allowed to react overnight. The reaction was monitored by TLC until the raw materials disappeared, and then the reaction was quenched by adding saturated NaHCO₃ solution. The resultant solution was extracted with dichloromethane. The organic phase was dried and concentrated, and the residue was subjected to column chromatography to obtain 37 mg of product, which was identified as the target compound by LC-MS. Total yield for two steps: 48%. ¹H NMR (400 MHz, DMSO-*d*₆) *δ* 10.94 (s, 1H), 9.30 (s, 1H), 7.47 (d, *J* = 8.3 Hz, 1H), 6.88 (q, *J* = 8.7 Hz, 3H), 6.82 - 6.72 (m, 1H), 6.64 (dd, *J* = 9.1, 5.8 Hz, 1H), 6.55 - 6.42 (m, 2H), 6.31 - 6.18 (m, 2H), 5.03 (dd, *J* = 13.3, 5.1 Hz, 1H), 4.29 (d, *J* = 16.9 Hz, 1H), 4.16 (d, *J =* 17.0 Hz, 1H), 4.08 (dd, *J* = 18.1, 8.0 Hz, 2H), 3.89 (t, *J* = 10.9 Hz, 1H), 3.61 - 3.63 (m, 5H), 3.29 - 3.22 (m, 1H), 3.05 - 2.76 (m, 2H), 2.69 - 2.51 (m, 4H), 2.33 (d, *J* = 17.3 Hz, 6H), 2.13 (d, *J* = 7.2 Hz, 2H), 2.04 - 1.88 (m, 2H), 1.73 - 1.75 (m, 6H), 1.29 - 1.22 (m, 2H). LC/MS (ESI+) calcd for C₄₃H₄₈F₃N₅O₅ ( [M+H]⁺ ) *m*/*z*: 772.4; found 772.4.

### Example 93: 3-deutero-3-(5-(7-((1-(4-((3S,4R)-3-(2,6-difluorophenyl)-7-hydroxychroman-4-yl)-2-fluorophenyl)piperidin-4-yl)methyl)-2,7-diazaspiro[3.5]nonane-2 -yl)-1-oxoisoinodolin-2-yl)piperidin-2,6-dione

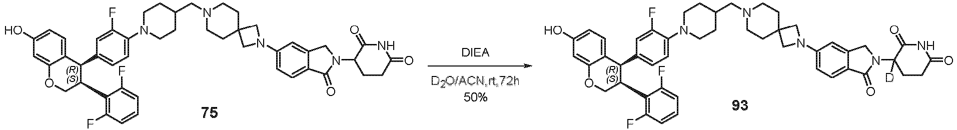

Compound **75** (100 mg, 0.12 mmol) was dissolved in the mixed solvent of 2 mL of deuterium oxide and 4 mL of acetonitrile, to which was added four drops of DIEA, and then the reaction was stirred for 72 h at room temperature. The reaction solution was diluted with DCM. The organic phase was respectively washed with water and saturated NaCl solution, dried over anhydrous Na₂SO₄ and concentrated. The residue was subjected to column chromatography to obtain the target compound (55 mg, 0.06 mmol), and the deuterated ratio was 84%, with a yield of 50%. LC/MS (ESI+) calcd for C₄₇H₄₇DF₃N₅O₅ (M + H⁺) *m*/*z*, 821.3; found 821.3. ¹H NMR (400 MHz, DMSO-*d*₆) *δ* 10.94 (s, 1H), 9.41 (s, 1H), 7.47 (d, *J =* 8.3 Hz, 1H), 7.38 - 7.29 (m, 1H), 6.96 (t, *J* = 9.3 Hz, 2H), 6.76 (t, *J* = 8.8 Hz, 1H), 6.69 (d, *J* = 9.1 Hz, 1H), 6.52 - 6.44 (m, 2H), 6.33 (ddt, *J* = 9.0, 5.0, 2.2 Hz, 3H), 6.23 (dd, *J* = 14.2, 2.0 Hz, 1H), 4.48 (d, J = 11.5 Hz, 1H), 4.29 (d, J = 16.5 Hz, 2H), 4.20 - 4.12 (m, 2H), 3.81 (dt, J = 11.6, 4.8 Hz, 1H), 3.62 (s, 4H), 3.22 (d, J = 8.7 Hz, 2H), 2.88 (d, J = 13.0 Hz, 1H), 2.57 (d, J = 18.7 Hz, 2H), 2.40 - 2.23 (m, 4H), 2.12 (d, J = 6.8 Hz, 2H), 1.97 - 1.87 (m, 1H), 1.74 (s, 6H), 1.59 (s, 1H), 1.22 (d, J = 7.7 Hz, 2H).

In Examples 94-100, compounds **94-100** could be obtained by a method similar to that of Example 93.

### Example 101. (cis-4-(4-(4-((2-(2-(2,6-dioxopiperidin-3-yl)-1-oxoisoinodolin-5-yl)-2,7-diazaspiro [3.5] nonan-7-yl)methyl)piperidin-1-yl)phenyl)-3-(2-fluorophenyl)pyran-7-yl)bor ic acid

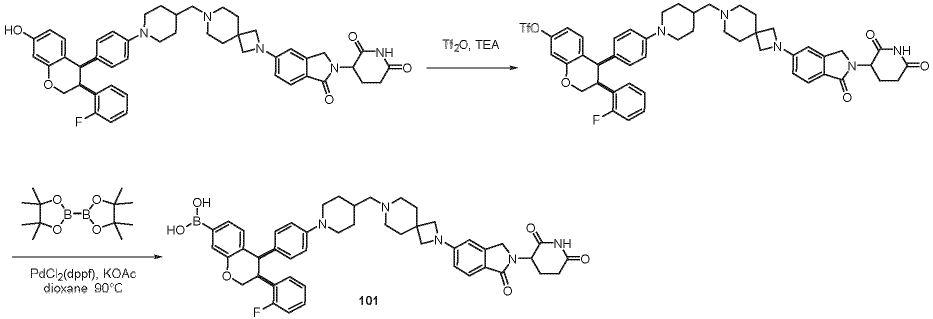

Using a method similar to that of Example 102, (*cis*)-4-(4-(4-((2-(2-(2,6-dioxopiperidin-3-yl)-1-oxoisoinodolin-5-yl)-2,7-diazaspiro[3.5]nonan-7-yl)methyl)piperidin-1-yl)phenyl)-3-(2-fluorophenyl)chroman-7-yltrifluoromethanesulfonate was prepared. (*cis*)-4-(4-(4-((2-(2-(2,6-dioxopiperidin-3-yl)-1-oxoisoinodolin-5-yl)-2,7-diazaspiro[3.5]nonan-7-yl)methyl)piperidin-1-yl)phenyl)-3-(2-fluorophenyl)chroman-7-yltrifluoromethanesulfonate (50 mg, 0.05 mmol) and bis(pinacolato)diboron (28 mg, 0.11 mmol) were dissolved in 1,4-dioxane (5 mL), to which were added PdCl₂(dppf) (4 mg, 0.005 mmol) and KOAc (20 mg, 0.11 mmol), and then the system was purged with Ar for three times. The reaction was allowed to react overnight at 90 °C. After completion of the reaction, the reaction was cooled to room temperature, and queched by adding water. The resultant solution was extracted with ethyl acetate. The organic phase was rotary evaporated to dry, and the residue was separated and purified by prep-TLC to obtain the target compound (10 mg), with a yield of 22%. LC/MS (ESI+) calcd for C₄₇H₅₁BFN₅O₆ ( [M+H]⁺ ) *m*/*z* 811.4; found 812.3.

¹H NMR (400 MHz, DMSO-*d*₆) *δ* 10.94 (s, 1H), 8.01 (s, 2H), 7.47 (d, *J =* 8.3 Hz, 1H), 7.36 (d, *J* = 1.3 Hz, 1H), 7.28 - 7.20 (m, 2H), 7.14 (dd, *J =* 8.0, 2.6 Hz, 1H), 6.93 - 6.82 (m, 2H), 6.61 (d, *J* = 8.4 Hz, 2H), 6.52 - 6.35 (m, 5H), 5.03 (dd, *J =* 13.2, 5.0 Hz, 1H), 4.43 (t, *J* = 11.1 Hz, 1H), 4.35 - 4.14 (m, 4H), 3.76 (dd, *J* = 10.3, 5.6 Hz, 1H), 3.62 (s, 4H), 3.53 (d, *J =* 11.5 Hz, 2H), 2.93 - 2.84 (m, 1H), 2.57 (d, *J* = 22.2 Hz, 3H), 2.38 - 2.23 (m, 4H), 2.10 (s, 2H), 1.96 (dd, *J =* 12.6, 6.3 Hz, 2H), 1.74 (s, 5H), 1.60 (s, 1H), 1.46 (d, *J* = 7.6 Hz, 1H), 1.11 (d, *J =* 12.3 Hz, 2H).

### Example 102. cis-4-(4-(4-((2-(2-(2,6-dioxopiperidin-3-yl)-1-oxoisoinodolin-5-yl)-2,7-diazaspiro [3.5] nonan-7-yl)methyl)piperidin-1-yl)phenyl)-3-(2-fluorophenyl)chroman-7-for mic acid

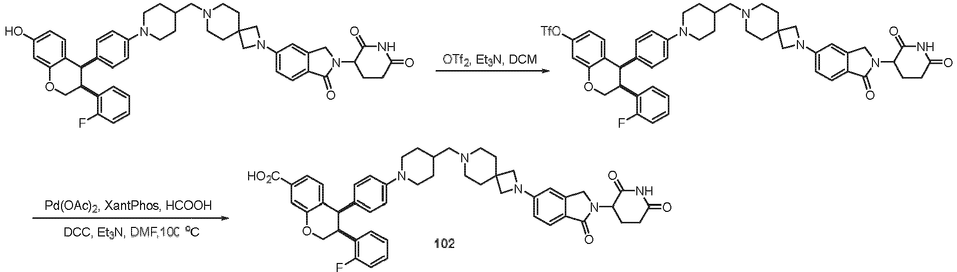

### Step 1: Synthesis of cis-4-(4-(4-((2-(2-(2,6-dioxopiperidin-3-yl)-1-oxoisoindol-5-yl)-2,7-diazaspiro[3.5]nonan-7-yl)methyl)piperidin-1-yl)phenyl)-3-(2-fluorophenyl)chroman-7-yltr ifluoromethanesulfonate

3-(5-(7-((1-(4-(*cis*-3-(2-fluorophenyl)-7-hydroxychroman-4-yl)phenyl)piperidin-4-yl)meth yl)-2,7-diazaspiro[3.5]nonane-2-yl)-1-oxoisoinodolin-2-yl)piperidin-2,6-dione (783 mg, 1 mmol) was dissolved in dichloromethane (15 mL), to which was added triethylamine (505 mg, 5 mmol), followd by slowly adding trifluoromethanesulfonic anhydride (846 mg, 3 mmol), and then the reaction was allowed to react overnight at room temperature. The reaction was no longer proceeding according to TLC detection, and then quenched by adding water. The resultant solution was extracted with dichloromethane. The organic phase was washed with saturated NaCl solution, and concentrated to dry. The residue was subjected to column chromatography to obtain 360 mg of product, which was identified as the target compound by LC-MS. Yield: 39%.

### Step 2: Synthesis of cis-4-(4-(4-((2-(2-(2,6-dioxopiperidin-3-yl)-1-oxoisoinodolin-5-yl)-2,7-diazaspiro[3.5]nonan-7-yl)methyl)piperidin-1-yl)phenyl)-3-(2-fluorophenyl)chroman-7-formic acid

Palladium(II) acetate (7 mg, 0.03 mmol) and XantPhos (17.3 mg, 0.03 mmol) were added into a sealed tube, and then the reaction system was purged with nitrogen. Subsequently, the solution of *cis*-4-(4-(4-((2-(2-(2,6-dioxopiperidin-3-yl)-1-oxoisoindol-5-yl)-2,7-diazaspiro[3.5]nonan-7-yl)methyl)piperidin-1-yl)phenyl)-3-(2-fluorophenyl)chroman-7-yltriflu oromethanesulfonate (305 mg, 0.3 mmol) in DMF (2 mL) and formic acid (86 mg, 1.5 mmol) were added into the sealed tube under nitrogen atmosphere. Then, triethylamine (90 mg, 0.9 mmol) and DCC (7 mg, 0.03 mmol) were also added. The tube was sealed, and the reaction was allowed to react overnight at 100 °C. The reaction was no longer proceeding according to TLC detection, and then cooled to room temperature, followed by filtering via diatomaceous earth. The filtrate was concentrated, and the residue was subjected to column chromatography to obtain 12 mg of product, with a yield of 5%. ¹H NMR (400 MHz, DMSO-*d*₆) *δ* 10.94 (s, 1H), 7.48 - 7.46 (m, 2H), 7.40 (dd, *J* = 8.0, 1.6 Hz, 1H), 7.27 - 7.22 (m, 1H), 7.15 (td, *J =* 10.4, 2.8 Hz, 1H), 7.03 (d, *J =* 8.0 Hz, 1H), 6.92 (td, *J =* 7.6, 1.2 Hz, 1H), 6.68 - 6.61 (m, 2H), 6.50 - 6.45 (m, 3H), 6.41 (d, *J* = 8.4 Hz, 2H), 5.02 (dd, *J =* 13.2, 4.8 Hz, 1H), 4.48 (t, *J* = 11.2 Hz, 1H), 4.40 - 4.35 (m, 2H), 4.29 (d, *J* = 17.2 Hz, 1H), 4.16 (d, *J* = 16.8 Hz, 1H), 3.81 - 3.76 (m, 1H), 3.62 (s, 4H), 3.57 - 3.48 (m, 3H), 3.36 - 3.34 (m, 1H), 2.94 - 2.85 (m, 1H), 2.60 - 2.51 (m, 2H), 2.48 - 2.46 (m, 2H), 2.39 - 2.30 (m, 4H), 2.11 (d, *J* = 7.2 Hz, 2H), 1.96 - 1.91 (m, 1H), 1.75 - 1.72 (m, 5H), 1.23 - 1.22 (m, 2H). LC/MS (ESI+) calcd for C₄₈H₅₀FN₅O₆ ( [M+H]⁺ ) *m*/*z*: 812.4; found 812.4.

### Example 103. N-(2,6-dioxopiperidin-3-yl)-2-fluoro-4-(7-((1-(4-(cis-3-(2-fluorophenyl)-7-hydroxychroman-4-yl)phenyl)piperidin-4-yl)methyl)-2,7-diazaspiro[3.5]nonane-2-yl)ben zamide

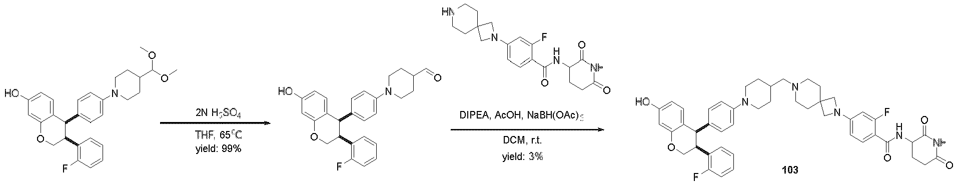

### Step 1: Synthesis of 1-(4-(cis-3-(2-fluorophenyl)-7-hydroxychroman-4-yl)phenyl) piperidine-4-carbaldehyde

cis-4-(4-(4-(dimethoxymethyl)piperidin-1-yl)phenyl)-3-(2-fluorophenyl)pyran-7-ol (100 mg, 0.23 mmol) was dissolved in THF (3 mL), to which was added H₂SO₄ (2N, 1mL), and then the system was purged with argon. The reaction was allowed to react for 30 min at 65 °C. The reaction was monitored by TLC until the raw materials disappeared. The reaction solution was cooled to room temperature, and adjusted to pH 7-8 with saturated NaHCO₃ solution. The resultant solution was extracted with ethyl acetate. The organic phase was washed with saturated NaCl solution, dried, and concentrated, to obtain 90 mg of crude product as pink oils. LC/MS (ESI+) calcd for C₂₇H₂₆FNO₃ ( [M+H]⁺ ) *m*/*z:* 432.2; found 450.2. Yield: 99%.

### Step 2: Synthesis of N-(2,6-dioxopiperidin-3-yl)-2-fluoro-4-(7-((1-(4-(cis-3-(2-fluorophenyl)-7-hydroxychroman-4-yl)phenyl)piperidin-4-yl)methyl)-2,7-diazaspiro[3.5]nonan e-2-yl)benzamide

The crude product N-(2,6-dioxopiperidin-3-yl)-2-fluoro-4-(2,7-diazaspiro[3.5]nonane-2-yl) benzamide trifluoroacetate (79 mg, 0.21 mmol) and 1-(4-(cis-3-(2-fluorophenyl)-7-hydroxychroman-4-yl)phenyl)piperidine-4-carbaldehyde (90 mg, 0.21 mmol) were dissolved in dichloromethane/methanol (4 mL, 4:1), to which was added glacial acetic acid (38 mg, 0.63 mmol), and then the reaction was stirred at room temperature for 1 h, followed by addition of sodium triacetoxyborohydride (134 mg, 0.63 mmol). The reaction was allowed to react overnight. The reaction was monitored by TLC until the raw materials disappeared, and then quenched by adding saturated NaHCO₃ solution. The resultant solution was extracted with dichloromethane. The organic phase was dried and concentrated, and the residue was subjected to column chromatography, to obtain 6 mg of white solids, with a total yield of 3% for two steps.

¹H NMR (400 MHz, DMSO-*d₆*) *δ* 10.84 (s, 1H), 9.32 (s, 1H), 7.97 (d, *J =* 7.1 Hz, 1H), 7.59 (d, *J* = 8.9 Hz, 1H), 7.22 (s, 1H), 7.17 - 7.11 (m, 1H), 6.99 (d, *J =* 10.7 Hz, 1H), 6.89 (t, *J* = 7.5 Hz, 1H), 6.67 (d, *J =* 8.4 Hz, 1H), 6.60 (d, *J* = 8.5 Hz, 2H), 6.54 (s, 1H), 6.41 (t, *J* = 9.2 Hz, 2H), 6.32 - 6.26 (m, 2H), 6.22 (d, *J =* 13.3 Hz, 1H), 4.74 - 4.68 (m, 1H), 4.35 (d, *J =* 10.9 Hz, 1H), 4.22 (d, *J =* 10.2 Hz, 1H), 4.03 (d, *J =* 7.2 Hz, 1H), 3.70 (s, 1H), 3.61 (s, 4H), 3.53 (d, *J* = 12.4 Hz, 3H), 2.69 - 2.66 (m, 1H), 2.35 - 2.31 (m, 1H), 2.28 (s, 3H), 2.16 - 2.04 (m, 3H), 2.00 (d, *J* = 7.5 Hz, 2H), 1.90 (s, 1H), 1.73 (s, 6H), 1.61 - 1.56 (m, 1H), 1.17 (t, *J* = 7.1 Hz, 2H). LC/MS (ESI+) calcd for C₄₆H₄₉F₂N₅O₅ ([M+H]⁺) *m*/*z:* 790.4; found 790.4.

The beneficial effects of the present invention were demonstrated with reference to the following experimental examples.

### Experimental example 1. The degradation activities of the compounds according to the present invention on ER (ELISA)

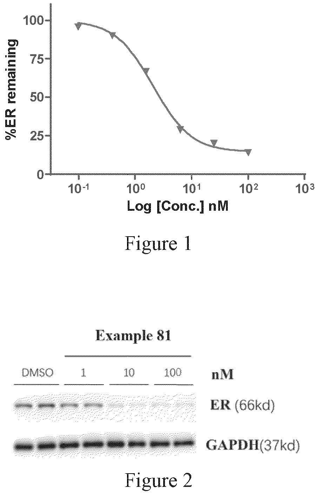

T47D breast cancer cells were subcultured in cell culture medium, and then the cells in good growth condition were seeded into 96-well plates at 80 µL/well, with 2.5×10⁴ cells per well. The cells were incubated in a 37 °C, 5% CO₂ incubator for 3 days. The drug was prepared into a 10 mM stock solution with dimethyl sulfoxide (DMSO). Prior to use, the stock solution was diluted with cell culture medium to obtain six final concentrations (100 nM, 25 nM, 6.25 nM, 1.56 nM, 0.39 nM, and 0.98 nM), and gently mixed. Additionally, negative control wells (only containing culture medium) and positive control wells (only containing cells and DMSO) were included. After the cells were incubated in the incubator for 24 h, the cells were rinsed once with ice-cold 1× PBS. The PBS was discarded, and 60 µL of ice-cold 1× cell lysis buffer was added to each well of the culture plate, followed by incubation on ice for 10 min. 50 µL of diluted sample was pipetted into each well of an ELISA plate. The plate was sealed with plastic film, and incubated in a 37°C incubator for 2 h. The film was removed. 200 µL of 1× washing buffer was added to each well, and then the plate was shaken for 5 min. The washing buffer was discarded, and the plate was pat-dried. The plate was washed four times. 100 µL of freshly prepared detection antibody (green) was added to each well of the ELISA plate. The plate was sealed with plastic film, and then incubated in a 37 °C incubator for 1 h. The film was removed, and the detection antibody was discarded. The plate was pat-dried. 200 µL of 1× washing buffer was added to each well, and then the plate was shaken for 5 min. The washing buffer was discarded, and the plate was pat-dried. The plate was washed four times. 100 µL of freshly prepared HRP-labeled secondary antibody (red) was added to each well of the ELISA plate. The plate was sealed with plastic film, and incubated in a 37 °C incubator for 30 min. The film was removed, the secondary antibody was discarded, and the plate was pat-dried, followed by adding 200 µL of 1× washing buffer to each well. The plate was shaken for 5 min, and the washing buffer was discarded before pat-drying the plate. The plate was washed four times. 100 µl of TMB substrate was added to each well of the ELISA plate, followed by incubating in a 37 °C incubator for 5 min. 100 µL of STOP solution was added to each well of the ELISA plate, and the plate was gently shaken for a few seconds. The bottom of each well was wiped with lint-free cloth. Within 30 min after adding the STOP solution, the absorbance was read at 450 nm. Use the formula: remaining ERα% = 100 * (OD value of the test well - OD value of the blank control well) / (OD value of the positive control well - OD value of the blank control well). The data were analyzed using the Dose-response equation in GraphPad Prism8 software to obtain the DC₅₀ and Dₘₐₓ values, and both of them are important indicators for measuring the degradation activities of compounds on target proteins. The DC₅₀ value represents the concentration of compound required to degrade 50% of the target protein. The Dₘₐₓ value represents the maximum percentage of target protein that can be degraded by the compound. The DC₅ₒ and Dₘₐₓ values of each compound are shown in Table 1. Taking Example 22 as an example, the experimental curve is shown in Figure 1.

**Table 1. The results for the degradation activities of the compounds according to the present invention on ER.**

| Example | DC₅₀ (nM) | Dₘₐₓ | | Example | DC₅₀ (nM) | Dₘₐₓ |
|---|---|---|---|---|---|---|
| 1 | A | B | | 47 | B | B |
| 2 | D | C | | 48 | A | B |
| 3 | D | C | | 49 | A | B |
| 5 | D | B | | 50 | A | B |
| 9 | A | B | | 51 | D | C |
| 11 | A | B | | 52 | D | C |
| 12 | B | A | | 53 | D | C |
| 13 | A | A | | 54 | D | C |
| 14 | A | A | | 55 | D | C |
| 15 | D | C | | 60 | A | B |
| 16 | A | B | | 63 | A | B |
| 17 | A | B | | 65 | A | B |
| 18 | A | A | | 66 | D | C |
| 19 | A | A | | 67 | A | A |
| 20 | A | A | | 68 | A | A |
| 21 | D | C | | 69 | A | A |
| 22 | A | A | | 70 | A | B |
| 23 | B | A | | 71 | D | C |
| 24 | D | C | | 72 | D | C |
| 25 | D | C | | 73 | A | B |
| 26 | A | B | | 74 | D | C |
| 27 | D | C | | 75 | A | A |
| 30 | A | B | | 76 | A | A |
| 32 | A | B | | 77 | C | B |
| 33 | A | B | | 78 | A | A |
| 34 | D | C | | 79 | A | B |
| 35 | B | B | | 81 | B | B |
| 36 | A | B | | 87 | A | B |
| 37 | A | B | | 88 | A | B |
| 38 | D | C | | 89 | A | B |
| 39 | D | C | | 90 | D | C |
| 40 | A | B | | 91 | A | A |
| 41 | D | C | | 92 | B | B |
| 42 | A | B | | | | |
| 43 | D | C | | | | |
| 44 | D | C | | | | |
| 45 | A | A | | | | |
| 46 | B | B | | | | |

| | | | | | | |
|---|---|---|---|---|---|---|
| Note: DC₅₀: A: <10 nM, B: 10-50 nM, C: 50-100 Nm, D: >100 nM. | | | | | | |

Dmax: A: > 75%, B: 75-50%, C: <50%.

As shown in Table 1, the compounds of the present invention exhibited good degradation activities on ER, in which compounds **12-13, 18-20, 22, 45,** 67-69, 75-76, 78, and **91** demonstrated better activities.

### Experimental example 2. Degradation activities of the compounds according to the present invention on ER (Western Blotting)

T47D breast cancer cells were subcultured in cell culture medium, and the cells in good growth condition were seeded into 6-well plate at 2 mL/well, with 2.5 × 10⁵ cells per well. The cells were incubated overnight in a 37 °C incubator under 5% CO₂. The drug was prepared into 10 mM stock solution with dimethyl sulfoxide (DMSO). Prior to use, the stock solution was diluted with DMSO in a ratio of 1:3. 2 µL of the diluted compound was added to the cell culture wells (to ensure a DMSO concentration of 0.1% in the culture system), and each concentration was repeated twice. The plate were gently shaken to mix. Additionally, the negative control wells (containing equal amount of DMSO) and the positive control wells were set up. After 24 hours of culture, the cells were lysed with RIPA cell lysis buffer to extract proteins, and the protein concentration was measured using a BCA kit. The protein loading buffer (5×) was added, and the plate was heated at 100 °C for 5 min. Then, the sample was stored at -20 °C. 30 µg of protein collected from each well was loaded onto a polyacrylamide gel for electrophoresis. The protein is transferred from the polyacrylamide gel to a PVDF membrane, blocked with 5% skim milk at room temperature for 1 h, and incubated overnight at 4 °C with primary antibodies Anti-ERα rabbit mAb (CST, Cat#: 8644S) and Anti-GAPDH rabbit mAb (CST, Cat#: 2118L). The membrane was washed three times with TBST solution, with 10 min for each time. The secondary antibody (horseradish peroxidase-labeled goat anti-rabbit IgG) was incubated at room temperature for 2 h, and then the membrane was washed three times with TBST solution, with 10 min for each time. Finally, ECL chromogenic solution was added for color development, and an automatic chemiluminescence instrument was used to take pictures. Images were collected and analyzed. The results showed that the compounds of the present invention had good degradation activity towards ER.

Taking Example 81 as an example, the ER degradation results obtained by western blotting are shown in Figure 2, indicating that the compound exhibited good ER degradation activity. The degradation activities of the compounds according to the present invention towards ER, as tested by western blotting, are presented in Table 2

**Table 2. The degradation activities of the compounds according to the present invention on ER tested by Western blot.**

| Example # | Compound concentration | Percentage reduction of ER | | Example # | Compound concentration | Percentage reduction of ER |
|---|---|---|---|---|---|---|
| 1 | 10 nM | 71% | | 55 | 100 nM | 25% |
| 2 | 1 nM | 53% | | 56 | 1 nM | 59% |
| 4 | 10 nM | 74% | | 57 | 1 nM | 77% |
| 8 | 1 nM | 47% | | 58 | 1 nM | 76% |
| 11 | 10 nM | 84% | | 59 | 10 nM | 54% |
| 12 | 10 nM | 84% | | 63 | 10 nM | 51% |
| 25 | 10 nM | 47% | | 79 | 1 nM | 70% |
| 26 | 1 nM | 56% | | 80 | 1 nM | 50% |
| 27 | 1 nM | 78% | | 81 | 10 nM | 82% |
| 30 | 1 nM | 62% | | 82 | 10 nM | 49% |
| 31 | 100 nM | 50% | | 83 | 1 nM | 87% |
| 32 | 10 nM | 81% | | 88 | 10 nM | 47% |
| 33 | 1 nM | 53% | | 89 | 1 nM | 52% |
| 34 | 100 nM | 51% | | | | |
| 53 | 1 nM | 39% | | | | |
| 54 | 100 nM | 57% | | | | |
| | | | | | | |

As shown in Table 2, the compounds of the present invention exhibited good degradation activities towards ER.

### Experimental example 3. Inhibitory activities of the compounds according to the present invention on the proliferation of breast cancer cells

After 1.6% agarose and 0.7% agarose were sterilized at high temperature, they were cooled to around 50 °C, and placed in a 42 °C water bath for later use. MCF-7 breast cancer cells were subcultured in complete cell culture medium, and cells in good growth condition were digested and counted for future use. 50 µL of 1.6% agarose mixed with 2× culture medium at a ratio of 1:1 was spread into the bottom of 96-well plate, and allowed to cool and solidify. 0.7% agarose was mixed with diluted cell suspension in a ratio of 1:1, and then 50 µL of the mixture of gel and cells was added to each well, with 2000 cells for each well; the mixture was allowed to cool and solidify at room temperature. 50 µL of culture medium was added to the plate containing cells, and the plate was placed in a 37°C, 5% CO₂ incubator and cultured for 24 h. The drug was prepared into 10 mM stock solution with dimethyl sulfoxide (DMSO). The stock solution was diluted in a ratio of 1:3, to obtain 9 concentration gradients. Then, each concentration of compound solution was further diluted with culture medium in 1:250 (to ensure a DMSO concentration of 0.1% in the culture system). Two replicate wells was set for each concentration. 50 µL of the diluted compound solution was added to the cell culture wells (with final concentrations of 10 µM, 3.3 µM, 1.1 µM, 0.37 µM...). The plate was gently shaken for mixing. Additionally, 3 negative control wells only containing cells and 3 blank control wells only containing culture medium were set up (each of the 6 wells was added with 50 µL of DMSO diluted with culture medium in a ration of 1:250). After 15 days of cultivation, the cell plates and CellTiter-Glo 3D Cell Viability were taken out and equilibrated to room temperature in advance. 80 µL of culture medium were aspirated and discarded from the cell plate, and 120 µL of CellTiter-Glo 3D Cell Viability solution was added to each well. The plates were centrifuged at 1200 rpm/min for 5 min, and then placed in the dark and kept for 25 min. The fluorescence values were measured using a multi-function microplate reader. The data were analyzed using the Dose-response-inhibition equation in GraphPad Prism8 software, and the IC₅₀ values were obtained, as shown in Table 3.

**Table 3. Inhibitory activities of the compounds according to the present invention on MCF-7 breast cancer cells.**

| Example | IC₅₀ (nM) | | Example | IC₅₀ (nM) |
|---|---|---|---|---|
| 4 | B | | 60 | A |
| 8 | B | | 63 | A |
| 9 | B | | 65 | B |
| 11 | A | | 70 | A |
| 14 | A | | 73 | A |
| 16 | A | | 74 | C |
| 17 | B | | 75 | A |
| 18 | A | | 76 | A |
| 22 | A | | 78 | A |
| 26 | A | | 79 | A |
| 30 | A | | 81 | B |
| 31 | B | | 83 | A |
| 32 | A | | 87 | A |
| 33 | A | | 88 | A |
| 40 | A | | 89 | A |
| 42 | B | | 93 | B |
| 44 | c | | 101 | B |
| 45 | A | | 102 | C |
| 49 | A | | 103 | C |
| 50 | A | | | |
| 57 | A | | | |
| 58 | A | | | |
| | | | | |

| | | | | |
|---|---|---|---|---|
| A: IC₅₀ < 10 nM; B: IC₅₀ = 11-50 nM; C: IC₅₀ > 50 nM | | | | |

As shown in Table 3, the compounds of the present invention had good inhibitory activities on the proliferation of MCF-7 breast cancer cells.

### Experimental example 4. Pharmacokinetics of the compounds according to the present invention

Pharmacokinetic experimental steps: A suitable amount of compound to be administered was weighed, and after the final volume required was calculated, a drug solution with corresponding concentration was prepared using the medium (DMSO/HS-15/Tween 80/D5W (5:5:2:88, v/v/v/v)). For intravenous administration, each drug was administered to 9 animals (female ICR mice, provided by Chengdu Dossy Experimental Animal Co., Ltd.) at a dose of 1 mg/kg (drug volume of 10 mL/kg, drug concentration of 0.1 mg/mL). After administration, samples were alternately collected from three animals of each group at time points of 5 min, 15 min, 30 min, 1 h, 2 h, 4 h, 8 h, 12 h, and 24 h. For oral gavage, each drug was administered to 9 animals (female ICR mice) at a dose of 3 mg/kg (drug volume of 10 mL/kg, drug concentration of 0.3 mg/mL). After administration, samples are alternately collected from three animals of each group at time points of 15 min, 30 min, 1 h, 2 h, 4 h, 6 h, 8 h, 12 h, and 24 h. The samples were anticoagulated with EDTA-K2 and centrifuged at 4 °C for 5 min to separate the plasma. The plasma samples were vortexed and mixed. A suitable amount of internal standard working solution was added to the 96-well plate containing samples used for ploting standard curve, samples used for quality control, or samples to be tested; A suitable amount of methanol was added to the blank sample, vortexed and mixed thoroughly, and then centrifuged for 15 min before the supernatant was collected and injected to LC-MS/MS for analysis. The plasma concentration at each time point was used to calculate the main pharmacokinetic parameters using the Winnolin 8.3 non-compartmental model.

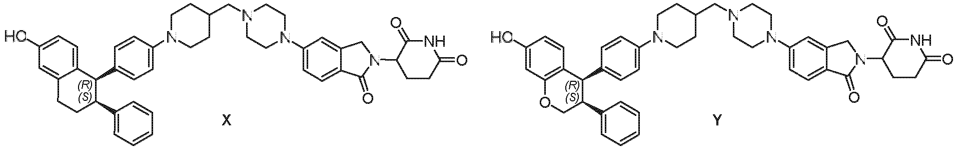

Compounds X (reference WO2018102725) and Y (reference US10800770) are disclosed ER Protac compounds. Compared to these compounds, the compounds of the present invention exhibited superior pharmacokinetics. The experimental results for oral administration at an exposure dose of 3 mg/kg (AUC) and the half-life (T_{1/2}) are shown in Table 4:

**Table 4. Results for the exposure (AUC) and half-life (T_{1/2}) of the compounds according to the present invention after oral administration at a dose of 3 mg/kg.**

| **Compound/Example** | **AUC_{inf} (µg*h/mL)** | **T_{1/2} (h)** |
|---|---|---|
| Control compound **X** | 1.3 | 2.3 |
| Control compound **Y** | 0.1 | 4.6 |
| 30 | B | A |
| 32 | B | A |
| 37 | A | A |
| 40 | A | A |
| 50 | B | A |
| 63 | A | A |
| 65 | A | A |
| 70 | A | A |
| 73 | B | A |
| 87 | B | A |
| 89 | B | A |

| | | |
|---|---|---|
| Note: AUC_{inf}: A: > 2.5 µg*h/mL, B: 1.4-2.5 µg*h/mL, T_{1/2}: A: >3.5 h, B: 2.5-3.5 h. | | |

### Experimental example 5. Pharmacodynamic experiment of the compounds according to the present invention in mouse models of human breast cancer

MCF-7 breast cancer cells were cultured in MEM medium containing 10% FBS, 0.01 mM NEAA, and 10 µg/mL insulin. MCF-7 cells in the exponential growth phase were collected and resuspended in PBS to a suitable concentration for subcutaneous injection into mice. The experimental animals were NOD/SCID female mice (purchased from GemPharmatech Co.Itd, Jiangsu). Before inoculation of tumor cells, a 0.36 mg/60-day slow-release estrogen tablet (17β-estradiol, Innovative Research of America, Sarasota, Florida, USA) was subcutaneously implanted on the right side of the back of the experimental animals. The day after estrogen tablet implantation, 2×10⁷ MCF-7 cells were inoculated into the right mammary fatty pad of the experimental mice, wherein the cells were resuspended in PBS and Matrigel (1:1) (0.2 mL/mouse). The tumor growth was regularly observed.

When the tumors in the tumor-bearing mice grew to an average volume of approximately 245.56 mm³, they were randomly grouped for administration based on tumor size. Grouping was performed using StudyDirector^{™} (version 3.1.399.19, supplier Studylog System, Inc., S. San Francisco, CA, USA). The day of grouping was designated as day 0, that is, the start of drug administration according to the experimental design. The experimental design involved once-daily oral gavage, including a vehicle group, a control group (compound X, dose 10 mg/kg), and groups of compounds according to the present invention (Example compounds **40, 70,** administered at low dose group 3 mg/kg, medium dose group 10 mg/kg, and high dose group 30 mg/kg; Example compounds **89, 73,** administered at medium dose group 10 mg/kg and high dose group 30 mg/kg).

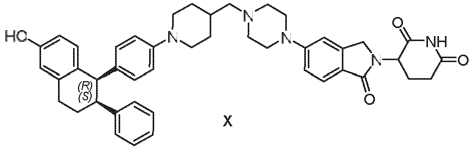

The test compound was dissolved in the medium [DMSO + HS-15 + Tween 80 + D5W (5:5:2:88, v/v/v/v)] to prepare a solution of 0.3 mg/mL (corresponding to a dose of 3 mg/kg), 1 mg/mL (corresponding to a dose of 10 mg/kg), or 3 mg/mL (corresponding to a dose of 30 mg/kg) for oral gavage. The vehicle group received DMSO plus medium by gavage administration.

After starting drug administration, the body weight and tumor size of mice were measured twice a week. The tumor volume calculation formula is: tumor volume (mm³) = 1/2 × (a × b²)

(wherein a represents the long diameter, while b represents the short diameter). In the experiment, data were collected using StudyDirectorTM software (version 3.1.399.19, supplier Studylog System, Inc.). The raw data were directly imported into the software after being measured using a balance and vernier caliper. The entire process including drug administration as well as measurement of tumor and body weight was performed in a biosafety cabinet or clean bench. The experiment lasted for 4 weeks and was terminated on day 28. The tumor growth inhibition (TGI) rate was calculated as follows: TGI% = (1 - the tumor volume of the test compound group / the tumor volume of the vehicle control group) * 100%.

At the end of the experiment, the average tumor volume in the vehicle control group reached 513 mm³, while the average tumor volume in the positive control group (compound **X,** dose 10 mg/kg) was 362 mm³, corresponding to a TGI of 29%. Both the low-dose groups (3 mg/kg) of Example compounds **40** and **70** showed TGI values of 29%-30%, demonstrating comparable efficacy to the 10 mg/kg dose group of compound **X.** The medium and high-dose groups (10 mg/kg, 30 mg/kg) of the compounds according to the present invention had TGI values of 40%-72%. A higher TGI indicated better tumor-suppressing efficacy. The results showed that the efficacy of the compound of the present invention was significantly better than that of the control compound **X.** No significant changes in body weight were observed in the vehicle group, control group, and experimental groups, indicating no significant toxic side effects but good pharmaceutical properties.

In summary, the present invention provided an aromatic compound, which could be used in the manufacture of ER degraders, as well as in the manufacture of medicaments for treating diseases related to estrogen receptors, such as medicaments for treating and/or preventing cancer, and said cancer could be breast cancer.

## Claims

1. A compound represented by formula (I), or an optical isomer thereof, or a salt thereof, or a hydrate thereof, or a solvate thereof: 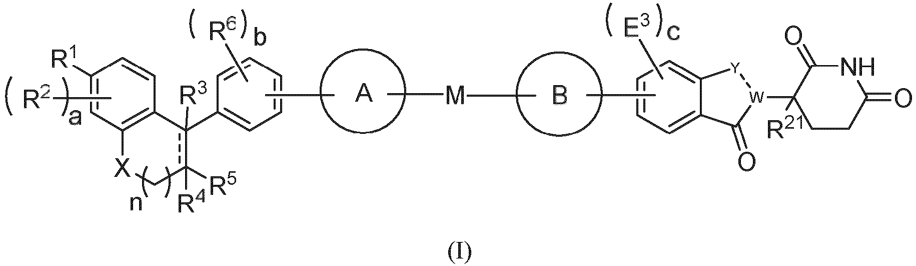 wherein,
R¹ is selected from the group consisting of H, C₁-C₆ alkyl, hydroxyl, amino, carboxyl, C₁-C₆ alkoxy, dihydroxyboranyl, and -OR';
R' is selected from the group consisting of phenyl and benzyl;
R² represents a substituent at any position of the benzene ring, and a represents the number of the substituent R²;
Each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
provided that the dashed line in the benzene ring represents a bond, R³ and R⁴ are independently selected from absence;
provided that the dashed line in the benzene ring is absent, R³ and R⁴ are each independently selected from the group consisting of H and C₁-C₆ alkyl; alternatively, R³ and R⁴ are linked to form 3-6-membered cycloalkyl;
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
n is 0, 1, or 2;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
provided that the dashed line between Y and W is absent, W represents NH, and Y represents halogen;
provided that the dashed line between Y and W is a bond, W represents N; and Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
R²¹ is selected from the group consisting of provided that the dashed line between Y and W is absent, W represents NH, and Y represents a halogen;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.

2. The compound according to claim 1, or an optical isomer thereof, or a salt thereof, or a hydrate thereof, or a solvate thereof, **characterized in that** the compound is as represented by formula (II): 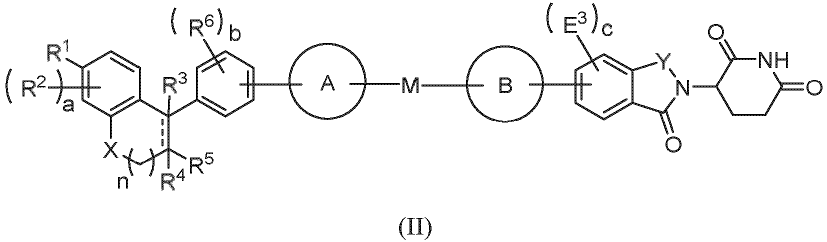 wherein,
R¹ is selected from the group consisting of H, C₁-C₆ alkyl, hydroxyl, amino, carboxyl, C₁-C₆ alkoxy, dihydroxyboranyl, and -OR';
R' is selected from the group consisting of phenyl and benzyl;
R² represents a substituent at any position of the benzene ring, and a represents the number of the substituent R²;
each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
provided that the dashed line in the benzene ring represents a bond, R³ and R⁴ are independently selected from absence;
provided that the dashed line in the benzene ring is absent, R³ and R⁴ are each independently selected from the group consisting of H and C₁-C₆ alkyl; alternatively, R³ and R⁴ are linked to form 3-6-membered cycloalkyl;
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
n is 0, 1, or 2;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.

3. The compound according to claim 2, or an optical isomer thereof, or a salt thereof, or a hydrate thereof, or a solvate thereof, **characterized in that** the compound is as represented by formula (III): 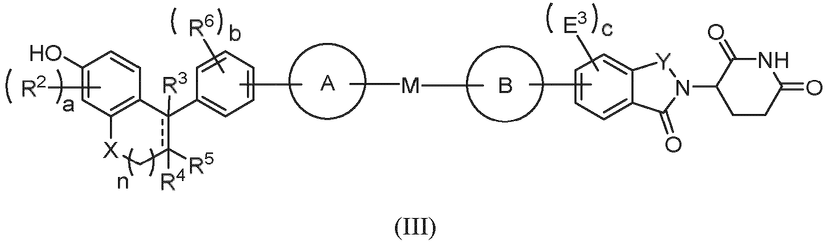 wherein,
R² represents a substituent at any position of the benzene ring, and a represents the number of the substituent R²;
each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
provided that the dashed line in the benzene ring represents a bond, R³ and R⁴ are independently selected from absence;
provided that the dashed line in the benzene ring is absent, R³ and R⁴ are each independently selected from the group consisting of H and C₁-C₆ alkyl; alternatively, R³ and R⁴ are linked to form 3-6-membered cycloalkyl;
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
n is 0, 1, or 2;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H an C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3. preferably,
R⁵ is selected from the group consisting of C₁-C₆ alkyl, 3-6-membered cycloalkyl, trifluoromethyl, trifluoroethyl, substituted or unsubstituted phenyl, substituted or unsubstituted thienyl, substituted or unsubstituted furanyl, substituted or unsubstituted pyrrolyl, substituted or unsubstituted pyrimidinyl, substituted or unsubstituted pyridazinyl, substituted or unsubstituted pyrazolyl, substituted or unsubstituted pyrazinyl; the substituent of said phenyl, thienyl, furanyl, pyrrolyl, pyrimidinyl, pyridazinyl, pyrazolyl, or pyrazinyl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

4. The compound according to claim 3, or an optical isomer thereof, or a salt thereof, or a hydrate thereof, or a solvate thereof, **characterized in that** the compound is as represented by formula (IV): 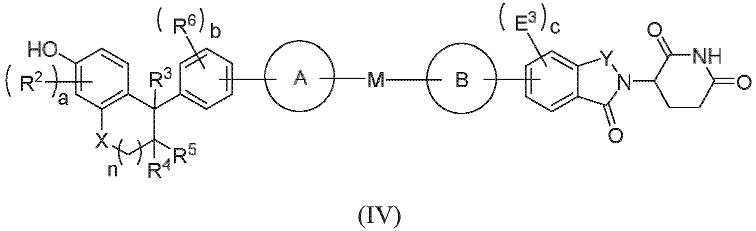 wherein,
R² represents a substituent at any position of the benzene ring, and a represents the number of the substituent R²;
each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
R³ and R⁴ are each independently selected from the group consisting of H and C₁-C₆ alkyl; alternatively, R³ and R⁴ are linked to form 3-6-membered cycloalkyl;
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
n is 0, 1, or 2;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.
Preferably,
R⁵ is selected from the group consisting of C₁-C₆ alkyl, 3-6-membered cycloalkyl, trifluoromethyl, trifluoroethyl, substituted or unsubstituted phenyl, substituted or unsubstituted thienyl, substituted or unsubstituted furanyl, substituted or unsubstituted pyrrolyl, substituted or unsubstituted pyrimidinyl, substituted or unsubstituted pyridazinyl, substituted or unsubstituted pyrazolyl, and substituted or unsubstituted pyrazinyl; the substituent of said phenyl, thienyl, furanyl, pyrrolyl, pyrimidinyl, pyridazinyl, pyrazolyl, or pyrazinyl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

5. The compound according to claim 4, or an optical isomer thereof, or a salt thereof, or a hydrate thereof, or a solvate thereof, **characterized in that** the compound is as represented by formula (V): 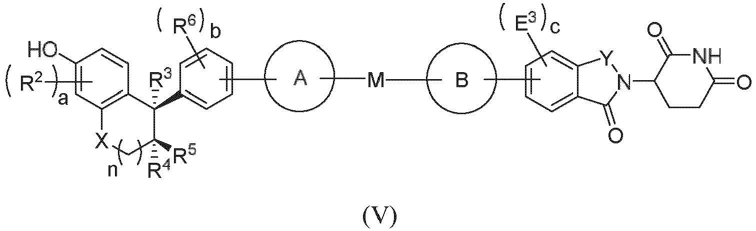 wherein,
R² represents a substituent at any position of the benzene ring, and a represents the number of the substituent R²;
each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
R³ and R⁴ are each independently selected from the group consisting of H and C₁-C₆ alkyl; alternatively, R³ and R⁴ are linked to form 3-6-membered cycloalkyl;
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
n is 0, 1, or 2;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.
Preferably,
R⁵ is selected from the group consisting of C₁-C₆ alkyl, 3-6-membered cycloalkyl, trifluoromethyl, trifluoroethyl, substituted or unsubstituted phenyl, substituted or unsubstituted thienyl, substituted or unsubstituted furanyl, substituted or unsubstituted pyrrolyl, substituted or unsubstituted pyrimidinyl, substituted or unsubstituted pyridazinyl, substituted or unsubstituted pyrazolyl, and substituted or unsubstituted pyrazinyl; the substituent of said phenyl, thienyl, furanyl, pyrrolyl, pyrimidinyl, pyridazinyl, pyrazolyl, or pyrazinyl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

6. The compound according to claim 5, or an optical isomer thereof, or a salt thereof, or a hydrate thereof, or a solvate thereof, **characterized in that** the compound is as represented by formula (VI): 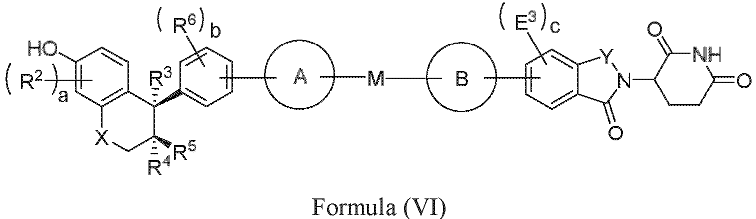 wherein
R² represents a substituent at any position of the benzene ring, and a represents the number of the substituent R²;
each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
R³ and R⁴ are each independently selected from the group consisting of H and C₁-C₆ alkyl; alternatively, R³ and R⁴ are linked to form 3-6-membered cycloalkyl;
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.
Preferably,
R⁵ is selected from the group consisting of C₁-C₆ alkyl, 3-6-membered cycloalkyl, trifluoromethyl, trifluoroethyl, substituted or unsubstituted phenyl, substituted or unsubstituted thienyl, substituted or unsubstituted furanyl, substituted or unsubstituted pyrrolyl, substituted or unsubstituted pyrimidinyl, substituted or unsubstituted pyridazinyl, substituted or unsubstituted pyrazolyl, and substituted or unsubstituted pyrazinyl; the substituent of said phenyl, thienyl, furanyl, pyrrolyl, pyrimidinyl, pyridazinyl, pyrazolyl, or pyrazinyl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

7. The compound according to claim 6, or an optical isomer thereof, or a salt thereof, or a hydrate thereof, or a solvate thereof, **characterized in that** the compound is as represented by formula (VII): 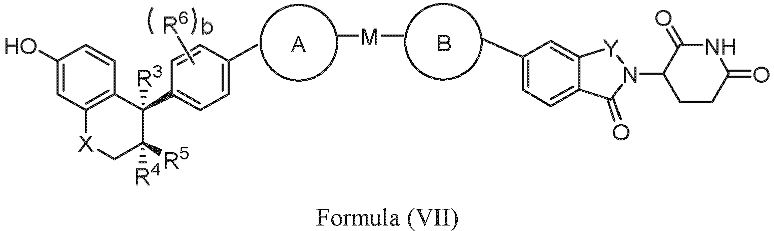 wherein
R³ and R⁴ are each independently selected from the group consisting of H and C₁-C₆ alkyl; alternatively, R³ and R⁴ are linked to form 3-6-membered cycloalkyl;
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.
Preferably,
R⁵ is selected from the group consisting of C₁-C₆ alkyl, 3-6-membered cycloalkyl, trifluoromethyl, trifluoroethyl, substituted or unsubstituted phenyl, substituted or unsubstituted thienyl, substituted or unsubstituted furanyl, substituted or unsubstituted pyrrolyl, substituted or unsubstituted pyrimidinyl, substituted or unsubstituted pyridazinyl, substituted or unsubstituted pyrazolyl, and substituted or unsubstituted pyrazinyl; the substituent of said phenyl, thienyl, furanyl, pyrrolyl, pyrimidinyl, pyridazinyl, pyrazolyl, or pyrazinyl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

8. The compound according to claim 7, or an optical isomer thereof, or a salt thereof, or a hydrate thereof, or a solvate thereof, **characterized in that** the compound is as represented by formula (VIII): 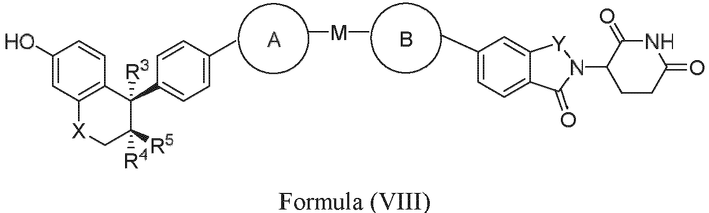 wherein,
R³ and R⁴ are each independently selected from the group consisting of H and C₁-C₆ alkyl; alternatively, R³ and R⁴ are linked to form 3-6-membered cycloalkyl;
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.
Preferably,
R⁵ is selected from the group consisting of C₁-C₆ alkyl, 3-6-membered cycloalkyl, trifluoromethyl, substituted or unsubstituted phenyl, substituted or unsubstituted thienyl, substituted or unsubstituted furanyl, substituted or unsubstituted pyrrolyl, substituted or unsubstituted pyrimidinyl, substituted or unsubstituted pyridazinyl, substituted or unsubstituted pyrazolyl, and substituted or unsubstituted pyrazinyl; the substituent of said phenyl, thienyl, furanyl, pyrrolyl, pyrimidinyl, pyridazinyl, pyrazolyl, or pyrazinyl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

9. The compound according to claim 7, or an optical isomer thereof, or a salt thereof, or a hydrate thereof, or a solvate thereof, **characterized in that** the compound is as represented by formula (IX): 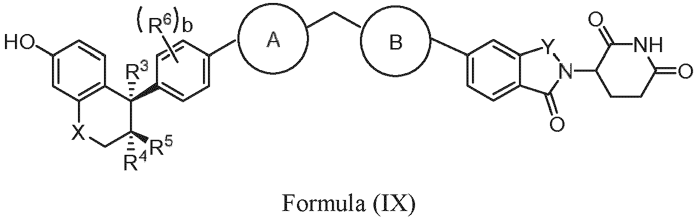 wherein,
R³ and R⁴ are each independently selected from the group consisting of H and C₁-C₆ alkyl; alternatively, R³ and R⁴ are linked to form 3-6-membered cycloalkyl;
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.
Preferably,
R⁵ is selected from the group consisting of C₁-C₆ alkyl, 3-6-membered cycloalkyl, trifluoromethyl, trifluoroethyl, substituted or unsubstituted phenyl, substituted or unsubstituted thienyl, substituted or unsubstituted furanyl, substituted or unsubstituted pyrrolyl, substituted or unsubstituted pyrimidinyl, substituted or unsubstituted pyridazinyl, substituted or unsubstituted pyrazolyl, and substituted or unsubstituted pyrazinyl; the substituent of said phenyl, thienyl, furanyl, pyrrolyl, pyrimidinyl, pyridazinyl, pyrazolyl, or pyrazinyl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

10. The compound according to claim 9, or an optical isomer thereof, or a salt thereof, or a hydrate thereof, or a solvate thereof, **characterized in that** the compound is as represented by formula (X): 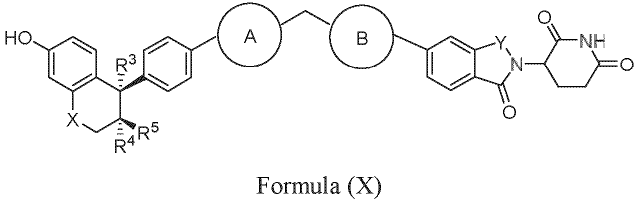 wherein,
R³ and R⁴ are each independently selected from the group consisting of H and C₁-C₆ alkyl; alternatively, R³ and R⁴ are linked to form 3-6-membered cycloalkyl;
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.
Preferably,
R⁵ is selected from the group consisting of C₁-C₆ alkyl, 3-6-membered cycloalkyl, trifluoromethyl, trifluoroethyl, substituted or unsubstituted phenyl, substituted or unsubstituted thienyl, substituted or unsubstituted furanyl, substituted or unsubstituted pyrrolyl, substituted or unsubstituted pyrimidinyl, substituted or unsubstituted pyridazinyl, substituted or unsubstituted pyrazolyl, and substituted or unsubstituted pyrazinyl; the substituent of said phenyl, thienyl, furanyl, pyrrolyl, pyrimidinyl, pyridazinyl, pyrazolyl, or pyrazinyl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

11. The compound according to claim 6, or an optical isomer thereof, or a salt thereof, or a hydrate thereof, or a solvate thereof, **characterized in that** the compound is as represented by formula (XI): 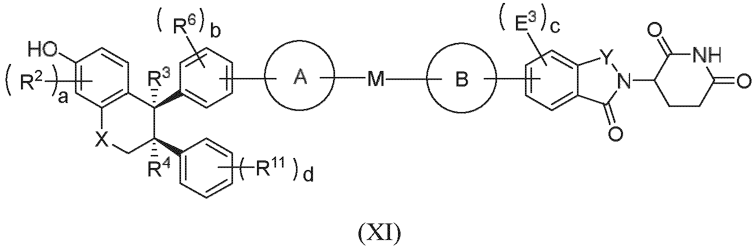 wherein,
R² represents a substituent at any position of the benzene ring, and a represents the number of the substituent R²;
each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
R³, R⁴ are each independently selected from the group consisting of H and C₁-C₆ alkyl; alternatively, R³ and R⁴ are linked to form 3-6-membered cycloalkyl;
R¹¹ represents a substituent at any position of the benzene ring, and d is the number of the substituent R¹¹;
each R¹¹ is independently selected from the group consisting of halogen, hydroxyl, amino, cyano, trifluoromethyl, C₁-C₆ alkyl, 3-6-membered cycloalkyl, and C₁-C₆ alkoxy;
d is 0, 1, 2, or 3;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.
Preferably,
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

12. The compound according to claim 11, or an optical isomer thereof, or a salt thereof, or a hydrate thereof, or a solvate thereof, **characterized in that** the compound is as represented by formula (XII): 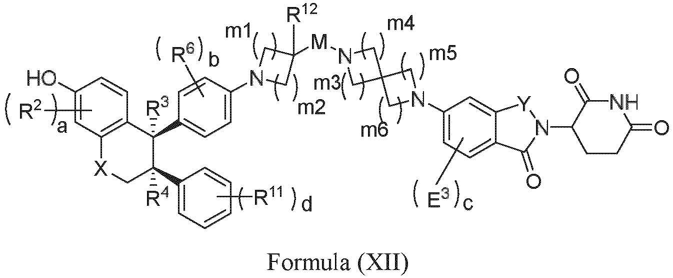 wherein,
R² represents a substituent at any position of the benzene ring, and a represents the number of the substituent R²;
each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
R³ and R⁴ are each independently selected from the group consisting of H and C₁-C₆ alkyl; alternatively, R³ and R⁴ are linked to form 3-6-membered cycloalkyl;
R¹¹ represents a substituent at any position of the benzene ring, and d is the number of the substituent R¹¹;
each R¹¹ is independently selected from the group consisting of halogen, hydroxyl, amino, cyano, trifluoromethyl, C₁-C₆ alkyl, 3-6-membered cycloalkyl, and C₁-C₆ alkoxy;
d is 0, 1, 2, or 3;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
R¹² is selected from the group consisting of H, halogen, C₁-C₆ alkyl, and hydroxyl;
m1, m2, m3, m4, m5, m6 are each independently selected from the group consisting of 0, 1, 2 or 3;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.
Preferably,
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl.

13. The compound according to claim 12, or an optical isomer thereof, or a salt thereof, or a hydrate thereof, or a solvate thereof, **characterized in that** the compound is as represented by formula (XIII): 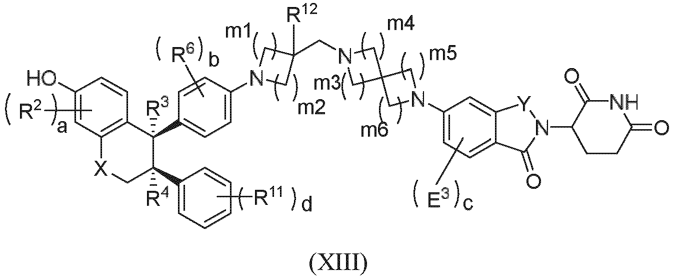 wherein,
R² represents a substituent at any position of the benzene ring, and a represents the number of the substituent R²;
each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
R³ and R⁴ are each independently selected from the group consisting of H and C₁-C₆ alkyl; alternatively, R³ and R⁴ are linked to form 3-6-membered cycloalkyl;
R¹¹ represents a substituent at any position of the benzene ring, and d is the number of the substituent R¹¹;
each R¹¹ is independently selected from the group consisting of halogen, hydroxyl, amino, cyano, trifluoromethyl, C₁-C₆ alkyl, 3-6-membered cycloalkyl, and C₁-C₆ alkoxy;
d is 0, 1, 2, or 3;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
R¹² is selected from the group consisting of H, halogen, C₁-C₆ alkyl, and hydroxyl;
m1, m2, m3, m4, m5, m6 are each independently selected from the group consisting of 0, 1, 2 or 3;
E³ represents a substituent at any position of the benzene ring, and c is the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; and the number of the heteroatom is 1, 2, or 3.
Preferably,
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl.

14. The compound according to claim 12, or an optical isomer thereof, or a salt thereof, or a hydrate thereof, or a solvate thereof, **characterized in that** the compound is as represented by formula (XIV): 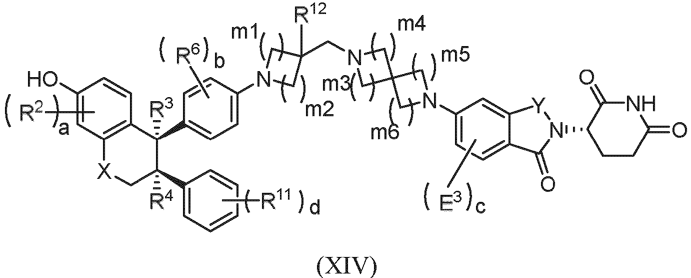 wherein,
R² represents a substituent at any position of the benzene ring, and a represents the number of the substituent R²;
each R² are each independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
R³ and R⁴ are each independently selected from the group consisting of H and C₁-C₆ alkyl; alternatively, R³ and R⁴ are linked to form 3-6-membered cycloalkyl;
R¹¹ represents a substituent at any position of the benzene ring, and d is the number of the substituent R¹¹;
each R¹¹ is independently selected from the group consisting of halogen, hydroxyl, amino, cyano, trifluoromethyl, C₁-C₆ alkyl, 3-6-membered cycloalkyl, and C₁-C₆ alkoxy;
d is 0, 1, 2, or 3;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
R¹² is selected from the group consisting of H, halogen, C₁-C₆ alkyl, and hydroxyl;
m1, m2, m3, m4, m5, m6 are each independently selected from the group consisting of 0, 1, 2 or 3;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.
Preferably,
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl.

15. The compound according to claim 6, or an optical isomer thereof, or a salt thereof, or a hydrate thereof, or a solvate thereof, **characterized in that** the compound is as represented by formula (XV): 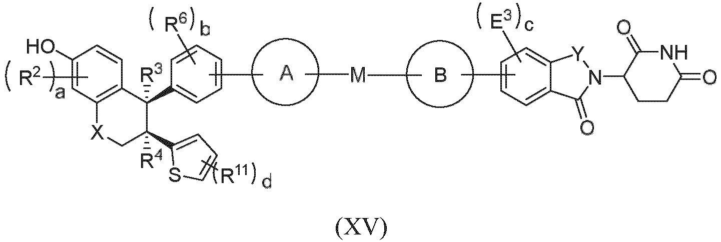 wherein,
R² represents a substituent at any position of the benzene ring, and a represents the number of the substituent R²;
each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
R³ and R⁴ are each independently selected from the group consisting of H and C₁-C₆ alkyl; alternatively, R³ and R⁴ are linked to form 3-6-membered cycloalkyl;
R¹¹ is a substituent at any position of the thiophene ring, and d is the number of the substituent R¹¹;
each R¹¹ is independently selected from the group consisting of halogen, hydroxyl, amino, cyano, trifluoromethyl, C₁-C₆ alkyl, 3-6-membered cycloalkyl, and C₁-C₆ alkoxy;
d is 0, 1, 2, or 3;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2}, C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.
Preferably,
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

16. The compound according to claim 6, or an optical isomer thereof, or a salt thereof, or a hydrate thereof, or a solvate thereof, **characterized in that** the compound is as represented by formula (XVI): 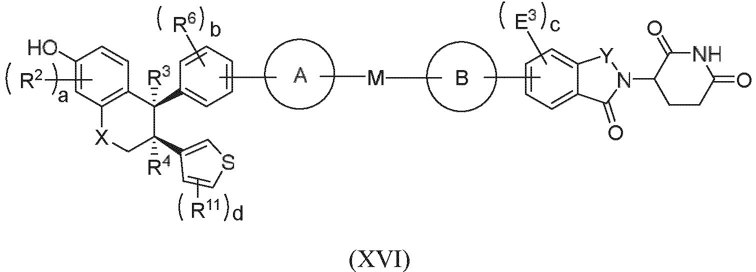 wherein,
R² represents a substituent at any position of the benzene ring, and a represents the number of the substituent R²;
each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
R³ and R⁴ are each independently selected from the group consisting of H and C₁-C₆ alkyl; alternatively, R³ and R⁴ are linked to form 3-6-membered cycloalkyl;
R¹¹ is a substituent at any position of the thiophene ring, and d is the number of the substituent R¹¹;
each R¹¹ is independently selected from the group consisting of halogen, hydroxyl, amino, cyano, trifluoromethyl, C₁-C₆ alkyl, 3-6-membered cycloalkyl, and C₁-C₆ alkoxy;
d is 0, 1, 2, or 3;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2} C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.
Preferably,
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

17. The compound according to claim 2, or an optical isomer thereof, or a salt thereof, or a hydrate thereof, or a solvate thereof, **characterized in that** the compound is as represented by formula (XVII): 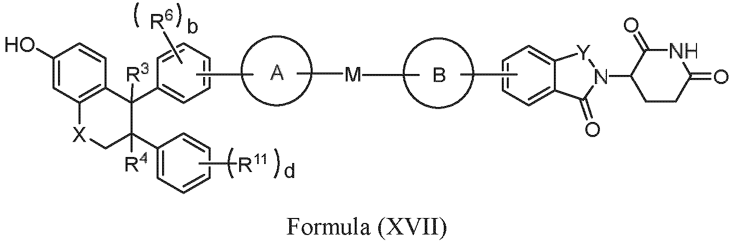 wherein,
R³ and R⁴ are each independently selected from the group consisting of H and C₁-C₆ alkyl; alternatively, R³ and R⁴ are linked to form 3-6-membered cycloalkyl;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R¹¹ represents a substituent at any position of the benzene ring, and d is the number of the substituent R¹¹; R⁶ represents a substituent at any position of the benzene ring, and b is the number of the substituent R⁶;
d is 0, 1, 2, or 3; b is 0, 1, 2, or 3; and at least one of d and b is not 0;
R¹¹ and R⁶ are each independently selected from the group consisting of halogen, hydroxyl, amino, cyano, trifluoromethyl, C₁-C₆ alkyl, 3-6-membered cycloalkyl, and C₁-C₆ alkoxy; and at least one of them is fluorine;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
Y is selected from the group consisting of absence, CR^{Y1}R^{Y2} C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heterocycloaryl is N; the number of the heteroatom is 1, 2, or 3.
Preferably,
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

18. The compound according to claim 17, or an optical isomer thereof, or a salt thereof, or a hydrate thereof, or a solvate thereof, **characterized in that** the compound is as represented by formula (XVIII): 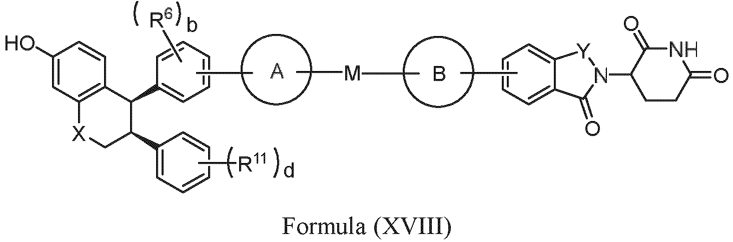 wherein,
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R¹¹ represents a substituent at any position of the benzene ring, and d is the number of the substituent R¹¹; R⁶ represents a substituent at any position of the benzene ring, and b is the number of the substituent R⁶;
d is 0, 1, 2, or 3; b is 0, 1, 2, or 3; and at least one of d and b is not 0;
R¹¹ and R⁶ are each independently selected from the group consisting of halogen, hydroxyl, amino, cyano, trifluoromethyl, C₁-C₆ alkyl, 3-6-membered cycloalkyl, and C₁-C₆ alkoxy; and at least one of them is fluorine;
ring A and ring B are each independently selected from the group consisting of 4-12-membered cycloalkyl and 4-12-membered heterocycloalkyl;
M is selected from the group consisting of absence and CR⁹R¹⁰;
R⁹ and R¹⁰ are each independently selected from the group consisting of H and C₁-C₆ alkyl;
Y is selected from the group consisting of absence, CR^{Y1}R^{Y2} C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heterocycloaryl is N; the number of the heteroatom is 1, 2, or 3.
Preferably,
X is selected from the group consisting of CH₂, CF₂ or O;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

19. The compound according to claim 2, or an optical isomer thereof, or a salt thereof, or a hydrate thereof, or a solvate thereof, **characterized in that** the compound is as represented by formula (XIX): 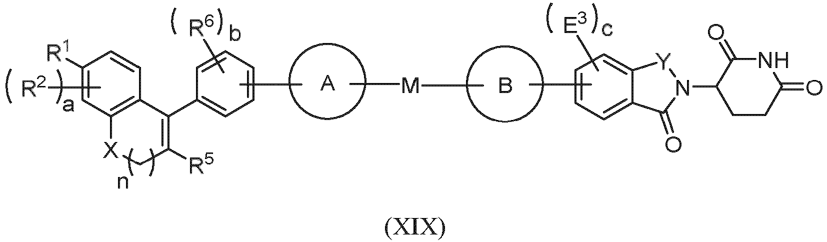 wherein,
R¹ is selected from the group consisting of H, C₁-C₆ alkyl, hydroxyl, amino or C₁-C₆ alkoxy, and dihydroxyboranyl;
R² represents a substituent at any position of the benzene ring, and a represents the number of the substituent R²;
each R² is independently selected from the group consisting of halogen, hydroxyl, amino, cyano, trifluoromethyl, C₁-C₆ alkyl, 3-6-membered cycloalkyl, and C₁-C₆ alkoxy;
a is 0, 1, 2, or 3;
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
n is 0, 1, or 2;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
E³ represents a substituent at any position of the benzene ring, and c represents the number of the substituent E³;
each E³ is independently selected from the group consisting of halogen, hydroxyl, amino, cyano, trifluoromethyl, C₁-C₆ alkyl, 3-6-membered cycloalkyl, and C₁-C₆ alkoxy; alternatively, any two E³ are linked to form 3-6-membered cycloalkyl, 6-10-membered aryl, 4-12-membered heterocycloalkyl, and 5-8-membered heteroaryl;
c is 0, 1, 2, or 3;
Y is selected from the group consisting of absence, CR^{Y1}R^{Y2} C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.
Preferably,
R⁵ is selected from the group consisting of C₁-C₆ alkyl, 3-6-membered cycloalkyl, trifluoromethyl, trifluoroethyl, substituted or unsubstituted phenyl, substituted or unsubstituted thienyl, substituted or unsubstituted furanyl, substituted or unsubstituted pyrrolyl, substituted or unsubstituted pyrimidinyl, substituted or unsubstituted pyridazinyl, substituted or unsubstituted pyrazolyl, and substituted or unsubstituted pyrazinyl; the substituent of said phenyl, thienyl, furanyl, pyrrolyl, pyrimidinyl, pyridazinyl, pyrazolyl, or pyrazinyl is selected from the group consisting of halogen, hydroxyl, amino, cyano, trifluoromethyl, C₁-C₆ alkyl, 3-6-membered cycloalkyl, and C₁-C₆ alkoxy.

20. The compound according to claim 19, or an optical isomer thereof, or a salt thereof, or a hydrate thereof, or a solvate thereof, **characterized in that** the compound is as represented by formula (XX): 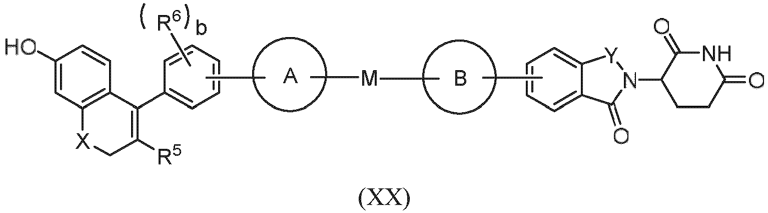 wherein,
R⁵ is selected from the group consisting of C₁-C₆ alkyl, 3-6-membered cycloalkyl, trifluoromethyl, trifluoroethyl, substituted or unsubstituted 6-10-membered aryl, and substituted or unsubstituted 5-8-membered heteroaryl; the substituent of the aryl or heteroaryl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R⁶ represents a substituent at any position of the benzene ring, and b represents the number of the substituent R⁶;
R⁶ are each independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
ring A and ring B are each independently selected from the group consisting of 4-12-membered cycloalkyl and 4-12-membered heterocycloalkyl;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
Y is selected from the group consisting of absence, CR^{Y1}R^{Y2} C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
the heteroatom in the heteroaryl is selected from the group consisting of O, N, or S; the number of the heteroatom is 1, 2, or 3;
the heteroatom in the heterocycloaryl is N; the number of the heteroatom is 1, 2, or 3.
Preferably,
R⁵ is selected from the group consisting of C₁-C₆ alkyl, 3-6-membered cycloalkyl, trifluoromethyl, trifluoroethyl, substituted or unsubstituted phenyl, substituted or unsubstituted thienyl, substituted or unsubstituted furanyl, substituted or unsubstituted pyrrolyl, substituted or unsubstituted pyrimidinyl, substituted or unsubstituted pyridazinyl, substituted or unsubstituted pyrazolyl, and substituted or unsubstituted pyrazinyl; the substituent of said phenyl, thienyl, furanyl, pyrrolyl, pyrimidinyl, pyridazinyl, pyrazolyl, or pyrazinyl is selected from the group consisting of halogen, hydroxyl, amino, cyano, trifluoromethyl, C₁-C₆ alkyl, 3-6-membered cycloalkyl, and C₁-C₆ alkoxy;
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl;
ring A and ring B are each independently selected from the group consisting of 4-12-membered cycloalkyl and 4-12-membered heterocycloalkyl; provided that both ring A and ring B are heterocycloalkyls, one of the rings contains one heteroatom N, while the other ring contains two heteroatoms N.

21. The compound according to claim 20, or an optical isomer thereof, or a salt thereof, or a hydrate thereof, or a solvate thereof, **characterized in that** the compound is as represented by formula (XXI): 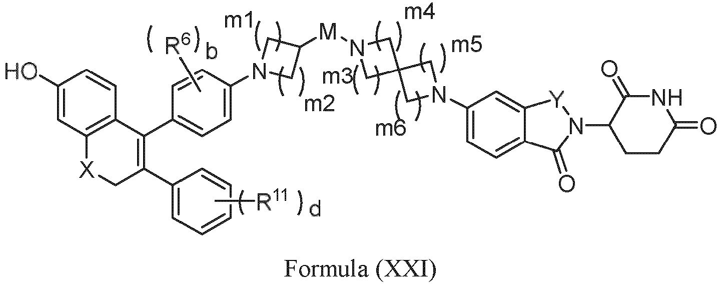 wherein,
R¹¹ represents a substituent at any position of the benzene ring, and d is the number of the substituent R¹¹;
each R¹¹ is independently selected from the group consisting of halogen, hydroxyl, amino, cyano, trifluoromethyl, C₁-C₆ alkyl, 3-6-membered cycloalkyl, and C₁-C₆ alkoxy;
d is 0, 1, 2, or 3;
X is selected from the group consisting of CR⁷R⁸, O, S, SO, SO₂, and NR⁷;
R⁷ and R⁸ are each independently selected from the group consisting of H, halogen, C₁-C₆ alkyl, and 3-6-membered cycloalkyl;
R⁶ represents a substituent at any position of the benzene ring, and b represents the number of the substituent R⁶;
each R⁶ is independently selected from the group consisting of halogen, hydroxyl, amino, cyano, trifluoromethyl, C₁-C₆ alkyl, 3-6-membered cycloalkyl, and C₁-C₆ alkoxy;
b is 0, 1, 2, or 3;
M is selected from the group consisting of absence, CR⁹R¹⁰, O, S, SO, SO₂, and NR⁹;
R⁹ and R¹⁰ are each independently selected from the group consisting of H and C₁-C₆ alkyl;
m1, m2, m3, m4, m5, and m6 are each independently selected from the group consisting of 0, 1, 2 or 3;
Y is selected from the group consisting of absence, CR^{Y1}R^{Y2} C(O), -CR¹¹=CR¹²-, -CR¹¹=N-, -N=N-, and -OC(O)-;
R^{Y1} and R^{Y2} are each independently selected from the group consisting of H and C₁-C₆ alkyl;
R¹¹ and R¹² are each independently selected from the group consisting of H and C₁-C₆ alkyl;
Preferably,
X is selected from the group consisting of CH₂, CF₂, O, S, SO, SO₂, and NR⁷;
R⁷ is selected from the group consisting of H, halogen, C₁-C₆ alkyl, and 3-6-membered cycloalkyl.

22. The compound according to any one of claims 1-11 or 15-20, or an optical isomer thereof, or a salt thereof, or a hydrate thereof, or a solvate thereof, **characterized in that** said ring A and ring B are each independently selected from the group consisting of the following structures:

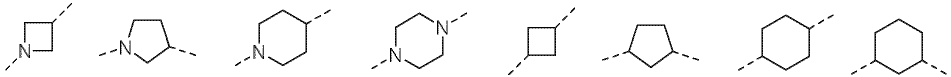

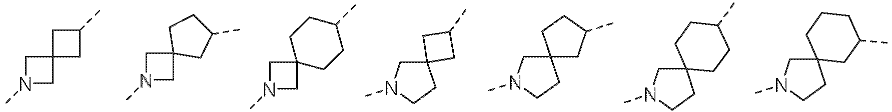

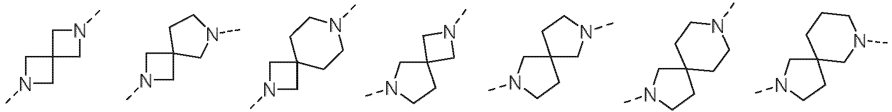

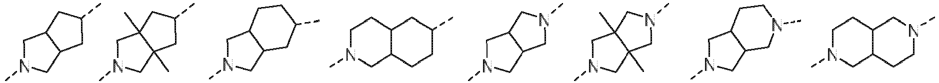

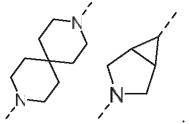

23. The compound according to any one of claims 1-21, or an optical isomer thereof, or a salt thereof, or a hydrate thereof, or a solvate thereof, **characterized in that** said 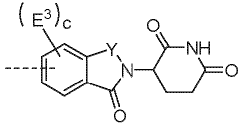 is selected from the group consisting of the following structures:

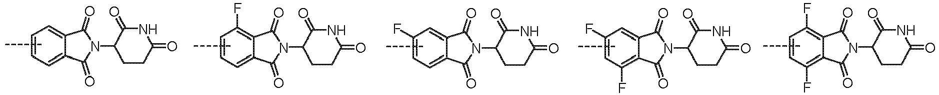

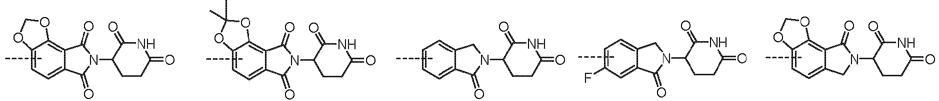

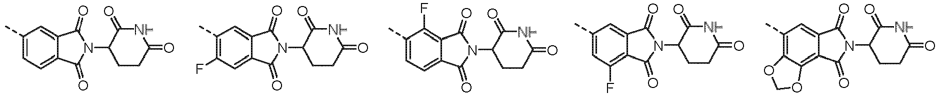

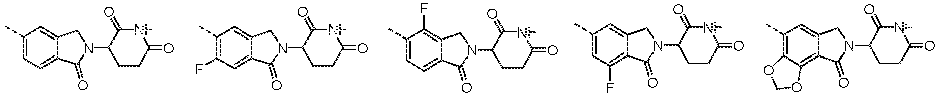

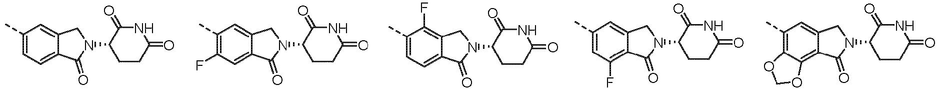

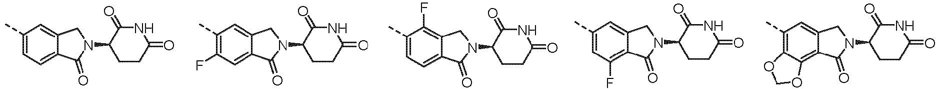

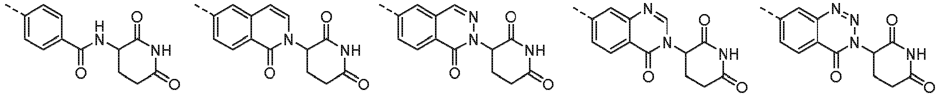

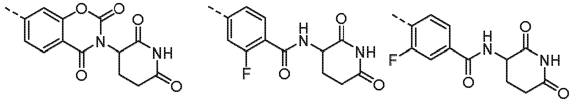

24. The compound according to any one of claims 1-21, or an optical isomer thereof, or a salt thereof, or a hydrate thereof, or a solvate thereof, **characterized in that** the compound is selected from the group consisting of the following compounds:

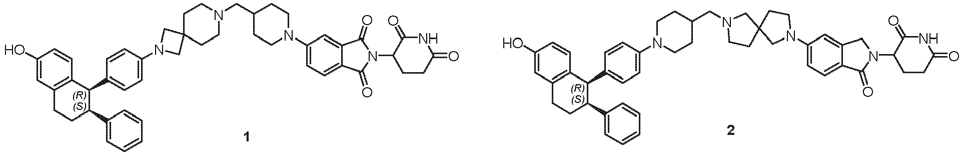

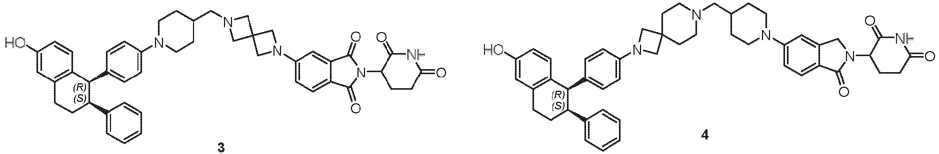

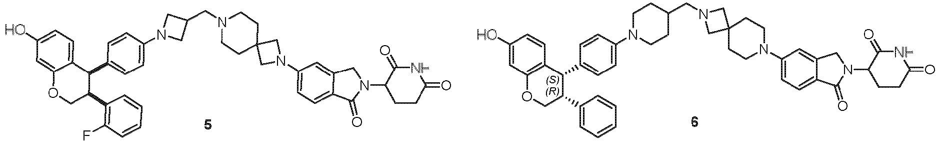

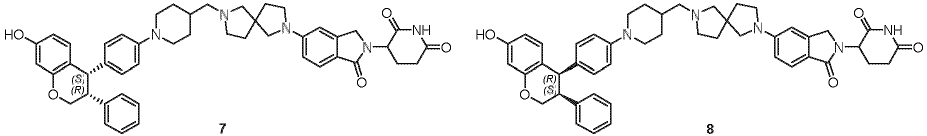

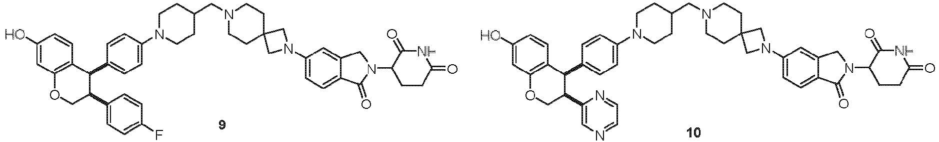

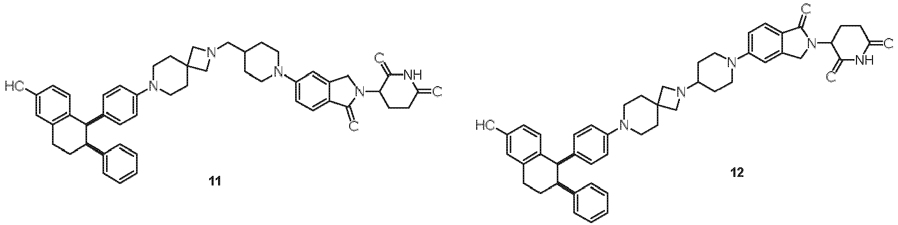

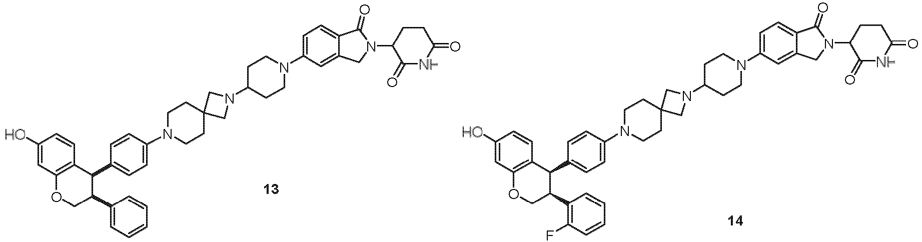

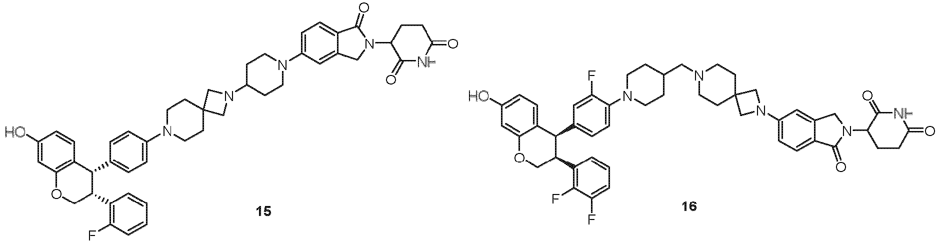

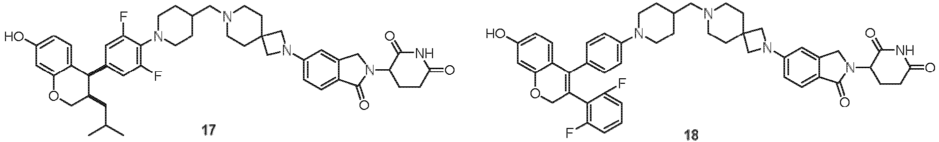

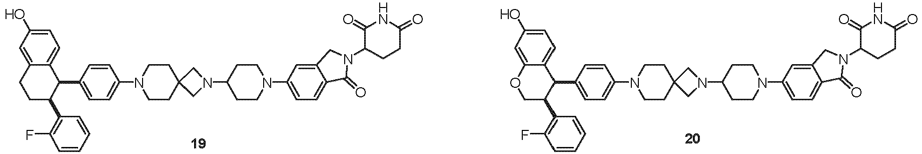

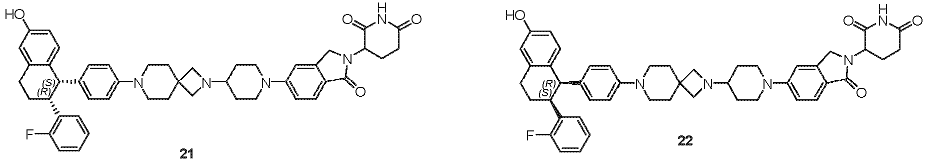

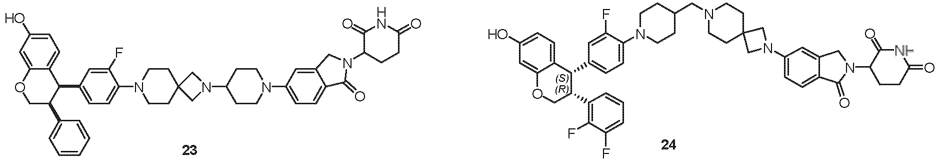

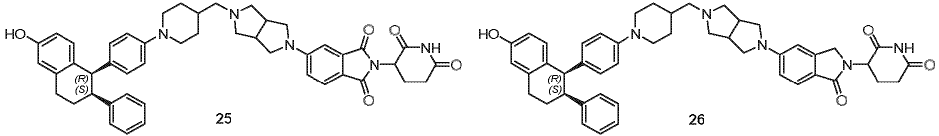

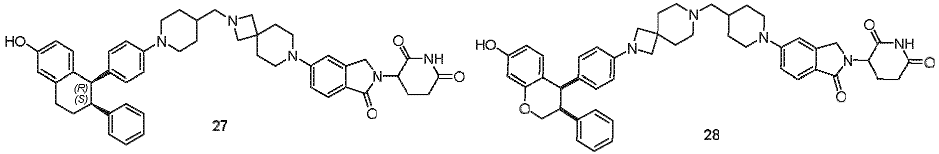

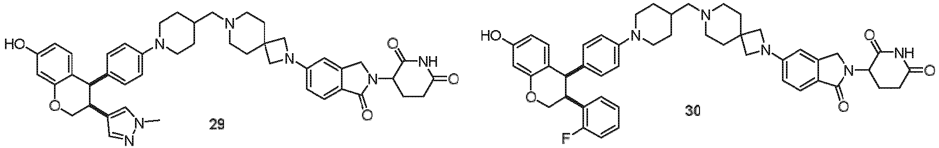

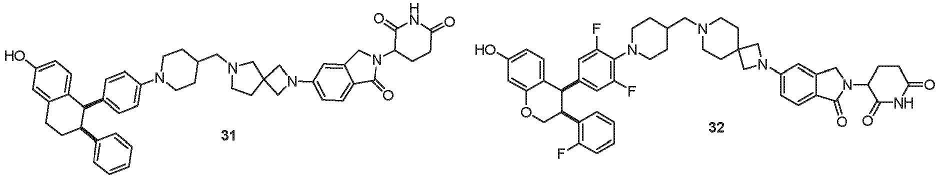

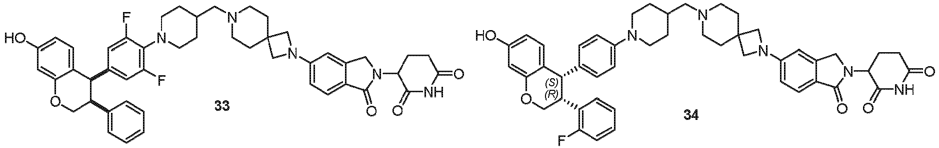

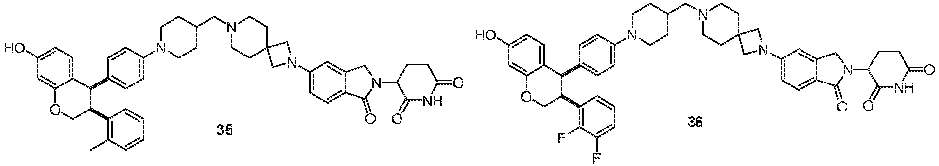

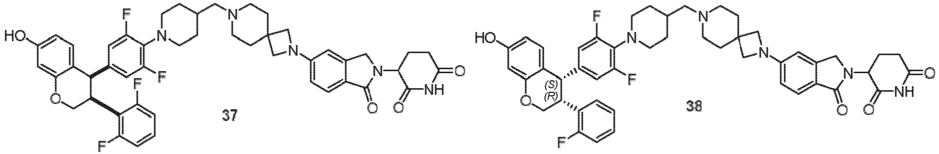

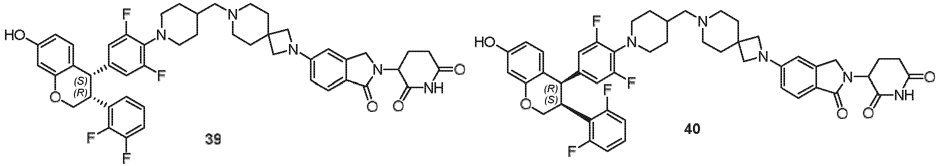

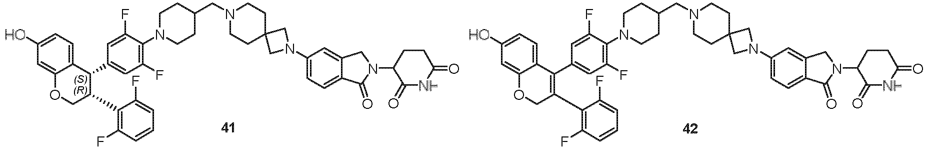

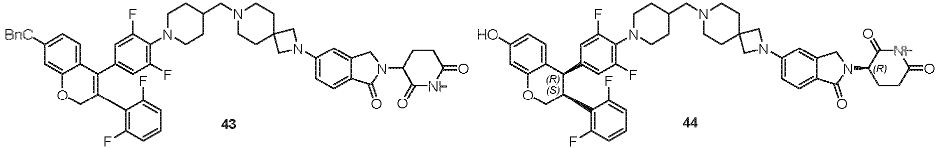

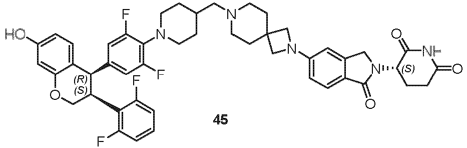

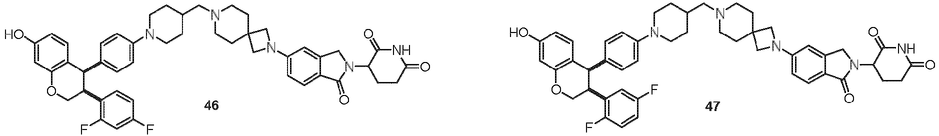

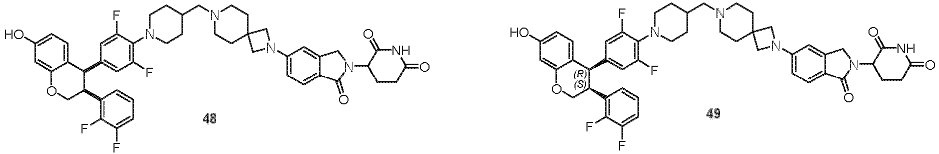

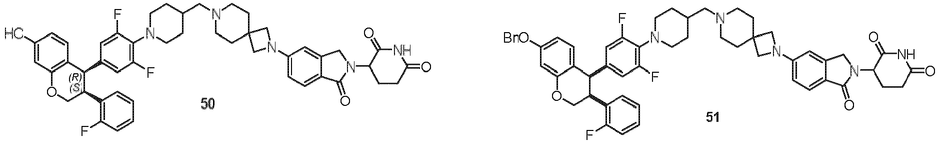

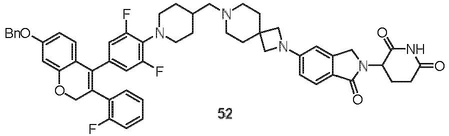

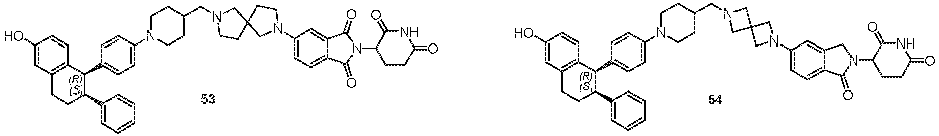

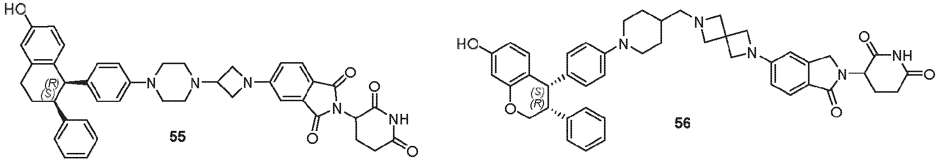

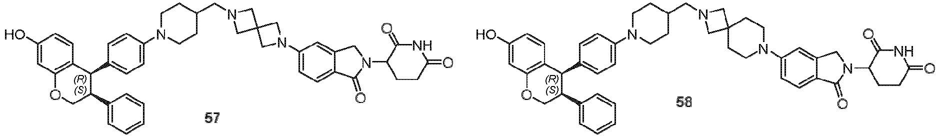

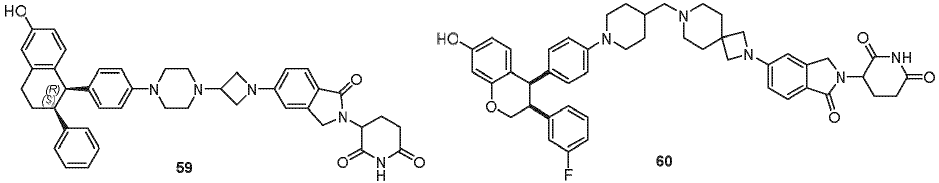

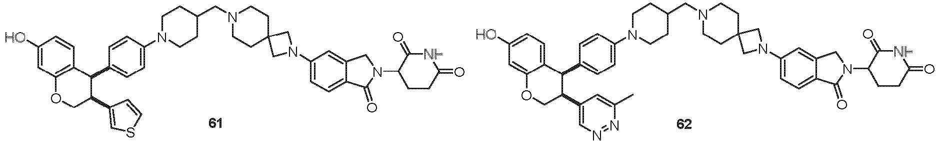

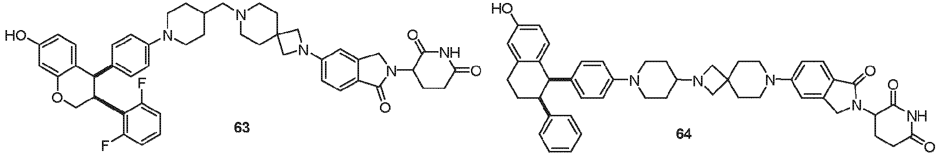

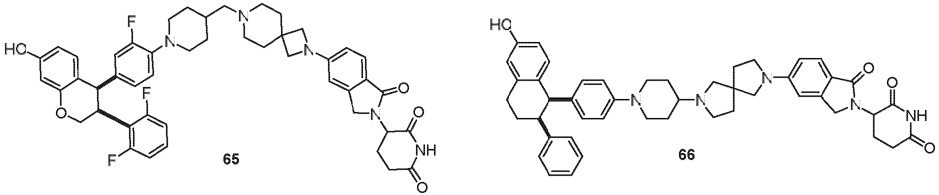

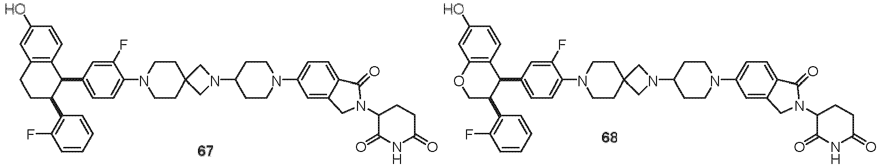

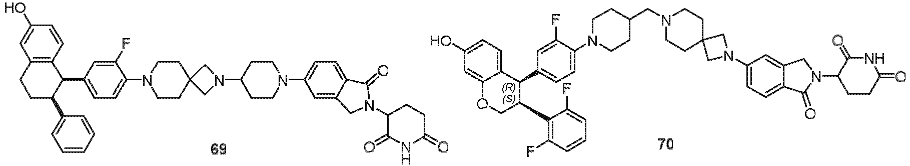

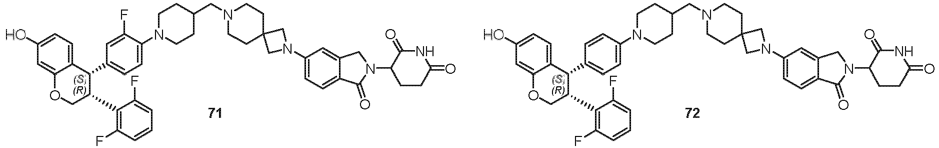

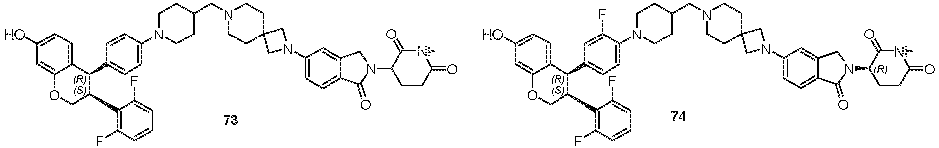

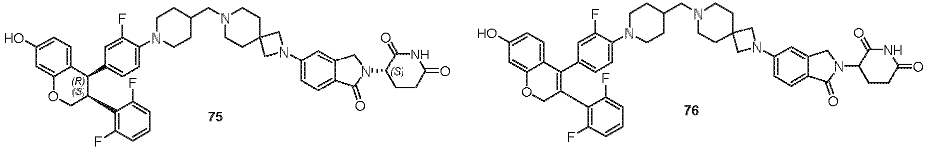

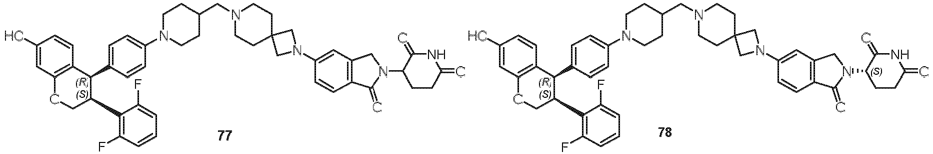

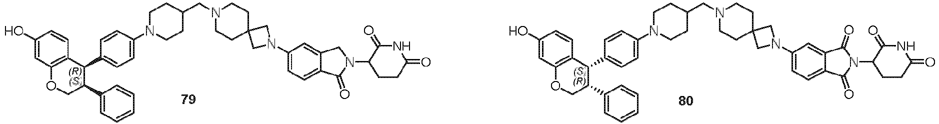

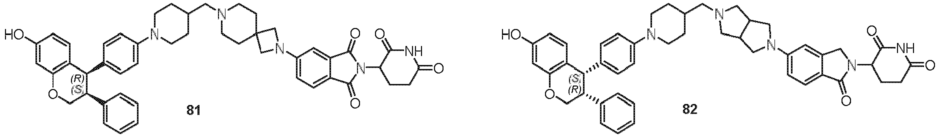

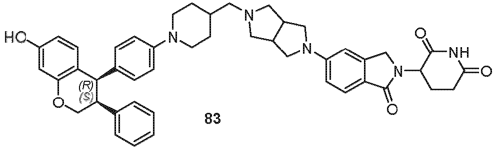

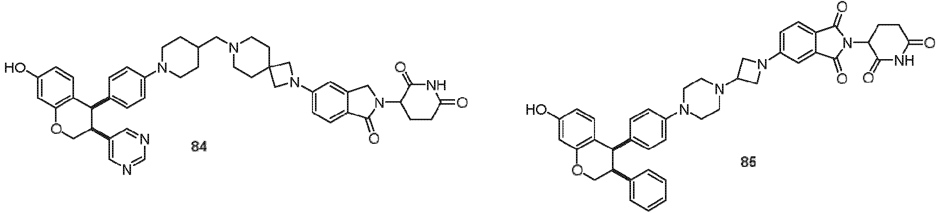

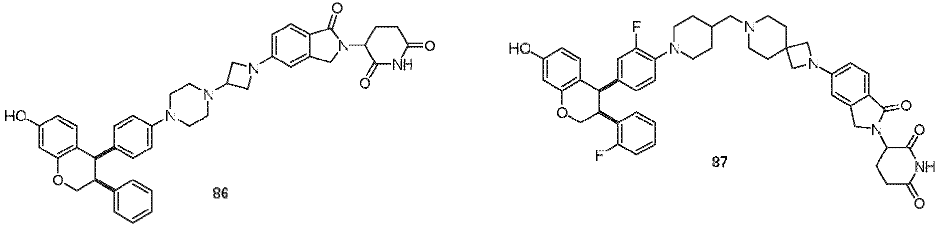

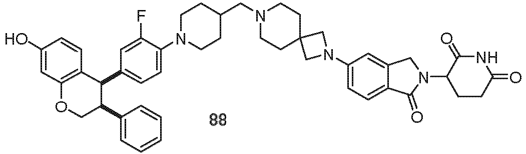

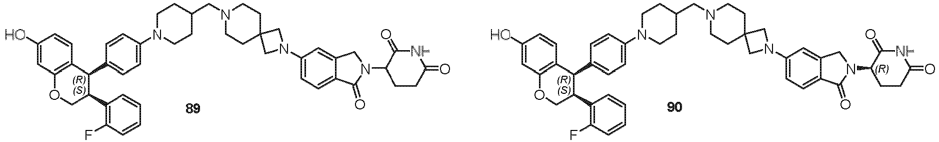

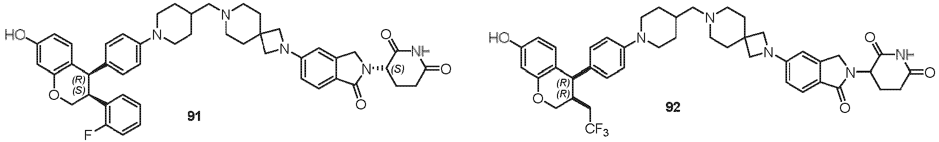

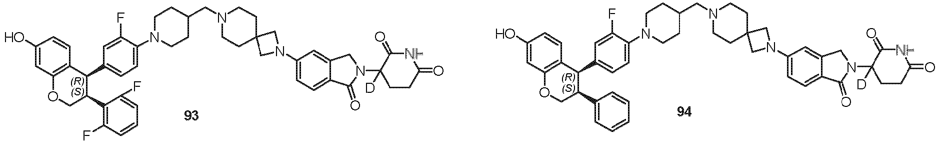

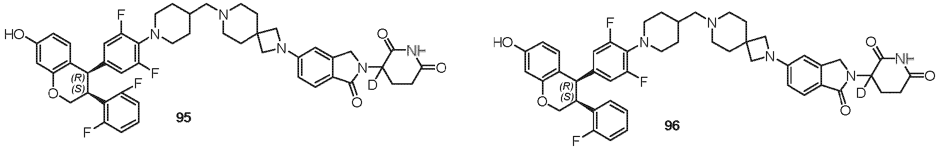

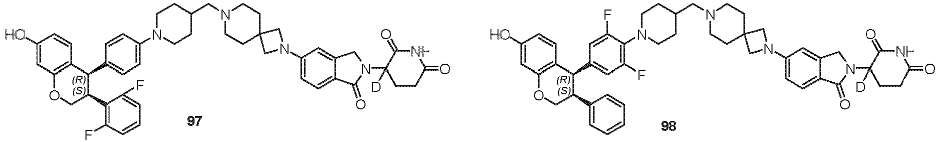

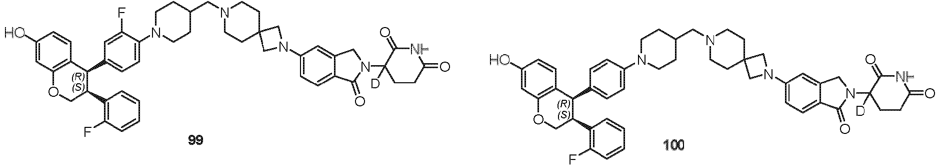

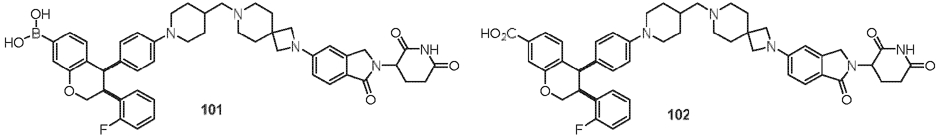

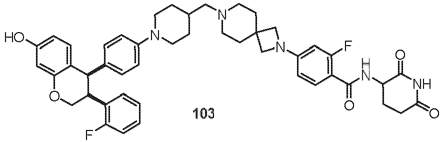

25. The compound according to any one of claims 1-21, or an optical isomer thereof, or a salt thereof, or a hydrate thereof, or a solvate thereof, **characterized in that** the salt is a pharmaceutically acceptable salt; the pharmaceutically acceptable salt is the phosphate, D-camphorsulfonate, hydrochloride, hydrobromide, hydrofluorate, sulfate, nitrate, formate, acetate, propionate, oxalate, malonate, succinate, fumarate, maleate, lactate, malate, tartrate, citrate, picrate, mesylate, phenylmethanesulfonate, benzenesulfonate, aspartate or glutamate of the compound.

26. The use of the compound according to any one of claims 1-25, or an optical isomer thereof, or a salt thereof, or a hydrate thereof, or a solvate thereof in the manufacture of estrogen receptor degraders.

27. The use of the compound according to any one of claims 1-25, or an optical isomer thereof, or a salt thereof, or a hydrate thereof, or a solvate thereof in the manufacture of medicaments for treating estrogen receptor-related diseases.

28. The use of the compound according to any one of claims 1-25, or an optical isomer thereof, or a salt thereof, or a hydrate thereof, or a solvate thereof in the manufacture of medicaments for the treatment and/or prevention of cancer.

29. The use of the compound according to any one of claims 1-25, or an optical isomer thereof, or a salt thereof, or a hydrate thereof, or a solvate thereof in the manufacture of medicaments for the treatment and/or prevention of bone cancer, colorectal cancer, endometrial cancer, prostate cancer, ovarian cancer, uterine cancer, cervical cancer, lung cancer, and breast cancer.

30. A medicament for the treatment of cancer, **characterized in that** it is formed by using the compound according to any one of claims 1-25, or an optical isomer thereof, or a salt thereof, or a hydrate thereof, or a solvate thereof as active ingredients, in combination with pharmaceutically acceptable excipients.
